# EUROPEAN PATENT SPECIFICATION

(11) **EP 2 996 706 B1**
(45) Date of publication and mention of the grant of the patent: **31.07.2019**
(21) Application number: 14769489.7
(22) Date of filing: 14.03.2014
(51) Int. Cl.: A61K 38/17, A61P 9/10, C07K 14/775

(54) **APOLIPOPROTEIN MIMETICS AND USES THEREOF**
APOLIPOPROTEINMIMETIKA UND VERWENDUNGEN DAVON
MIMÉTIQUES D'APOLIPOPROTÉINE ET LEURS UTILISATIONS

(30) Priority: 14.03.2013 US 201361782956 P; 14.06.2013 US 201361834992 P
(43) Date of publication of application: 23.03.2016
(73) Proprietor: UAB Research Foundation, Birmingham, AL 35294-0111 (US); Lipimetix Development, LLC, Natick, MA 01760 (US)
(72) Inventor: ANANTHARAMAIAH, Gattadahalli M., Birmingham, AL 35225 (US); GOLDBERG, Dennis, Sudbury, MA 01776 (US)
(74) Representative: CH Kilger Anwaltspartnerschaft mbB
(86) International application number: PCT/US2014/027719
(87) International publication number: WO 2014/152776

(56) References cited:
- WO-A1-2016/018665
- US-A1- 2010 286 025
- US-A1- 2013 005 645
- GARBER DAVID W ET AL: "Atherosclerosis and vascular disease: Effects of peptide mimetics of apolipoproteins", CURRENT PHARMACEUTICAL BIOTECHNOLOGY, BENTHAM SCIENCE PUBLISHERS, NL, vol. 7, no. 4, 1 August 2006 (2006-08-01), pages 235-240, XP008157674, ISSN: 1389-2010, DOI: 10.2174/138920106777950834
- NAVAB ET AL.: 'A novel method for oral delivery of apolipoprotein mimetic peptides synthesized from all L-amino acids.' J LIPID RES. vol. 50, no. 8, 01 January 2009, pages 1538 - 1547, XP055056762 DOI: 10.1194/JLR.M800539-JLR2

### SEQUENCE LISTING

<110> Anantharamaiah, G.M.
   Goldberg, Dennis
<120> APOLIPOPROTEIN MIMETICS AND USES THEREOF
<130> 21085.0184P1
<150> 61/782,956
   <151> 2013-03-14
<150> 61/834,992
   <151> 2013-06-14
<160> 619
<170> PatentIn version 3.5
<210> 1
   <211> 28
   <212> PRT
   <213> Artificial Sequence
<220>
   <223> synthetic construct; ApoE mimetic
<400> 1
   Leu Arg Lys Leu Arg Lys Arg Leu Leu Arg Asp Trp Leu Lys Ala Phe
   1               5                   10                  15
   Tyr Asp Lys Val Ala Glu Lys Leu Lys Glu Ala Phe
               20                  25
<210> 2
   <211> 28
   <212> PRT
   <213> Artificial Sequence
<220>
   <223> synthetic construct; reverse analog of ApoE mimetic
<400> 2
   Arg Leu Leu Arg Lys Arg Leu Lys Arg Leu Asp Trp Leu Lys Ala Phe
   1               5                   10                  15
   Tyr Asp Lys Val Ala Glu Lys Leu Lys Glu Ala Phe
               20                  25
<210> 3
   <211> 28
   <212> PRT
   <213> Artificial Sequence
<220>
   <223> synthetic construct; reverse analog of ApoE mimetic
<400> 3
   Leu Arg Lys Leu Arg Lys Arg Leu Leu Arg Phe Ala Glu Lys Leu Lys
   1               5                   10                  15
   Glu Ala Val Lys Asp Tyr Phe Ala Lys Leu Trp Asp
               20                  25
<210> 4
   <211> 10
   <212> PRT
   <213> Artificial Sequence
<220>
   <223> synthetic construct; receptor binding domain of human Apo E
<400> 4
   Leu Arg Lys Leu Arg Lys Arg Leu Leu Arg
   1               5                   10
<210> 5
   <211> 18
   <212> PRT
   <213> Artificial Sequence
<220>
   <223> synthetic construct; lipid-associating peptide
<400> 5
   Asp Trp Leu Lys Ala Phe Tyr Asp Lys Val Ala Glu Lys Leu Lys Glu
   1               5                   10                  15
   Ala Phe
<210> 6
   <211> 10
   <212> PRT
   <213> Artificial Sequence
<220>
   <223> synthetic construct; analog of receptor binding domain of human Apo E
<400> 6
   Leu Arg Arg Leu Arg Arg Arg Leu Leu Arg
   1               5                   10
<210> 7
   <211> 10
   <212> PRT
   <213> Artificial Sequence
<220>
   <223> synthetic construct; Rat ApoE mimetic
<400> 7
   Leu Arg Lys Met Arg Lys Arg Leu Met Arg
   1               5                   10
<210> 8
   <211> 10
   <212> PRT
   <213> Artificial Sequence
<220>
   <223> synthetic construct; Bovine Apo E mimetic
<400> 8
   Leu Arg Lys Leu Pro Lys Arg Leu Leu Arg
   1               5                   10
<210> 9
   <211> 10
   <212> PRT
   <213> Artificial Sequence
<220>
   <223> synthetic construct; Pig ApoE mimetic
<400> 9
   Leu Arg Asn Val Arg Lys Arg Leu Val Arg
   1               5                   10
<210> 10
   <211> 10
   <212> PRT
   <213> Artificial Sequence
<220>
   <223> synthetic construct; Dog ApoE mimetic
<400> 10
   Met Arg Lys Leu Arg Lys Arg Val Leu Arg
   1 5 10
<210> 11
   <211> 10
   <212> PRT
   <213> Artificial Sequence
<220>
   <223> synthetic construct; R modified ApoE mimetic
<400> 11
   Leu Arg Arg Leu Arg Arg Arg Leu Leu Arg
   1 5 10
<210> 12
   <211> 10
   <212> PRT
   <213> Artificial Sequence
<220>
   <223> synthetic construct; F modified ApoE mimetic
<400> 12
   Leu Arg Lys Leu Arg Lys Arg Phe Phe Arg
   1 5 10
<210> 13
   <211> 9
   <212> PRT
   <213> Artificial Sequence
<220>
   <223> synthetic construct; ApoB mimetic
<400> 13
   Arg Leu Thr Arg Lys Arg Gly Leu Lys
   1 5
<210> 14
   <211> 18
   <212> PRT
   <213> Artificial Sequence
<220>
   <223> synthetic construct; class A peptide
<400> 14
   Asp Trp Phe Lys Ala Phe Tyr Asp Lys Val Ala Glu Lys Leu Lys Glu
   1 5 10 15
   Ala Phe
<210> 15
   <211> 18
   <212> PRT
   <213> Artificial Sequence
<220>
   <223> synthetic construct; class A peptide
<400> 15
   Asp Trp Leu Lys Ala Phe Tyr Asp Lys Val Ala Glu Lys Phe Lys Glu
   1 5 10 15
   Ala Phe
<210> 16
   <211> 18
   <212> PRT
   <213> Artificial Sequence
<220>
   <223> synthetic construct; class A peptide
<400> 16
   Asp Trp Phe Lys Ala Phe Tyr Asp Lys Val Ala Glu Lys Phe Lys Glu
   1 5 10 15
   Ala Phe
<210> 17
   <211> 18
   <212> PRT
   <213> Artificial Sequence
<220>
   <223> synthetic construct; class A peptide
<400> 17
   Asp Trp Leu Lys Ala Phe Tyr Asp Lys Val Phe Glu Lys Phe Lys Glu
   1 5 10 15
   Phe Phe
<210> 18
   <211> 18
   <212> PRT
   <213> Artificial Sequence
<220>
   <223> synthetic construct; class A peptide
<400> 18
   Asp Trp Leu Lys Ala Phe Tyr Asp Lys Phe Phe Glu Lys Phe Lys Glu
   1 5 10 15
   Phe Phe
<210> 19
   <211> 18
   <212> PRT
   <213> Artificial Sequence
<220>
   <223> synthetic construct; class A peptide
<400> 19
   Asp Trp Phe Lys Ala Phe Tyr Asp Lys Phe Phe Glu Lys Phe Lys Glu
   1 5 10 15
   Phe Phe
<210> 20
   <211> 18
   <212> PRT
   <213> Artificial Sequence
<220>
   <223> synthetic construct; class A peptide
<400> 20
   Asp Trp Leu Lys Ala Phe Tyr Asp Lys Val Ala Glu Lys Leu Lys Glu
   1 5 10 15
   Phe Phe
<210> 21
   <211> 18
   <212> PRT
   <213> Artificial Sequence
<220>
   <223> synthetic construct; class A peptide
<400> 21
   Asp Trp Leu Lys Ala Phe Tyr Asp Lys Val Phe Glu Lys Leu Lys Glu
   1 5 10 15
   Phe Phe
<210> 22
   <211> 18
   <212> PRT
   <213> Artificial Sequence
<220>
   <223> synthetic construct; class A peptide
<400> 22
   Asp Trp Leu Lys Ala Phe Tyr Asp Lys Val Phe Glu Lys Phe Lys Glu
   1               5                   10                  15
   Phe Phe
<210> 23
   <211> 18
   <212> PRT
   <213> Artificial Sequence
<220>
   <223> synthetic construct; class A peptide
<400> 23
   Glu Trp Leu Lys Leu Phe Tyr Glu Lys Val Leu Glu Lys Phe Lys Glu
   1               5                   10                  15
   Ala Phe
<210> 24
   <211> 19
   <212> PRT
   <213> Artificial Sequence
<220>
   <223> synthetic construct; class A peptide
<400> 24
   Glu Trp Leu Lys Ala Phe Tyr Ile Asp Lys Val Ala Glu Lys Phe Lys
   1               5                   10                  15
   Glu Ala Phe
<210> 25
   <211> 19
   <212> PRT
   <213> Artificial Sequence
<220>
   <223> synthetic construct; class A peptide
<400> 25
   Glu Glu Trp Leu Lys Ala Phe Tyr Asp Lys Val Ala Glu Lys Leu Lys
   1 5 10 15
   Glu Phe Phe
<210> 26
   <211> 18
   <212> PRT
   <213> Artificial Sequence
<220>
   <223> synthetic construct; class A peptide
<400> 26
   Glu Trp Leu Lys Ala Phe Tyr Asp Lys Val Phe Glu Lys Phe Lys Glu
   1 5 10 15
   Ala Phe
<210> 27
   <211> 18
   <212> PRT
   <213> Artificial Sequence
<220>
   <223> synthetic construct; class A peptide
<400> 27
   Glu Trp Leu Lys Ala Phe Tyr Asp Lys Val Phe Glu Lys Leu Lys Glu
   1 5 10 15
   Phe Phe
<210> 28
   <211> 18
   <212> PRT
   <213> Artificial Sequence
<220>
   <223> synthetic construct; class A peptide
<400> 28
   Glu Trp Leu Lys Ala Phe Tyr Asp Lys Val Ala Glu Lys Phe Lys Glu
   1 5 10 15
   Phe Phe
<210> 29
   <211> 18
   <212> PRT
   <213> Artificial Sequence
<220>
   <223> synthetic construct; class A peptide
<400> 29
   Glu Trp Leu Lys Ala Phe Tyr Asp Lys Val Phe Glu Lys Phe Lys Glu
   1               5                   10                  15
   Phe Phe
<210> 30
   <211> 14
   <212> PRT
   <213> Artificial Sequence
<220>
   <223> synthetic construct; class A peptide
<400> 30
   Ala Phe Tyr Asp Lys Val Ala Glu Lys Leu Lys Glu Ala Phe
   1               5                   10
<210> 31
   <211> 14
   <212> PRT
   <213> Artificial Sequence
<220>
   <223> synthetic construct; class A peptide
<400> 31
   Ala Phe Tyr Asp Lys Val Ala Glu Lys Phe Lys Glu Ala Phe
   1               5                   10
<210> 32
   <211> 14
   <212> PRT
   <213> Artificial Sequence
<220>
   <223> synthetic construct; class A peptide
<400> 32
   Ala Phe Tyr Asp Lys Val Ala Glu Lys Phe Lys Glu Ala Phe
   1               5                   10
<210> 33
   <211> 14
   <212> PRT
   <213> Artificial Sequence
<220>
   <223> synthetic construct; class A peptide
<400> 33
   Ala Phe Tyr Asp Lys Phe Phe Glu Lys Phe Lys Glu Phe Phe
   1               5                   10
<210> 34
   <211> 14
   <212> PRT
   <213> Artificial Sequence
<220>
   <223> synthetic construct; class A peptide
<400> 34
   Ala Phe Tyr Asp Lys Phe Phe Glu Lys Phe Lys Glu Phe Phe
   1               5                   10
<210> 35
   <211> 14
   <212> PRT
   <213> Artificial Sequence
<220>
   <223> synthetic construct; class A peptide
<400> 35
   Ala Phe Tyr Asp Lys Val Ala Glu Lys Phe Lys Glu Ala Phe
   1               5                   10
<210> 36
   <211> 14
   <212> PRT
   <213> Artificial Sequence
<220>
   <223> synthetic construct; class A peptide
<400> 36
   Ala Phe Tyr Asp Lys Val Ala Glu Lys Leu Lys Glu Phe Phe
   1               5                   10
<210> 37
   <211> 14
   <212> PRT
   <213> Artificial Sequence
<220>
   <223> synthetic construct; class A peptide
<400> 37
   Ala Phe Tyr Asp Lys Val Phe Glu Lys Phe Lys Glu Ala Phe
   1               5                   10
<210> 38
   <211> 14
   <212> PRT
   <213> Artificial Sequence
<220>
   <223> synthetic construct; class A peptide
<400> 38
   Ala Phe Tyr Asp Lys Val Phe Glu Lys Leu Lys Glu Phe Phe
   1               5                   10
<210> 39
   <211> 14
   <212> PRT
   <213> Artificial Sequence
<220>
   <223> synthetic construct; class A peptide
<400> 39
   Ala Phe Tyr Asp Lys Val Ala Glu Lys Phe Lys Glu Phe Phe
   1               5                   10
<210> 40
   <211> 14
   <212> PRT
   <213> Artificial Sequence
<220>
   <223> synthetic construct; class A peptide
<400> 40
   Lys Ala Phe Tyr Asp Lys Val Phe Glu Lys Phe Lys Glu Phe
   1               5                   10
<210> 41
   <211> 14
   <212> PRT
   <213> Artificial Sequence
<220>
   <223> synthetic construct; class A peptide
<400> 41
   Leu Phe Tyr Glu Lys Val Leu Glu Lys Phe Lys Glu Ala Phe
   1               5                   10
<210> 42
   <211> 14
   <212> PRT
   <213> Artificial Sequence
<220>
   <223> synthetic construct; class A peptide
<400> 42
   Ala Phe Tyr Asp Lys Val Ala Glu Lys Phe Lys Glu Ala Phe
   1               5                   10
<210> 43
   <211> 14
   <212> PRT
   <213> Artificial Sequence
<220>
   <223> synthetic construct; class A peptide
<400> 43
   Ala Phe Tyr Asp Lys Val Ala Glu Lys Leu Lys Glu Phe Phe
   1               5                   10
<210> 44
   <211> 14
   <212> PRT
   <213> Artificial Sequence
<220>
   <223> synthetic construct; class A peptide
<400> 44
   Ala Phe Tyr Asp Lys Val Phe Glu Lys Phe Lys Glu Ala Phe
   1               5                   10
<210> 45
   <211> 14
   <212> PRT
   <213> Artificial Sequence
<220>
   <223> synthetic construct; class A peptide
<400> 45
   Ala Phe Tyr Asp Lys Val Phe Glu Lys Leu Lys Glu Phe Phe
   1               5                   10
<210> 46
   <211> 14
   <212> PRT
   <213> Artificial Sequence
<220>
   <223> synthetic construct; class A peptide
<400> 46
   Ala Phe Tyr Asp Lys Val Ala Glu Lys Phe Lys Glu Phe Phe
   1               5                   10
<210> 47
   <211> 14
   <212> PRT
   <213> Artificial Sequence
<220>
   <223> synthetic construct; class A peptide
<400> 47
   Ala Phe Tyr Asp Lys Val Phe Glu Lys Phe Lys Glu Phe Phe
   1 5 10
<210> 48
   <211> 18
   <212> PRT
   <213> Artificial Sequence
<220>
   <223> synthetic construct; class A peptide
<400> 48
   Asp Trp Leu Lys Ala Leu Tyr Asp Lys Val Ala Glu Lys Leu Lys Glu
   1 5 10 15
   Ala Leu
<210> 49
   <211> 18
   <212> PRT
   <213> Artificial Sequence
<220>
   <223> synthetic construct; class A peptide
<400> 49
   Asp Trp Phe Lys Ala Phe Tyr Glu Lys Val Ala Glu Lys Leu Lys Glu
   1 5 10 15
   Phe Phe
<210> 50
   <211> 18
   <212> PRT
   <213> Artificial Sequence
<220>
   <223> synthetic construct; class A peptide
<400> 50
   Asp Trp Phe Lys Ala Phe Tyr Glu Lys Phe Phe Glu Lys Phe Lys Glu
   1               5                   10                  15
   Phe Phe
<210> 51
   <211> 18
   <212> PRT
   <213> Artificial Sequence
<220>
   <223> synthetic construct; class A peptide
<400> 51
   Glu Trp Leu Lys Ala Leu Tyr Glu Lys Val Ala Glu Lys Leu Lys Glu
   1               5                   10                  15
   Ala Leu
<210> 52
   <211> 18
   <212> PRT
   <213> Artificial Sequence
<220>
   <223> synthetic construct; class A peptide
<400> 52
   Glu Trp Leu Lys Ala Phe Tyr Glu Lys Val Ala Glu Lys Leu Lys Glu
   1               5                   10                  15
   Ala Phe
<210> 53
   <211> 18
   <212> PRT
   <213> Artificial Sequence
<220>
   <223> synthetic construct; class A peptide
<400> 53
   Glu Trp Phe Lys Ala Phe Tyr Glu Lys Val Ala Glu Lys Leu Lys Glu
   1               5                   10                  15
   Phe Phe
<210> 54
   <211> 18
   <212> PRT
   <213> Artificial Sequence
<220>
   <223> synthetic construct; class A peptide
<400> 54
   Glu Trp Leu Lys Ala Phe Tyr Glu Lys Val Phe Glu Lys Phe Lys Glu
   1 5 10 15
   Phe Phe
<210> 55
   <211> 18
   <212> PRT
   <213> Artificial Sequence
<220>
   <223> synthetic construct; class A peptide
<400> 55
   Glu Trp Leu Lys Ala Phe Tyr Glu Lys Phe Phe Glu Lys Phe Lys Glu
   1 5 10 15
   Phe Phe
<210> 56
   <211> 18
   <212> PRT
   <213> Artificial Sequence
<220>
   <223> synthetic construct; class A peptide
<400> 56
   Glu Trp Phe Lys Ala Phe Tyr Glu Lys Phe Phe Glu Lys Phe Lys Glu
   1 5 10 15
   Phe Phe
<210> 57
   <211> 18
   <212> PRT
   <213> Artificial Sequence
<220>
   <223> synthetic construct; class A peptide
<400> 57
   Asp Phe Leu Lys Ala Trp Tyr Asp Lys Val Ala Glu Lys Leu Lys Glu
   1 5 10 15
   Ala Trp
<210> 58
   <211> 18
   <212> PRT
   <213> Artificial Sequence
<220>
   <223> synthetic construct; class A peptide
<400> 58
   Glu Phe Leu Lys Ala Trp Tyr Glu Lys Val Ala Glu Lys Leu Lys Glu
   1               5                   10                  15
   Ala Trp
<210> 59
   <211> 18
   <212> PRT
   <213> Artificial Sequence
<220>
   <223> synthetic construct; class A peptide
<400> 59
   Asp Phe Trp Lys Ala Trp Tyr Asp Lys Val Ala Glu Lys Leu Lys Glu
   1               5                   10                  15
   Trp Trp
<210> 60
   <211> 18
   <212> PRT
   <213> Artificial Sequence
<220>
   <223> synthetic construct; class A peptide
<400> 60
   Glu Phe Trp Lys Ala Trp Tyr Glu Lys Val Ala Glu Lys Leu Lys Glu
   1               5                   10                  15
   Trp Trp
<210> 61
   <211> 18
   <212> PRT
   <213> Artificial Sequence
<220>
   <223> synthetic construct; class A peptide
<400> 61
   Asp Lys Leu Lys Ala Phe Tyr Asp Lys Val Phe Glu Trp Ala Lys Glu
   1 5 10 15
   Ala Phe
<210> 62
   <211> 18
   <212> PRT
   <213> Artificial Sequence
<220>
   <223> synthetic construct; class A peptide
<400> 62
   Asp Lys Trp Lys Ala Val Tyr Asp Lys Phe Ala Glu Ala Phe Lys Glu
   1 5 10 15
   Phe Leu
<210> 63
   <211> 18
   <212> PRT
   <213> Artificial Sequence
<220>
   <223> synthetic construct; class A peptide
<400> 63
   Glu Lys Leu Lys Ala Phe Tyr Glu Lys Val Phe Glu Trp Ala Lys Glu
   1 5 10 15
   Ala Phe
<210> 64
   <211> 18
   <212> PRT
   <213> Artificial Sequence
<220>
   <223> synthetic construct; class A peptide
<400> 64
   Glu Lys Trp Lys Ala Val Tyr Glu Lys Phe Ala Glu Ala Phe Lys Glu
   1 5 10 15
   Phe Leu
<210> 65
   <211> 18
   <212> PRT
   <213> Artificial Sequence
<220>
   <223> synthetic construct; class A peptide
<400> 65
   Asp Trp Leu Lys Ala Phe Val Asp Lys Phe Ala Glu Lys Phe Lys Glu
   1               5                   10                  15
   Ala Tyr
<210> 66
   <211> 18
   <212> PRT
   <213> Artificial Sequence
<220>
   <223> synthetic construct; class A peptide
<400> 66
   Glu Lys Trp Lys Ala Val Tyr Glu Lys Phe Ala Glu Ala Phe Lys Glu
   1               5                   10                  15
   Phe Leu
<210> 67
   <211> 18
   <212> PRT
   <213> Artificial Sequence
<220>
   <223> synthetic construct; class A peptide
<400> 67
   Asp Trp Leu Lys Ala Phe Val Tyr Asp Lys Val Phe Lys Leu Lys Glu
   1               5                   10                  15
   Phe Phe
<210> 68
   <211> 18
   <212> PRT
   <213> Artificial Sequence
<220>
   <223> synthetic construct; class A peptide
<400> 68
   Glu Trp Leu Lys Ala Phe Val Tyr Glu Lys Val Phe Lys Leu Lys Glu
   1               5                   10                  15
   Phe Phe
<210> 69
   <211> 18
   <212> PRT
   <213> Artificial Sequence
<220>
   <223> synthetic construct; class A peptide
<400> 69
   Asp Trp Leu Arg Ala Phe Tyr Asp Lys Val Ala Glu Lys Leu Lys Glu
   1               5                   10                  15
   Ala Phe
<210> 70
   <211> 18
   <212> PRT
   <213> Artificial Sequence
<220>
   <223> synthetic construct; class A peptide
<400> 70
   Glu Trp Leu Arg Ala Phe Tyr Glu Lys Val Ala Glu Lys Leu Lys Glu
   1               5                   10                  15
   Ala Phe
<210> 71
   <211> 18
   <212> PRT
   <213> Artificial Sequence
<220>
   <223> synthetic construct; class A peptide
<400> 71
   Asp Trp Leu Lys Ala Phe Tyr Asp Arg Val Ala Glu Lys Leu Lys Glu
   1               5                   10                  15
   Ala Phe
<210> 72
   <211> 18
   <212> PRT
   <213> Artificial Sequence
<220>
   <223> synthetic construct; class A peptide
<400> 72
   Glu Trp Leu Lys Ala Phe Tyr Glu Arg Val Ala Glu Lys Leu Lys Glu
   1 5 10 15
   Ala Phe
<210> 73
   <211> 18
   <212> PRT
   <213> Artificial Sequence
<220>
   <223> synthetic construct; class A peptide
<400> 73
   Asp Trp Leu Lys Ala Phe Tyr Asp Lys Val Ala Glu Arg Leu Lys Glu
   1 5 10 15
   Ala Phe
<210> 74
   <211> 18
   <212> PRT
   <213> Artificial Sequence
<220>
   <223> synthetic construct; class A peptide
<400> 74
   Glu Trp Leu Lys Ala Phe Tyr Glu Lys Val Ala Glu Arg Leu Lys Glu
   1 5 10 15
   Ala Phe
<210> 75
   <211> 18
   <212> PRT
   <213> Artificial Sequence
<220>
   <223> synthetic construct; class A peptide
<400> 75
   Asp Trp Leu Lys Ala Phe Tyr Asp Lys Val Ala Glu Lys Leu Arg Glu
   1 5 10 15
   Ala Phe
<210> 76
   <211> 18
   <212> PRT
   <213> Artificial Sequence
<220>
   <223> synthetic construct; class A peptide
<400> 76
   Glu Trp Leu Lys Ala Phe Tyr Glu Lys Val Ala Glu Lys Leu Arg Glu
   1 5 10 15
   Ala Phe
<210> 77
   <211> 18
   <212> PRT
   <213> Artificial Sequence
<220>
   <223> synthetic construct; class A peptide
<400> 77
   Asp Trp Leu Lys Ala Phe Tyr Asp Arg Val Ala Glu Arg Leu Lys Glu
   1 5 10 15
   Ala Phe
<210> 78
   <211> 18
   <212> PRT
   <213> Artificial Sequence
<220>
   <223> synthetic construct; class A peptide
<400> 78
   Glu Trp Leu Lys Ala Phe Tyr Glu Arg Val Ala Glu Arg Leu Lys Glu
   1 5 10 15
   Ala Phe
<210> 79
   <211> 18
   <212> PRT
   <213> Artificial Sequence
<220>
   <223> synthetic construct; class A peptide
<400> 79
   Asp Trp Leu Arg Ala Phe Tyr Asp Lys Val Ala Glu Lys Leu Arg Glu
   1 5 10 15
   Ala Phe
<210> 80
   <211> 18
   <212> PRT
   <213> Artificial Sequence
<220>
   <223> synthetic construct; class A peptide
<400> 80
   Glu Trp Leu Arg Ala Phe Tyr Glu Lys Val Ala Glu Lys Leu Arg Glu
   1               5                   10                  15
   Ala Phe
<210> 81
   <211> 18
   <212> PRT
   <213> Artificial Sequence
<220>
   <223> synthetic construct; class A peptide
<400> 81
   Asp Trp Leu Arg Ala Phe Tyr Asp Arg Val Ala Glu Lys Leu Lys Glu
   1               5                   10                  15
   Ala Phe
<210> 82
   <211> 18
   <212> PRT
   <213> Artificial Sequence
<220>
   <223> synthetic construct; class A peptide
<400> 82
   Glu Trp Leu Arg Ala Phe Tyr Glu Arg Val Ala Glu Lys Leu Lys Glu
   1               5                   10                  15
   Ala Phe
<210> 83
   <211> 18
   <212> PRT
   <213> Artificial Sequence
<220>
   <223> synthetic construct; class A peptide
<400> 83
   Asp Trp Leu Lys Ala Phe Tyr Asp Lys Val Ala Glu Arg Leu Arg Glu
   1               5                   10                  15
   Ala Phe
<210> 84
   <211> 18
   <212> PRT
   <213> Artificial Sequence
<220>
   <223> synthetic construct; class A peptide
<400> 84
   Glu Trp Leu Lys Ala Phe Tyr Glu Lys Val Ala Glu Arg Leu Arg Glu
   1               5                   10                  15
   Ala Phe
<210> 85
   <211> 18
   <212> PRT
   <213> Artificial Sequence
<220>
   <223> synthetic construct; class A peptide
<400> 85
   Asp Trp Leu Arg Ala Phe Tyr Asp Lys Val Ala Glu Arg Leu Lys Glu
   1               5                   10                  15
   Ala Phe
<210> 86
   <211> 18
   <212> PRT
   <213> Artificial Sequence
<220>
   <223> synthetic construct; class A peptide
<400> 86
   Glu Trp Leu Arg Ala Phe Tyr Glu Lys Val Ala Glu Arg Leu Lys Glu
   1               5                   10                  15
   Ala Phe
<210> 87
   <211> 37
   <212> PRT
   <213> Artificial Sequence
<220>
   <223> synthetic construct; class A peptide
<400> 87
   Asp Trp Leu Lys Ala Phe Tyr Asp Lys Val Ala Glu Lys Leu Lys Glu
   1               5                   10                  15
   Ala Phe Pro Asp Trp Leu Lys Ala Phe Tyr Asp Lys Val Ala Glu Lys
               20                  25                  30
   Leu Lys Glu Ala Phe
           35
<210> 88
   <211> 37
   <212> PRT
   <213> Artificial Sequence
<220>
   <223> synthetic construct; class A peptide
<400> 88
   Asp Trp Leu Lys Ala Phe Tyr Asp Lys Val Ala Glu Lys Leu Lys Glu
   1               5                   10                  15
   Phe Phe Pro Asp Trp Leu Lys Ala Phe Tyr Asp Lys Val Ala Glu Lys
               20                  25                  30
   Leu Lys Glu Phe Phe
           35
<210> 89
   <211> 37
   <212> PRT
   <213> Artificial Sequence
<220>
   <223> synthetic construct; class A peptide
<400> 89
   Asp Trp Phe Lys Ala Phe Tyr Asp Lys Val Ala Glu Lys Leu Lys Glu
   1               5                   10                  15
   Ala Phe Pro Asp Trp Phe Lys Ala Phe Tyr Asp Lys Val Ala Glu Lys
               20                  25                  30
   Leu Lys Glu Ala Phe
           35
<210> 90
   <211> 37
   <212> PRT
   <213> Artificial Sequence
<220>
   <223> synthetic construct; class A peptide
<400> 90
   Asp Lys Leu Lys Ala Phe Tyr Asp Lys Val Phe Glu Trp Ala Lys Glu
   1               5                   10                  15
   Ala Phe Pro Asp Lys Leu Lys Ala Phe Tyr Asp Lys Val Phe Glu Trp
               20                  25                  30
   Leu Lys Glu Ala Phe
           35
<210> 91
   <211> 37
   <212> PRT
   <213> Artificial Sequence
<220>
   <223> synthetic construct; class A peptide
<400> 91
   Asp Lys Trp Lys Ala Val Tyr Asp Lys Phe Ala Glu Ala Phe Lys Glu
   1               5                   10                  15
   Phe Leu Pro Asp Lys Trp Lys Ala Val Tyr Asp Lys Phe Ala Glu Ala
               20                  25                  30
   Phe Lys Glu Phe Leu
           35
<210> 92
   <211> 37
   <212> PRT
   <213> Artificial Sequence
<220>
   <223> synthetic construct; class A peptide
<400> 92
   Asp Trp Phe Lys Ala Phe Tyr Asp Lys Val Ala Glu Lys Phe Lys Glu
   1 5 10 15
   Ala Phe Pro Asp Trp Phe Lys Ala Phe Tyr Asp Lys Val Ala Glu Lys
   20 25 30
   Phe Lys Glu Ala Phe
   35
<210> 93
   <211> 37
   <212> PRT
   <213> Artificial Sequence
<220>
   <223> synthetic construct; class A peptide
<400> 93
   Asp Trp Leu Lys Ala Phe Val Tyr Asp Lys Val Phe Lys Leu Lys Glu
   1 5 10 15
   Phe Phe Pro Asp Trp Leu Lys Ala Phe Val Tyr Asp Lys Val Phe Lys
   20 25 30
   Leu Lys Glu Phe Phe
   35
<210> 94
   <211> 37
   <212> PRT
   <213> Artificial Sequence
<220>
   <223> synthetic construct; class A peptide
<400> 94
   Asp Trp Leu Lys Ala Phe Tyr Asp Lys Phe Ala Glu Lys Phe Lys Glu
   1 5 10 15
   Phe Phe Pro Asp Trp Leu Lys Ala Phe Tyr Asp Lys Phe Ala Glu Lys
   20 25 30
   Phe Lys Glu Phe Phe
   35
<210> 95
   <211> 18
   <212> PRT
   <213> Artificial Sequence
<220>
   <223> synthetic construct; class A peptide
<400> 95
   Glu Trp Phe Lys Ala Phe Tyr Glu Lys Val Ala Glu Lys Phe Lys Glu
   1               5                   10                  15
   Ala Phe
<210> 96
   <211> 14
   <212> PRT
   <213> Artificial Sequence
<220>
   <223> synthetic construct; class A peptide
<400> 96
   Asp Trp Phe Lys Ala Phe Tyr Asp Lys Val Ala Glu Lys Phe
   1               5                   10
<210> 97
   <211> 14
   <212> PRT
   <213> Artificial Sequence
<220>
   <223> synthetic construct; class A peptide
<400> 97
   Phe Lys Ala Phe Tyr Asp Lys Val Ala Glu Lys Phe Lys Glu
   1               5                   10
<210> 98
   <211> 14
   <212> PRT
   <213> Artificial Sequence
<220>
   <223> synthetic construct; class A peptide
<400> 98
   Phe Lys Ala Phe Tyr Glu Lys Val Ala Glu Lys Phe Lys Glu
   1               5                   10
<210> 99
   <211> 14
   <212> PRT
   <213> Artificial Sequence
<220>
   <223> synthetic construct; class A peptide
<400> 99
   Phe Lys Ala Phe Tyr Asp Lys Val Ala Glu Lys Phe Lys Glu
   1 5 10
<210> 100
   <211> 14
   <212> PRT
   <213> Artificial Sequence
<220>
   <223> synthetic construct; class A peptide
<400> 100
   Phe Lys Ala Phe Tyr Glu Lys Val Ala Glu Lys Phe Lys Glu
   1 5 10
<210> 101
   <211> 18
   <212> PRT
   <213> Artificial Sequence
<220>
   <223> synthetic construct; class A peptide
<400> 101
   Asp Trp Phe Lys Ala Phe Tyr Asp Lys Val Ala Glu Lys Phe Lys Glu
   1 5 10 15
   Ala Phe
<210> 102
   <211> 18
   <212> PRT
   <213> Artificial Sequence
<220>
   <223> synthetic construct; class A peptide
<400> 102
   Glu Trp Phe Lys Ala Phe Tyr Glu Lys Val Ala Glu Lys Phe Lys Glu
   1 5 10 15
   Ala Phe
<210> 103
   <211> 14
   <212> PRT
   <213> Artificial Sequence
<220>
   <223> synthetic construct; class A peptide
<400> 103
   Ala Phe Tyr Asp Lys Val Ala Glu Lys Phe Lys Glu Ala Phe
   1 5 10
<210> 104
   <211> 14
   <212> PRT
   <213> Artificial Sequence
<220>
   <223> synthetic construct; class A peptide
<400> 104
   Asp Trp Phe Lys Ala Phe Tyr Asp Lys Val Ala Glu Lys Phe
   1 5 10
<210> 105
   <211> 18
   <212> PRT
   <213> Artificial Sequence
<220>
   <223> synthetic construct; class A peptide
<400> 105
   Asp Trp Leu Lys Ala Phe Tyr Asp Lys Val Phe Glu Lys Phe Lys Glu
   1 5 10 15
   Phe Phe
<210> 106
   <211> 18
   <212> PRT
   <213> Artificial Sequence
<220>
   <223> synthetic construct; class A peptide
<400> 106
   Asp Trp Leu Lys Ala Phe Tyr Asp Lys Val Phe Glu Lys Phe Lys Glu
   1 5 10 15
   Phe Phe
<210> 107
   <211> 18
   <212> PRT
   <213> Artificial Sequence
<220>
   <223> synthetic construct; class A peptide
<400> 107
   Glu Trp Leu Lys Ala Phe Tyr Glu Lys Val Phe Glu Lys Phe Lys Glu
   1               5                   10                  15
   Phe Phe
<210> 108
   <211> 18
   <212> PRT
   <213> Artificial Sequence
<220>
   <223> synthetic construct; class A peptide
<400> 108
   Glu Trp Leu Lys Ala Phe Tyr Glu Lys Val Phe Glu Lys Phe Lys Glu
   1               5                   10                  15
   Phe Phe
<210> 109
   <211> 14
   <212> PRT
   <213> Artificial Sequence
<220>
   <223> synthetic construct; class A peptide
<400> 109
   Ala Phe Tyr Asp Lys Val Phe Glu Lys Phe Lys Glu Phe Phe
   1               5                   10
<210> 110
   <211> 14
   <212> PRT
   <213> Artificial Sequence
<220>
   <223> synthetic construct; class A peptide
<400> 110
   Ala Phe Tyr Asp Lys Val Phe Glu Lys Phe Lys Glu Phe Phe
   1               5                   10
<210> 111
   <211> 14
   <212> PRT
   <213> Artificial Sequence
<220>
   <223> synthetic construct; class A peptide
<400> 111
   Ala Phe Tyr Glu Lys Val Phe Glu Lys Phe Lys Glu Phe Phe
   1               5                   10
<210> 112
   <211> 14
   <212> PRT
   <213> Artificial Sequence
<220>
   <223> synthetic construct; class A peptide
<400> 112
   Ala Phe Tyr Glu Lys Val Phe Glu Lys Phe Lys Glu Phe Phe
   1               5                   10
<210> 113
   <211> 14
   <212> PRT
   <213> Artificial Sequence
<220>
   <223> synthetic construct; class A peptide
<400> 113
   Asp Trp Leu Lys Ala Phe Tyr Asp Lys Val Phe Glu Lys Phe
   1               5                   10
<210> 114
   <211> 14
   <212> PRT
   <213> Artificial Sequence
<220>
   <223> synthetic construct; class A peptide
<400> 114
   Asp Trp Leu Lys Ala Phe Tyr Asp Lys Val Phe Glu Lys Phe
   1               5                   10
<210> 115
   <211> 14
   <212> PRT
   <213> Artificial Sequence
<220>
   <223> synthetic construct; class A peptide
<400> 115
   Glu Trp Leu Lys Ala Phe Tyr Glu Lys Val Phe Glu Lys Phe
   1               5                   10
<210> 116
   <211> 14
   <212> PRT
   <213> Artificial Sequence
<220>
   <223> synthetic construct; class A peptide
<400> 116
   Glu Trp Leu Lys Ala Phe Tyr Glu Lys Val Phe Glu Lys Phe
   1 5 10
<210> 117
   <211> 14
   <212> PRT
   <213> Artificial Sequence
<220>
   <223> synthetic construct; class A peptide
<400> 117
   Leu Lys Ala Phe Tyr Asp Lys Val Phe Glu Lys Phe Lys Glu
   1 5 10
<210> 118
   <211> 14
   <212> PRT
   <213> Artificial Sequence
<220>
   <223> synthetic construct; class A peptide
<400> 118
   Leu Lys Ala Phe Tyr Asp Lys Val Phe Glu Lys Phe Lys Glu
   1 5 10
<210> 119
   <211> 14
   <212> PRT
   <213> Artificial Sequence
<220>
   <223> synthetic construct; class A peptide
<400> 119
   Leu Lys Ala Phe Tyr Glu Lys Val Phe Glu Lys Phe Lys Glu
   1 5 10
<210> 120
   <211> 14
   <212> PRT
   <213> Artificial Sequence
<220>
   <223> synthetic construct; class A peptide
<400> 120
   Leu Lys Ala Phe Tyr Glu Lys Val Phe Glu Lys Phe Lys Glu
   1               5                   10
<210> 121
   <211> 18
   <212> PRT
   <213> Artificial Sequence
<220>
   <223> synthetic construct; modified class A peptide
<400> 121
   Asp Lys Trp Lys Ala Val Tyr Asp Lys Phe Ala Glu Ala Phe Lys Glu
   1               5                   10                  15
   Phe Leu
<210> 122
   <211> 18
   <212> PRT
   <213> Artificial Sequence
<220>
   <223> synthetic construct; modified class A peptide
<400> 122
   Asp Lys Leu Lys Ala Phe Tyr Asp Lys Val Phe Glu Trp Ala Lys Glu
   1               5                   10                  15
   Ala Phe
<210> 123
   <211> 18
   <212> PRT
   <213> Artificial Sequence
<220>
   <223> synthetic construct; Class A amphipathic helical peptides
<400> 123
   Glu Trp Phe Lys Ala Phe Tyr Glu Lys Val Ala Asp Lys Phe Lys Asp
   1               5                   10                  15
   Ala Phe
<210> 124
   <211> 18
   <212> PRT
   <213> Artificial Sequence
<220>
   <223> synthetic construct; Class A amphipathic helical peptides
<400> 124
   Glu Trp Phe Lys Ala Phe Tyr Asp Lys Val Ala Asp Lys Phe Lys Glu
   1               5                   10                  15
   Ala Phe
<210> 125
   <211> 18
   <212> PRT
   <213> Artificial Sequence
<220>
   <223> synthetic construct; Class A amphipathic helical peptides
<400> 125
   Asp Trp Phe Lys Ala Phe Tyr Glu Lys Val Ala Asp Lys Phe Lys Glu
   1               5                   10                  15
   Ala Phe
<210> 126
   <211> 18
   <212> PRT
   <213> Artificial Sequence
<220>
   <223> synthetic construct; Class A amphipathic helical peptides
<400> 126
   Asp Trp Phe Lys Ala Phe Tyr Glu Lys Val Ala Glu Lys Phe Lys Asp
   1               5                   10                  15
   Ala Phe
<210> 127
   <211> 18
   <212> PRT
   <213> Artificial Sequence
<220>
   <223> synthetic construct; Class A amphipathic helical peptides
<400> 127
   Asp Phe Trp Lys Ala Phe Tyr Asp Lys Val Ala Glu Lys Phe Lys Glu
   1               5                   10                  15
   Ala Phe
<210> 128
   <211> 18
   <212> PRT
   <213> Artificial Sequence
<220>
   <223> synthetic construct; Class A amphipathic helical peptides
<400> 128
   Glu Phe Trp Lys Ala Phe Tyr Glu Lys Val Ala Asp Lys Phe Lys Asp
   1               5                   10                  15
   Ala Phe
<210> 129
   <211> 18
   <212> PRT
   <213> Artificial Sequence
<220>
   <223> synthetic construct; Class A amphipathic helical peptides
<400> 129
   Glu Phe Trp Lys Ala Phe Tyr Asp Lys Val Ala Asp Lys Phe Lys Glu
   1               5                   10                  15
   Ala Phe
<210> 130
   <211> 18
   <212> PRT
   <213> Artificial Sequence
<220>
   <223> synthetic construct; Class A amphipathic helical peptides
<400> 130
   Asp Phe Trp Lys Ala Phe Tyr Glu Lys Val Ala Asp Lys Phe Lys Glu
   1               5                   10                  15
   Ala Phe
<210> 131
   <211> 18
   <212> PRT
   <213> Artificial Sequence
<220>
   <223> synthetic construct; Class A amphipathic helical peptides
<400> 131
   Asp Phe Trp Lys Ala Phe Tyr Glu Lys Val Ala Glu Lys Phe Lys Asp
   1               5                   10                  15
   Ala Phe
<210> 132
   <211> 18
   <212> PRT
   <213> Artificial Sequence
<220>
   <223> synthetic construct; Class A amphipathic helical peptides
<400> 132
   Asp Trp Phe Lys Ala Tyr Phe Asp Lys Val Ala Glu Lys Phe Lys Glu
   1               5                   10                  15
   Ala Phe
<210> 133
   <211> 18
   <212> PRT
   <213> Artificial Sequence
<220>
   <223> synthetic construct; Class A amphipathic helical peptides
<400> 133
   Glu Trp Phe Lys Ala Tyr Phe Glu Lys Val Ala Asp Lys Phe Lys Asp
   1               5                   10                  15
   Ala Phe
<210> 134
   <211> 18
   <212> PRT
   <213> Artificial Sequence
<220>
   <223> synthetic construct; Class A amphipathic helical peptides
<400> 134
   Glu Trp Phe Lys Ala Tyr Phe Asp Lys Val Ala Asp Lys Phe Lys Glu
   1               5                   10                  15
   Ala Phe
<210> 135
   <211> 18
   <212> PRT
   <213> Artificial Sequence
<220>
   <223> synthetic construct; Class A amphipathic helical peptides
<400> 135
   Asp Trp Phe Lys Ala Tyr Phe Glu Lys Val Ala Asp Lys Phe Lys Glu
   1 5 10 15
   Ala Phe
<210> 136
   <211> 18
   <212> PRT
   <213> Artificial Sequence
<220>
   <223> synthetic construct; Class A amphipathic helical peptides
<400> 136
   Asp Trp Phe Lys Ala Tyr Phe Glu Lys Val Ala Glu Lys Phe Lys Asp
   1 5 10 15
   Ala Phe
<210> 137
   <211> 18
   <212> PRT
   <213> Artificial Sequence
<220>
   <223> synthetic construct; Class A amphipathic helical peptides
<400> 137
   Asp Trp Phe Lys Ala Phe Val Asp Lys Tyr Ala Glu Lys Phe Lys Glu
   1 5 10 15
   Ala Phe
<210> 138
   <211> 18
   <212> PRT
   <213> Artificial Sequence
<220>
   <223> synthetic construct; Class A amphipathic helical peptides
<400> 138
   Glu Trp Phe Lys Ala Phe Val Glu Lys Tyr Ala Asp Lys Phe Lys Asp
   1 5 10 15
   Ala Phe
<210> 139
   <211> 18
   <212> PRT
   <213> Artificial Sequence
<220>
   <223> synthetic construct; Class A amphipathic helical peptides
<400> 139
   Glu Trp Phe Lys Ala Phe Val Asp Lys Tyr Ala Asp Lys Phe Lys Glu
   1               5                   10                  15
   Ala Phe
<210> 140
   <211> 18
   <212> PRT
   <213> Artificial Sequence
<220>
   <223> synthetic construct; Class A amphipathic helical peptides
<400> 140
   Asp Trp Phe Lys Ala Phe Val Glu Lys Tyr Ala Asp Lys Phe Lys Glu
   1               5                   10                  15
   Ala Phe
<210> 141
   <211> 18
   <212> PRT
   <213> Artificial Sequence
<220>
   <223> synthetic construct; Class A amphipathic helical peptides
<400> 141
   Asp Trp Phe Lys Ala Phe Val Glu Lys Tyr Ala Glu Lys Phe Lys Asp
   1               5                   10                  15
   Ala Phe
<210> 142
   <211> 18
   <212> PRT
   <213> Artificial Sequence
<220>
   <223> synthetic construct; Class A amphipathic helical peptides
<400> 142
   Asp Trp Phe Lys Ala Phe Tyr Asp Lys Ala Val Glu Lys Phe Lys Glu
   1 5 10 15
   Ala Phe
<210> 143
   <211> 18
   <212> PRT
   <213> Artificial Sequence
<220>
   <223> synthetic construct; Class A amphipathic helical peptides
<400> 143
   Glu Trp Phe Lys Ala Phe Tyr Glu Lys Ala Val Asp Lys Phe Lys Asp
   1 5 10 15
   Ala Phe
<210> 144
   <211> 18
   <212> PRT
   <213> Artificial Sequence
<220>
   <223> synthetic construct; Class A amphipathic helical peptides
<400> 144
   Glu Trp Phe Lys Ala Phe Tyr Asp Lys Ala Val Asp Lys Phe Lys Glu
   1 5 10 15
   Ala Phe
<210> 145
   <211> 18
   <212> PRT
   <213> Artificial Sequence
<220>
   <223> synthetic construct; Class A amphipathic helical peptides
<400> 145
   Asp Trp Phe Lys Ala Phe Tyr Glu Lys Ala Val Asp Lys Phe Lys Glu
   1 5 10 15
   Ala Phe
<210> 146
   <211> 18
   <212> PRT
   <213> Artificial Sequence
<220>
   <223> synthetic construct; Class A amphipathic helical peptides
<400> 146
   Asp Trp Phe Lys Ala Phe Tyr Glu Lys Ala Val Glu Lys Phe Lys Asp
   1 5 10 15
   Ala Phe
<210> 147
   <211> 18
   <212> PRT
   <213> Artificial Sequence
<220>
   <223> synthetic construct; Class A amphipathic helical peptides
<400> 147
   Asp Trp Phe Lys Ala Phe Tyr Asp Lys Val Phe Glu Lys Ala Lys Glu
   1 5 10 15
   Ala Phe
<210> 148
   <211> 18
   <212> PRT
   <213> Artificial Sequence
<220>
   <223> synthetic construct; Class A amphipathic helical peptides
<400> 148
   Glu Trp Phe Lys Ala Phe Tyr Glu Lys Val Phe Asp Lys Ala Lys Asp
   1 5 10 15
   Ala Phe
<210> 149
   <211> 18
   <212> PRT
   <213> Artificial Sequence
<220>
   <223> synthetic construct; Class A amphipathic helical peptides
<400> 149
   Glu Trp Phe Lys Ala Phe Tyr Asp Lys Val Phe Asp Lys Ala Lys Glu
   1 5 10 15
   Ala Phe
<210> 150
   <211> 18
   <212> PRT
   <213> Artificial Sequence
<220>
   <223> synthetic construct; Class A amphipathic helical peptides
<400> 150
   Asp Trp Phe Lys Ala Phe Tyr Glu Lys Val Phe Asp Lys Ala Lys Glu
   1               5                   10                  15
   Ala Phe
<210> 151
   <211> 18
   <212> PRT
   <213> Artificial Sequence
<220>
   <223> synthetic construct; Class A amphipathic helical peptides
<400> 151
   Asp Trp Phe Lys Ala Phe Tyr Glu Lys Val Phe Glu Lys Ala Lys Asp
   1               5                   10                  15
   Ala Phe
<210> 152
   <211> 18
   <212> PRT
   <213> Artificial Sequence
<220>
   <223> synthetic construct; Class A amphipathic helical peptides
<400> 152
   Asp Trp Phe Lys Ala Phe Tyr Asp Lys Val Ala Glu Lys Ala Lys Glu
   1               5                   10                  15
   Phe Phe
<210> 153
   <211> 18
   <212> PRT
   <213> Artificial Sequence
<220>
   <223> synthetic construct; Class A amphipathic helical peptides
<400> 153
   Glu Trp Phe Lys Ala Phe Tyr Glu Lys Val Ala Asp Lys Ala Lys Asp
   1               5                   10                  15
   Phe Phe
<210> 154
   <211> 18
   <212> PRT
   <213> Artificial Sequence
<220>
   <223> synthetic construct; Class A amphipathic helical peptides
<400> 154
   Glu Trp Phe Lys Ala Phe Tyr Asp Lys Val Ala Asp Lys Ala Lys Glu
   1               5                   10                  15
   Phe Phe
<210> 155
   <211> 18
   <212> PRT
   <213> Artificial Sequence
<220>
   <223> synthetic construct; Class A amphipathic helical peptides
<400> 155
   Asp Trp Phe Lys Ala Phe Tyr Glu Lys Val Ala Asp Lys Ala Lys Glu
   1               5                   10                  15
   Phe Phe
<210> 156
   <211> 18
   <212> PRT
   <213> Artificial Sequence
<220>
   <223> synthetic construct; Class A amphipathic helical peptides
<400> 156
   Asp Trp Phe Lys Ala Phe Tyr Glu Lys Val Ala Glu Lys Ala Lys Asp
   1               5                   10                  15
   Phe Phe
<210> 157
   <211> 18
   <212> PRT
   <213> Artificial Sequence
<220>
   <223> synthetic construct; Class A amphipathic helical peptides
<400> 157
   Asp Trp Phe Lys Ala Phe Tyr Asp Lys Val Ala Glu Lys Phe Lys Glu
   1               5                   10                  15
   Phe Ala
<210> 158
   <211> 18
   <212> PRT
   <213> Artificial Sequence
<220>
   <223> synthetic construct; Class A amphipathic helical peptides
<400> 158
   Glu Trp Phe Lys Ala Phe Tyr Glu Lys Val Ala Asp Lys Phe Lys Asp
   1               5                   10                  15
   Phe Ala
<210> 159
   <211> 18
   <212> PRT
   <213> Artificial Sequence
<220>
   <223> synthetic construct; Class A amphipathic helical peptides
<400> 159
   Glu Trp Phe Lys Ala Phe Tyr Asp Lys Val Ala Asp Lys Phe Lys Glu
   1               5                   10                  15
   Phe Ala
<210> 160
   <211> 18
   <212> PRT
   <213> Artificial Sequence
<220>
   <223> synthetic construct; Class A amphipathic helical peptides
<400> 160
   Asp Trp Phe Lys Ala Phe Tyr Glu Lys Val Ala Asp Lys Phe Lys Glu
   1               5                   10                  15
   Phe Ala
<210> 161
   <211> 18
   <212> PRT
   <213> Artificial Sequence
<220>
   <223> synthetic construct; Class A amphipathic helical peptides
<400> 161
   Asp Trp Phe Lys Ala Phe Tyr Glu Lys Val Ala Glu Lys Phe Lys Asp
   1 5 10 15
   Phe Ala
<210> 162
   <211> 18
   <212> PRT
   <213> Artificial Sequence
<220>
   <223> synthetic construct; Class A amphipathic helical peptides
<400> 162
   Asp Ala Phe Lys Ala Phe Tyr Asp Lys Val Ala Glu Lys Phe Lys Glu
   1 5 10 15
   Trp Phe
<210> 163
   <211> 18
   <212> PRT
   <213> Artificial Sequence
<220>
   <223> synthetic construct; Class A amphipathic helical peptides
<400> 163
   Glu Ala Phe Lys Ala Phe Tyr Glu Lys Val Ala Asp Lys Phe Lys Asp
   1 5 10 15
   Trp Phe
<210> 164
   <211> 18
   <212> PRT
   <213> Artificial Sequence
<220>
   <223> synthetic construct; Class A amphipathic helical peptides
<400> 164
   Glu Ala Phe Lys Ala Phe Tyr Asp Lys Val Ala Asp Lys Phe Lys Glu
   1 5 10 15
   Trp Phe
<210> 165
   <211> 18
   <212> PRT
   <213> Artificial Sequence
<220>
   <223> synthetic construct; Class A amphipathic helical peptides
<400> 165
   Asp Ala Phe Lys Ala Phe Tyr Glu Lys Val Ala Asp Lys Phe Lys Glu
   1 5 10 15
   Trp Phe
<210> 166
   <211> 18
   <212> PRT
   <213> Artificial Sequence
<220>
   <223> synthetic construct; Class A amphipathic helical peptides
<400> 166
   Asp Ala Phe Lys Ala Phe Tyr Glu Lys Val Ala Glu Lys Phe Lys Asp
   1 5 10 15
   Trp Phe
<210> 167
   <211> 18
   <212> PRT
   <213> Artificial Sequence
<220>
   <223> synthetic construct; Class A amphipathic helical peptides
<400> 167
   Asp Ala Phe Lys Ala Phe Tyr Asp Lys Val Trp Glu Lys Phe Lys Glu
   1 5 10 15
   Ala Phe
<210> 168
   <211> 18
   <212> PRT
   <213> Artificial Sequence
<220>
   <223> synthetic construct; Class A amphipathic helical peptides
<400> 168
   Glu Ala Phe Lys Ala Phe Tyr Glu Lys Val Trp Asp Lys Phe Lys Asp
   1               5                   10                  15
   Ala Phe
<210> 169
   <211> 18
   <212> PRT
   <213> Artificial Sequence
<220>
   <223> synthetic construct; Class A amphipathic helical peptides
<400> 169
   Glu Ala Phe Lys Ala Phe Tyr Asp Lys Val Trp Asp Lys Phe Lys Glu
   1               5                   10                  15
   Ala Phe
<210> 170
   <211> 18
   <212> PRT
   <213> Artificial Sequence
<220>
   <223> synthetic construct; Class A amphipathic helical peptides
<400> 170
   Asp Ala Phe Lys Ala Phe Tyr Glu Lys Val Trp Asp Lys Phe Lys Glu
   1               5                   10                  15
   Ala Phe
<210> 171
   <211> 18
   <212> PRT
   <213> Artificial Sequence
<220>
   <223> synthetic construct; Class A amphipathic helical peptides
<400> 171
   Asp Ala Phe Lys Ala Phe Tyr Glu Lys Val Trp Glu Lys Phe Lys Asp
   1               5                   10                  15
   Ala Phe
<210> 172
   <211> 18
   <212> PRT
   <213> Artificial Sequence
<220>
   <223> synthetic construct; Class A amphipathic helical peptides
<400> 172
   Asp Tyr Phe Lys Ala Phe Trp Asp Lys Val Ala Glu Lys Phe Lys Glu
   1               5                   10                  15
   Ala Phe
<210> 173
   <211> 18
   <212> PRT
   <213> Artificial Sequence
<220>
   <223> synthetic construct; Class A amphipathic helical peptides
<400> 173
   Glu Tyr Phe Lys Ala Phe Trp Glu Lys Val Ala Asp Lys Phe Lys Asp
   1               5                   10                  15
   Ala Phe
<210> 174
   <211> 18
   <212> PRT
   <213> Artificial Sequence
<220>
   <223> synthetic construct; Class A amphipathic helical peptides
<400> 174
   Glu Tyr Phe Lys Ala Phe Trp Asp Lys Val Ala Asp Lys Phe Lys Glu
   1               5                   10                  15
   Ala Phe
<210> 175
   <211> 18
   <212> PRT
   <213> Artificial Sequence
<220>
   <223> synthetic construct; Class A amphipathic helical peptides
<400> 175
   Asp Tyr Phe Lys Ala Phe Trp Glu Lys Val Ala Asp Lys Phe Lys Glu
   1 5 10 15
   Ala Phe
<210> 176
   <211> 18
   <212> PRT
   <213> Artificial Sequence
<220>
   <223> synthetic construct; Class A amphipathic helical peptides
<400> 176
   Asp Tyr Phe Lys Ala Phe Trp Glu Lys Val Ala Glu Lys Phe Lys Asp
   1 5 10 15
   Ala Phe
<210> 177
   <211> 18
   <212> PRT
   <213> Artificial Sequence
<220>
   <223> synthetic construct; Class A amphipathic helical peptides
<400> 177
   Asp Trp Ala Lys Ala Phe Tyr Asp Lys Val Ala Glu Lys Phe Lys Glu
   1 5 10 15
   Phe Phe
<210> 178
   <211> 18
   <212> PRT
   <213> Artificial Sequence
<220>
   <223> synthetic construct; Class A amphipathic helical peptides
<400> 178
   Glu Trp Ala Lys Ala Phe Tyr Glu Lys Val Ala Asp Lys Phe Lys Asp
   1 5 10 15
   Phe Phe
<210> 179
   <211> 18
   <212> PRT
   <213> Artificial Sequence
<220>
   <223> synthetic construct; Class A amphipathic helical peptides
<400> 179
   Glu Trp Ala Lys Ala Phe Tyr Asp Lys Val Ala Asp Lys Phe Lys Glu
   1               5                   10                  15
   Phe Phe
<210> 180
   <211> 18
   <212> PRT
   <213> Artificial Sequence
<220>
   <223> synthetic construct; Class A amphipathic helical peptides
<400> 180
   Asp Trp Ala Lys Ala Phe Tyr Glu Lys Val Ala Asp Lys Phe Lys Glu
   1               5                   10                  15
   Phe Phe
<210> 181
   <211> 18
   <212> PRT
   <213> Artificial Sequence
<220>
   <223> synthetic construct; Class A amphipathic helical peptides
<400> 181
   Asp Trp Ala Lys Ala Phe Tyr Glu Lys Val Ala Glu Lys Phe Lys Asp
   1               5                   10                  15
   Phe Phe
<210> 182
   <211> 18
   <212> PRT
   <213> Artificial Sequence
<220>
   <223> synthetic construct; Class A amphipathic helical peptides
<400> 182
   Asp Trp Phe Lys Ala Ala Tyr Asp Lys Val Ala Glu Lys Phe Lys Glu
   1               5                   10                  15
   Phe Phe
<210> 183
   <211> 18
   <212> PRT
   <213> Artificial Sequence
<220>
   <223> synthetic construct; Class A amphipathic helical peptides
<400> 183
   Glu Trp Phe Lys Ala Ala Tyr Glu Lys Val Ala Asp Lys Phe Lys Asp
   1               5                   10                  15
   Phe Phe
<210> 184
   <211> 18
   <212> PRT
   <213> Artificial Sequence
<220>
   <223> synthetic construct; Class A amphipathic helical peptides
<400> 184
   Glu Trp Phe Lys Ala Ala Tyr Asp Lys Val Ala Asp Lys Phe Lys Glu
   1               5                   10                  15
   Phe Phe
<210> 185
   <211> 18
   <212> PRT
   <213> Artificial Sequence
<220>
   <223> synthetic construct; Class A amphipathic helical peptides
<400> 185
   Asp Trp Phe Lys Ala Ala Tyr Glu Lys Val Ala Asp Lys Phe Lys Glu
   1               5                   10                  15
   Phe Phe
<210> 186
   <211> 18
   <212> PRT
   <213> Artificial Sequence
<220>
   <223> synthetic construct; Class A amphipathic helical peptides
<400> 186
   Asp Trp Phe Lys Ala Ala Tyr Glu Lys Val Ala Glu Lys Phe Lys Asp
   1 5 10 15
   Phe Phe
<210> 187
   <211> 18
   <212> PRT
   <213> Artificial Sequence
<220>
   <223> synthetic construct; Class A amphipathic helical peptides
<400> 187
   Asp Trp Phe Lys Ala Phe Ala Asp Lys Val Ala Glu Lys Phe Lys Glu
   1 5 10 15
   Tyr Phe
<210> 188
   <211> 18
   <212> PRT
   <213> Artificial Sequence
<220>
   <223> synthetic construct; Class A amphipathic helical peptides
<400> 188
   Glu Trp Phe Lys Ala Phe Ala Glu Lys Val Ala Asp Lys Phe Lys Asp
   1 5 10 15
   Tyr Phe
<210> 189
   <211> 18
   <212> PRT
   <213> Artificial Sequence
<220>
   <223> synthetic construct; Class A amphipathic helical peptides
<400> 189
   Glu Trp Phe Lys Ala Phe Ala Asp Lys Val Ala Asp Lys Phe Lys Glu
   1 5 10 15
   Tyr Phe
<210> 190
   <211> 18
   <212> PRT
   <213> Artificial Sequence
<220>
   <223> synthetic construct; Class A amphipathic helical peptides
<400> 190
   Asp Trp Phe Lys Ala Phe Ala Glu Lys Val Ala Asp Lys Phe Lys Glu
   1 5 10 15
   Tyr Phe
<210> 191
   <211> 18
   <212> PRT
   <213> Artificial Sequence
<220>
   <223> synthetic construct; Class A amphipathic helical peptides
<400> 191
   Asp Trp Phe Lys Ala Phe Ala Glu Lys Val Ala Glu Lys Phe Lys Asp
   1 5 10 15
   Tyr Phe
<210> 192
   <211> 18
   <212> PRT
   <213> Artificial Sequence
<220>
   <223> synthetic construct; Class A amphipathic helical peptides
<400> 192
   Asp Trp Phe Lys Ala Phe Tyr Asp Lys Ala Ala Glu Lys Phe Lys Glu
   1 5 10 15
   Val Phe
<210> 193
   <211> 18
   <212> PRT
   <213> Artificial Sequence
<220>
   <223> synthetic construct; Class A amphipathic helical peptides
<400> 193
   Glu Trp Phe Lys Ala Phe Tyr Glu Lys Ala Ala Asp Lys Phe Lys Asp
   1 5 10 15
   Val Phe
<210> 194
   <211> 18
   <212> PRT
   <213> Artificial Sequence
<220>
   <223> synthetic construct; Class A amphipathic helical peptides
<400> 194
   Glu Trp Phe Lys Ala Phe Tyr Asp Lys Ala Ala Asp Lys Phe Lys Glu
   1 5 10 15
   Val Phe
<210> 195
   <211> 18
   <212> PRT
   <213> Artificial Sequence
<220>
   <223> synthetic construct; Class A amphipathic helical peptides
<400> 195
   Asp Trp Phe Lys Ala Phe Tyr Glu Lys Ala Ala Asp Lys Phe Lys Glu
   1 5 10 15
   Val Phe
<210> 196
   <211> 18
   <212> PRT
   <213> Artificial Sequence
<220>
   <223> synthetic construct; Class A amphipathic helical peptides
<400> 196
   Asp Trp Phe Lys Ala Phe Tyr Glu Lys Ala Ala Glu Lys Phe Lys Asp
   1 5 10 15
   Val Phe
<210> 197
   <211> 18
   <212> PRT
   <213> Artificial Sequence
<220>
   <223> synthetic construct; Class A amphipathic helical peptides
<400> 197
   Asp Trp Tyr Lys Ala Phe Phe Asp Lys Val Ala Glu Lys Phe Lys Glu
   1               5                   10                  15
   Ala Phe
<210> 198
   <211> 18
   <212> PRT
   <213> Artificial Sequence
<220>
   <223> synthetic construct; Class A amphipathic helical peptides
<400> 198
   Glu Trp Tyr Lys Ala Phe Phe Glu Lys Val Ala Asp Lys Phe Lys Asp
   1               5                   10                  15
   Ala Phe
<210> 199
   <211> 18
   <212> PRT
   <213> Artificial Sequence
<220>
   <223> synthetic construct; Class A amphipathic helical peptides
<400> 199
   Glu Trp Tyr Lys Ala Phe Phe Asp Lys Val Ala Asp Lys Phe Lys Glu
   1               5                   10                  15
   Ala Phe
<210> 200
   <211> 18
   <212> PRT
   <213> Artificial Sequence
<220>
   <223> synthetic construct; Class A amphipathic helical peptides
<400> 200
   Asp Trp Tyr Lys Ala Phe Phe Glu Lys Val Ala Asp Lys Phe Lys Glu
   1               5                   10                  15
   Ala Phe
<210> 201
   <211> 18
   <212> PRT
   <213> Artificial Sequence
<220>
   <223> synthetic construct; Class A amphipathic helical peptides
<400> 201
   Asp Trp Tyr Lys Ala Phe Phe Glu Lys Val Ala Glu Lys Phe Lys Asp
   1               5                   10                  15
   Ala Phe
<210> 202
   <211> 18
   <212> PRT
   <213> Artificial Sequence
<220>
   <223> synthetic construct; Class A amphipathic helical peptides
<400> 202
   Asp Trp Val Lys Ala Phe Tyr Asp Lys Phe Ala Glu Lys Phe Lys Glu
   1               5                   10                  15
   Ala Phe
<210> 203
   <211> 18
   <212> PRT
   <213> Artificial Sequence
<220>
   <223> synthetic construct; Class A amphipathic helical peptides
<400> 203
   Glu Trp Val Lys Ala Phe Tyr Glu Lys Phe Ala Asp Lys Phe Lys Asp
   1               5                   10                  15
   Ala Phe
<210> 204
   <211> 18
   <212> PRT
   <213> Artificial Sequence
<220>
   <223> synthetic construct; Class A amphipathic helical peptides
<400> 204
   Glu Trp Val Lys Ala Phe Tyr Asp Lys Phe Ala Asp Lys Phe Lys Glu
   1               5                   10                  15
   Ala Phe
<210> 205
   <211> 18
   <212> PRT
   <213> Artificial Sequence
<220>
   <223> synthetic construct; Class A amphipathic helical peptides
<400> 205
   Asp Trp Val Lys Ala Phe Tyr Glu Lys Phe Ala Asp Lys Phe Lys Glu
   1 5 10 15
   Ala Phe
<210> 206
   <211> 18
   <212> PRT
   <213> Artificial Sequence
<220>
   <223> synthetic construct; Class A amphipathic helical peptides
<400> 206
   Asp Trp Val Lys Ala Phe Tyr Glu Lys Phe Ala Glu Lys Phe Lys Asp
   1 5 10 15
   Ala Phe
<210> 207
   <211> 18
   <212> PRT
   <213> Artificial Sequence
<220>
   <223> synthetic construct; Class A amphipathic helical peptides
<400> 207
   Asp Trp Phe Lys Ala Phe Phe Asp Lys Val Ala Glu Lys Tyr Lys Glu
   1 5 10 15
   Ala Phe
<210> 208
   <211> 18
   <212> PRT
   <213> Artificial Sequence
<220>
   <223> synthetic construct; Class A amphipathic helical peptides
<400> 208
   Glu Trp Phe Lys Ala Phe Phe Glu Lys Val Ala Asp Lys Tyr Lys Asp
   1 5 10 15
   Ala Phe
<210> 209
   <211> 18
   <212> PRT
   <213> Artificial Sequence
<220>
   <223> synthetic construct; Class A amphipathic helical peptides
<400> 209
   Glu Trp Phe Lys Ala Phe Phe Asp Lys Val Ala Asp Lys Tyr Lys Glu
   1 5 10 15
   Ala Phe
<210> 210
   <211> 18
   <212> PRT
   <213> Artificial Sequence
<220>
   <223> synthetic construct; Class A amphipathic helical peptides
<400> 210
   Asp Trp Phe Lys Ala Phe Phe Glu Lys Val Ala Asp Lys Tyr Lys Glu
   1 5 10 15
   Ala Phe
<210> 211
   <211> 18
   <212> PRT
   <213> Artificial Sequence
<220>
   <223> synthetic construct; Class A amphipathic helical peptides
<400> 211
   Asp Trp Phe Lys Ala Phe Phe Glu Lys Val Ala Asp Lys Tyr Lys Glu
   1 5 10 15
   Ala Phe
<210> 212
   <211> 18
   <212> PRT
   <213> Artificial Sequence
<220>
   <223> synthetic construct; Class A amphipathic helical peptides
<400> 212
   Asp Trp Phe Lys Ala Phe Phe Asp Lys Val Ala Glu Lys Phe Lys Glu
   1               5                   10                  15
   Ala Tyr
<210> 213
   <211> 18
   <212> PRT
   <213> Artificial Sequence
<220>
   <223> synthetic construct; Class A amphipathic helical peptides
<400> 213
   Glu Trp Phe Lys Ala Phe Phe Glu Lys Val Ala Asp Lys Phe Lys Asp
   1               5                   10                  15
   Ala Tyr
<210> 214
   <211> 18
   <212> PRT
   <213> Artificial Sequence
<220>
   <223> synthetic construct; Class A amphipathic helical peptides
<400> 214
   Glu Trp Phe Lys Ala Phe Phe Asp Lys Val Ala Asp Lys Phe Lys Glu
   1               5                   10                  15
   Ala Tyr
<210> 215
   <211> 18
   <212> PRT
   <213> Artificial Sequence
<220>
   <223> synthetic construct; Class A amphipathic helical peptides
<400> 215
   Asp Trp Phe Lys Ala Phe Phe Glu Lys Val Ala Asp Lys Phe Lys Glu
   1               5                   10                  15
   Ala Tyr
<210> 216
   <211> 18
   <212> PRT
   <213> Artificial Sequence
<220>
   <223> synthetic construct; Class A amphipathic helical peptides
<400> 216
   Asp Trp Phe Lys Ala Phe Phe Glu Lys Val Ala Glu Lys Phe Lys Asp
   1               5                   10                  15
   Ala Tyr
<210> 217
   <211> 18
   <212> PRT
   <213> Artificial Sequence
<220>
   <223> synthetic construct; Class A amphipathic helical peptides
<400> 217
   Asp Trp Phe Lys Ala Phe Tyr Asp Lys Phe Ala Glu Lys Phe Lys Glu
   1               5                   10                  15
   Ala Val
<210> 218
   <211> 18
   <212> PRT
   <213> Artificial Sequence
<220>
   <223> synthetic construct; Class A amphipathic helical peptides
<400> 218
   Glu Trp Phe Lys Ala Phe Tyr Glu Lys Phe Ala Asp Lys Phe Lys Asp
   1               5                   10                  15
   Ala Val
<210> 219
   <211> 18
   <212> PRT
   <213> Artificial Sequence
<220>
   <223> synthetic construct; Class A amphipathic helical peptides
<400> 219
   Glu Trp Phe Lys Ala Phe Tyr Asp Lys Phe Ala Asp Lys Phe Lys Glu
   1               5                   10                  15
   Ala Val
<210> 220
   <211> 18
   <212> PRT
   <213> Artificial Sequence
<220>
   <223> synthetic construct; Class A amphipathic helical peptides
<400> 220
   Asp Trp Phe Lys Ala Phe Tyr Glu Lys Phe Ala Asp Lys Phe Lys Glu
   1               5                   10                  15
   Ala Val
<210> 221
   <211> 18
   <212> PRT
   <213> Artificial Sequence
<220>
   <223> synthetic construct; Class A amphipathic helical peptides
<400> 221
   Asp Trp Phe Lys Ala Phe Tyr Glu Lys Phe Ala Glu Lys Phe Lys Asp
   1               5                   10                  15
   Ala Val
<210> 222
   <211> 18
   <212> PRT
   <213> Artificial Sequence
<220>
   <223> synthetic construct; Class A amphipathic helical peptides
<400> 222
   Asp Lys Phe Lys Ala Phe Tyr Asp Lys Val Ala Glu Lys Phe Trp Glu
   1               5                   10                  15
   Ala Phe
<210> 223
   <211> 18
   <212> PRT
   <213> Artificial Sequence
<220>
   <223> synthetic construct; Class A amphipathic helical peptides
<400> 223
   Glu Lys Phe Lys Ala Phe Tyr Glu Lys Val Ala Asp Lys Phe Trp Asp
   1               5                   10                  15
   Ala Phe
<210> 224
   <211> 18
   <212> PRT
   <213> Artificial Sequence
<220>
   <223> synthetic construct; Class A amphipathic helical peptides
<400> 224
   Glu Lys Phe Lys Ala Phe Tyr Asp Lys Val Ala Asp Lys Phe Trp Glu
   1               5                   10                  15
   Ala Phe
<210> 225
   <211> 18
   <212> PRT
   <213> Artificial Sequence
<220>
   <223> synthetic construct; Class A amphipathic helical peptides
<400> 225
   Asp Lys Phe Lys Ala Phe Tyr Glu Lys Val Ala Asp Lys Phe Trp Glu
   1               5                   10                  15
   Ala Phe
<210> 226
   <211> 18
   <212> PRT
   <213> Artificial Sequence
<220>
   <223> synthetic construct; Class A amphipathic helical peptides
<400> 226
   Asp Lys Phe Lys Ala Phe Tyr Glu Lys Val Ala Glu Lys Phe Trp Asp
   1               5                   10                  15
   Ala Phe
<210> 227
   <211> 18
   <212> PRT
   <213> Artificial Sequence
<220>
   <223> synthetic construct; Class A amphipathic helical peptides
<400> 227
   Asp Lys Trp Lys Ala Phe Tyr Asp Lys Val Ala Glu Lys Phe Phe Glu
   1               5                   10                  15
   Ala Phe
<210> 228
   <211> 18
   <212> PRT
   <213> Artificial Sequence
<220>
   <223> synthetic construct; Class A amphipathic helical peptides
<400> 228
   Glu Lys Trp Lys Ala Phe Tyr Glu Lys Val Ala Asp Lys Phe Phe Asp
   1               5                   10                  15
   Ala Phe
<210> 229
   <211> 18
   <212> PRT
   <213> Artificial Sequence
<220>
   <223> synthetic construct; Class A amphipathic helical peptides
<400> 229
   Glu Lys Trp Lys Ala Phe Tyr Asp Lys Val Ala Asp Lys Phe Phe Glu
   1               5                   10                  15
   Ala Phe
<210> 230
   <211> 18
   <212> PRT
   <213> Artificial Sequence
<220>
   <223> synthetic construct; Class A amphipathic helical peptides
<400> 230
   Asp Lys Trp Lys Ala Phe Tyr Glu Lys Val Ala Asp Lys Phe Phe Glu
   1               5                   10                  15
   Ala Phe
<210> 231
   <211> 18
   <212> PRT
   <213> Artificial Sequence
<220>
   <223> synthetic construct; Class A amphipathic helical peptides
<400> 231
   Asp Lys Trp Lys Ala Phe Tyr Glu Lys Val Ala Glu Lys Phe Phe Asp
   1               5                   10                  15
   Ala Phe
<210> 232
   <211> 18
   <212> PRT
   <213> Artificial Sequence
<220>
   <223> synthetic construct; Class A amphipathic helical peptides
<400> 232
   Asp Lys Phe Lys Ala Phe Tyr Asp Lys Trp Ala Glu Val Phe Lys Glu
   1               5                   10                  15
   Ala Phe
<210> 233
   <211> 18
   <212> PRT
   <213> Artificial Sequence
<220>
   <223> synthetic construct; Class A amphipathic helical peptides
<400> 233
   Glu Lys Phe Lys Ala Phe Tyr Glu Lys Trp Ala Asp Val Phe Lys Asp
   1               5                   10                  15
   Ala Phe
<210> 234
   <211> 18
   <212> PRT
   <213> Artificial Sequence
<220>
   <223> synthetic construct; Class A amphipathic helical peptides
<400> 234
   Glu Lys Phe Lys Ala Phe Tyr Asp Lys Trp Ala Asp Val Phe Lys Glu
   1 5 10 15
   Ala Phe
<210> 235
   <211> 18
   <212> PRT
   <213> Artificial Sequence
<220>
   <223> synthetic construct; Class A amphipathic helical peptides
<400> 235
   Asp Lys Phe Lys Ala Phe Tyr Glu Lys Trp Ala Asp Val Phe Lys Glu
   1 5 10 15
   Ala Phe
<210> 236
   <211> 18
   <212> PRT
   <213> Artificial Sequence
<220>
   <223> synthetic construct; Class A amphipathic helical peptides
<400> 236
   Asp Lys Phe Lys Ala Phe Tyr Glu Lys Trp Ala Glu Val Phe Lys Asp
   1 5 10 15
   Ala Phe
<210> 237
   <211> 18
   <212> PRT
   <213> Artificial Sequence
<220>
   <223> synthetic construct; Class A amphipathic helical peptides
<400> 237
   Asp Lys Phe Lys Ala Phe Tyr Asp Lys Val Ala Glu Phe Trp Lys Glu
   1 5 10 15
   Ala Phe
<210> 238
   <211> 18
   <212> PRT
   <213> Artificial Sequence
<220>
   <223> synthetic construct; Class A amphipathic helical peptides
<400> 238
   Glu Lys Phe Lys Ala Phe Tyr Glu Lys Val Ala Asp Phe Trp Lys Asp
   1               5                   10                  15
   Ala Phe
<210> 239
   <211> 18
   <212> PRT
   <213> Artificial Sequence
<220>
   <223> synthetic construct; Class A amphipathic helical peptides
<400> 239
   Glu Lys Phe Cys Ala Phe Tyr Asp Lys Val Ala Asp Phe Trp Lys Glu
   1               5                   10                  15
   Ala Phe
<210> 240
   <211> 18
   <212> PRT
   <213> Artificial Sequence
<220>
   <223> synthetic construct; Class A amphipathic helical peptides
<400> 240
   Asp Lys Phe Lys Ala Phe Tyr Glu Lys Val Ala Asp Phe Trp Lys Glu
   1               5                   10                  15
   Ala Phe
<210> 241
   <211> 18
   <212> PRT
   <213> Artificial Sequence
<220>
   <223> synthetic construct; Class A amphipathic helical peptides
<400> 241
   Asp Lys Phe Lys Ala Phe Tyr Glu Lys Val Ala Glu Phe Trp Lys Asp
   1               5                   10                  15
   Ala Phe
<210> 242
   <211> 18
   <212> PRT
   <213> Artificial Sequence
<220>
   <223> synthetic construct; Class A amphipathic helical peptides
<400> 242
   Phe Ala Glu Lys Phe Lys Glu Ala Val Lys Asp Tyr Phe Ala Lys Phe
   1               5                   10                  15
   Trp Asp
<210> 243
   <211> 18
   <212> PRT
   <213> Artificial Sequence
<220>
   <223> synthetic construct; Class A amphipathic helical peptides
<400> 243
   Phe Ala Asp Lys Phe Lys Asp Ala Val Lys Glu Tyr Phe Ala Lys Phe
   1               5                   10                  15
   Trp Glu
<210> 244
   <211> 18
   <212> PRT
   <213> Artificial Sequence
<220>
   <223> synthetic construct; Class A amphipathic helical peptides
<400> 244
   Phe Ala Asp Lys Phe Lys Glu Ala Val Lys Asp Tyr Phe Ala Lys Phe
   1               5                   10                  15
   Trp Glu
<210> 245
   <211> 18
   <212> PRT
   <213> Artificial Sequence
<220>
   <223> synthetic construct; Class A amphipathic helical peptides
<400> 245
   Phe Ala Glu Lys Phe Lys Asp Ala Val Lys Glu Tyr Phe Ala Lys Phe
   1               5                   10                  15
   Trp Asp
<210> 246
   <211> 18
   <212> PRT
   <213> Artificial Sequence
<220>
   <223> synthetic construct; Class A amphipathic helical peptides
<400> 246
   Phe Ala Glu Lys Phe Lys Asp Ala Val Lys Asp Tyr Phe Ala Lys Phe
   1               5                   10                  15
   Trp Glu
<210> 247
   <211> 18
   <212> PRT
   <213> Artificial Sequence
<220>
   <223> synthetic construct; Class A amphipathic helical peptides
<400> 247
   Phe Trp Glu Lys Phe Lys Glu Ala Val Lys Asp Tyr Phe Ala Lys Phe
   1               5                   10                  15
   Ala Asp
<210> 248
   <211> 18
   <212> PRT
   <213> Artificial Sequence
<220>
   <223> synthetic construct; Class A amphipathic helical peptides
<400> 248
   Phe Trp Asp Lys Phe Lys Asp Ala Val Lys Glu Tyr Phe Ala Lys Phe
   1               5                   10                  15
   Ala Glu
<210> 249
   <211> 18
   <212> PRT
   <213> Artificial Sequence
<220>
   <223> synthetic construct; Class A amphipathic helical peptides
<400> 249
   Phe Ala Asp Lys Phe Lys Glu Ala Val Lys Asp Tyr Phe Ala Lys Phe
   1               5                   10                  15
   Trp Glu
<210> 250
   <211> 18
   <212> PRT
   <213> Artificial Sequence
<220>
   <223> synthetic construct; Class A amphipathic helical peptides
<400> 250
   Phe Ala Glu Lys Phe Lys Asp Ala Val Lys Glu Tyr Phe Ala Lys Phe
   1               5                   10                  15
   Trp Asp
<210> 251
   <211> 18
   <212> PRT
   <213> Artificial Sequence
<220>
   <223> synthetic construct; Class A amphipathic helical peptides
<400> 251
   Phe Ala Glu Lys Phe Lys Asp Ala Val Lys Asp Tyr Phe Ala Lys Phe
   1               5                   10                  15
   Trp Glu
<210> 252
   <211> 18
   <212> PRT
   <213> Artificial Sequence
<220>
   <223> synthetic construct; Class A amphipathic helical peptides
<400> 252
   Phe Phe Glu Lys Phe Lys Glu Ala Val Lys Asp Tyr Phe Ala Lys Ala
   1 5 10 15
   Trp Asp
<210> 253
   <211> 18
   <212> PRT
   <213> Artificial Sequence
<220>
   <223> synthetic construct; Class A amphipathic helical peptides
<400> 253
   Phe Phe Asp Lys Phe Lys Asp Ala Val Lys Glu Tyr Phe Ala Lys Ala
   1 5 10 15
   Trp Glu
<210> 254
   <211> 18
   <212> PRT
   <213> Artificial Sequence
<220>
   <223> synthetic construct; Class A amphipathic helical peptides
<400> 254
   Phe Phe Asp Lys Phe Lys Glu Ala Val Lys Asp Tyr Phe Ala Lys Ala
   1 5 10 15
   Trp Glu
<210> 255
   <211> 18
   <212> PRT
   <213> Artificial Sequence
<220>
   <223> synthetic construct; Class A amphipathic helical peptides
<400> 255
   Phe Phe Glu Lys Phe Lys Asp Ala Val Lys Glu Tyr Phe Ala Lys Ala
   1 5 10 15
   Trp Asp
<210> 256
   <211> 18
   <212> PRT
   <213> Artificial Sequence
<220>
   <223> synthetic construct; Class A amphipathic helical peptides
<400> 256
   Phe Phe Glu Lys Phe Lys Asp Ala Val Lys Asp Tyr Phe Ala Lys Ala
   1 5 10 15
   Trp Glu
<210> 257
   <211> 18
   <212> PRT
   <213> Artificial Sequence
<220>
   <223> synthetic construct; Class A amphipathic helical peptides
<400> 257
   Phe Ala Glu Lys Ala Lys Glu Phe Val Lys Asp Tyr Phe Ala Lys Phe
   1 5 10 15
   Trp Asp
<210> 258
   <211> 18
   <212> PRT
   <213> Artificial Sequence
<220>
   <223> synthetic construct; Class A amphipathic helical peptides
<400> 258
   Phe Ala Asp Lys Ala Lys Asp Phe Val Lys Glu Tyr Phe Ala Lys Phe
   1 5 10 15
   Trp Glu
<210> 259
   <211> 18
   <212> PRT
   <213> Artificial Sequence
<220>
   <223> synthetic construct; Class A amphipathic helical peptides
<400> 259
   Phe Ala Asp Lys Ala Lys Glu Phe Val Lys Asp Tyr Phe Ala Lys Phe
   1 5 10 15
   Trp Glu
<210> 260
   <211> 18
   <212> PRT
   <213> Artificial Sequence
<220>
   <223> synthetic construct; Class A amphipathic helical peptides
<400> 260
   Phe Ala Glu Lys Ala Lys Asp Phe Val Lys Glu Tyr Phe Ala Lys Phe
   1               5                   10                  15
   Trp Asp
<210> 261
   <211> 18
   <212> PRT
   <213> Artificial Sequence
<220>
   <223> synthetic construct; Class A amphipathic helical peptides
<400> 261
   Phe Ala Glu Lys Ala Lys Asp Phe Val Lys Asp Tyr Phe Ala Lys Phe
   1               5                   10                  15
   Trp Glu
<210> 262
   <211> 18
   <212> PRT
   <213> Artificial Sequence
<220>
   <223> synthetic construct; Class A amphipathic helical peptides
<400> 262
   Phe Ala Glu Lys Phe Lys Glu Val Ala Lys Asp Tyr Phe Ala Lys Phe
   1               5                   10                  15
   Trp Asp
<210> 263
   <211> 18
   <212> PRT
   <213> Artificial Sequence
<220>
   <223> synthetic construct; Class A amphipathic helical peptides
<400> 263
   Phe Ala Asp Lys Phe Lys Asp Val Ala Lys Glu Tyr Phe Ala Lys Phe
   1 5 10 15
   Trp Glu
<210> 264
   <211> 18
   <212> PRT
   <213> Artificial Sequence
<220>
   <223> synthetic construct; Class A amphipathic helical peptides
<400> 264
   Phe Ala Asp Lys Phe Lys Glu Val Ala Lys Asp Tyr Phe Ala Lys Phe
   1 5 10 15
   Trp Glu
<210> 265
   <211> 18
   <212> PRT
   <213> Artificial Sequence
<220>
   <223> synthetic construct; Class A amphipathic helical peptides
<400> 265
   Phe Ala Glu Lys Phe Lys Asp Val Ala Lys Glu Tyr Phe Ala Lys Phe
   1 5 10 15
   Trp Asp
<210> 266
   <211> 18
   <212> PRT
   <213> Artificial Sequence
<220>
   <223> synthetic construct; Class A amphipathic helical peptides
<400> 266
   Phe Ala Glu Lys Phe Lys Asp Val Ala Lys Asp Tyr Phe Ala Lys Phe
   1 5 10 15
   Trp Glu
<210> 267
   <211> 18
   <212> PRT
   <213> Artificial Sequence
<220>
   <223> synthetic construct; Class A amphipathic helical peptides
<400> 267
   Phe Ala Asp Lys Phe Lys Glu Ala Tyr Lys Asp Val Phe Ala Lys Phe
   1 5 10 15
   Trp Glu
<210> 268
   <211> 18
   <212> PRT
   <213> Artificial Sequence
<220>
   <223> synthetic construct; Class A amphipathic helical peptides
<400> 268
   Phe Ala Glu Lys Phe Lys Asp Ala Tyr Lys Glu Val Phe Ala Lys Phe
   1 5 10 15
   Trp Asp
<210> 269
   <211> 18
   <212> PRT
   <213> Artificial Sequence
<220>
   <223> synthetic construct; Class A amphipathic helical peptides
<400> 269
   Phe Ala Glu Lys Phe Lys Asp Ala Tyr Lys Asp Val Phe Ala Lys Phe
   1 5 10 15
   Trp Glu
<210> 270
   <211> 18
   <212> PRT
   <213> Artificial Sequence
<220>
   <223> synthetic construct; Class A amphipathic helical peptides
<400> 270
   Phe Ala Glu Lys Phe Lys Glu Ala Val Lys Asp Phe Tyr Ala Lys Phe
   1 5 10 15
   Trp Asp
<210> 271
   <211> 18
   <212> PRT
   <213> Artificial Sequence
<220>
   <223> synthetic construct; Class A amphipathic helical peptides
<400> 271
   Phe Ala Asp Lys Phe Lys Asp Ala Val Lys Glu Phe Tyr Ala Lys Phe
   1               5                   10                  15
   Trp Glu
<210> 272
   <211> 18
   <212> PRT
   <213> Artificial Sequence
<220>
   <223> synthetic construct; Class A amphipathic helical peptides
<400> 272
   Phe Ala Asp Lys Phe Lys Glu Ala Val Lys Asp Phe Tyr Ala Lys Phe
   1               5                   10                  15
   Trp Glu
<210> 273
   <211> 18
   <212> PRT
   <213> Artificial Sequence
<220>
   <223> synthetic construct; Class A amphipathic helical peptides
<400> 273
   Phe Ala Glu Lys Phe Lys Asp Ala Val Lys Glu Phe Tyr Ala Lys Phe
   1               5                   10                  15
   Trp Asp
<210> 274
   <211> 18
   <212> PRT
   <213> Artificial Sequence
<220>
   <223> synthetic construct; Class A amphipathic helical peptides
<400> 274
   Phe Ala Glu Lys Phe Lys Asp Ala Val Lys Asp Phe Tyr Ala Lys Phe
   1 5 10 15
   Trp Glu
<210> 275
   <211> 18
   <212> PRT
   <213> Artificial Sequence
<220>
   <223> synthetic construct; Class A amphipathic helical peptides
<400> 275
   Phe Ala Glu Lys Phe Trp Glu Ala Val Lys Asp Tyr Phe Ala Lys Phe
   1 5 10 15
   Lys Asp
<210> 276
   <211> 18
   <212> PRT
   <213> Artificial Sequence
<220>
   <223> synthetic construct; Class A amphipathic helical peptides
<400> 276
   Phe Ala Asp Lys Phe Trp Asp Ala Val Lys Glu Tyr Phe Ala Lys Phe
   1 5 10 15
   Lys Glu
<210> 277
   <211> 18
   <212> PRT
   <213> Artificial Sequence
<220>
   <223> synthetic construct; Class A amphipathic helical peptides
<400> 277
   Phe Ala Asp Lys Phe Trp Glu Ala Val Lys Asp Tyr Phe Ala Lys Phe
   1 5 10 15
   Lys Glu
<210> 278
   <211> 18
   <212> PRT
   <213> Artificial Sequence
<220>
   <223> synthetic construct; Class A amphipathic helical peptides
<400> 278
   Phe Ala Glu Lys Phe Trp Asp Ala Val Lys Glu Tyr Phe Ala Lys Phe
   1               5                   10                  15
   Lys Asp
<210> 279
   <211> 18
   <212> PRT
   <213> Artificial Sequence
<220>
   <223> synthetic construct; Class A amphipathic helical peptides
<400> 279
   Phe Ala Glu Lys Phe Trp Asp Ala Val Lys Asp Tyr Phe Ala Lys Phe
   1               5                   10                  15
   Lys Glu
<210> 280
   <211> 18
   <212> PRT
   <213> Artificial Sequence
<220>
   <223> synthetic construct; Class A amphipathic helical peptides
<400> 280
   Ala Phe Glu Lys Phe Lys Glu Ala Val Lys Asp Tyr Phe Ala Lys Phe
   1               5                   10                  15
   Trp Asp
<210> 281
   <211> 18
   <212> PRT
   <213> Artificial Sequence
<220>
   <223> synthetic construct; Class A amphipathic helical peptides
<400> 281
   Ala Phe Asp Lys Phe Lys Asp Ala Val Lys Glu Tyr Phe Ala Lys Phe
   1               5                   10                  15
   Trp Glu
<210> 282
   <211> 18
   <212> PRT
   <213> Artificial Sequence
<220>
   <223> synthetic construct; Class A amphipathic helical peptides
<400> 282
   Ala Phe Asp Lys Phe Lys Glu Ala Val Lys Asp Tyr Phe Ala Lys Phe
   1 5 10 15
   Trp Glu
<210> 283
   <211> 18
   <212> PRT
   <213> Artificial Sequence
<220>
   <223> synthetic construct; Class A amphipathic helical peptides
<400> 283
   Ala Phe Glu Lys Phe Lys Asp Ala Val Lys Glu Tyr Phe Ala Lys Phe
   1 5 10 15
   Trp Asp
<210> 284
   <211> 18
   <212> PRT
   <213> Artificial Sequence
<220>
   <223> synthetic construct; Class A amphipathic helical peptides
<400> 284
   Ala Phe Glu Lys Phe Lys Asp Ala Val Lys Asp Tyr Phe Ala Lys Phe
   1 5 10 15
   Trp Glu
<210> 285
   <211> 18
   <212> PRT
   <213> Artificial Sequence
<220>
   <223> synthetic construct; Class A amphipathic helical peptides
<400> 285
   Val Ala Glu Lys Phe Lys Glu Ala Phe Lys Asp Tyr Phe Ala Lys Phe
   1 5 10 15
   Trp Asp
<210> 286
   <211> 18
   <212> PRT
   <213> Artificial Sequence
<220>
   <223> synthetic construct; Class A amphipathic helical peptides
<400> 286
   Val Ala Asp Lys Phe Lys Asp Ala Phe Lys Glu Tyr Phe Ala Lys Phe
   1 5 10 15
   Trp Glu
<210> 287
   <211> 18
   <212> PRT
   <213> Artificial Sequence
<220>
   <223> synthetic construct; Class A amphipathic helical peptides
<400> 287
   Val Ala Asp Lys Phe Lys Glu Ala Phe Lys Asp Tyr Phe Ala Lys Phe
   1 5 10 15
   Trp Glu
<210> 288
   <211> 18
   <212> PRT
   <213> Artificial Sequence
<220>
   <223> synthetic construct; Class A amphipathic helical peptides
<400> 288
   Val Ala Glu Lys Phe Lys Asp Ala Phe Lys Glu Tyr Phe Ala Lys Phe
   1 5 10 15
   Trp Asp
<210> 289
   <211> 18
   <212> PRT
   <213> Artificial Sequence
<220>
   <223> synthetic construct; Class A amphipathic helical peptides
<400> 289
   Val Ala Glu Lys Phe Lys Asp Ala Phe Lys Asp Tyr Phe Ala Lys Phe
   1               5                   10                  15
   Trp Glu
<210> 290
   <211> 18
   <212> PRT
   <213> Artificial Sequence
<220>
   <223> synthetic construct; Class A amphipathic helical peptides
<400> 290
   Tyr Ala Glu Lys Phe Lys Glu Ala Val Lys Asp Phe Phe Ala Lys Phe
   1               5                   10                  15
   Trp Asp
<210> 291
   <211> 18
   <212> PRT
   <213> Artificial Sequence
<220>
   <223> synthetic construct; Class A amphipathic helical peptides
<400> 291
   Tyr Ala Asp Lys Phe Lys Asp Ala Val Lys Glu Phe Phe Ala Lys Phe
   1               5                   10                  15
   Trp Glu
<210> 292
   <211> 18
   <212> PRT
   <213> Artificial Sequence
<220>
   <223> synthetic construct; Class A amphipathic helical peptides
<400> 292
   Tyr Ala Asp Lys Phe Lys Glu Ala Val Lys Asp Phe Phe Ala Lys Phe
   1               5                   10                  15
   Trp Glu
<210> 293
   <211> 18
   <212> PRT
   <213> Artificial Sequence
<220>
   <223> synthetic construct; Class A amphipathic helical peptides
<400> 293
   Tyr Ala Glu Lys Phe Lys Asp Ala Val Lys Glu Phe Phe Ala Lys Phe
   1               5                   10                  15
   Trp Asp
<210> 294
   <211> 18
   <212> PRT
   <213> Artificial Sequence
<220>
   <223> synthetic construct; Class A amphipathic helical peptides
<400> 294
   Tyr Ala Glu Lys Phe Lys Asp Ala Val Lys Asp Phe Phe Ala Lys Phe
   1               5                   10                  15
   Trp Glu
<210> 295
   <211> 18
   <212> PRT
   <213> Artificial Sequence
<220>
   <223> synthetic construct; Class A amphipathic helical peptides
<400> 295
   Ala Ala Glu Lys Phe Lys Glu Phe Val Lys Asp Tyr Phe Ala Lys Phe
   1               5                   10                  15
   Trp Asp
<210> 296
   <211> 18
   <212> PRT
   <213> Artificial Sequence
<220>
   <223> synthetic construct; Class A amphipathic helical peptides
<400> 296
   Ala Ala Asp Lys Phe Lys Asp Phe Val Lys Glu Tyr Phe Ala Lys Phe
   1 5 10 15
   Trp Glu
<210> 297
   <211> 18
   <212> PRT
   <213> Artificial Sequence
<220>
   <223> synthetic construct; Class A amphipathic helical peptides
<400> 297
   Ala Ala Asp Lys Phe Lys Glu Phe Val Lys Asp Tyr Phe Ala Lys Phe
   1 5 10 15
   Trp Glu
<210> 298
   <211> 18
   <212> PRT
   <213> Artificial Sequence
<220>
   <223> synthetic construct; Class A amphipathic helical peptides
<400> 298
   Ala Ala Glu Lys Phe Lys Asp Phe Val Lys Glu Tyr Phe Ala Lys Phe
   1 5 10 15
   Trp Asp
<210> 299
   <211> 18
   <212> PRT
   <213> Artificial Sequence
<220>
   <223> synthetic construct; Class A amphipathic helical peptides
<400> 299
   Ala Ala Glu Lys Phe Lys Asp Phe Val Lys Asp Tyr Phe Ala Lys Phe
   1 5 10 15
   Trp Glu
<210> 300
   <211> 18
   <212> PRT
   <213> Artificial Sequence
<220>
   <223> synthetic construct; Class A amphipathic helical peptides
<400> 300
   Phe Phe Glu Lys Ala Lys Glu Ala Val Lys Asp Tyr Phe Ala Lys Phe
   1               5                   10                  15
   Trp Asp
<210> 301
   <211> 18
   <212> PRT
   <213> Artificial Sequence
<220>
   <223> synthetic construct; Class A amphipathic helical peptides
<400> 301
   Phe Phe Asp Lys Ala Lys Asp Ala Val Lys Glu Tyr Phe Ala Lys Phe
   1               5                   10                  15
   Trp Glu
<210> 302
   <211> 18
   <212> PRT
   <213> Artificial Sequence
<220>
   <223> synthetic construct; Class A amphipathic helical peptides
<400> 302
   Phe Phe Asp Lys Ala Lys Glu Ala Val Lys Asp Tyr Phe Ala Lys Phe
   1               5                   10                  15
   Trp Glu
<210> 303
   <211> 16
   <212> PRT
   <213> Artificial Sequence
<220>
   <223> synthetic construct; Class A amphipathic helical peptides
<400> 303
   Glu Lys Ala Lys Asp Ala Val Lys Glu Tyr Phe Ala Lys Phe Trp Asp
   1               5                   10                  15
<210> 304
   <211> 16
   <212> PRT
   <213> Artificial Sequence
<220>
   <223> synthetic construct; Class A amphipathic helical peptides
<400> 304
   Glu Lys Ala Lys Asp Ala Val Lys Asp Tyr Phe Ala Lys Phe Trp Glu
   1               5                   10                  15
<210> 305
   <211> 18
   <212> PRT
   <213> Artificial Sequence
<220>
   <223> synthetic construct; Class A amphipathic helical peptides
<400> 305
   Phe Tyr Glu Lys Phe Lys Glu Ala Val Lys Asp Ala Phe Ala Lys Phe
   1               5                   10                  15
   Trp Asp
<210> 306
   <211> 18
   <212> PRT
   <213> Artificial Sequence
<220>
   <223> synthetic construct; Class A amphipathic helical peptides
<400> 306
   Phe Tyr Asp Lys Phe Lys Asp Ala Val Lys Glu Ala Phe Ala Lys Phe
   1               5                   10                  15
   Trp Glu
<210> 307
   <211> 18
   <212> PRT
   <213> Artificial Sequence
<220>
   <223> synthetic construct; Class A amphipathic helical peptides
<400> 307
   Phe Tyr Asp Lys Phe Lys Glu Ala Val Lys Asp Ala Phe Ala Lys Phe
   1               5                   10                  15
   Trp Glu
<210> 308
   <211> 18
   <212> PRT
   <213> Artificial Sequence
<220>
   <223> synthetic construct; Class A amphipathic helical peptides
<400> 308
   Phe Tyr Glu Lys Phe Lys Asp Ala Val Lys Glu Ala Phe Ala Lys Phe
   1               5                   10                  15
   Trp Asp
<210> 309
   <211> 18
   <212> PRT
   <213> Artificial Sequence
<220>
   <223> synthetic construct; Class A amphipathic helical peptides
<400> 309
   Phe Tyr Glu Lys Phe Lys Asp Ala Val Lys Asp Ala Phe Ala Lys Phe
   1               5                   10                  15
   Trp Glu
<210> 310
   <211> 18
   <212> PRT
   <213> Artificial Sequence
<220>
   <223> synthetic construct; Class A amphipathic helical peptides
<400> 310
   Phe Val Glu Lys Phe Lys Glu Ala Ala Lys Asp Tyr Phe Ala Lys Phe
   1               5                   10                  15
   Trp Asp
<210> 311
   <211> 18
   <212> PRT
   <213> Artificial Sequence
<220>
   <223> synthetic construct; Class A amphipathic helical peptides
<400> 311
   Phe Val Asp Lys Phe Lys Asp Ala Ala Lys Glu Tyr Phe Ala Lys Phe
   1               5                   10                  15
   Trp Glu
<210> 312
   <211> 18
   <212> PRT
   <213> Artificial Sequence
<220>
   <223> synthetic construct; Class A amphipathic helical peptides
<400> 312
   Phe Val Asp Lys Phe Lys Glu Ala Ala Lys Asp Tyr Phe Ala Lys Phe
   1               5                   10                  15
   Trp Glu
<210> 313
   <211> 18
   <212> PRT
   <213> Artificial Sequence
<220>
   <223> synthetic construct; Class A amphipathic helical peptides
<400> 313
   Phe Val Glu Lys Phe Lys Asp Ala Ala Lys Glu Tyr Phe Ala Lys Phe
   1               5                   10                  15
   Trp Asp
<210> 314
   <211> 18
   <212> PRT
   <213> Artificial Sequence
<220>
   <223> synthetic construct; Class A amphipathic helical peptides
<400> 314
   Phe Val Glu Lys Phe Lys Asp Ala Ala Lys Asp Tyr Phe Ala Lys Phe
   1               5                   10                  15
   Trp Glu
<210> 315
   <211> 18
   <212> PRT
   <213> Artificial Sequence
<220>
   <223> synthetic construct; Class A amphipathic helical peptides
<400> 315
   Phe Ala Glu Lys Tyr Lys Glu Ala Val Lys Asp Phe Phe Ala Lys Phe
   1               5                   10                  15
   Trp Asp
<210> 316
   <211> 18
   <212> PRT
   <213> Artificial Sequence
<220>
   <223> synthetic construct; Class A amphipathic helical peptides
<400> 316
   Phe Ala Asp Lys Tyr Lys Asp Ala Val Lys Glu Phe Phe Ala Lys Phe
   1               5                   10                  15
   Trp Glu
<210> 317
   <211> 18
   <212> PRT
   <213> Artificial Sequence
<220>
   <223> synthetic construct; Class A amphipathic helical peptides
<400> 317
   Phe Ala Asp Lys Tyr Lys Glu Ala Val Lys Asp Phe Phe Ala Lys Phe
   1               5                   10                  15
   Trp Glu
<210> 318
   <211> 18
   <212> PRT
   <213> Artificial Sequence
<220>
   <223> synthetic construct; Class A amphipathic helical peptides
<400> 318
   Phe Ala Glu Lys Tyr Lys Asp Ala Val Lys Glu Phe Phe Ala Lys Phe
   1 5 10 15
   Trp Asp
<210> 319
   <211> 18
   <212> PRT
   <213> Artificial Sequence
<220>
   <223> synthetic construct; Class A amphipathic helical peptides
<400> 319
   Phe Ala Glu Lys Tyr Lys Asp Ala Val Lys Asp Phe Phe Ala Lys Phe
   1 5 10 15
   Trp Glu
<210> 320
   <211> 18
   <212> PRT
   <213> Artificial Sequence
<220>
   <223> synthetic construct; Class A amphipathic helical peptides
<400> 320
   Phe Ala Glu Lys Val Lys Glu Ala Phe Lys Asp Tyr Phe Ala Lys Phe
   1 5 10 15
   Trp Asp
<210> 321
   <211> 18
   <212> PRT
   <213> Artificial Sequence
<220>
   <223> synthetic construct; Class A amphipathic helical peptides
<400> 321
   Phe Ala Asp Lys Val Lys Asp Ala Phe Lys Glu Tyr Phe Ala Lys Phe
   1 5 10 15
   Trp Glu
<210> 322
   <211> 18
   <212> PRT
   <213> Artificial Sequence
<220>
   <223> synthetic construct; Class A amphipathic helical peptides
<400> 322
   Phe Ala Asp Lys Val Lys Glu Ala Phe Lys Asp Tyr Phe Ala Lys Phe
   1 5 10 15
   Trp Glu
<210> 323
   <211> 18
   <212> PRT
   <213> Artificial Sequence
<220>
   <223> synthetic construct; Class A amphipathic helical peptides
<400> 323
   Phe Ala Glu Lys Val Lys Asp Ala Phe Lys Glu Tyr Phe Ala Lys Phe
   1 5 10 15
   Trp Asp
<210> 324
   <211> 18
   <212> PRT
   <213> Artificial Sequence
<220>
   <223> synthetic construct; Class A amphipathic helical peptides
<400> 324
   Phe Ala Glu Lys Val Lys Asp Ala Phe Lys Asp Tyr Phe Ala Lys Phe
   1 5 10 15
   Trp Glu
<210> 325
   <211> 18
   <212> PRT
   <213> Artificial Sequence
<220>
   <223> synthetic construct; Class A amphipathic helical peptides
<400> 325
   Phe Ala Glu Lys Phe Lys Glu Tyr Val Lys Asp Ala Phe Ala Lys Phe
   1 5 10 15
   Trp Asp
<210> 326
   <211> 18
   <212> PRT
   <213> Artificial Sequence
<220>
   <223> synthetic construct; Class A amphipathic helical peptides
<400> 326
   Phe Ala Asp Lys Phe Lys Asp Tyr Val Lys Glu Ala Phe Ala Lys Phe
   1               5                   10                  15
   Trp Glu
<210> 327
   <211> 18
   <212> PRT
   <213> Artificial Sequence
<220>
   <223> synthetic construct; Class A amphipathic helical peptides
<400> 327
   Phe Ala Asp Lys Phe Lys Glu Tyr Val Lys Asp Ala Phe Ala Lys Phe
   1               5                   10                  15
   Trp Glu
<210> 328
   <211> 18
   <212> PRT
   <213> Artificial Sequence
<220>
   <223> synthetic construct; Class A amphipathic helical peptides
<400> 328
   Phe Ala Glu Lys Phe Lys Asp Tyr Val Lys Glu Ala Phe Ala Lys Phe
   1               5                   10                  15
   Trp Asp
<210> 329
   <211> 18
   <212> PRT
   <213> Artificial Sequence
<220>
   <223> synthetic construct; Class A amphipathic helical peptides
<400> 329
   Phe Ala Glu Lys Phe Lys Asp Tyr Val Lys Asp Ala Phe Ala Lys Phe
   1               5                   10                  15
   Trp Glu
<210> 330
   <211> 18
   <212> PRT
   <213> Artificial Sequence
<220>
   <223> synthetic construct; Class A amphipathic helical peptides
<400> 330
   Phe Ala Glu Lys Phe Lys Glu Ala Phe Lys Asp Tyr Val Ala Lys Phe
   1               5                   10                  15
   Trp Asp
<210> 331
   <211> 18
   <212> PRT
   <213> Artificial Sequence
<220>
   <223> synthetic construct; Class A amphipathic helical peptides
<400> 331
   Phe Ala Asp Lys Phe Lys Asp Ala Phe Lys Glu Tyr Val Ala Lys Phe
   1               5                   10                  15
   Trp Glu
<210> 332
   <211> 18
   <212> PRT
   <213> Artificial Sequence
<220>
   <223> synthetic construct; Class A amphipathic helical peptides
<400> 332
   Phe Ala Asp Lys Phe Lys Glu Ala Phe Lys Asp Tyr Val Ala Lys Phe
   1               5                   10                  15
   Trp Glu
<210> 333
   <211> 18
   <212> PRT
   <213> Artificial Sequence
<220>
   <223> synthetic construct; Class A amphipathic helical peptides
<400> 333
   Phe Ala Glu Lys Phe Lys Asp Ala Phe Lys Glu Tyr Val Ala Lys Phe
   1               5                   10                  15
   Trp Asp
<210> 334
   <211> 18
   <212> PRT
   <213> Artificial Sequence
<220>
   <223> synthetic construct; Class A amphipathic helical peptides
<400> 334
   Phe Ala Glu Lys Phe Lys Asp Ala Phe Lys Asp Tyr Val Ala Lys Phe
   1               5                   10                  15
   Trp Glu
<210> 335
   <211> 18
   <212> PRT
   <213> Artificial Sequence
<220>
   <223> synthetic construct; Class A amphipathic helical peptides
<400> 335
   Phe Ala Glu Lys Phe Lys Glu Ala Phe Lys Asp Tyr Phe Ala Lys Val
   1               5                   10                  15
   Trp Asp
<210> 336
   <211> 18
   <212> PRT
   <213> Artificial Sequence
<220>
   <223> synthetic construct; Class A amphipathic helical peptides
<400> 336
   Phe Ala Asp Lys Phe Lys Asp Ala Phe Lys Glu Tyr Phe Ala Lys Val
   1               5                   10                  15
   Trp Glu
<210> 337
   <211> 18
   <212> PRT
   <213> Artificial Sequence
<220>
   <223> synthetic construct; Class A amphipathic helical peptides
<400> 337
   Phe Ala Asp Lys Phe Lys Glu Ala Phe Lys Asp Tyr Phe Ala Lys Val
   1               5                   10                  15
   Trp Glu
<210> 338
   <211> 18
   <212> PRT
   <213> Artificial Sequence
<220>
   <223> synthetic construct; Class A amphipathic helical peptides
<400> 338
   Phe Ala Glu Lys Phe Lys Asp Ala Phe Lys Glu Tyr Phe Ala Lys Val
   1               5                   10                  15
   Trp Asp
<210> 339
   <211> 18
   <212> PRT
   <213> Artificial Sequence
<220>
   <223> synthetic construct; Class A amphipathic helical peptides
<400> 339
   Phe Ala Glu Lys Phe Lys Asp Ala Phe Lys Asp Tyr Phe Ala Lys Val
   1               5                   10                  15
   Trp Glu
<210> 340
   <211> 18
   <212> PRT
   <213> Artificial Sequence
<220>
   <223> synthetic construct; Class A amphipathic helical peptides
<400> 340
   Phe Ala Glu Lys Phe Lys Glu Ala Val Lys Asp Phe Phe Ala Lys Tyr
   1 5 10 15
   Trp Asp
<210> 341
   <211> 18
   <212> PRT
   <213> Artificial Sequence
<220>
   <223> synthetic construct; Class A amphipathic helical peptides
<400> 341
   Phe Ala Asp Lys Phe Lys Asp Ala Val Lys Glu Phe Phe Ala Lys Tyr
   1 5 10 15
   Trp Glu
<210> 342
   <211> 18
   <212> PRT
   <213> Artificial Sequence
<220>
   <223> synthetic construct; Class A amphipathic helical peptides
<400> 342
   Phe Ala Asp Lys Phe Lys Glu Ala Val Lys Asp Phe Phe Ala Lys Tyr
   1 5 10 15
   Trp Glu
<210> 343
   <211> 18
   <212> PRT
   <213> Artificial Sequence
<220>
   <223> synthetic construct; Class A amphipathic helical peptides
<400> 343
   Phe Ala Glu Lys Phe Lys Asp Ala Val Lys Glu Phe Phe Ala Lys Tyr
   1 5 10 15
   Trp Asp
<210> 344
   <211> 18
   <212> PRT
   <213> Artificial Sequence
<220>
   <223> synthetic construct; Class A amphipathic helical peptides
<400> 344
   Phe Ala Glu Lys Phe Lys Asp Ala Val Lys Asp Phe Phe Ala Lys Tyr
   1               5                   10                  15
   Trp Glu
<210> 345
   <211> 18
   <212> PRT
   <213> Artificial Sequence
<220>
   <223> synthetic construct; Class A amphipathic helical peptides
<400> 345
   Trp Ala Glu Lys Phe Phe Glu Ala Val Lys Asp Tyr Phe Ala Lys Phe
   1               5                   10                  15
   Lys Asp
<210> 346
   <211> 18
   <212> PRT
   <213> Artificial Sequence
<220>
   <223> synthetic construct; Class A amphipathic helical peptides
<400> 346
   Trp Ala Asp Lys Phe Phe Asp Ala Val Lys Glu Tyr Phe Ala Lys Phe
   1               5                   10                  15
   Lys Glu
<210> 347
   <211> 18
   <212> PRT
   <213> Artificial Sequence
<220>
   <223> synthetic construct; Class A amphipathic helical peptides
<400> 347
   Trp Ala Asp Lys Phe Phe Glu Ala Val Lys Asp Tyr Phe Ala Lys Phe
   1               5                   10                  15
   Lys Glu
<210> 348
   <211> 18
   <212> PRT
   <213> Artificial Sequence
<220>
   <223> synthetic construct; Class A amphipathic helical peptides
<400> 348
   Trp Ala Glu Lys Phe Phe Asp Ala Val Lys Glu Tyr Phe Ala Lys Phe
   1               5                   10                  15
   Lys Asp
<210> 349
   <211> 18
   <212> PRT
   <213> Artificial Sequence
<220>
   <223> synthetic construct; Class A amphipathic helical peptides
<400> 349
   Trp Ala Glu Lys Phe Phe Asp Ala Val Lys Asp Tyr Phe Ala Lys Phe
   1               5                   10                  15
   Lys Glu
<210> 350
   <211> 18
   <212> PRT
   <213> Artificial Sequence
<220>
   <223> synthetic construct; Class A amphipathic helical peptides
<400> 350
   Phe Ala Glu Lys Trp Phe Glu Ala Val Lys Asp Tyr Phe Ala Lys Phe
   1               5                   10                  15
   Lys Asp
<210> 351
   <211> 18
   <212> PRT
   <213> Artificial Sequence
<220>
   <223> synthetic construct; Class A amphipathic helical peptides
<400> 351
   Phe Ala Asp Lys Trp Phe Asp Ala Val Lys Glu Tyr Phe Ala Lys Phe
   1 5 10 15
   Lys Glu
<210> 352
   <211> 18
   <212> PRT
   <213> Artificial Sequence
<220>
   <223> synthetic construct; Class A amphipathic helical peptides
<400> 352
   Phe Ala Asp Lys Trp Phe Glu Ala Val Lys Asp Tyr Phe Ala Lys Phe
   1 5 10 15
   Lys Glu
<210> 353
   <211> 18
   <212> PRT
   <213> Artificial Sequence
<220>
   <223> synthetic construct; Class A amphipathic helical peptides
<400> 353
   Phe Ala Glu Lys Trp Phe Asp Ala Val Lys Glu Tyr Phe Ala Lys Phe
   1 5 10 15
   Lys Asp
<210> 354
   <211> 18
   <212> PRT
   <213> Artificial Sequence
<220>
   <223> synthetic construct; Class A amphipathic helical peptides
<400> 354
   Phe Ala Glu Lys Trp Phe Asp Ala Val Lys Asp Tyr Phe Ala Lys Phe
   1 5 10 15
   Lys Glu
<210> 355
   <211> 18
   <212> PRT
   <213> Artificial Sequence
<220>
   <223> synthetic construct; Class A amphipathic helical peptides
<400> 355
   Phe Ala Glu Lys Phe Val Glu Ala Trp Lys Asp Tyr Phe Ala Lys Phe
   1               5                   10                  15
   Lys Asp
<210> 356
   <211> 18
   <212> PRT
   <213> Artificial Sequence
<220>
   <223> synthetic construct; Class A amphipathic helical peptides
<400> 356
   Phe Ala Asp Lys Phe Val Asp Ala Trp Lys Glu Tyr Phe Ala Lys Phe
   1               5                   10                  15
   Lys Glu
<210> 357
   <211> 18
   <212> PRT
   <213> Artificial Sequence
<220>
   <223> synthetic construct; Class A amphipathic helical peptides
<400> 357
   Phe Ala Asp Lys Phe Val Glu Ala Trp Lys Asp Tyr Phe Ala Lys Phe
   1               5                   10                  15
   Lys Glu
<210> 358
   <211> 18
   <212> PRT
   <213> Artificial Sequence
<220>
   <223> synthetic construct; Class A amphipathic helical peptides
<400> 358
   Phe Ala Glu Lys Phe Val Asp Ala Trp Lys Glu Tyr Phe Ala Lys Phe
   1               5                   10                  15
   Lys Asp
<210> 359
   <211> 18
   <212> PRT
   <213> Artificial Sequence
<220>
   <223> synthetic construct; Class A amphipathic helical peptides
<400> 359
   Phe Ala Glu Lys Phe Val Asp Ala Trp Lys Asp Tyr Phe Ala Lys Phe
   1 5 10 15
   Lys Glu
<210> 360
   <211> 18
   <212> PRT
   <213> Artificial Sequence
<220>
   <223> synthetic construct; Class A amphipathic helical peptides
<400> 360
   Phe Tyr Glu Lys Phe Ala Glu Ala Val Lys Asp Trp Phe Ala Lys Phe
   1 5 10 15
   Lys Asp
<210> 361
   <211> 18
   <212> PRT
   <213> Artificial Sequence
<220>
   <223> synthetic construct; Class A amphipathic helical peptides
<400> 361
   Phe Tyr Asp Lys Phe Ala Asp Ala Val Lys Glu Trp Phe Ala Lys Phe
   1 5 10 15
   Lys Glu
<210> 362
   <211> 18
   <212> PRT
   <213> Artificial Sequence
<220>
   <223> synthetic construct; Class A amphipathic helical peptides
<400> 362
   Phe Tyr Asp Lys Phe Ala Glu Ala Val Lys Asp Trp Phe Ala Lys Phe
   1               5                   10                  15
   Lys Glu
<210> 363
   <211> 18
   <212> PRT
   <213> Artificial Sequence
<220>
   <223> synthetic construct; Class A amphipathic helical peptides
<400> 363
   Phe Tyr Glu Lys Phe Ala Asp Ala Val Lys Glu Trp Phe Ala Lys Phe
   1               5                   10                  15
   Lys Asp
<210> 364
   <211> 18
   <212> PRT
   <213> Artificial Sequence
<220>
   <223> synthetic construct; Class A amphipathic helical peptides
<400> 364
   Phe Tyr Glu Lys Phe Ala Asp Ala Val Lys Asp Trp Phe Ala Lys Phe
   1               5                   10                  15
   Lys Glu
<210> 365
   <211> 18
   <212> PRT
   <213> Artificial Sequence
<220>
   <223> synthetic construct; class A or class Y amphipathic helix peptide
<220>
   <221> misc
   <222> (1)..(1)
   <223> D can be Asp or Glu
<220>
   <221> misc
   <222> (2)..(3)
   <223> Xaa can be Leu, norLeu, Val, Ile, Trp, Phe, Tyr, beta-Nal, or alpha-Nal
<220>
   <221> misc
   <222> (5)..(5)
   <223> Y can be Ala, His, Ser, Gln, Asn, or Thr
<220>
   <221> misc
   <222> (6)..(7)
   <223> Xaa can be Leu, norLeu, Val, Ile, Trp, Phe, Tyr, beta-Nal, or alpha-Nal
<220>
   <221> misc
   <222> (8)..(8)
   <223> D can be Asp or Glu
<220>
   <221> misc
   <222> (10)..(10)
   <223> Xaa can be Leu, norLeu, Val, Ile, Trp, Phe, Tyr, beta-Nal, or alpha-Nal
<220>
   <221> misc
   <222> (11)..(11)
   <223> Y can be Ala, His, Ser, Gln, Asn, or Thr
<220>
   <221> misc
   <222> (12)..(12)
   <223> D can be Asp or Glu
<220>
   <221> misc
   <222> (14)..(14)
   <223> Xaa can be Leu, norLeu, Val, Ile, Trp, Phe, Tyr, beta-Nal, or alpha-Nal
<220>
   <221> misc
   <222> (16)..(16)
   <223> D can be Asp or Glu
<220>
   <221> misc
   <222> (17)..(17)
   <223> Y can be Ala, His, Ser, Gln, Asn, or Thr
<220>
   <221> misc
   <222> (18)..(18)
   <223> Xaa can be Leu, norLeu, Val, Ile, Trp, Phe, Tyr, beta-Nal, or alpha-Nal
<400> 365
   Asp Xaa Xaa Lys Tyr Xaa Xaa Asp Lys Xaa Tyr Asp Lys Xaa Lys Asp
   1 5 10 15
   Tyr Xaa
<210> 366
   <211> 18
   <212> PRT
   <213> Artificial Sequence
<220>
   <223> synthetic construct; peptide mimetic
<400> 366
   Asp Trp Phe Lys His Phe Tyr Asp Lys Val Ala Glu Lys Phe Lys Glu
   1               5                   10                  15
   Ala Phe
<210> 367
   <211> 18
   <212> PRT
   <213> Artificial Sequence
<220>
   <223> synthetic construct; peptide mimetic
<400> 367
   Glu Trp Phe Lys His Phe Tyr Glu Lys Val Ala Asp Lys Phe Lys Asp
   1               5                   10                  15
   Ala Phe
<210> 368
   <211> 18
   <212> PRT
   <213> Artificial Sequence
<220>
   <223> synthetic construct; peptide mimetic
<400> 368
   Glu Trp Phe Lys His Phe Tyr Asp Lys Val Ala Glu Lys Phe Lys Glu
   1               5                   10                  15
   Ala Phe
<210> 369
   <211> 18
   <212> PRT
   <213> Artificial Sequence
<220>
   <223> synthetic construct; peptide mimetic
<400> 369
   Asp Trp Phe Lys His Phe Tyr Glu Lys Val Ala Glu Lys Phe Lys Glu
   1 5 10 15
   Ala Phe
<210> 370
   <211> 18
   <212> PRT
   <213> Artificial Sequence
<220>
   <223> synthetic construct; peptide mimetic
<400> 370
   Asp Trp Phe Lys His Phe Tyr Asp Lys Val Ala Asp Lys Phe Lys Glu
   1 5 10 15
   Ala Phe
<210> 371
   <211> 18
   <212> PRT
   <213> Artificial Sequence
<220>
   <223> synthetic construct; peptide mimetic
<400> 371
   Asp Trp Phe Lys His Phe Tyr Asp Lys Val Ala Glu Lys Phe Lys Asp
   1 5 10 15
   Ala Phe
<210> 372
   <211> 18
   <212> PRT
   <213> Artificial Sequence
<220>
   <223> synthetic construct; peptide mimetic
<400> 372
   Asp Trp His Lys Phe Phe Tyr Asp Lys Val Ala Glu Lys Phe Lys Glu
   1 5 10 15
   Ala Phe
<210> 373
   <211> 18
   <212> PRT
   <213> Artificial Sequence
<220>
   <223> synthetic construct; peptide mimetic
<400> 373
   Glu Trp His Lys Phe Phe Tyr Glu Lys Val Ala Asp Lys Phe Lys Asp
   1 5 10 15
   Ala Phe
<210> 374
   <211> 18
   <212> PRT
   <213> Artificial Sequence
<220>
   <223> synthetic construct; peptide mimetic
<400> 374
   Glu Trp His Lys Phe Phe Tyr Asp Lys Val Ala Glu Lys Phe Lys Glu
   1 5 10 15
   Ala Phe
<210> 375
   <211> 18
   <212> PRT
   <213> Artificial Sequence
<220>
   <223> synthetic construct; peptide mimetic
<400> 375
   Asp Trp His Lys Phe Phe Tyr Glu Lys Val Ala Glu Lys Phe Lys Glu
   1 5 10 15
   Ala Phe
<210> 376
   <211> 18
   <212> PRT
   <213> Artificial Sequence
<220>
   <223> synthetic construct; peptide mimetic
<400> 376
   Asp Trp His Lys Phe Phe Tyr Asp Lys Val Ala Asp Lys Phe Lys Glu
   1 5 10 15
   Ala Phe
<210> 377
   <211> 18
   <212> PRT
   <213> Artificial Sequence
<220>
   <223> synthetic construct; peptide mimetic
<400> 377
   Asp Trp His Lys Phe Phe Tyr Asp Lys Val Ala Glu Lys Phe Lys Asp
   1 5 10 15
   Ala Phe
<210> 378
   <211> 18
   <212> PRT
   <213> Artificial Sequence
<220>
   <223> synthetic construct; peptide mimetic
<400> 378
   Asp Trp Phe Lys Phe His Tyr Asp Lys Val Ala Glu Lys Phe Lys Glu
   1 5 10 15
   Ala Phe
<210> 379
   <211> 18
   <212> PRT
   <213> Artificial Sequence
<220>
   <223> synthetic construct; peptide mimetic
<400> 379
   Glu Trp Phe Lys Phe His Tyr Glu Lys Val Ala Asp Lys Phe Lys Asp
   1 5 10 15
   Ala Phe
<210> 380
   <211> 18
   <212> PRT
   <213> Artificial Sequence
<220>
   <223> synthetic construct; peptide mimetic
<400> 380
   Glu Trp Phe Lys Phe His Tyr Asp Lys Val Ala Glu Lys Phe Lys Glu
   1               5                   10                  15
   Ala Phe
<210> 381
   <211> 18
   <212> PRT
   <213> Artificial Sequence
<220>
   <223> synthetic construct; peptide mimetic
<400> 381
   Asp Trp Phe Lys Phe His Tyr Glu Lys Val Ala Glu Lys Phe Lys Glu
   1               5                   10                  15
   Ala Phe
<210> 382
   <211> 18
   <212> PRT
   <213> Artificial Sequence
<220>
   <223> synthetic construct; peptide mimetic
<400> 382
   Asp Trp Phe Lys Phe His Tyr Asp Lys Val Ala Asp Lys Phe Lys Glu
   1               5                   10                  15
   Ala Phe
<210> 383
   <211> 18
   <212> PRT
   <213> Artificial Sequence
<220>
   <223> synthetic construct; peptide mimetic
<400> 383
   Asp Trp Phe Lys Phe His Tyr Asp Lys Val Ala Glu Lys Phe Lys Asp
   1 5 10 15
   Ala Phe
<210> 384
   <211> 18
   <212> PRT
   <213> Artificial Sequence
<220>
   <223> synthetic construct; peptide mimetic
<400> 384
   Asp Trp Phe Lys Val Phe Tyr Asp Lys His Ala Glu Lys Phe Lys Glu
   1 5 10 15
   Ala Phe
<210> 385
   <211> 18
   <212> PRT
   <213> Artificial Sequence
<220>
   <223> synthetic construct; peptide mimetic
<400> 385
   Glu Trp Phe Lys Val Phe Tyr Glu Lys His Ala Asp Lys Phe Lys Asp
   1 5 10 15
   Ala Phe
<210> 386
   <211> 18
   <212> PRT
   <213> Artificial Sequence
<220>
   <223> synthetic construct; peptide mimetic
<400> 386
   Glu Trp Phe Lys Val Phe Tyr Asp Lys His Ala Glu Lys Phe Lys Glu
   1 5 10 15
   Ala Phe
<210> 387
   <211> 18
   <212> PRT
   <213> Artificial Sequence
<220>
   <223> synthetic construct; peptide mimetic
<400> 387
   Asp Trp Phe Lys Val Phe Tyr Glu Lys His Ala Glu Lys Phe Lys Glu
   1 5 10 15
   Ala Phe
<210> 388
   <211> 18
   <212> PRT
   <213> Artificial Sequence
<220>
   <223> synthetic construct; peptide mimetic
<400> 388
   Asp Trp Phe Lys Val Phe Tyr Asp Lys His Ala Asp Lys Phe Lys Glu
   1 5 10 15
   Ala Phe
<210> 389
   <211> 18
   <212> PRT
   <213> Artificial Sequence
<220>
   <223> synthetic construct; peptide mimetic
<400> 389
   Asp Trp Phe Lys Val Phe Tyr Asp Lys His Ala Glu Lys Phe Lys Asp
   1 5 10 15
   Ala Phe
<210> 390
   <211> 18
   <212> PRT
   <213> Artificial Sequence
<220>
   <223> synthetic construct; peptide mimetic
<400> 390
   Asp Trp Phe Lys Ala Phe Tyr Asp Lys Val Ala Glu Lys Phe Lys Glu
   1 5 10 15
   His Phe
<210> 391
   <211> 18
   <212> PRT
   <213> Artificial Sequence
<220>
   <223> synthetic construct; peptide mimetic
<400> 391
   Glu Trp Phe Lys Ala Phe Tyr Glu Lys Val Ala Asp Lys Phe Lys Asp
   1 5 10 15
   His Phe
<210> 392
   <211> 18
   <212> PRT
   <213> Artificial Sequence
<220>
   <223> synthetic construct; peptide mimetic
<400> 392
   Glu Trp Phe Lys Ala Phe Tyr Asp Lys Val Ala Glu Lys Phe Lys Glu
   1 5 10 15
   His Phe
<210> 393
   <211> 18
   <212> PRT
   <213> Artificial Sequence
<220>
   <223> synthetic construct; peptide mimetic
<400> 393
   Asp Trp Phe Lys Ala Phe Tyr Glu Lys Val Ala Glu Lys Phe Lys Glu
   1 5 10 15
   His Phe
<210> 394
   <211> 18
   <212> PRT
   <213> Artificial Sequence
<220>
   <223> synthetic construct; peptide mimetic
<400> 394
   Asp Trp Phe Lys Ala Phe Tyr Asp Lys Val Ala Asp Lys Phe Lys Glu
   1               5                   10                  15
   His Phe
<210> 395
   <211> 18
   <212> PRT
   <213> Artificial Sequence
<220>
   <223> synthetic construct; peptide mimetic
<400> 395
   Asp Trp Phe Lys Ala Phe Tyr Asp Lys Val Ala Glu Lys Phe Lys Asp
   1               5                   10                  15
   His Phe
<210> 396
   <211> 18
   <212> PRT
   <213> Artificial Sequence
<220>
   <223> synthetic construct; peptide mimetic
<400> 396
   Asp Trp Phe Lys Ala Phe Tyr Asp Lys Val Ala Glu Lys Phe Lys Glu
   1               5                   10                  15
   Phe His
<210> 397
   <211> 18
   <212> PRT
   <213> Artificial Sequence
<220>
   <223> synthetic construct; peptide mimetic
<400> 397
   Glu Trp Phe Lys Ala Phe Tyr Glu Lys Val Ala Asp Lys Phe Lys Asp
   1 5 10 15
   Phe His
<210> 398
   <211> 18
   <212> PRT
   <213> Artificial Sequence
<220>
   <223> synthetic construct; peptide mimetic
<400> 398
   Glu Trp Phe Lys Ala Phe Tyr Asp Lys Val Ala Glu Lys Phe Lys Glu
   1 5 10 15
   Phe His
<210> 399
   <211> 18
   <212> PRT
   <213> Artificial Sequence
<220>
   <223> synthetic construct; peptide mimetic
<400> 399
   Asp Trp Phe Lys Ala Phe Tyr Asp Lys Val Ala Glu Lys Phe Lys Glu
   1 5 10 15
   Phe His
<210> 400
   <211> 18
   <212> PRT
   <213> Artificial Sequence
<220>
   <223> synthetic construct; peptide mimetic
<400> 400
   Asp Trp Phe Lys Ala Phe Tyr Glu Lys Val Ala Glu Lys Phe Lys Glu
   1 5 10 15
   Phe His
<210> 401
   <211> 18
   <212> PRT
   <213> Artificial Sequence
<220>
   <223> synthetic construct; peptide mimetic
<400> 401
   Asp Trp Phe Lys Ala Phe Tyr Asp Lys Val Ala Glu Lys Phe Lys Glu
   1               5                   10                  15
   Phe His
<210> 402
   <211> 18
   <212> PRT
   <213> Artificial Sequence
<220>
   <223> synthetic construct; peptide mimetic
<400> 402
   Asp Trp Phe Lys Ala Phe Tyr Asp Lys Val Ala Glu Lys Phe Lys Asp
   1               5                   10                  15
   Phe His
<210> 403
   <211> 18
   <212> PRT
   <213> Artificial Sequence
<220>
   <223> synthetic construct; peptide mimetic
<400> 403
   Phe Ala Glu Lys Phe Lys Glu Ala Val Lys Asp Tyr Phe Ala Lys Phe
   1               5                   10                  15
   Trp Asp
<210> 404
   <211> 18
   <212> PRT
   <213> Artificial Sequence
<220>
   <223> synthetic construct; peptide mimetic
<400> 404
   Phe His Glu Lys Phe Lys Glu Ala Val Lys Asp Tyr Phe Ala Lys Phe
   1 5 10 15
   Trp Asp
<210> 405
   <211> 18
   <212> PRT
   <213> Artificial Sequence
<220>
   <223> synthetic construct; peptide mimetic
<400> 405
   Phe His Glu Lys Phe Lys Glu Ala Val Lys Glu Tyr Phe Ala Lys Phe
   1 5 10 15
   Trp Glu
<210> 406
   <211> 18
   <212> PRT
   <213> Artificial Sequence
<220>
   <223> synthetic construct; peptide mimetic
<400> 406
   Phe His Asp Lys Phe Lys Asp Ala Val Lys Asp Tyr Phe Ala Lys Phe
   1 5 10 15
   Trp Asp
<210> 407
   <211> 18
   <212> PRT
   <213> Artificial Sequence
<220>
   <223> synthetic construct; peptide mimetic
<400> 407
   Phe His Asp Lys Phe Lys Asp Ala Val Lys Glu Tyr Phe Ala Lys Phe
   1 5 10 15
   Trp Glu
<210> 408
   <211> 18
   <212> PRT
   <213> Artificial Sequence
<220>
   <223> synthetic construct; peptide mimetic
<400> 408
   Phe His Asp Lys Phe Lys Glu Ala Val Lys Asp Tyr Phe Ala Lys Phe
   1 5 10 15
   Trp Asp
<210> 409
   <211> 18
   <212> PRT
   <213> Artificial Sequence
<220>
   <223> synthetic construct; peptide mimetic
<400> 409
   Phe His Glu Lys Phe Lys Asp Ala Val Lys Asp Tyr Phe Ala Lys Phe
   1 5 10 15
   Trp Asp
<210> 410
   <211> 18
   <212> PRT
   <213> Artificial Sequence
<220>
   <223> synthetic construct; peptide mimetic
<400> 410
   Phe His Glu Lys Phe Lys Glu Ala Val Lys Glu Tyr Phe Ala Lys Phe
   1 5 10 15
   Trp Asp
<210> 411
   <211> 18
   <212> PRT
   <213> Artificial Sequence
<220>
   <223> synthetic construct; peptide mimetic
<400> 411
   Phe His Glu Lys Phe Lys Glu Ala Val Lys Asp Tyr Phe Ala Lys Phe
   1 5 10 15
   Trp Glu
<210> 412
   <211> 18
   <212> PRT
   <213> Artificial Sequence
<220>
   <223> synthetic construct; peptide mimetic
<400> 412
   His Phe Glu Lys Phe Lys Glu Ala Val Lys Asp Tyr Phe Ala Lys Phe
   1 5 10 15
   Trp Asp
<210> 413
   <211> 18
   <212> PRT
   <213> Artificial Sequence
<220>
   <223> synthetic construct; peptide mimetic
<400> 413
   His Phe Asp Lys Phe Lys Asp Ala Val Lys Glu Tyr Phe Ala Lys Phe
   1 5 10 15
   Trp Glu
<210> 414
   <211> 18
   <212> PRT
   <213> Artificial Sequence
<220>
   <223> synthetic construct; peptide mimetic
<400> 414
   His Phe Glu Lys Phe Lys Glu Ala Val Lys Glu Tyr Phe Ala Lys Phe
   1 5 10 15
   Trp Glu
<210> 415
   <211> 18
   <212> PRT
   <213> Artificial Sequence
<220>
   <223> synthetic construct; peptide mimetic
<400> 415
   His Phe Asp Lys Phe Lys Glu Ala Val Lys Asp Tyr Phe Ala Lys Phe
   1               5                   10                  15
   Trp Asp
<210> 416
   <211> 18
   <212> PRT
   <213> Artificial Sequence
<220>
   <223> synthetic construct; peptide mimetic
<400> 416
   His Phe Glu Lys Phe Lys Asp Ala Val Lys Asp Tyr Phe Ala Lys Phe
   1               5                   10                  15
   Trp Asp
<210> 417
   <211> 18
   <212> PRT
   <213> Artificial Sequence
<220>
   <223> synthetic construct; peptide mimetic
<400> 417
   His Phe Glu Lys Phe Lys Glu Ala Val Lys Glu Tyr Phe Ala Lys Phe
   1               5                   10                  15
   Trp Asp
<210> 418
   <211> 18
   <212> PRT
   <213> Artificial Sequence
<220>
   <223> synthetic construct; peptide mimetic
<400> 418
   His Phe Glu Lys Phe Lys Glu Ala Val Lys Asp Tyr Phe Ala Lys Phe
   1               5                   10                  15
   Trp Glu
<210> 419
   <211> 18
   <212> PRT
   <213> Artificial Sequence
<220>
   <223> synthetic construct; peptide mimetic
<400> 419
   Phe Phe Glu Lys His Lys Glu Ala Val Lys Asp Tyr Phe Ala Lys Phe
   1               5                   10                  15
   Trp Asp
<210> 420
   <211> 18
   <212> PRT
   <213> Artificial Sequence
<220>
   <223> synthetic construct; peptide mimetic
<400> 420
   Phe Phe Asp Lys His Lys Asp Ala Val Lys Glu Tyr Phe Ala Lys Phe
   1               5                   10                  15
   Trp Glu
<210> 421
   <211> 18
   <212> PRT
   <213> Artificial Sequence
<220>
   <223> synthetic construct; peptide mimetic
<400> 421
   Phe Phe Glu Lys His Lys Glu Ala Val Lys Glu Tyr Phe Ala Lys Phe
   1               5                   10                  15
   Trp Glu
<210> 422
   <211> 18
   <212> PRT
   <213> Artificial Sequence
<220>
   <223> synthetic construct; peptide mimetic
<400> 422
   Phe Phe Asp Lys His Lys Asp Ala Val Lys Asp Tyr Phe Ala Lys Phe
   1               5                   10                  15
   Trp Asp
<210> 423
   <211> 18
   <212> PRT
   <213> Artificial Sequence
<220>
   <223> synthetic construct; peptide mimetic
<400> 423
   Phe Phe Asp Lys His Lys Glu Ala Val Lys Asp Tyr Phe Ala Lys Phe
   1               5                   10                  15
   Trp Asp
<210> 424
   <211> 18
   <212> PRT
   <213> Artificial Sequence
<220>
   <223> synthetic construct; peptide mimetic
<400> 424
   Phe Phe Glu Lys His Lys Glu Ala Val Lys Glu Tyr Phe Ala Lys Phe
   1               5                   10                  15
   Trp Asp
<210> 425
   <211> 18
   <212> PRT
   <213> Artificial Sequence
<220>
   <223> synthetic construct; peptide mimetic
<400> 425
   Phe Phe Glu Lys His Lys Glu Ala Val Lys Asp Tyr Phe Ala Lys Phe
   1               5                   10                  15
   Trp Glu
<210> 426
   <211> 18
   <212> PRT
   <213> Artificial Sequence
<220>
   <223> synthetic construct; peptide mimetic
<400> 426
   Phe Val Glu Lys Phe Lys Glu Ala His Lys Asp Tyr Phe Ala Lys Phe
   1               5                   10                  15
   Trp Asp
<210> 427
   <211> 18
   <212> PRT
   <213> Artificial Sequence
<220>
   <223> synthetic construct; peptide mimetic
<400> 427
   Phe Val Asp Lys Phe Lys Asp Ala His Lys Glu Tyr Phe Ala Lys Phe
   1               5                   10                  15
   Trp Glu
<210> 428
   <211> 18
   <212> PRT
   <213> Artificial Sequence
<220>
   <223> synthetic construct; peptide mimetic
<400> 428
   Phe Val Glu Lys Phe Lys Glu Ala His Lys Glu Tyr Phe Ala Lys Phe
   1               5                   10                  15
   Trp Glu
<210> 429
   <211> 18
   <212> PRT
   <213> Artificial Sequence
<220>
   <223> synthetic construct; peptide mimetic
<400> 429
   Phe Val Asp Lys Phe Lys Asp Ala His Lys Asp Tyr Phe Ala Lys Phe
   1               5                   10                  15
   Trp Asp
<210> 430
   <211> 18
   <212> PRT
   <213> Artificial Sequence
<220>
   <223> synthetic construct; peptide mimetic
<400> 430
   Phe Val Asp Lys Phe Lys Glu Ala His Lys Asp Tyr Phe Ala Lys Phe
   1               5                   10                  15
   Trp Asp
<210> 431
   <211> 18
   <212> PRT
   <213> Artificial Sequence
<220>
   <223> synthetic construct; peptide mimetic
<400> 431
   Phe Val Glu Lys Phe Lys Asp Ala His Lys Asp Tyr Phe Ala Lys Phe
   1               5                   10                  15
   Trp Asp
<210> 432
   <211> 18
   <212> PRT
   <213> Artificial Sequence
<220>
   <223> synthetic construct; peptide mimetic
<400> 432
   Phe Val Glu Lys Phe Lys Glu Ala His Lys Glu Tyr Phe Ala Lys Phe
   1 5 10 15
   Trp Asp
<210> 433
   <211> 18
   <212> PRT
   <213> Artificial Sequence
<220>
   <223> synthetic construct; peptide mimetic
<400> 433
   Phe Val Glu Lys Phe Lys Glu Ala His Lys Asp Tyr Phe Ala Lys Phe
   1 5 10 15
   Trp Glu
<210> 434
   <211> 18
   <212> PRT
   <213> Artificial Sequence
<220>
   <223> synthetic construct; peptide mimetic
<400> 434
   Phe Ala Glu Lys Phe Lys Glu His Val Lys Asp Tyr Phe Ala Lys Phe
   1 5 10 15
   Trp Asp
<210> 435
   <211> 18
   <212> PRT
   <213> Artificial Sequence
<220>
   <223> synthetic construct; peptide mimetic
<400> 435
   Phe Ala Asp Lys Phe Lys Asp His Val Lys Glu Tyr Phe Ala Lys Phe
   1 5 10 15
   Trp Glu
<210> 436
   <211> 18
   <212> PRT
   <213> Artificial Sequence
<220>
   <223> synthetic construct; peptide mimetic
<400> 436
   Phe Ala Glu Lys Phe Lys Glu His Val Lys Glu Tyr Phe Ala Lys Phe
   1 5 10 15
   Trp Glu
<210> 437
   <211> 18
   <212> PRT
   <213> Artificial Sequence
<220>
   <223> synthetic construct; peptide mimetic
<400> 437
   Phe Ala Asp Lys Phe Lys Asp His Val Lys Asp Tyr Phe Ala Lys Phe
   1 5 10 15
   Trp Asp
<210> 438
   <211> 18
   <212> PRT
   <213> Artificial Sequence
<220>
   <223> synthetic construct; peptide mimetic
<400> 438
   Phe Ala Asp Lys Phe Lys Glu His Val Lys Asp Tyr Phe Ala Lys Phe
   1 5 10 15
   Trp Asp
<210> 439
   <211> 18
   <212> PRT
   <213> Artificial Sequence
<220>
   <223> synthetic construct; peptide mimetic
<400> 439
   Phe Ala Glu Lys Phe Lys Asp His Val Lys Asp Tyr Phe Ala Lys Phe
   1 5 10 15
   Trp Asp
<210> 440
   <211> 18
   <212> PRT
   <213> Artificial Sequence
<220>
   <223> synthetic construct; peptide mimetic
<400> 440
   Phe Ala Glu Lys Phe Lys Glu His Val Lys Glu Tyr Phe Ala Lys Phe
   1 5 10 15
   Trp Asp
<210> 441
   <211> 18
   <212> PRT
   <213> Artificial Sequence
<220>
   <223> synthetic construct; peptide mimetic
<400> 441
   Phe Ala Glu Lys Phe Lys Glu His Val Lys Asp Tyr Phe Ala Lys Phe
   1 5 10 15
   Trp Glu
<210> 442
   <211> 18
   <212> PRT
   <213> Artificial Sequence
<220>
   <223> synthetic construct; peptide mimetic
<400> 442
   Phe Ala Glu Lys Phe Lys Glu Phe Val Lys Asp Tyr His Ala Lys Phe
   1 5 10 15
   Trp Asp
<210> 443
   <211> 18
   <212> PRT
   <213> Artificial Sequence
<220>
   <223> synthetic construct; peptide mimetic
<400> 443
   Phe Ala Asp Lys Phe Lys Asp Phe Val Lys Glu Tyr His Ala Lys Phe
   1 5 10 15
   Trp Glu
<210> 444
   <211> 18
   <212> PRT
   <213> Artificial Sequence
<220>
   <223> synthetic construct; peptide mimetic
<400> 444
   Phe Ala Asp Lys Phe Lys Glu Phe Val Lys Asp Tyr His Ala Lys Phe
   1 5 10 15
   Trp Asp
<210> 445
   <211> 18
   <212> PRT
   <213> Artificial Sequence
<220>
   <223> synthetic construct; peptide mimetic
<400> 445
   Phe Ala Glu Lys Phe Lys Asp Phe Val Lys Asp Tyr His Ala Lys Phe
   1 5 10 15
   Trp Asp
<210> 446
   <211> 18
   <212> PRT
   <213> Artificial Sequence
<220>
   <223> synthetic construct; peptide mimetic
<400> 446
   Phe Ala Asp Lys Phe Lys Asp Phe Val Lys Asp Tyr His Ala Lys Phe
   1 5 10 15
   Trp Asp
<210> 447
   <211> 18
   <212> PRT
   <213> Artificial Sequence
<220>
   <223> synthetic construct; peptide mimetic
<400> 447
   Phe Ala Glu Lys Phe Lys Glu Phe Val Lys Glu Tyr His Ala Lys Phe
   1 5 10 15
   Trp Glu
<210> 448
   <211> 18
   <212> PRT
   <213> Artificial Sequence
<220>
   <223> synthetic construct; peptide mimetic
<400> 448
   Phe Ala Glu Lys Phe Lys Glu Phe Val Lys Glu Tyr His Ala Lys Phe
   1 5 10 15
   Trp Asp
<210> 449
   <211> 18
   <212> PRT
   <213> Artificial Sequence
<220>
   <223> synthetic construct; peptide mimetic
<400> 449
   Phe Ala Glu Lys Phe Lys Glu Phe Val Lys Asp Tyr His Ala Lys Phe
   1 5 10 15
   Trp Glu
<210> 450
   <211> 18
   <212> PRT
   <213> Artificial Sequence
<220>
   <223> synthetic construct; peptide mimetic
<400> 450
   Phe Ala Glu Lys Phe Lys Glu Phe Val Lys Asp Tyr Phe Ala Lys His
   1               5                   10                  15
   Trp Asp
<210> 451
   <211> 18
   <212> PRT
   <213> Artificial Sequence
<220>
   <223> synthetic construct; peptide mimetic
<400> 451
   Phe Ala Asp Lys Phe Lys Asp Phe Val Lys Glu Tyr Phe Ala Lys His
   1               5                   10                  15
   Trp Glu
<210> 452
   <211> 18
   <212> PRT
   <213> Artificial Sequence
<220>
   <223> synthetic construct; peptide mimetic
<400> 452
   Phe Ala Glu Lys Phe Lys Glu Phe Val Lys Glu Tyr Phe Ala Lys His
   1               5                   10                  15
   Trp Glu
<210> 453
   <211> 18
   <212> PRT
   <213> Artificial Sequence
<220>
   <223> synthetic construct; peptide mimetic
<400> 453
   Phe Ala Asp Lys Phe Lys Asp Phe Val Lys Asp Tyr Phe Ala Lys His
   1 5 10 15
   Trp Asp
<210> 454
   <211> 18
   <212> PRT
   <213> Artificial Sequence
<220>
   <223> synthetic construct; peptide mimetic
<400> 454
   Phe Ala Asp Lys Phe Lys Glu Phe Val Lys Asp Tyr Phe Ala Lys His
   1 5 10 15
   Trp Asp
<210> 455
   <211> 18
   <212> PRT
   <213> Artificial Sequence
<220>
   <223> synthetic construct; peptide mimetic
<400> 455
   Phe Ala Glu Lys Phe Lys Asp Phe Val Lys Asp Tyr Phe Ala Lys His
   1 5 10 15
   Trp Asp
<210> 456
   <211> 18
   <212> PRT
   <213> Artificial Sequence
<220>
   <223> synthetic construct; peptide mimetic
<400> 456
   Phe Ala Glu Lys Phe Lys Glu Phe Val Lys Glu Tyr Phe Ala Lys His
   1 5 10 15
   Trp Asp
<210> 457
   <211> 18
   <212> PRT
   <213> Artificial Sequence
<220>
   <223> synthetic construct; peptide mimetic
<400> 457
   Phe Ala Glu Lys Phe Lys Glu Phe Val Lys Asp Tyr Phe Ala Lys His
   1               5                   10                  15
   Trp Glu
<210> 458
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 458
   Asp Trp Phe Lys Ala Phe Tyr Asp Lys Val Ala Glu Lys Phe Lys Glu
   1               5                   10                  15
   Ala Phe
<210> 459
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 459
   Glu Trp Phe Lys Ala Phe Tyr Glu Lys Val Ala Asp Lys Phe Lys Asp
   1 5 10 15
   Ala Phe
<210> 460
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 460
   Glu Trp Phe Lys Ala Phe Tyr Glu Lys Val Ala Glu Lys Phe Lys Glu
   1               5                   10                  15
   Ala Phe
<210> 461
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 461
   Asp Trp Phe Lys Ala Phe Tyr Asp Lys Val Ala Asp Lys Phe Lys Asp
   1               5                   10                  15
   Ala Phe
<210> 462
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 462
   Glu Trp Phe Lys Ala Phe Tyr Asp Lys Val Ala Glu Lys Phe Lys Glu
   1 5 10 15
   Ala Phe
<210> 463
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 463
   Asp Trp Phe Lys Ala Phe Tyr Glu Lys Val Ala Glu Lys Phe Lys Glu
   1 5 10 15
   Ala Phe
<210> 464
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 464
   Asp Trp Phe Lys Ala Phe Tyr Asp Lys Val Ala Asp Lys Phe Lys Glu
   1               5                   10                  15
   Ala Phe
<210> 465
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 465
   Asp Trp Phe Lys Ala Phe Tyr Asp Lys Val Ala Glu Lys Phe Lys Asp
   1               5                   10                  15
   Ala Phe
<210> 466
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 466
   Asp Trp Phe Lys Ala Phe Tyr Asp Lys Val Ala Glu Lys Phe Lys Glu
   1               5                   10                  15
   Ala Phe
<210> 467
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 467
   Asp Trp Phe Lys Ala Phe Tyr Asp Lys Val Ala Glu Lys Phe Lys Glu
   1               5                   10                  15
   Ala Phe
<210> 468
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 468
   Asp Trp Phe Lys Ala Phe Tyr Asp Lys Val Ala Glu Lys Phe Lys Glu
   1 5 10 15
   Ala Phe
<210> 469
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 469
   Asp Trp Phe Lys Ala Phe Tyr Asp Lys Val Ala Glu Lys Phe Lys Glu
   1 5 10 15
   Ala Phe
<210> 470
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 470
   Asp Trp Phe Lys Ala Phe Tyr Asp Lys Val Ala Glu Lys Phe Lys Glu
   1               5                   10                  15
   Ala Phe
<210> 471
   <211> 18
   <212> PRT
   <213> Artificial Sequence
<220>
   <223> synthetic construct, Nph peptide mimetic
<220>
   <221> misc
   <222> (3)..(3)
   <223> The phenyl group comprises a para nitro moiety
<220>
   <221> misc
   <222> (6)..(6)
   <223> The phenyl group comprises a para nitro moiety
<220>
   <221> misc
   <222> (14)..(14)
   <223> The phenyl group comprises a para nitro moiety
<220>
   <221> misc
   <222> (18)..(18)
   <223> The phenyl group comprises a para nitro moiety
<400> 471
   Asp Trp Phe Lys Ala Phe Tyr Asp Lys Val Ala Glu Lys Phe Lys Glu
   1               5                   10                  15
   Ala Phe
<210> 472
   <211> 18
   <212> PRT
   <213> Artificial Sequence
<220>
   <223> synthetic construct, Nph peptide mimetic
<220>
   <221> misc
   <222> (1)..(1)
   <223> The phenyl group comprises a para nitro moiety
<220>
   <221> misc
   <222> (5)..(5)
   <223> The phenyl group comprises a para nitro moiety
<220>
   <221> misc
   <222> (13)..(13)
   <223> The phenyl group comprises a para nitro moiety
<220>
   <221> misc
   <222> (16)..(16)
   <223> The phenyl group comprises a para nitro moiety
<400> 472
   Phe Ala Glu Lys Phe Lys Glu Ala Val Lys Asp Tyr Phe Ala Lys Phe
   1               5                   10                  15
   Trp Asp
<210> 473
   <211> 18
   <212> PRT
   <213> Artificial Sequence
<220>
   <223> synthetic construct, Nph peptide mimetic
<220>
   <221> misc
   <222> (1)..(1)
   <223> The phenyl group comprises a para nitro moiety
<220>
   <221> misc
   <222> (5)..(5)
   <223> The phenyl group comprises a para nitro moiety
<400> 473
   Phe Ala Glu Lys Phe Lys Glu Ala Val Lys Asp Tyr Phe Ala Lys Phe
   1 5 10 15
   Trp Asp
<210> 474
   <211> 18
   <212> PRT
   <213> Artificial Sequence
<220>
   <223> synthetic construct, Nph peptide mimetic
<220>
   <221> misc
   <222> (13)..(13)
   <223> The phenyl group comprises a para nitro moiety
<220>
   <221> misc
   <222> (16)..(16)
   <223> The phenyl group comprises a para nitro moiety
<400> 474
   Phe Ala Glu Lys Phe Lys Glu Ala Val Lys Asp Tyr Phe Ala Lys Phe
   1 5 10 15
   Trp Asp
<210> 475
   <211> 18
   <212> PRT
   <213> Artificial Sequence
<220>
   <223> synthetic construct, Nph peptide mimetic
<220>
   <221> misc
   <222> (1)..(1)
   <223> The phenyl group comprises a para nitro moiety
<400> 475
   Phe Ala Glu Lys Phe Lys Glu Ala Val Lys Asp Tyr Phe Ala Lys Phe
   1 5 10 15
   Trp Asp
<210> 476
   <211> 18
   <212> PRT
   <213> Artificial Sequence
<220>
   <223> synthetic construct, Nph peptide mimetic
<220>
   <221> misc
   <222> (5)..(5)
   <223> The phenyl group comprises a para nitro moiety
<400> 476
   Phe Ala Glu Lys Phe Lys Glu Ala Val Lys Asp Tyr Phe Ala Lys Phe
   1               5                   10                  15
   Trp Asp
<210> 477
   <211> 18
   <212> PRT
   <213> Artificial Sequence
<220>
   <223> synthetic construct, Nph peptide mimetic
<220>
   <221> misc
   <222> (13)..(13)
   <223> The phenyl group comprises a para nitro moiety
<400> 477
   Phe Ala Glu Lys Phe Lys Glu Ala Val Lys Asp Tyr Phe Ala Lys Phe
   1               5                   10                  15
   Trp Asp
<210> 478
   <211> 18
   <212> PRT
   <213> Artificial Sequence
<220>
   <223> synthetic construct, Nph peptide mimetic
<220>
   <221> misc
   <222> (16)..(16)
   <223> The phenyl group comprises a para nitro moiety
<400> 478
   Phe Ala Glu Lys Glu Lys Glu Ala Val Lys Asp Tyr Phe Ala Lys Phe
   1               5                   10                  15
   Trp Asp
<210> 479
   <211> 18
   <212> PRT
   <213> Artificial Sequence
<220>
   <223> synthetic construct; peptide mimetic
<400> 479
   Phe Ala Glu Lys Phe Lys Glu Ala Val Lys Glu Tyr Phe Ala Lys Phe
   1               5                   10                  15
   Trp Glu
<210> 480
   <211> 18
   <212> PRT
   <213> Artificial Sequence
<220>
   <223> synthetic construct; peptide mimetic
<400> 480
   Phe Ala Asp Lys Phe Lys Asp Ala Val Lys Asp Tyr Phe Ala Lys Phe
   1               5                   10                  15
   Trp Asp
<210> 481
   <211> 18
   <212> PRT
   <213> Artificial Sequence
<220>
   <223> synthetic construct; peptide mimetic
<400> 481
   Phe Ala Glu Arg Phe Arg Glu Ala Val Lys Asp Tyr Phe Ala Lys Phe
   1               5                   10                  15
   Trp Asp
<210> 482
   <211> 18
   <212> PRT
   <213> Artificial Sequence
<220>
   <223> synthetic construct; peptide mimetic
<400> 482
   Phe Ala Glu Lys Phe Arg Glu Ala Val Lys Asp Tyr Phe Ala Lys Phe
   1 5 10 15
   Trp Asp
<210> 483
   <211> 18
   <212> PRT
   <213> Artificial Sequence
<220>
   <223> synthetic construct; peptide mimetic
<400> 483
   Phe Ala Glu Lys Phe Lys Glu Ala Val Arg Asp Tyr Phe Ala Lys Phe
   1 5 10 15
   Trp Asp
<210> 484
   <211> 18
   <212> PRT
   <213> Artificial Sequence
<220>
   <223> synthetic construct; peptide mimetic
<400> 484
   Phe Ala Glu Lys Phe Lys Glu Ala Val Lys Asp Tyr Phe Ala Arg Phe
   1 5 10 15
   Trp Asp
<210> 485
   <211> 18
   <212> PRT
   <213> Artificial Sequence
<220>
   <223> synthetic construct; peptide mimetic
<400> 485
   Phe Ala Glu Lys Phe Arg Glu Ala Val Lys Asp Tyr Glu Ala Lys Phe
   1 5 10 15
   Trp Asp
<210> 486
   <211> 18
   <212> PRT
   <213> Artificial Sequence
<220>
   <223> synthetic construct; peptide mimetic
<400> 486
   Leu Phe Glu Lys Phe Ala Glu Ala Phe Lys Asp Tyr Val Ala Lys Trp
   1 5 10 15
   Lys Asp
<210> 487
   <211> 18
   <212> PRT
   <213> Artificial Sequence
<220>
   <223> synthetic construct; peptide mimetic
<400> 487
   Leu Phe Glu Arg Phe Ala Glu Ala Phe Lys Asp Tyr Val Ala Lys Trp
   1 5 10 15
   Lys Asp
<210> 488
   <211> 18
   <212> PRT
   <213> Artificial Sequence
<220>
   <223> synthetic construct; peptide mimetic
<400> 488
   Leu Phe Glu Lys Phe Ala Glu Ala Phe Arg Asp Tyr Val Ala Lys Trp
   1 5 10 15
   Lys Asp
<210> 489
   <211> 18
   <212> PRT
   <213> Artificial Sequence
<220>
   <223> synthetic construct; peptide mimetic
<400> 489
   Leu Phe Glu Lys Phe Ala Glu Ala Phe Lys Asp Tyr Val Ala Arg Trp
   1 5 10 15
   Lys Asp
<210> 490
   <211> 18
   <212> PRT
   <213> Artificial Sequence
<220>
   <223> synthetic construct; peptide mimetic
<400> 490
   Leu Phe Glu Lys Phe Ala Glu Ala Phe Lys Asp Tyr Val Ala Lys Trp
   1 5 10 15
   Arg Asp
<210> 491
   <211> 18
   <212> PRT
   <213> Artificial Sequence
<220>
   <223> synthetic construct; peptide mimetic
<400> 491
   Leu Phe Glu Lys Phe Ala Glu Ala Phe Lys Glu Tyr Val Ala Lys Trp
   1 5 10 15
   Lys Glu
<210> 492
   <211> 18
   <212> PRT
   <213> Artificial Sequence
<220>
   <223> synthetic construct; peptide mimetic
<400> 492
   Leu Phe Asp Lys Phe Ala Asp Ala Phe Lys Asp Tyr Val Ala Lys Trp
   1 5 10 15
   Lys Asp
<210> 493
   <211> 18
   <212> PRT
   <213> Artificial Sequence
<220>
   <223> synthetic construct; peptide mimetic
<400> 493
   Leu Phe Asp Lys Phe Ala Glu Ala Phe Lys Asp Tyr Val Ala Lys Trp
   1               5                   10                  15
   Lys Asp
<210> 494
   <211> 18
   <212> PRT
   <213> Artificial Sequence
<220>
   <223> synthetic construct; peptide mimetic
<400> 494
   Leu Phe Glu Lys Phe Ala Asp Ala Phe Lys Asp Tyr Val Ala Lys Trp
   1               5                   10                  15
   Lys Asp
<210> 495
   <211> 18
   <212> PRT
   <213> Artificial Sequence
<220>
   <223> synthetic construct; peptide mimetic
<400> 495
   Leu Phe Glu Lys Phe Ala Glu Ala Phe Lys Glu Tyr Val Ala Lys Trp
   1               5                   10                  15
   Lys Asp
<210> 496
   <211> 18
   <212> PRT
   <213> Artificial Sequence
<220>
   <223> synthetic construct; peptide mimetic
<400> 496
   Leu Phe Glu Lys Phe Ala Glu Ala Phe Lys Asp Tyr Val Ala Lys Trp
   1               5                   10                  15
   Lys Glu
<210> 497
   <211> 18
   <212> PRT
   <213> Artificial Sequence
<220>
   <223> synthetic construct; peptide mimetic
<400> 497
   Phe Ala Glu Lys Ala Trp Glu Phe Val Lys Asp Tyr Phe Ala Lys Leu
   1               5                   10                  15
   Lys Asp
<210> 498
   <211> 18
   <212> PRT
   <213> Artificial Sequence
<220>
   <223> synthetic construct; peptide mimetic
<400> 498
   Phe Ala Glu Arg Ala Trp Glu Phe Val Lys Asp Tyr Phe Ala Lys Leu
   1               5                   10                  15
   Lys Asp
<210> 499
   <211> 18
   <212> PRT
   <213> Artificial Sequence
<220>
   <223> synthetic construct; peptide mimetic
<400> 499
   Phe Ala Glu Lys Ala Trp Glu Phe Val Lys Asp Tyr Phe Ala Lys Leu
   1               5                   10                  15
   Lys Asp
<210> 500
   <211> 18
   <212> PRT
   <213> Artificial Sequence
<220>
   <223> synthetic construct; peptide mimetic
<400> 500
   Phe Ala Glu Lys Ala Trp Glu Phe Val Lys Asp Tyr Phe Ala Lys Leu
   1               5                   10                  15
   Lys Asp
<210> 501
   <211> 18
   <212> PRT
   <213> Artificial Sequence
<220>
   <223> synthetic construct; peptide mimetic
<400> 501
   Phe Ala Glu Lys Ala Trp Glu Phe Val Lys Asp Tyr Phe Ala Lys Leu
   1               5                   10                  15
   Arg Asp
<210> 502
   <211> 18
   <212> PRT
   <213> Artificial Sequence
<220>
   <223> synthetic construct; peptide mimetic
<400> 502
   Phe Ala Glu Lys Ala Trp Glu Phe Val Lys Glu Tyr Phe Ala Lys Leu
   1               5                   10                  15
   Lys Glu
<210> 503
   <211> 18
   <212> PRT
   <213> Artificial Sequence
<220>
   <223> synthetic construct; peptide mimetic
<400> 503
   Phe Ala Asp Lys Ala Trp Asp Phe Val Lys Asp Tyr Phe Ala Lys Leu
   1               5                   10                  15
   Lys Asp
<210> 504
   <211> 18
   <212> PRT
   <213> Artificial Sequence
<220>
   <223> synthetic construct; peptide mimetic
<400> 504
   Phe Ala Asp Lys Ala Trp Glu Phe Val Lys Asp Tyr Phe Ala Lys Leu
   1               5                   10                  15
   Lys Asp
<210> 505
   <211> 18
   <212> PRT
   <213> Artificial Sequence
<220>
   <223> synthetic construct; peptide mimetic
<400> 505
   Phe Ala Glu Lys Ala Trp Asp Phe Val Lys Asp Tyr Phe Ala Lys Leu
   1               5                   10                  15
   Lys Asp
<210> 506
   <211> 18
   <212> PRT
   <213> Artificial Sequence
<220>
   <223> synthetic construct; peptide mimetic
<400> 506
   Phe Ala Glu Lys Ala Trp Glu Phe Val Lys Glu Tyr Phe Ala Lys Leu
   1               5                   10                  15
   Lys Asp
<210> 507
   <211> 18
   <212> PRT
   <213> Artificial Sequence
<220>
   <223> synthetic construct; peptide mimetic
<400> 507
   Phe Ala Glu Lys Ala Trp Glu Phe Val Lys Asp Tyr Phe Ala Lys Leu
   1               5                   10                  15
   Lys Glu
<210> 508
   <211> 18
   <212> PRT
   <213> Artificial Sequence
<220>
   <223> synthetic construct; peptide mimetic
<400> 508
   Phe Phe Glu Lys Phe Lys Glu Phe Val Lys Asp Tyr Phe Ala Lys Leu
   1 5 10 15
   Trp Asp
<210> 509
   <211> 18
   <212> PRT
   <213> Artificial Sequence
<220>
   <223> synthetic construct; peptide mimetic
<400> 509
   Phe Phe Glu Lys Phe Lys Glu Phe Val Lys Glu Tyr Phe Ala Lys Leu
   1 5 10 15
   Trp Glu
<210> 510
   <211> 18
   <212> PRT
   <213> Artificial Sequence
<220>
   <223> synthetic construct; peptide mimetic
<400> 510
   Phe Phe Asp Lys Phe Lys Asp Phe Val Lys Asp Tyr Phe Ala Lys Leu
   1 5 10 15
   Trp Asp
<210> 511
   <211> 18
   <212> PRT
   <213> Artificial Sequence
<220>
   <223> synthetic construct; peptide mimetic
<400> 511
   Phe Phe Glu Arg Phe Lys Glu Phe Val Lys Asp Tyr Phe Ala Lys Leu
   1 5 10 15
   Trp Asp
<210> 512
   <211> 18
   <212> PRT
   <213> Artificial Sequence
<220>
   <223> synthetic construct; peptide mimetic
<400> 512
   Phe Phe Glu Lys Phe Arg Glu Phe Val Lys Asp Tyr Phe Ala Lys Leu
   1               5                   10                  15
   Trp Asp
<210> 513
   <211> 18
   <212> PRT
   <213> Artificial Sequence
<220>
   <223> synthetic construct; peptide mimetic
<400> 513
   Phe Phe Glu Lys Phe Lys Glu Phe Val Arg Asp Tyr Phe Ala Lys Leu
   1               5                   10                  15
   Trp Asp
<210> 514
   <211> 18
   <212> PRT
   <213> Artificial Sequence
<220>
   <223> synthetic construct; peptide mimetic
<400> 514
   Phe Phe Glu Lys Phe Lys Glu Phe Val Lys Asp Tyr Phe Ala Arg Leu
   1               5                   10                  15
   Trp Asp
<210> 515
   <211> 18
   <212> PRT
   <213> Artificial Sequence
<220>
   <223> synthetic construct; peptide mimetic
<400> 515
   Phe Phe Asp Lys Phe Lys Glu Phe Val Lys Asp Tyr Phe Ala Lys Leu
   1 5 10 15
   Trp Asp
<210> 516
   <211> 18
   <212> PRT
   <213> Artificial Sequence
<220>
   <223> synthetic construct; peptide mimetic
<400> 516
   Phe Phe Glu Lys Phe Lys Asp Phe Val Lys Asp Tyr Phe Ala Lys Leu
   1 5 10 15
   Trp Asp
<210> 517
   <211> 18
   <212> PRT
   <213> Artificial Sequence
<220>
   <223> synthetic construct; peptide mimetic
<400> 517
   Phe Phe Glu Lys Phe Lys Glu Phe Val Lys Glu Tyr Phe Ala Lys Leu
   1 5 10 15
   Trp Asp
<210> 518
   <211> 18
   <212> PRT
   <213> Artificial Sequence
<220>
   <223> synthetic construct; peptide mimetic
<400> 518
   Phe Phe Glu Lys Phe Lys Glu Phe Val Lys Asp Tyr Phe Ala Lys Leu
   1 5 10 15
   Trp Glu
<210> 519
   <211> 18
   <212> PRT
   <213> Artificial Sequence
<220>
   <223> synthetic construct; peptide mimetic
<400> 519
   Phe Leu Glu Lys Phe Lys Glu Phe Val Lys Asp Tyr Phe Ala Lys Phe
   1               5                   10                  15
   Trp Asp
<210> 520
   <211> 18
   <212> PRT
   <213> Artificial Sequence
<220>
   <223> synthetic construct; peptide mimetic
<400> 520
   Phe Leu Glu Lys Phe Lys Glu Phe Val Lys Glu Tyr Phe Ala Lys Phe
   1               5                   10                  15
   Trp Glu
<210> 521
   <211> 18
   <212> PRT
   <213> Artificial Sequence
<220>
   <223> synthetic construct; peptide mimetic
<400> 521
   Phe Leu Asp Lys Phe Lys Glu Phe Val Lys Asp Tyr Phe Ala Lys Phe
   1               5                   10                  15
   Trp Asp
<210> 522
   <211> 18
   <212> PRT
   <213> Artificial Sequence
<220>
   <223> synthetic construct; peptide mimetic
<400> 522
   Phe Leu Asp Lys Phe Lys Glu Phe Val Lys Asp Tyr Phe Ala Lys Phe
   1               5                   10                  15
   Trp Asp
<210> 523
   <211> 18
   <212> PRT
   <213> Artificial Sequence
<220>
   <223> synthetic construct; peptide mimetic
<400> 523
   Phe Leu Glu Lys Phe Lys Asp Phe Val Lys Asp Tyr Phe Ala Lys Phe
   1               5                   10                  15
   Trp Asp
<210> 524
   <211> 18
   <212> PRT
   <213> Artificial Sequence
<220>
   <223> synthetic construct; peptide mimetic
<400> 524
   Phe Leu Glu Lys Phe Lys Glu Phe Val Lys Glu Tyr Phe Ala Lys Phe
   1               5                   10                  15
   Trp Asp
<210> 525
   <211> 18
   <212> PRT
   <213> Artificial Sequence
<220>
   <223> synthetic construct; peptide mimetic
<400> 525
   Phe Leu Glu Lys Phe Lys Glu Phe Val Lys Asp Tyr Phe Ala Lys Phe
   1               5                   10                  15
   Trp Glu
<210> 526
   <211> 18
   <212> PRT
   <213> Artificial Sequence
<220>
   <223> synthetic construct; peptide mimetic
<400> 526
   Phe Leu Glu Arg Phe Lys Glu Phe Val Lys Asp Tyr Phe Ala Lys Phe
   1 5 10 15
   Trp Asp
<210> 527
   <211> 18
   <212> PRT
   <213> Artificial Sequence
<220>
   <223> synthetic construct; peptide mimetic
<400> 527
   Phe Leu Glu Lys Phe Arg Glu Phe Val Lys Asp Tyr Phe Ala Lys Phe
   1 5 10 15
   Trp Asp
<210> 528
   <211> 18
   <212> PRT
   <213> Artificial Sequence
<220>
   <223> synthetic construct; peptide mimetic
<400> 528
   Phe Leu Glu Lys Phe Lys Glu Phe Val Arg Asp Tyr Phe Ala Lys Phe
   1 5 10 15
   Trp Asp
<210> 529
   <211> 18
   <212> PRT
   <213> Artificial Sequence
<220>
   <223> synthetic construct; peptide mimetic
<400> 529
   Phe Leu Glu Lys Phe Lys Glu Phe Val Lys Asp Tyr Phe Ala Arg Phe
   1 5 10 15
   Trp Asp
<210> 530
   <211> 18
   <212> PRT
   <213> Artificial Sequence
<220>
   <223> synthetic construct; peptide mimetic
<400> 530
   Phe Phe Glu Lys Phe Lys Glu Phe Phe Lys Asp Tyr Phe Ala Lys Leu
   1 5 10 15
   Trp Asp
<210> 531
   <211> 18
   <212> PRT
   <213> Artificial Sequence
<220>
   <223> synthetic construct; peptide mimetic
<400> 531
   Phe Phe Glu Lys Phe Lys Glu Phe Phe Lys Glu Tyr Phe Ala Lys Leu
   1 5 10 15
   Trp Glu
<210> 532
   <211> 18
   <212> PRT
   <213> Artificial Sequence
<220>
   <223> synthetic construct; peptide mimetic
<400> 532
   Phe Phe Asp Lys Phe Lys Asp Phe Phe Lys Asp Tyr Phe Ala Lys Leu
   1 5 10 15
   Trp Asp
<210> 533
   <211> 18
   <212> PRT
   <213> Artificial Sequence
<220>
   <223> synthetic construct; peptide mimetic
<400> 533
   Phe Phe Glu Arg Phe Lys Glu Phe Phe Lys Asp Tyr Phe Ala Lys Leu
   1 5 10 15
   Trp Asp
<210> 534
   <211> 18
   <212> PRT
   <213> Artificial Sequence
<220>
   <223> synthetic construct; peptide mimetic
<400> 534
   Phe Phe Glu Lys Phe Arg Glu Phe Phe Lys Asp Tyr Phe Ala Lys Leu
   1               5                   10                  15
   Trp Asp
<210> 535
   <211> 18
   <212> PRT
   <213> Artificial Sequence
<220>
   <223> synthetic construct; peptide mimetic
<400> 535
   Phe Phe Glu Lys Phe Lys Glu Phe Phe Arg Asp Tyr Phe Ala Lys Leu
   1               5                   10                  15
   Trp Asp
<210> 536
   <211> 18
   <212> PRT
   <213> Artificial Sequence
<220>
   <223> synthetic construct; peptide mimetic
<400> 536
   Phe Phe Glu Arg Phe Lys Glu Phe Phe Lys Asp Tyr Phe Ala Arg Leu
   1               5                   10                  15
   Trp Asp
<210> 537
   <211> 18
   <212> PRT
   <213> Artificial Sequence
<220>
   <223> synthetic construct; peptide mimetic
<400> 537
   Phe Phe Asp Lys Phe Lys Glu Phe Phe Lys Asp Tyr Phe Ala Lys Leu
   1 5 10 15
   Trp Asp
<210> 538
   <211> 18
   <212> PRT
   <213> Artificial Sequence
<220>
   <223> synthetic construct; peptide mimetic
<400> 538
   Phe Phe Glu Lys Glu Lys Asp Phe Phe Lys Asp Tyr Phe Ala Lys Leu
   1 5 10 15
   Trp Asp
<210> 539
   <211> 18
   <212> PRT
   <213> Artificial Sequence
<220>
   <223> synthetic construct; peptide mimetic
<400> 539
   Phe Phe Glu Lys Phe Lys Glu Phe Phe Lys Glu Tyr Phe Ala Lys Leu
   1 5 10 15
   Trp Asp
<210> 540
   <211> 18
   <212> PRT
   <213> Artificial Sequence
<220>
   <223> synthetic construct; peptide mimetic
<400> 540
   Phe Phe Glu Lys Phe Lys Glu Phe Phe Lys Asp Tyr Phe Ala Lys Leu
   1 5 10 15
   Trp Glu
<210> 541
   <211> 18
   <212> PRT
   <213> Artificial Sequence
<220>
   <223> synthetic construct; peptide mimetic
<400> 541
   Phe Ala Glu Lys Phe Lys Glu Ala Val Lys Asp Tyr Phe Ala Lys Phe
   1 5 10 15
   Trp Asp
<210> 542
   <211> 18
   <212> PRT
   <213> Artificial Sequence
<220>
   <223> synthetic construct; peptide mimetic
<400> 542
   Asp Lys Trp Lys Ala Val Tyr Asp Lys Phe Ala Glu Ala Phe Lys Glu
   1 5 10 15
   Phe Phe
<210> 543
   <211> 18
   <212> PRT
   <213> Artificial Sequence
<220>
   <223> synthetic construct; peptide mimetic
<400> 543
   Glu Lys Trp Lys Ala Val Tyr Glu Lys Phe Ala Glu Ala Phe Lys Glu
   1 5 10 15
   Phe Phe
<210> 544
   <211> 18
   <212> PRT
   <213> Artificial Sequence
<220>
   <223> synthetic construct; peptide mimetic
<400> 544
   Asp Lys Trp Lys Ala Val Tyr Asp Lys Phe Ala Asp Ala Phe Lys Asp
   1 5 10 15
   Phe Phe
<210> 545
   <211> 18
   <212> PRT
   <213> Artificial Sequence
<220>
   <223> synthetic construct; peptide mimetic
<400> 545
   Asp Arg Trp Lys Ala Val Tyr Asp Lys Phe Ala Glu Ala Phe Lys Glu
   1 5 10 15
   Phe Phe
<210> 546
   <211> 18
   <212> PRT
   <213> Artificial Sequence
<220>
   <223> synthetic construct; peptide mimetic
<400> 546
   Asp Lys Trp Arg Ala Val Tyr Asp Lys Phe Ala Glu Ala Phe Lys Glu
   1 5 10 15
   Phe Phe
<210> 547
   <211> 18
   <212> PRT
   <213> Artificial Sequence
<220>
   <223> synthetic construct; peptide mimetic
<400> 547
   Asp Lys Trp Lys Ala Val Tyr Asp Arg Phe Ala Glu Ala Phe Lys Glu
   1 5 10 15
   Phe Phe
<210> 548
   <211> 18
   <212> PRT
   <213> Artificial Sequence
<220>
   <223> synthetic construct; peptide mimetic
<400> 548
   Asp Lys Trp Lys Ala Val Tyr Asp Lys Phe Ala Glu Ala Phe Arg Glu
   1 5 10 15
   Phe Phe
<210> 549
   <211> 18
   <212> PRT
   <213> Artificial Sequence
<220>
   <223> synthetic construct; peptide mimetic
<400> 549
   Phe Phe Glu Lys Phe Ala Glu Ala Phe Lys Asp Tyr Val Ala Lys Trp
   1 5 10 15
   Lys Asp
<210> 550
   <211> 18
   <212> PRT
   <213> Artificial Sequence
<220>
   <223> synthetic construct; peptide mimetic
<400> 550
   Phe Phe Glu Lys Phe Ala Glu Ala Phe Lys Glu Tyr Val Ala Lys Trp
   1 5 10 15
   Lys Glu
<210> 551
   <211> 18
   <212> PRT
   <213> Artificial Sequence
<220>
   <223> synthetic construct; peptide mimetic
<400> 551
   Phe Phe Asp Lys Phe Ala Asp Ala Phe Lys Asp Tyr Val Ala Lys Trp
   1 5 10 15
   Lys Asp
<210> 552
   <211> 18
   <212> PRT
   <213> Artificial Sequence
<220>
   <223> synthetic construct; peptide mimetic
<400> 552
   Phe Phe Glu Arg Phe Ala Glu Ala Phe Lys Asp Tyr Val Ala Lys Trp
   1               5                   10                  15
   Lys Asp
<210> 553
   <211> 18
   <212> PRT
   <213> Artificial Sequence
<220>
   <223> synthetic construct; peptide mimetic
<400> 553
   Glu Phe Glu Arg Phe Ala Glu Ala Phe Arg Asp Tyr Val Ala Lys Trp
   1               5                   10                  15
   Lys Asp
<210> 554
   <211> 18
   <212> PRT
   <213> Artificial Sequence
<220>
   <223> synthetic construct; peptide mimetic
<400> 554
   Phe Phe Glu Lys Phe Ala Glu Ala Phe Lys Asp Tyr Val Ala Arg Trp
   1               5                   10                  15
   Lys Asp
<210> 555
   <211> 18
   <212> PRT
   <213> Artificial Sequence
<220>
   <223> synthetic construct; peptide mimetic
<400> 555
   Phe Phe Glu Arg Phe Ala Glu Ala Phe Lys Asp Tyr Val Ala Lys Trp
   1               5                   10                  15
   Arg Asp
<210> 556
   <211> 18
   <212> PRT
   <213> Artificial Sequence
<220>
   <223> synthetic construct; peptide mimetic
<400> 556
   Phe Phe Asp Lys Phe Ala Glu Ala Phe Lys Asp Tyr Val Ala Lys Trp
   1               5                   10                  15
   Lys Asp
<210> 557
   <211> 18
   <212> PRT
   <213> Artificial Sequence
<220>
   <223> synthetic construct; peptide mimetic
<400> 557
   Phe Phe Glu Lys Phe Ala Asp Ala Phe Lys Asp Tyr Val Ala Lys Trp
   1               5                   10                  15
   Lys Asp
<210> 558
   <211> 18
   <212> PRT
   <213> Artificial Sequence
<220>
   <223> synthetic construct; peptide mimetic
<400> 558
   Phe Phe Glu Arg Phe Ala Glu Ala Phe Lys Glu Tyr Val Ala Lys Trp
   1               5                   10                  15
   Lys Asp
<210> 559
   <211> 18
   <212> PRT
   <213> Artificial Sequence
<220>
   <223> synthetic construct; peptide mimetic
<400> 559
   Phe Phe Glu Arg Phe Ala Glu Ala Phe Lys Asp Tyr Val Ala Lys Trp
   1 5 10 15
   Lys Glu
<210> 560
   <211> 18
   <212> PRT
   <213> Artificial Sequence
<220>
   <223> synthetic construct; peptide mimetic
<400> 560
   Phe Phe Glu Lys Phe Lys Glu Phe Phe Lys Asp Tyr Phe Ala Lys Phe
   1 5 10 15
   Trp Asp
<210> 561
   <211> 18
   <212> PRT
   <213> Artificial Sequence
<220>
   <223> synthetic construct; peptide mimetic
<400> 561
   Phe Phe Asp Lys Phe Lys Asp Phe Phe Lys Asp Tyr Phe Ala Lys Phe
   1 5 10 15
   Trp Asp
<210> 562
   <211> 18
   <212> PRT
   <213> Artificial Sequence
<220>
   <223> synthetic construct; peptide mimetic
<400> 562
   Phe Phe Glu Lys Phe Lys Glu Phe Phe Lys Glu Tyr Phe Ala Lys Phe
   1 5 10 15
   Trp Glu
<210> 563
   <211> 18
   <212> PRT
   <213> Artificial Sequence
<220>
   <223> synthetic construct; peptide mimetic
<400> 563
   Phe Phe Glu Arg Phe Lys Glu Phe Phe Lys Asp Tyr Phe Ala Lys Phe
   1               5                   10                  15
   Trp Asp
<210> 564
   <211> 18
   <212> PRT
   <213> Artificial Sequence
<220>
   <223> synthetic construct; peptide mimetic
<400> 564
   Phe Phe Glu Lys Phe Arg Glu Phe Phe Lys Asp Tyr Phe Ala Lys Phe
   1               5                   10                  15
   Trp Asp
<210> 565
   <211> 18
   <212> PRT
   <213> Artificial Sequence
<220>
   <223> synthetic construct; peptide mimetic
<400> 565
   Phe Phe Glu Lys Phe Lys Glu Phe Phe Arg Asp Tyr Phe Ala Lys Phe
   1               5                   10                  15
   Trp Asp
<210> 566
   <211> 18
   <212> PRT
   <213> Artificial Sequence
<220>
   <223> synthetic construct; peptide mimetic
<400> 566
   Phe Phe Glu Lys Phe Lys Glu Phe Phe Lys Asp Tyr Phe Ala Arg Phe
   1               5                   10                  15
   Trp Asp
<210> 567
   <211> 18
   <212> PRT
   <213> Artificial Sequence
<220>
   <223> synthetic construct; peptide mimetic
<400> 567
   Phe Phe Asp Lys Phe Lys Glu Phe Phe Lys Asp Tyr Phe Ala Lys Phe
   1               5                   10                  15
   Trp Asp
<210> 568
   <211> 18
   <212> PRT
   <213> Artificial Sequence
<220>
   <223> synthetic construct; peptide mimetic
<400> 568
   Phe Phe Glu Lys Phe Lys Asp Phe Phe Lys Asp Tyr Phe Ala Lys Phe
   1               5                   10                  15
   Trp Asp
<210> 569
   <211> 18
   <212> PRT
   <213> Artificial Sequence
<220>
   <223> synthetic construct; peptide mimetic
<400> 569
   Phe Phe Glu Lys Phe Lys Glu Phe Phe Lys Glu Tyr Phe Ala Lys Phe
   1               5                   10                  15
   Trp Asp
<210> 570
   <211> 18
   <212> PRT
   <213> Artificial Sequence
<220>
   <223> synthetic construct; peptide mimetic
<400> 570
   Phe Phe Glu Lys Phe Lys Glu Phe Phe Lys Asp Tyr Phe Ala Lys Phe
   1               5                   10                  15
   Trp Glu
<210> 571
   <211> 18
   <212> PRT
   <213> Artificial Sequence
<220>
   <223> synthetic construct; epsilon-methylLys analog
<220>
   <221> misc
   <222> (4)..(4)
   <223> Lys at position 4 is epsilon methylated
<220>
   <221> misc
   <222> (9)..(9)
   <223> Lys at position 9 is epsilon methylated
<220>
   <221> misc
   <222> (13)..(13)
   <223> Lys at position 13 is epsilon methylated
<220>
   <221> misc
   <222> (15)..(15)
   <223> Lys at position 15 is epsilon methylated
<400> 571
   Asp Trp Phe Lys Ala Phe Tyr Asp Lys Val Ala Glu Lys Phe Lys Glu
   1               5                   10                  15
   Ala Phe
<210> 572
   <211> 18
   <212> PRT
   <213> Artificial Sequence
<220>
   <223> synthetic construct; epsilon-methylLys analog
<220>
   <221> misc
   <222> (4)..(4)
   <223> Lys at position 4 is epsilon methylated
<220>
   <221> misc
   <222> (9)..(9)
   <223> Lys at position 9 is epsilon methylated
<220>
   <221> misc
   <222> (13)..(13)
   <223> Lys at position 13 is epsilon methylated
<220>
   <221> misc
   <222> (15)..(15)
   <223> Lys at position 15 is epsilon methylated
<400> 572
   Asp Trp Phe Lys Ala Phe Tyr Asp Lys Val Ala Glu Lys Phe Lys Glu
   1               5                   10                  15
   Ala Phe
<210> 573
   <211> 18
   <212> PRT
   <213> Artificial Sequence
<220>
   <223> synthetic construct; epsilon-N-dimethylLys analog
<220>
   <221> misc
   <222> (4)..(4)
   <223> Lys at position 4 is and epsilon-N-Dimethyl-Lys
<220>
   <221> misc
   <222> (9)..(9)
   <223> Lys at position 9 is and epsilon-N-Dimethyl-Lys
<220>
   <221> misc
   <222> (13)..(13)
   <223> Lys at position 9 is and epsilon-N-Dimethyl-Lys
<220>
   <221> misc
   <222> (15)..(15)
   <223> Lys at position 9 is and epsilon-N-Dimethyl-Lys
<400> 573
   Asp Trp Phe Lys Ala Phe Tyr Asp Lys Val Ala Glu Lys Phe Lys Glu
   1               5                   10                  15
   Ala Phe
<210> 574
   <211> 18
   <212> PRT
   <213> Artificial Sequence
<220>
   <223> synthetic construct; epsilon-N-dimethylLys analog
<220>
   <221> misc
   <222> (4)..(4)
   <223> Lys at position 4 is and epsilon-N-Dimethyl-Lys
<220>
   <221> misc
   <222> (9)..(9)
   <223> Lys at position 9 is and epsilon-N-Dimethyl-Lys
<220>
   <221> misc
   <222> (13)..(13)
   <223> Lys at position 13 is and epsilon-N-Dimethyl-Lys
<220>
   <221> misc
   <222> (15)..(15)
   <223> Lys at position 15 is and epsilon-N-Dimethyl-Lys
<400> 574
   Asp Trp Phe Lys Ala Phe Tyr Asp Lys Val Ala Glu Lys Phe Lys Glu
   1               5                   10                  15
   Ala Phe
<210> 575
   <211> 18
   <212> PRT
   <213> Artificial Sequence
<220>
   <223> synthetic construct; epsilon-N-dimethylLys analog
<220>
   <221> misc
   <222> (4)..(4)
   <223> Lys at position 4 is and epsilon-N-Dimethyl-Lys
<220>
   <221> misc
   <222> (9)..(9)
   <223> Lys at position 9 is and epsilon-N-Dimethyl-Lys
<400> 575
   Asp Trp Phe Lys Ala Phe Tyr Asp Lys Val Ala Glu Lys Phe Lys Glu
   1 5 10 15
   Ala Phe
<210> 576
   <211> 18
   <212> PRT
   <213> Artificial Sequence
<220>
   <223> synthetic construct; epsilon-N-diethylLys analog
<220>
   <221> misc
   <222> (4)..(4)
   <223> Lys at position 4 is and epsilon-N-Diethyl-Lys
<220>
   <221> misc
   <222> (9)..(9)
   <223> Lys at position 9 is and epsilon-N-Diethyl-Lys
<220>
   <221> misc
   <222> (13)..(13)
   <223> Lys at position 13 is and epsilon-N-Diethyl-Lys
<220>
   <221> misc
   <222> (15)..(15)
   <223> Lys at position 15 is and epsilon-N-Diethyl-Lys
<400> 576
   Asp Trp Phe Lys Ala Phe Tyr Asp Lys Val Ala Glu Lys Phe Lys Glu
   1 5 10 15
   Ala Phe
<210> 577
   <211> 18
   <212> PRT
   <213> Artificial Sequence
<220>
   <223> synthetic construct; epsilon-N-diethylLys analog
<220>
   <221> misc
   <222> (4)..(4)
   <223> Lys at position 4 is and epsilon-N-Diethyl-Lys
<220>
   <221> misc
   <222> (9)..(9)
   <223> Lys at position 9 is and epsilon-N-Diethyl-Lys
<220>
   <221> misc
   <222> (13)..(13)
   <223> Lys at position 13 is and epsilon-N-Diethyl-Lys
<220>
   <221> misc
   <222> (15)..(15)
   <223> Lys at position 15 is and epsilon-N-Diethyl-Lys
<400> 577
   Asp Trp Phe Lys Ala Phe Tyr Asp Lys Val Ala Glu Lys Phe Lys Glu
   1               5                   10                  15
   Ala Phe
<210> 578
   <211> 18
   <212> PRT
   <213> Artificial Sequence
<220>
   <223> synthetic construct; epsilon-N-diethylLys analog
<220>
   <221> misc
   <222> (4)..(4)
   <223> Lys at position 4 is and epsilon-N-Diethyl-Lys
<220>
   <221> misc
   <222> (9)..(9)
   <223> Lys at position 9 is and epsilon-N-Diethyl-Lys
<220>
   <221> misc
   <222> (13)..(13)
   <223> Lys at position 13 is and epsilon-N-Diethyl-Lys
<220>
   <221> misc
   <222> (13)..(13)
   <223> Lys at position 15 is and epsilon-N-Diethyl-Lys
<400> 578
   Asp Trp Phe Lys Ala Phe Tyr Asp Lys Val Ala Glu Lys Phe Lys Glu
   1               5                   10                  15
   Ala Phe
<210> 579
   <211> 18
   <212> PRT
   <213> Artificial Sequence
<220>
   <223> synthetic construct; epsilon-N-monomethylLys analog
<220>
   <221> misc
   <222> (4)..(4)
   <223> Lys at position 4 is and epsilon-N-monomethyl-Lys
<220>
   <221> misc
   <222> (9)..(9)
   <223> Lys at position 9 is and epsilon-N-monomiethyl-Lys
<220>
   <221> misc
   <222> (13)..(13)
   <223> Lys at position 13 is and epsilon-N-monomethyl-Lys
<220>
   <221> misc
   <222> (15)..(15)
   <223> Lys at position 15 is and epsilon-N-monomethyl-Lys
<400> 579
   Asp Trp Phe Lys Ala Phe Tyr Asp Lys Val Ala Glu Lys Phe Lys Glu
   1 5 10 15
   Ala Phe
<210> 580
   <211> 18
   <212> PRT
   <213> Artificial Sequence
<220>
   <223> synthetic construct; epsilon-N-monomethylLys analog
<220>
   <221> misc
   <222> (4)..(4)
   <223> Lys at position 4 is and epsilon-N-monomethyl-Lys
<220>
   <221> misc
   <222> (9)..(9)
   <223> Lys at position 9 is and epsilon-N-monomethyl-Lys
<220>
   <221> misc
   <222> (13)..(13)
   <223> Lys at position 13 is and epsilon-N-monomethyl-Lys
<220>
   <221> misc
   <222> (15)..(15)
   <223> Lys at position 15 is and epsilon-N-monomethyl-Lys
<400> 580
   Asp Trp Phe Lys Ala Phe Tyr Asp Lys Val Ala Glu Lys Phe Lys Glu
   1               5                   10                  15
   Ala Phe
<210> 581
   <211> 18
   <212> PRT
   <213> Artificial Sequence
<220>
   <223> synthetic construct; epsilon-N-monomethylLys analog
<220>
   <221> misc
   <222> (4)..(4)
   <223> Lys at position 4 is and epsilon-N-monomethyl-Lys
<220>
   <221> misc
   <222> (9)..(9)
   <223> Lys at position 9 is and epsilon-N-monomethyl-Lys
<220>
   <221> misc
   <222> (13)..(13)
   <223> Lys at position 13 is and epsilon-N-monomethyl-Lys
<220>
   <221> misc
   <222> (15)..(15)
   <223> Lys at position 15 is and epsilon-N-monomethyl-Lys
<400> 581
   Asp Trp Phe Lys Ala Phe Tyr Asp Lys Val Ala Glu Lys Phe Lys Glu
   1               5                   10                  15
   Ala Phe
<210> 582
   <211> 18
   <212> PRT
   <213> Artificial Sequence
<220>
   <223> synthetic construct; epsilon-N-ethylLys analog
<220>
   <221> misc
   <222> (4)..(4)
   <223> Lys at position 4 is and epsilon-N-ethyl-Lys
<220>
   <221> misc
   <222> (9)..(9)
   <223> Lys at position 9 is and epsilon-N-ethyl-Lys
<220>
   <221> misc
   <222> (13)..(13)
   <223> Lys at position 13 is and epsilon-N-ethyl-Lys
<220>
   <221> misc
   <222> (15)..(15)
   <223> Lys at position 15 is and epsilon-N-ethyl-Lys
<400> 582
   Asp Trp Phe Lys Ala Phe Tyr Asp Lys Val Ala Glu Lys Phe Lys Glu
   1               5                   10                  15
   Ala Phe
<210> 583
   <211> 18
   <212> PRT
   <213> Artificial Sequence
<220>
   <223> synthetic construct; epsilon-N-ethylLys analog
<220>
   <221> misc
   <222> (4)..(4)
   <223> Lys at position 4 is and epsilon-N-ethyl-Lys
<220>
   <221> misc
   <222> (9)..(9)
   <223> Lys at position 9 is and epsilon-N-ethyl-Lys
<220>
   <221> misc
   <222> (13)..(13)
   <223> Lys at position 13 is and epsilon-N-ethyl-Lys
<220>
   <221> misc
   <222> (15)..(15)
   <223> Lys at position 15 is and epsilon-N-ethyl-Lys
<400> 583
   Asp Trp Phe Lys Ala Phe Tyr Asp Lys Val Ala Glu Lys Phe Lys Glu
   1 5 10 15
   Ala Phe
<210> 584
   <211> 18
   <212> PRT
   <213> Artificial Sequence
<220>
   <223> synthetic construct; epsilon-N-ethylLys analog
<220>
   <221> misc
   <222> (4)..(4)
   <223> Lys at position 4 is and epsilon-N-ethyl-Lys
<220>
   <221> misc
   <222> (9)..(9)
   <223> Lys at position 9 is and epsilon-N-ethyl-Lys
<220>
   <221> misc
   <222> (13)..(13)
   <223> Lys at position 13 is and epsilon-N-ethyl-Lys
<220>
   <221> misc
   <222> (15)..(15)
   <223> Lys at position 15 is and epsilon-N-ethyl-Lys
<400> 584
   Asp Trp Phe Lys Ala Phe Tyr Asp Lys Val Ala Glu Lys Phe Lys Glu
   1 5 10 15
   Ala Phe
<210> 585
   <211> 18
   <212> PRT
   <213> Artificial Sequence
<220>
   <223> synthetic construct; homoLys analog
<220>
   <221> misc
   <222> (4)..(4)
   <223> Lys at position 4 is a homoLys
<220>
   <221> misc
   <222> (9)..(9)
   <223> Lys at position 9 is a homoLys
<220>
   <221> misc
   <222> (13)..(13)
   <223> Lys at position 13 is a homoLys
<220>
   <221> misc
   <222> (15)..(15)
   <223> Lys at position 15 is a homoLys
<400> 585
   Asp Trp Phe Lys Ala Phe Tyr Asp Lys Val Ala Glu Lys Phe Lys Glu
   1               5                   10                  15
   Ala Phe
<210> 586
   <211> 18
   <212> PRT
   <213> Artificial Sequence
<220>
   <223> synthetic construct; homoLys analog
<220>
   <221> misc
   <222> (4)..(4)
   <223> Lys at position 4 is homoLys
<220>
   <221> misc
   <222> (9)..(9)
   <223> Lys at position 9 is homoLys
<220>
   <221> misc
   <222> (13)..(13)
   <223> Lys at position 13 is homoLys
<220>
   <221> misc
   <222> (15)..(15)
   <223> Lys at position 15 is homoLys
<400> 586
   Asp Trp Phe Lys Ala Phe Tyr Asp Lys Val Ala Glu Lys Phe Lys Glu
   1               5                   10                  15
   Ala Phe
<210> 587
   <211> 18
   <212> PRT
   <213> Artificial Sequence
<220>
   <223> synthetic construct; homoLys analog
<220>
   <221> misc
   <222> (4)..(4)
   <223> Lys at position 4 is homoLys
<220>
   <221> misc
   <222> (9)..(9)
   <223> Lys at position 9 is homoLys
<220>
   <221> misc
   <222> (13)..(13)
   <223> Lys at position 13 is homoLys
<220>
   <221> misc
   <222> (15)..(15)
   <223> Lys at position 13 is homoLys
<400> 587
   Asp Trp Phe Lys Ala Phe Tyr Asp Lys Val Ala Glu Lys Phe Lys Glu
   1               5                   10                  15
   Ala Phe
<210> 588
   <211> 18
   <212> PRT
   <213> Artificial Sequence
<220>
   <223> synthetic construct; homoLys analog
<220>
   <221> misc
   <222> (4)..(4)
   <223> Lys at position 4 is homoLys
<220>
   <221> misc
   <222> (9)..(9)
   <223> Lys at position 9 is homoLys
<220>
   <221> misc
   <222> (13)..(13)
   <223> Lys at position 13 is homoLys
<220>
   <221> misc
   <222> (15)..(15)
   <223> Lys at position 15 is homoLys
<400> 588
   Asp Trp Phe Lys Ala Phe Tyr Asp Lys Val Ala Glu Lys Phe Lys Glu
   1 5 10 15
   Ala Phe
<210> 589
   <211> 18
   <212> PRT
   <213> Artificial Sequence
<220>
   <223> synthetic construct; homoLys analog
<220>
   <221> misc
   <222> (4)..(4)
   <223> Lys at position 4 is homoLys
<220>
   <221> misc
   <222> (9)..(9)
   <223> Lys at position 9 is homoLys
<220>
   <221> misc
   <222> (13)..(13)
   <223> Lys at position 13 is homoLys
<220>
   <221> misc
   <222> (15)..(15)
   <223> Lys at position 15 is homoLys
<400> 589
   Asp Trp Phe Lys Ala Phe Tyr Asp Lys Val Ala Glu Lys Phe Lys Glu
   1 5 10 15
   Ala Phe
<210> 590
   <211> 18
   <212> PRT
   <213> Artificial Sequence
<220>
   <223> synthetic construct; homoLys analog
<220>
   <221> misc
   <222> (4)..(4)
   <223> Lys at position 4 is homoLys
<220>
   <221> misc
   <222> (9)..(9)
   <223> Lys at position 9 is homoLys
<220>
   <221> misc
   <222> (13)..(13)
   <223> Lys at position 13 is homoLys
<220>
   <221> misc
   <222> (15)..(15)
   <223> Lys at position 15 is homoLys
<400> 590
   Asp Trp Phe Lys Ala Phe Tyr Asp Lys Val Ala Glu Lys Phe Lys Glu
   1               5                   10                  15
   Ala Phe
<210> 591
   <211> 18
   <212> PRT
   <213> Artificial Sequence
<220>
   <223> synthetic construct; homoLys analog
<220>
   <221> misc
   <222> (4)..(4)
   <223> Lys at position 4 is homoLys
<220>
   <221> misc
   <222> (9)..(9)
   <223> Lys at position 9 is homoLys
<220>
   <221> misc
   <222> (13)..(13)
   <223> Lys at position 13 is homoLys
<220>
   <221> misc
   <222> (15)..(15)
   <223> Lys at position 15 is homoLys
<400> 591
   Asp Trp Phe Lys Ala Phe Tyr Asp Lys Val Ala Glu Lys Phe Lys Glu
   1               5                   10                  15
   Ala Phe
<210> 592
   <211> 18
   <212> PRT
   <213> Artificial Sequence
<220>
   <223> synthetic construct; homoLys analog
<220>
   <221> misc
   <222> (4)..(4)
   <223> Lys at position 4 is homoLys
<220>
   <221> misc
   <222> (9)..(9)
   <223> Lys at position 9 is homoLys
<220>
   <221> misc
   <222> (13)..(13)
   <223> Lys at position 13 is homoLys
<220>
   <221> misc
   <222> (15)..(15)
   <223> Lys at position 15 is homoLys
<400> 592
   Asp Trp Phe Lys Ala Phe Tyr Asp Lys Val Ala Glu Lys Phe Lys Glu
   1               5                   10                  15
   Ala Phe
<210> 593
   <211> 18
   <212> PRT
   <213> Artificial Sequence
<220>
   <223> synthetic construct; homoLys analog
<220>
   <221> misc
   <222> (4)..(4)
   <223> Lys at position 4 is homoLys
<220>
   <221> misc
   <222> (9)..(9)
   <223> Lys at position 9 is homoLys
<220>
   <221> misc
   <222> (13)..(13)
   <223> Lys at position 13 is homoLys
<220>
   <221> misc
   <222> (15)..(15)
   <223> Lys at position 15 is homoLys
<400> 593
   Asp Trp Phe Lys Ala Phe Tyr Asp Lys Val Ala Glu Lys Phe Lys Glu
   1               5                   10                  15
   Ala Phe
<210> 594
   <211> 18
   <212> PRT
   <213> Artificial Sequence
<220>
   <223> synthetic construct; homoLys analog
<220>
   <221> misc
   <222> (4)..(4)
   <223> Lys at position 4 is homoLys
<220>
   <221> misc
   <222> (9)..(9)
   <223> Lys at position 9 is homoLys
<220>
   <221> misc
   <222> (13)..(13)
   <223> Lys at position 13 is homoLys
<220>
   <221> misc
   <222> (15)..(15)
   <223> Lys at position 15 is homoLys
<400> 594
   Asp Trp Phe Lys Ala Phe Tyr Asp Lys Val Ala Glu Lys Phe Lys Glu
     1               5                   10                  15
   Ala Phe
<210> 595
   <211> 18
   <212> PRT
   <213> Artificial Sequence
<220>
   <223> synthetic construct; homoLys analog
<220>
   <221> misc
   <222> (4)..(4)
   <223> Lys at position 4 is homoLys
<220>
   <221> misc
   <222> (9)..(9)
   <223> Lys at position 9 is homoLys
<220>
   <221> misc
   <222> (13)..(13)
   <223> Lys at position 13 is homoLys
<220>
   <221> misc
   <222> (15)..(15)
   <223> Lys at position 15 is homoLys
<400> 595
   Asp Trp Phe Lys Ala Phe Tyr Asp Lys Val Ala Glu Lys Phe Lys Glu
   1 5 10 15
   Ala Phe
<210> 596
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-dimethyl-ornithine
<220>
   <221> misc
   <222> (9)..(9)
   <223> Xaa at position 9 is a delta-N-dimethyl-ornithine
<220>
   <221> misc
   <222> (13)..(13)
   <223> Xaa at position 13 is a delta-N-dimethyl-ornithine
<220>
   <221> misc
   <222> (15)..(15)
   <223> Xaa at position 15 is a delta-N-dimethyl-ornithine
<400> 596
   Asp Trp Phe Xaa Ala Phe Tyr Asp Xaa Val Ala Glu Xaa Phe Xaa Glu
   1               5                   10                  15
   Ala Phe
<210> 597
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-dimethyl-ornithine
<220>
   <221> misc
   <222> (9)..(9)
   <223> Xaa at position 9 is a delta-N-dimethyl-ornithine
<220>
   <221> misc
   <222> (13)..(13)
   <223> Xaa at position 13 is a delta-N-dimethyl-ornithine
<220>
   <221> misc
   <222> (15)..(15)
   <223> Xaa at position 15 is a delta-N-dimethyl-ornithine
<400> 597
   Asp Trp Phe Xaa Ala Phe Tyr Asp Xaa Val Ala Glu Xaa Phe Xaa Glu
   1               5                   10                  15
   Ala Phe
<210> 598
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-dimethyl-ornithine
<220>
   <221> misc
   <222> (9)..(9)
   <223> Xaa at position 9 is a delta-N-dimethyl-ornithine
<220>
   <221> misc
   <222> (13)..(13)
   <223> Xaa at position 13 is a delta-N-dimethyl-ornithine
<220>
   <221> misc
   <222> (15)..(15)
   <223> Xaa at position 15 is a delta-N-dimethyl-ornithine
<400> 598
   Asp Trp Phe Xaa Ala Phe Tyr Asp Xaa Val Ala Glu Xaa Phe Xaa Glu
   1               5                   10                  15
   Ala Phe
<210> 599
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-dimethyl-ornithine
<220>
   <221> misc
   <222> (9)..(9)
   <223> Xaa at position 9 is a delta-N-dimethyl-ornithine
<220>
   <221> misc
   <222> (13)..(13)
   <223> Xaa at position 13 is a delta-N-dimethyl-ornithine
<220>
   <221> misc
   <222> (15)..(15)
   <223> Xaa at position 15 is a delta-N-dimethyl-ornithine
<400> 599
   Asp Trp Phe Xaa Ala Phe Tyr Asp Xaa Val Ala Glu Xaa Phe Xaa Glu
   1 5 10 15
   Ala Phe
<210> 600
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-diethyl-ornithine
<220>
   <221> misc
   <222> (9)..(9)
   <223> Xaa at position 9 is a delta-N-diethyl-ornithine
<220>
   <221> misc
   <222> (13)..(13)
   <223> Xaa at position 13 is a delta-N-diethyl-ornithine
<220>
   <221> misc
   <222> (15)..(15)
   <223> Xaa at position 15 is a delta-N-diethyl-ornithine
<400> 600
   Asp Trp Phe Xaa Ala Phe Tyr Asp Xaa Val Ala Glu Xaa Phe Xaa Glu
   1 5 10 15
   Ala Phe
<210> 601
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-methyl-ornithine
<220>
   <221> misc
   <222> (9)..(9)
   <223> Xaa at position 9 is a delta-N-methyl-ornithine
<220>
   <221> misc
   <222> (13)..(13)
   <223> Xaa at position 13 is a delta-N-methyl-ornithine
<220>
   <221> misc
   <222> (15)..(15)
   <223> Xaa at position 15 is a delta-N-methyl-ornithine
<400> 601
   Asp Trp Phe Xaa Ala Phe Tyr Asp Xaa Val Ala Glu Xaa Phe Xaa Glu
   1               5                   10                  15
   Ala Phe
<210> 602
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-methyl-ornithine
<220>
   <221> misc
   <222> (9)..(9)
   <223> Xaa at position 9 is a delta-N-methyl-ornithine
<220>
   <221> misc
   <222> (13)..(13)
   <223> Xaa at position 13 is a delta-N-methyl-ornithine
<220>
   <221> misc
   <222> (15)..(15)
   <223> Xaa at position 15 is a delta-N-methyl-ornithine
<400> 602
   Asp Trp Phe Xaa Ala Phe Tyr Asp Xaa Val Ala Glu Xaa Phe Xaa Glu
   1               5                   10                  15
   Ala Phe
<210> 603
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-methyl-ornithine
<220>
   <221> misc
   <222> (9)..(9)
   <223> Xaa at position 9 is a delta-N-methyl-ornithine
<220>
   <221> misc
   <222> (13)..(13)
   <223> Xaa at position 13 is a delta-N-methyl-ornithine
<220>
   <221> misc
   <222> (15)..(15)
   <223> Xaa at position 15 is a delta-N-methyl-ornithine
<400> 603
   Asp Trp Phe Xaa Ala Phe Tyr Asp Xaa Val Ala Glu Xaa Phe Xaa Glu
   1               5                   10                  15
   Ala Phe
<210> 604
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-ethyl-ornithine
<220>
   <221> misc
   <222> (9)..(9)
   <223> Xaa at position 9 is a delta-N-ethyl-ornithine
<220>
   <221> misc
   <222> (13)..(13)
   <223> Xaa at position 13 is a delta-N-ethyl-ornithine
<220>
   <221> misc
   <222> (15)..(15)
   <223> Xaa at position 15 is a delta-N-ethyl-ornithine
<400> 604
   Asp Trp Phe Xaa Ala Phe Tyr Asp Xaa Val Ala Glu Xaa Phe Xaa Glu
   1               5                   10                  15
   Ala Phe
<210> 605
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-ethyl-ornithine
<220>
   <221> misc
   <222> (9)..(9)
   <223> Xaa at position 9 is a delta-N-ethyl-ornithine
<220>
   <221> misc
   <222> (13)..(13)
   <223> Xaa at position 13 is a delta-N-ethyl-ornithine
<220>
   <221> misc
   <222> (15)..(15)
   <223> Xaa at position 15 is a delta-N-ethyl-ornithine
<400> 605
   Asp Trp Phe Xaa Ala Phe Tyr Asp Xaa Val Ala Glu Xaa Phe Xaa Glu
   1               5                   10                  15
   Ala Phe
<210> 606
   <211> 18
   <212> PRT
   <213> Artificial Sequence
<220>
   <223> synthetic construct; ornithine analogs
<220>
   <221> misc
   <222> (4)..(4)
   <223> Xaa at position 4 is a delta-N-ethyl-ornithine
<220>
   <221> misc
   <222> (9)..(9)
   <223> Xaa at position 9 is a delta-N-ethyl-ornithine
<220>
   <221> misc
   <222> (13)..(13)
   <223> Xaa at position 13 is a delta-N-ethyl-ornithine
<220>
   <221> misc
   <222> (15)..(15)
   <223> Xaa at position 15 is a delta-N-ethyl-ornithine
<400> 606
   Asp Trp Phe Xaa Ala Phe Tyr Asp Xaa Val Ala Glu Xaa Phe Xaa Glu
   1 5 10 15
   Ala Phe
<210> 607
   <211> 10
   <212> PRT
   <213> Artificial Sequence
<220>
   <223> synthetic construct; receptor binding domains of ApoE
<400> 607
   Arg Leu Leu Arg Lys Arg Leu Lys Arg Leu
   1 5 10
<210> 608
   <211> 10
   <212> PRT
   <213> Artificial Sequence
<220>
   <223> synthetic construct; receptor binding domain of ApoE
<400> 608
   Arg Met Leu Arg Lys Arg Met Lys Arg Leu
   1 5 10
<210> 609
   <211> 10
   <212> PRT
   <213> Artificial Sequence
<220>
   <223> synthetic construct; receptor binding domain of ApoE
<400> 609
   Arg Leu Leu Arg Lys Pro Leu Lys Arg Leu
   1 5 10
<210> 610
   <211> 10
   <212> PRT
   <213> Artificial Sequence
<220>
   <223> synthetic construct; receptor binding domain of ApoE
<400> 610
   Arg Val Leu Arg Lys Arg Val Asn Arg Leu
   1 5 10
<210> 611
   <211> 10
   <212> PRT
   <213> Artificial Sequence
<220>
   <223> synthetic construct; receptor binding domain of ApoE
<400> 611
   Arg Leu Val Arg Lys Arg Leu Lys Arg Met
   1 5 10
<210> 612
   <211> 10
   <212> PRT
   <213> Artificial Sequence
<220>
   <223> synthetic construct; receptor binding domain of ApoE
<400> 612
   Arg Leu Leu Arg Arg Arg Leu Arg Arg Leu
   1 5 10
<210> 613
   <211> 10
   <212> PRT
   <213> Artificial Sequence
<220>
   <223> synthetic construct; receptor binding domain of ApoE
<400> 613
   Arg Phe Phe Arg Lys Arg Leu Lys Arg Leu
   1 5 10
<210> 614
   <211> 18
   <212> PRT
   <213> Artificial Sequence
<220>
   <223> synthetic construct; Lipid-Associating Peptide
<400> 614
   Phe Ala Glu Lys Leu Lys Glu Ala Val Lys Asp Tyr Phe Ala Lys Leu
   1               5                   10                  15
   Trp Asp
<210> 615
   <211> 18
   <212> PRT
   <213> Artificial Sequence
<220>
   <223> synthetic construct; Lipid-Associating Peptide
<400> 615
   Phe Ala Glu Lys Phe Lys Glu Ala Val Lys Asp Tyr Phe Ala Lys Phe
   1               5                   10                  15
   Trp Asp
<210> 616
   <211> 18
   <212> PRT
   <213> Artificial Sequence
<220>
   <223> synthetic construct; Lipid-Associating Peptide
<400> 616
   Lys Ala Phe Glu Glu Val Leu Ala Lys Lys Phe Tyr Asp Lys Ala Leu
   1               5                   10                  15
   Trp Asp
<210> 617
   <211> 10
   <212> PRT
   <213> Artificial Sequence
<220>
   <223> synthetic construct; receptor binding domain of ApoE
<400> 617
   Leu Arg Leu Leu Arg Lys Leu Lys Arg Arg
   1 5 10
<210> 618
   <211> 18
   <212> PRT
   <213> Artificial Sequence
<220>
   <223> synthetic construct; peptide mimetic
<400> 618
   Asp Trp Leu Lys Ala Phe Tyr Asp Lys Val Phe Glu Lys Phe Lys Glu
   1 5 10 15
   Ala Phe
<210> 619
   <211> 18
   <212> PRT
   <213> Artificial Sequence
<220>
   <223> synthetic construct; peptide mimetic
<400> 619
   Asp Trp Leu Lys Ala Phe Tyr Asp Lys Val Ala Glu Lys Phe Lys Glu
   1 5 10 15
   Phe Phe

## Description

This invention was made with government support under R01 HL090803 awarded by The National Institutes of Health. The government has certain rights in the invention.

### FIELD OF THE INVENTION

The disclosed invention relates generally to the treatment of atherosclerosis. In particular, the treatment pertains to a specific dosing regimen that includes a treatment cycle followed by a rest phase that provides prolonged therapeutic effects even after the treatment is withdrawn.

### BACKGROUND

In the United States, heart disease is the leading cause of death in both men and women. Several causative factors are implicated in the development of cardiovascular disease including hereditary predisposition to the disease, gender, lifestyle factors such as smoking and diet, age, hypertension, and hyperlipidemia, including hypercholesterolemia. Several of these factors, particularly hyperlipidemia and hypercholesteremia (high blood cholesterol concentrations) provide a significant risk factor associated with atherosclerosis.

Atherosclerosis is associated with an inflammatory response caused by the accumulation of low-density lipoprotein (LDL) molecules in blood vessels. It can be asymptomatic for years. Atherosclerosis causes hardening and narrowing of blood vessels. There are several treatments for atherosclerosis, such as lifestyle change, medication and medical procedures. Statins are a well-known treatment for atherosclerosis. Statins have proven to reduce cardiac risk however the withdrawal of statin therapy abrogates the protective effect (Heeschen et al. Circulation. 105:1446-1452, 2002).

A study suggested that apolipoprotein may contribute to the removal of cholesterol from peripheral tissues to the liver, anti-inflammatory and anti-oxidative activities, and modulation of vascular function, which might be correlated with the protection of a risk of atherosclerosis and other vascular diseases. Peptide analogs have been found to be maximally effective in mimicking the positive qualities of apolipoprotein; this peptide inhibited atherosclerosis, reduced inflammation and oxidation, and improved vascular function in a number of animal models, and when synthesized with D-amino acid is orally bioavailable (US 2010/286025A1, Anantharamaiah et al.).

The current approach to treating atherosclerosis is to provide earlier intervention and life-long treatment. This approach is problematic as it requires identifying asymptomatic patients early in their life cycle and, since risk increases with age, maintaining therapy for the duration of their life. Further, the most efficacious currently available therapies are unable to prevent major cardiac events in all patients whether as primary or secondary interventions. Therefore, there is a need for therapies that can provide rapid benefit in reducing atherosclerosis and have long-term effects that do not require constant administration. The compositions and methods disclosed herein provide an atherosclerosis therapy with sustained therapeutic effects even after the treatment is withdrawn.

### BRIEF SUMMARY

Dosing regimens containing at least one treatment cycle followed by a rest phase are provided. Also provided are methods of treating atherosclerosis involving administering to a subject an effective amount of an Apo E mimetic for at least one treatment cycle followed by a rest phase. The treatment cycles and rest phases of the dosing regimens and the methods of treating can be the same.

In both the dosing regimens and the methods of treating, the treatment cycle contains the administration of an effective amount of an Apo E mimetic to cause an immediate benefit illustrated by a decrease in atherosclerosis, a decrease in artery wall stiffness, a decrease in isolated systolic hypertension, a decrease in arterial inflammation, an increase in anti-oxidant capability of the HDL fraction and/or an improvement in myocardial function, and to allow for a sustained therapeutic effect after withdrawal of the Apo E mimetic. The treatment cycle can include the administration of an effective amount of the Apo E mimetic once a week for four weeks or once a week for three months. In some instances, the treatment cycle includes the administration of an effective amount of the Apo E mimetic once every two weeks for up to 12 weeks.

The rest phase is a period of time wherein the Apo E mimetic is not administered. The rest phase can be at least four weeks. An atherosclerosis therapeutic other than an Apo E mimetic can be administered during the rest phase. The atherosclerosis therapeutic other than an Apo E mimetic can be a conventional lipid lowering therapy, such as a statin or bile acid sequestrant, and/or a therapeutic such as a PCSK9 inhibitor, a VLDL synthesis inhibitor and/or a CETP inhibitor.

Both the dosing regimen and the methods of treating can further include a second treatment cycle after the rest phase. The second treatment cycle can be administered after a four week rest phase or one or more years from the end of the initial treatment cycle.

The Apo E mimetic can be hE 18A (LRKLRKRLLRDWLKAFYDKVAEKLKEAF (SEQ ID NO:1)).

Additional advantages of the disclosed method and compositions will be set forth in part in the description which follows, and in part will be understood from the description, or may be learned by practice of the disclosed method and compositions. The advantages of the disclosed method and compositions will be realized and attained by means of the elements and combinations particularly pointed out in the appended claims. It is to be understood that both the foregoing general description and the following detailed description are exemplary and explanatory only and are not restrictive of the invention as claimed.

### BRIEF DESCRIPTION OF THE DRAWINGS

The accompanying drawings, which are incorporated in and constitute a part of this specification, illustrate several embodiments of the disclosed method and compositions and together with the description, serve to explain the principles of the disclosed method and compositions.
Figures 1A, 1B, and 1C show the design of mR18L. A) 18L; B) m18L; and C) mR18L.
Figure 2 is a schematic diagram of the experimental protocol used in Apo E null mice.
Figures 3A and 3B show the amount of plasma cholesterol (mg/dl). A) Bar graph showing the plasma cholesterol after six weeks of western diet compared to after two weeks of chow after the six weeks of western diet. B) Line graph showing the plasma cholesterol over a 30 day period of treatment with mR18L, hE18A or control.
Figure 4 is a bar graph of plasma triglycerides (mg/dl) versus different treatments (saline, mR18L or hE18A).
Figures 5A and 5B are bar graphs of reactive oxygen species (ROS) and paraoxanase (PON) versus the different treatments, respectively.
Figures 6A and 6B are a combination of a line graph showing the absorbance in the different elutions and polyacrylamide gels were stained to show PON-1 activity in the HDL fractions (29-32). A) Control treated; B) Ac-he18A-NH₂ treated. The plasma cholesterol lipoprotein fractions were analyzed by the CLiP method. The solid lines show the cholesterol content of the various lipoprotein fractions, displayed as dashed lines.
Figure 7 is a line graph showing the plasma cholesterol levels (mg/dl) over a 60 day time period. Peptide was administered during the first 30 days The final time point is four weeks after the end of treatment with saline, mR18L or hE18A. Cholesterol levels in all treatment groups were the same at the end of the study.
Figures 8A and 8B are bar graphs of PON activity versus different treatments and ROS versus different treatments, respectively. The samples were from four weeks after treatment.
Figures 9A and 9B show that aortic lesions were reduced in hE18A treated mice even after treatment had been withdrawn for four weeks. A) Bar graph showing percent lesion area versus different treatments. B) Stained tissue samples showing the *en face* lesion area.
Figure 10 shows that lesions in the aortic sinus were reduced in hE18A treated mice even after treatment had been withdrawn for four weeks. mR18L had no effect on aortic sinus lesions at study termination.
Figure 11 shows a trend towards decreased macrophage coverage in the aortic sinus of mice treated with hE18A, relative to the other treatment groups, at study termination.
Figure 12 is a schematic diagram of the study design used in Apo E -/- mice.
Figures 13A and 13B are graphs showing the plasma lipid levels after treatment with a control or an Apo E mimetic. A) cholesterol; B) triglycerides. Each graph shows the lipid levels after the treatment has been withdrawn for four weeks (control and hE18A) and the lipid levels immediately after four weeks of treatment (control Term. 1 and hE18A Term. 1).
Figures 14 is a bar graph showing the liver cholesteryl ester and free cholesterol.
Figure 15 is a diagram of the aorta showing the regions that were analyzed for plaques or lesions.
Figure 16 is a bar graph of percent plaque in the aorta versus the different treatments. "Term 1" represents the results after the initial four weeks of treatment. "Term 2" represents the results after treatment has been withdrawn for four weeks.
Figures 17 is a bar graph showing plaque area in the aortic arch at week 5 and week 8.
Figures 18A and 18B are graphs showing the diastolic (A) and the systolic (B) area.

### DETAILED DESCRIPTION

The disclosed method and compositions may be understood more readily by reference to the following detailed description of particular embodiments and the Example included therein and to the Figures and their previous and following description.

It is to be understood that the disclosed method and compositions are not limited to specific synthetic methods, specific analytical techniques, or to particular reagents unless otherwise specified, and, as such, may vary. It is also to be understood that the terminology used herein is for the purpose of describing particular embodiments only and is not intended to be limiting.

### A. Definitions

The terminology used herein is for the purpose of describing particular embodiments only and is not intended to be limiting.

As used in the specification and the appended claims, the singular forms "a," "an" and "the" can include plural referents unless the context clearly dictates otherwise. Thus, for example, reference to "a compound" includes mixtures of compounds, reference to "a pharmaceutical carrier" includes mixtures of two or more such carriers, and the like.

Ranges may be expressed herein as from "about" one particular value, and/or to "about" another particular value. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%. When such a range is expressed, another embodiment includes from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

As used herein, the term "amino acid sequence" refers to a list of abbreviations, letters, characters or words representing amino acid residues. The amino acid abbreviations used herein are conventional one letter codes for the amino acids and are expressed as follows: A, alanine; C, cysteine; D aspartic acid; E, glutamic acid; F, phenylalanine; G, glycine; H histidine; I isoleucine; K, lysine; L, leucine; M, methionine; N, asparagine; P, proline; Q, glutamine; R, arginine; S, serine; T, threonine; V, valine; W, tryptophan; Y, tyrosine;.

As used herein, the term "Apo E mimetic" is interchangeable with apolipoprotein-E mimicking peptide. Apo E mimetics are peptides that are related to, characteristic of, or mimic Apo E. Apo E mimetics include Apo E peptides (i.e. peptides derived from full length Apo E).

"Dosing regimen" as used herein refers to at least one treatment cycle followed by at least one rest phase. A dosing regimen can include more than one treatment cycle and more than one rest phase. For example, a dosing regimen can be a three month treatment cycle followed by a one year rest phase. Another example can be a six month treatment cycle followed by a six month rest phase and then a three month treatment cycle followed by a one year rest phase.

As used herein, the term "treatment cycle" refers to the administration of Apo E mimetics for an established period of time. A treatment cycle includes a wide range of dosages of Apo E mimetics as well as different lengths of time for administering the Apo E mimetics. For example, a treatment cycle can be a three month period wherein an Apo E mimetic is administered twice a week for the three month period.

"Dose" or "dosage" as used herein refers to a specific quantity of a therapeutic agent, such as an Apo E mimetic, that is taken at specific times.

As used herein, "effective amount" is meant to mean a sufficient amount of the composition or Apo E mimetic to provide the desired effect. For example, an effective amount of an Apo E mimetic can be an amount that provides a therapeutic affect and provides sustained therapeutic effects after withdrawal of the treatment. An effective amount of an Apo E mimetic is an amount that is able to cause a benefit illustrated by a decrease in atherosclerosis, a decrease in artery wall stiffness, a decrease in isolated systolic hypertension, a decrease in arterial inflammation, an increase in anti-oxidant capability of the HDL fraction and/or an improvement in myocardial function, as well as an amount that allows for a sustained therapeutic effect after withdrawal of the Apo E mimetic. The exact amount required will vary from subject to subject, depending on the species, age, and general condition of the subject, the severity of disease (or underlying genetic defect) that is being treated, the particular compound used, its mode of administration, and the like. Thus, it is not possible to specify an exact "effective amount." However, an appropriate "effective amount" may be determined by one of ordinary skill in the art using only routine experimentation.

As used herein, "sustained therapeutic effect" is a therapeutic effect that persists after the therapeutic has been withdrawn. For example, the sustained therapeutic effect is maintained even after the acute cholesterol lowering effect is gone.

"Rest phase" as used herein refers to a period of time wherein an Apo E mimetic is not administered.

Atherosclerotic burden as used herein is the amount of atherosclerosis in the arteries of a patient. This may include the coronary, carotid, peripheral and other arteries. The atheroma may be complex lesions with a smooth muscle and collagen containing fibrous cap, areas of calcification, cholesterol crystals and cholesterol laden macropahges (foam cells) and/or less complex and more unstable lesions with less calcification and a thinner fibrous cap, and more foam cells and cholesterol (unstable lesions). The unstable lesions may intrude into the lumen of the artery or expand away from the lumen of the artery.

"Peptide" as used herein refers to any peptide, oligopeptide, polypeptide, gene product, expression product, or protein. A peptide is comprised of consecutive amino acids. The term "peptide" encompasses naturally occurring or synthetic molecules.

The word "or" as used herein means any one member of a particular list and also includes any combination of members of that list.

As used herein, "reverse oriented", "reversed orientation", "reverse analog" or "reverse sequence" refers to a peptide, or a portion of the peptide, has a reverse amino acid sequence as compared to a non-reverse oriented peptide (i.e., the original sequence is read (or written) from right to left). For example, if one peptide has the amino acid sequence ABCDE, its reverse analog or a peptide having its reverse sequence is as follows: EDCBA. In a dual domain peptide for example, Ac-hE-18A-NH2, either the hE sequence is read from right to left or the 18A sequence is read from right to left. For a reverse analog of, LRKLRKRLLR- DWLKAFYDKVAEKLKEAF (SEQ ID NO:1) can be RLLRKRLKRL-DWLKAFYDKVAEKLKEAF (SEQ ID NO:2) or LRKLRKRLLR-FAEKLKEAVKDYFAKLWD (SEQ ID NO:3).

As used herein a "dual-domain peptide", a "dual-domain synthetic peptide", or a "dual-domain Apo E mimicking peptide" is meant to mean a peptide comprising a lipid-associating peptide/domain and a receptor binding peptide/domain.

As used herein a "single-domain peptide", a "single-domain synthetic peptide", or a "single-domain Apo E mimicking peptide" is meant to mean a peptide comprising either a lipid-associating peptide/domain or a receptor binding peptide/domain, but not both.

As used herein "domain switched", "switched domain", or "switched" peptide is meant to mean that the lipid-associating peptide is covalently linked to the receptor binding domain of apolipoprotein E such that the lipid-associating peptide is at the N-terminus of the synthetic apolipoprotein E-mimicking peptide. For example, the peptide 18A-hE is exemplary of a domain switched peptide.

As used herein, "scrambled" "scrambled version", or "scrambled peptide" is meant to mean that the composition of the amino acid sequence is the same as the unscrambled peptide, however the sequence of the amino acids is altered thus rendering the peptide unable to form either an α-amphipathic helix or does not possess lipid associating (or HSPG associating) properties. However, in some cases, as described in this invention, the scrambled peptide remains able to form a different helical structure, such as a π-helix. For example, if one peptide has the amino acid sequence ABCDE, the scrambled version of the peptide could have the amino acid sequence DEABC. Scrambled peptides are often denoted as having a "Sc" prior to the portion of the peptide that is scrambled. For example, Sc-hE-18A denoted that the hE portion of the peptide is scrambled.

As used herein, "sample" is meant to mean an animal; a tissue or organ from an animal; a cell (either within a subject, taken directly from a subject, or a cell maintained in culture or from a cultured cell line); a cell lysate (or lysate fraction) or cell extract; or a solution containing one or more molecules derived from a cell or cellular material (e.g. a polypeptide or nucleic acid), which is assayed as described herein. A sample may also be any body fluid or excretion (for example, but not limited to, blood, urine, stool, saliva, tears, bile) that contains cells or cell components.

As used herein, "subject" refers to the target of administration, e.g. an animal. Thus the subject of the disclosed methods can be a vertebrate, such as a mammal. For example, the subject can be a human. The term does not denote a particular age or sex. Subject can be used interchangeably with "individual" or "patient".

As used herein, "modulate" is meant to mean to alter, by increasing or decreasing.

As used herein "lipid binding domain E" and "lipid-associating peptide" are used interchangeably. As used herein, both terms can mean the lipid binding domain of apolipoprotein E.

As used herein, "isolated polypeptide" or "purified polypeptide" is meant to mean a polypeptide (or a fragment thereof) that is substantially free from the materials with which the polypeptide is normally associated in nature. The polypeptides of the invention, or fragments thereof, can be obtained, for example, by extraction from a natural source (for example, a mammalian cell), by expression of a recombinant nucleic acid encoding the polypeptide (for example, in a cell or in a cell-free translation system), or by chemically synthesizing the polypeptide. In addition, polypeptide fragments may be obtained by any of these methods, or by cleaving full length proteins and/or polypeptides.

As used herein, "treat" is meant to mean administer one of the disclosed compositions to a subject, such as a human or other mammal (for example, an animal model), that has atherosclerosis, in order to prevent or delay a worsening of the effects of the disease or condition, or to partially or fully reverse the effects of the disease.

As used herein, "prevent" is meant to mean minimize the chance that a subject who has an increased susceptibility for developing atherosclerosis will develop atherosclerosis.

As used herein, "lipoprotein" or "lipoproteins" is meant to mean a biochemical assembly that contains both proteins and lipids. The lipids or their derivatives may be covalently or non-covalently bound to the proteins. Many enzymes, transporters, structural proteins, antigens, adhesins, and toxins are lipoproteins. Examples include the high density and low density lipoproteins of the blood, the transmembrane proteins of the mitochondrion and the chloroplast, and bacterial lipoproteins

As used herein, "high-density lipoprotein" (HDL) is meant to mean a class of lipoproteins, varying somewhat in their size (8-11 nm in diameter), that can transport cholesterol. HDL cholesterol is cholesterol that is associated with HDLs. About one-fourth to one-third of blood cholesterol is carried by high-density lipoprotein (HDL). HDL cholesterol is known as "good" cholesterol, because high levels of HDL seem to protect against heart attack. Low levels of HDL (less than 40 mg/dL in men and less than 50 mg/dL in women) also increase the risk of heart disease. Medical experts think that HDL tends to carry cholesterol away from the arteries and back to the liver, where it is passed from the body. Some experts believe that that HDL removes excess cholesterol from arterial plaque, thus slowing its buildup

As used herein, "very Low Density Lipoproteins" (VLDL) is meant to mean a lipoprotein subclass. It is assembled in the liver from cholesterol and apolipoproteins. It is converted in the bloodstream to low density lipoprotein (LDL). VLDL particles have a diameter of 30-80 nm. VLDL transports endogenous products where chylomicrons transport exogenous (dietary) products.

As used herein, "low-density lipoprotein" or "LDL" is mean to mean a lipoprotein that varies in size (approx. 22 nm) and can contain a changing number of triglycerides and cholesteryl esters they actually have a mass and size distribution. Each native LDL particle contains a single apolipoproteinB-100 molecule (Apo B-100, a protein with 4536 amino acid amino acid residues) and a phospholipid coat that circles the triglycerides and cholesteryl esters, keeping them soluble in the aqueous environment. LDL is commonly referred to as bad cholesterol. LDL cholesterol is cholesterol that is associated with LDLs. When too much LDL cholesterol circulates in the blood, it can slowly build up in the inner walls of the arteries that feed the heart and brain. Together with other substances, it can form plaque, a thick, hard deposit that can narrow the arteries and make them less flexible. This condition is known as atherosclerosis. If a clot forms and blocks a narrowed artery, then heart attack or stroke can result.

Cholesterol cannot dissolve in the blood. It has to be transported to and from the cells by carriers called lipoproteins. LDLs and HDLs along with triglyceride-rich lipoproteins (VLDL) and Lp(a) cholesterol, make up your total cholesterol count, which can be determined through a blood test.

The phrase "nucleic acid" as used herein refers to a naturally occurring or synthetic oligonucleotide or polynucleotide, whether DNA or RNA or DNA-RNA hybrid, single-stranded or double-stranded, sense or antisense, which is capable of hybridization to a complementary nucleic acid by Watson-Crick base-pairing. Nucleic acids of the invention can also include nucleotide analogs (e.g., BrdU), and non-phosphodiester internucleoside linkages (e.g., peptide nucleic acid (PNA) or thiodiester linkages). In particular, nucleic acids can include, without limitation, DNA, RNA, cDNA, gDNA, ssDNA, dsDNA or any combination thereof

Unless defined otherwise, all technical and scientific terms used herein have the same meanings as commonly understood by one of skill in the art to which the disclosed method and compositions belong. Although any methods and materials similar or equivalent to those described herein can be used in the practice or testing of the present method and compositions, the particularly useful methods, devices, and materials are as described..

### B. Dosing Regimens

Disclosed herein are dosing regimens comprising at least one treatment cycle of an effective amount of an Apo E mimetic followed by a rest phase. The rest phase of the dosing regimen is a period of time where the Apo E mimetic is not administered.

Disclosed herein are dosing regimens comprising at least one treatment cycle followed by a rest phase, wherein the treatment cycle comprises administering an effective amount of an Apo E mimetic to allow for a sustained therapeutic effect after withdrawal of the Apo E mimetic, wherein the Apo E mimetic is not administered during the rest phase. Not only does an effective amount of Apo E mimetic result in sustained therapeutic effects, but it is also an amount sufficient to cause an acute beneficial effect. Thus, the effects of the Apo E mimetic can be measured and seen during the treatment cycle, at the end of the treatment cycle and during the rest phase. The sustained therapeutic effects are the therapeutic effects seen even after the acute cholesterol lowering effect is gone.

Disclosed herein are dosing regimens comprising at least one treatment cycle followed by a rest phase, wherein the treatment cycle comprises administering an effective amount of an Apo E mimetic to allow for a sustained therapeutic effect after withdrawal of the Apo E mimetic, wherein the Apo E mimetic is not administered during the rest phase, wherein the treatment cycle comprises administration of an effective amount of the Apo E mimetic once a week for three months or wherein the treatment cycle comprises administration of an effective amount of the Apo E mimetic once every two weeks for up to 12 weeks.

Disclosed herein are dosing regimens comprising at least one treatment cycle followed by a rest phase, wherein the treatment cycle comprises administering an effective amount of an Apo E mimetic to allow for a sustained therapeutic effect after withdrawal of the Apo E mimetic, wherein the Apo E mimetic is not administered during the rest phase, wherein the dosing regimen further comprises a second treatment cycle after the rest phase.

Disclosed herein are dosing regimens comprising at least one treatment cycle followed by a rest phase, wherein the treatment cycle comprises administering an effective amount of an Apo E mimetic to allow for a sustained therapeutic effect after withdrawal of the Apo E mimetic, wherein the ApoE mimetic consists of the amino acid sequence LRKLRKRLLRDWLKAFYDKVAEKLKEAF (SEQ ID NO:1), wherein the Apo E mimetic is not administered during the rest phase. In some instances, the amino acid sequence LRKLRKRLLRDWLKAFYDKVAEKLKEAF (SEQ ID NO:1), is end protected with an acetyl group protecting the amino terminus and an amide group protecting the carboxyl terminus.

The dosing regimen can further include a second treatment cycle after the rest phase. A second rest phase can occur after the second treatment cycle. In some instances a third, fourth, fifth, sixth, seventh, eighth, ninth or tenth treatment cycle can be administered wherein each treatment cycle is followed by a rest phase. In one aspect, the dosing regimen includes infinite treatment cycles, each followed by a rest phase. For example, a subject may be prescribed a dosing regimen that involves consecutive treatment cycles followed by rest phases for the duration of their life.

In one aspect, a second dosing regimen can be prescribed based on the re-occurrence of atherosclerotic lesions or other atherosclerosis factors. The second dosing regimen can be administered 1, 2, 3, 4, 5 years or more than 5 years after the initial dosing regimen was administered. The second dosing regimen can be the same as the initial dosing regimen or can be different. For example, the initial dosing regimen can be a three month treatment cycle followed by a one year rest phase. After the one year rest phase the subject can be tested and if atherosclerotic lesions are building up again then a second dosing regimen consisting of another three month treatment cycle followed by a rest phase or a six month treatment cycle followed by a rest phase can be prescribed. The dose of Apo E mimetic can vary between the initial dosing regimen and any additionally prescribed dosing regimens.

### 1. Treatment Cycle

The treatment cycle can include the administration of different dosages of Apo E mimetic as well as administration at different time points. The Apo E mimetic can be administered for varying amounts of time for up to 6 months. In some instances, the administration can occur for up to one, two, three, four, five or six months. For example, the Apo E mimetic can be administered once a week for 4, 6, 8, 10, 12, 14, 16, 18, 20, 22, or 24 weeks.

The length of time for each treatment cycle can vary depending on the amount of Apo E administered per dosage. A treatment cycle can include the administration of Apo E mimetic once, twice or three times a week. In some aspects, the Apo E mimetic can be administered daily. In some aspects, the Apo E mimetic can be administered once every two weeks or even once a month. In some instances, the Apo E mimetic can be administered every two weeks for 4, 6, 8, 10, 12, 14, 16, 18, 20, 22, or 24 weeks. For example, the treatment cycle can include administering an Apo E mimetic once a week for four weeks or once every two weeks for up to six months. Thus, each treatment cycle includes an established length of time for administration as well as an established dosing schedule during that time frame.

In one aspect, more than one Apo E mimetic can be administered during the treatment cycles. The more than one Apo E mimetic can be formulated together or in separate compositions. In some instances, one or more Apo E mimetic is administered in combination with one or more other therapeutic agents, such as cholesterol lowering drugs.

### 2. Rest Phase

The disclosed dosing regimen includes at least one treatment cycle followed by a rest phase. The rest phase is a period of time wherein Apo E mimetic is not administered and the length of the period of time can vary. The length of the rest phase is dependent on how long the sustained therapeutic effects of the Apo E mimetic administered during the treatment cycle last. In some instances the rest phase can be at least 1, 2, 3, 4, 5, 6, 7, 8, 9, 10, 11, or 12 months. In some instances the rest phase can be at least 1, 2, 3, 4, 5, 6, 7, 8, 9, or 10 years. For example, the rest phase can be at least four weeks (one month).

One way to determine how long the rest phase should last is to test the subject to determine the progression of the atherosclerosis burden in the subject's arteries. If the atherosclerosis burden has progressed to a level that increases the risk of cardiovascular disease, then the subject can be prescribed a second dosing regimen. If the atherosclerosis burden is stable then the rest phase can be prolonged. Subjects can be tested on a regular basis. For example, a subject can be tested every 3, 6, 9, 12, 18, 24, 30 or 36 months.

In one aspect, the rest phase can be decreased or extended depending on the dose of Apo E mimetic administered and the reduction in atherosclerosis achieved during the treatment cycle. For example, the rest phase can be extended if the dose of Apo E mimetic during the treatment cycle is increased and the atherosclerosis burden is substantially reduced. The length of the rest phase can also vary based on the length of the treatment cycle. For instance, if a subject receives a certain dose of Apo E mimetic once a week for three months then the rest phase may be shorter than a subject that receives the same dose of Apo E mimetic once a week for six months.

Although an Apo E mimetic is not administered during the rest phase, an atherosclerosis therapeutic other than an Apo E mimetic can be administered during the rest phase. The atherosclerosis therapeutic other than an Apo E mimetic can be a conventional lipid lowering therapy, such as a statin, a bile acid sequestrant or a fibrate, or a novel anti-atherosclerosis therapeutic like a CETP inhibitor, a VLDL synthesis inhibitor, a PCSK9 inhibitor, and/or an arterial inflammation inhibitor.

In some instances, the beneficial effects of the Apo E mimetic can still be present in a subject even after the treatment cycle is complete. In one instance, the half-life of the Apo E mimetic is less than 1, 2, 3, 4, 5, 10, 15, 20, 25, or 30 days. In some instances the Apo E mimetic is no longer detectable in a subject after the treatment cycle is complete. Thus, the long-term therapeutic effects are not from residual Apo E mimetic.

### 3. Dose

The dose or dosage of Apo E mimetic can vary depending on many factors, such as but not limited to, age, condition, sex and extent of the disease in the patient, route of administration, length of treatment cycle, or whether other drugs are included in the regimen, and can be determined by one of skill in the art.

Effective dosages can be determined empirically, and making such determinations is within the skill in the art. The dosage ranges for the administration of the compositions are those large enough to produce the desired effect in which the disease is treated. For example, the dosage can be an amount effective to provide therapeutic effects and provide or allow for sustained therapeutic effects even after the treatment (i.e. Apo E mimetic) is withdrawn. The therapeutic effects can be, but are not limited to, a reduction in atherosclerotic lesions, decrease in arterial stiffness, decrease in isolated systolic hypertension, increase in vasoresponsiveness, improvement in cardiac function, increased blood flow to the limbs, decreased claudication, increased ankle brachial pressure index, improved healing of distal limb wounds. The therapeutic effects can be measured by markers of arterial inflammation such as, but not limited to, C-reactive protein. The therapeutic effects can be measured by atherosclerosis imaging techniques, including MRI, intravascular ultrasound, ultrafast imaging CT scans, B-mode ultrasonography, virtual histology intravascular ultrasound, optical coherence tomography, or other known methods.

The dosage should not be so large as to cause adverse side effects, such as unwanted cross-reactions, anaphylactic reactions, and the like. The dosage can be adjusted by the individual physician in the event of any counter-indications. Dosage can vary, and can be administered in one or more dose administrations daily, for one or several days. Guidance can be found in the literature for appropriate dosages for given classes of pharmaceutical products.

Suitable dosages include, but are not limited to amounts between 0.01 mg/kg and 10 mg/kg. For example, disclosed herein are methods involving administering one or more of the disclosed Apo E mimetics to a subject, wherein the Apo E mimetic is administered in an amount of about 0.15 mg/kg to about 5 mg/kg.

The Apo E mimetic dose can be administered as a bolus injection or as an infusion over one or more hours.

### 4. Apo E mimetics

Disclosed are apolipoprotein-E mimicking peptides or Apo E mimetics. Nonlimiting examples of the Apo E mimetics are provided herein. The Apo E mimetics can be single domain or dual domain peptides. Compositions containing the Apo E mimetics are also disclosed.

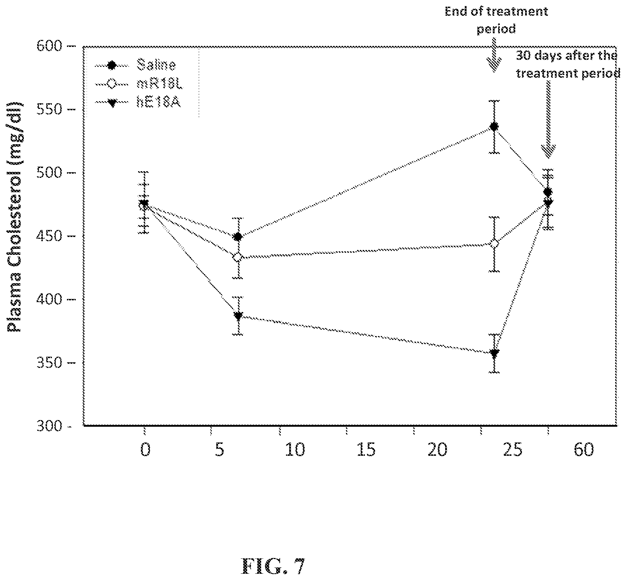

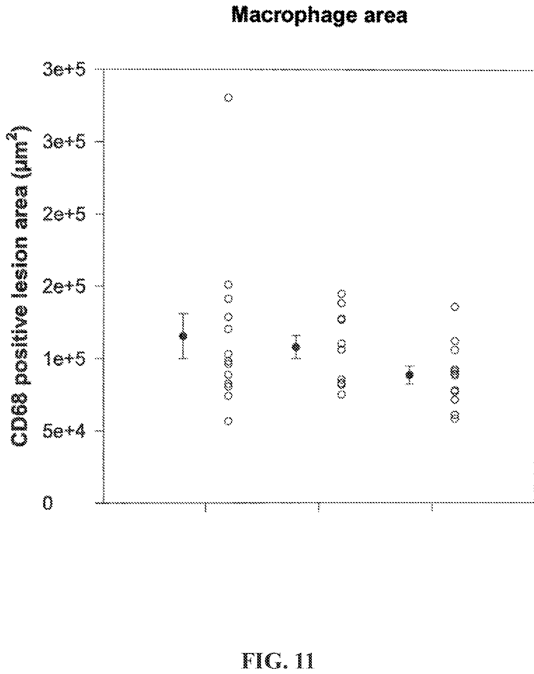

Apolipoprotein E mimetics have both direct cholesterol lowering effects by providing an alternative ligand for receptors on the liver to clear atherogenic Apolipoprotein B containing lipoproteins (LDL, VDLD and β-VLDL), and direct beneficial effects on the artery wall. New, more effective methods of imaging coronary atherosclerosis allow for direct measurement of benefits to the artery wall (Van Velzen, et al. Hellenic J Cardiol 50: 245-263, 2009). The Apo E mimetics can enhance the removal of cholesterol from the artery wall, working in conjunction with HDL, increasing the formation of lipid poor preβ-HDL that accept cholesterol from macrophages. The Apo E mimetics can stimulate macrophage-mediated clearance of dead and dying cells in the artery wall (efferocytosis), improve the quality of HDL by increasing PON-1 levels and bringing down plasma lipid hydroperoxide levels, decrease macrophage content in atherosclerotic lesions resulting in more stable lesions, and decrease inflammation in the artery wall. As a result, the Apo E mimetics reduce the size of atherosclerotic lesions more rapidly and to a far greater extent than the statins (HMG-CoA reductase inhibitors). Figures 7 and 11 show that atherosclerotic lesion regression persists in Apo E mimetic treated animals even when cholesterol levels are the same as in saline treated animals. Thus, the effects cannot be simply explained by cholesterol lowering.

### i. Apolipoprotein E

Apolipoprotein E (Apo E) plays an important role in the metabolism of triglyceride-rich lipoproteins, such as very low density lipoprotein (VLDL) and chylomicrons. Apolipoprotein E mediates the high affinity binding of Apo E-containing lipoproteins to the low density lipoprotein (LDL) receptor (Apo B, E receptor) and the members of its gene family, including LDL receptor related protein (LRP), very low density lipoprotein receptor (VLDLR) and the Apo E2 receptor (Apo E2R) (Mahley, R. W., (1988) Science 240, 622-630). The putative and complex role of Apo E in atherosclerosis has been emphasized by several observations: (i) mice that over express human Apo E have lower levels of total plasma cholesterol levels (Shimono, H. N., et al., (1992) Eur. J. Clin. Invest. 90, 2084-2991), (ii) intravenous injection of human Apo E into cholesterol-fed rabbits protects these animals from atherosclerosis (Yamada, et al., (1989) Proc. Natl. Acad. Sci. U.S.A. 86, 665-669), and (iii) loss of the Apo E gene in mice produces spontaneous atherosclerosis (Zhang, S. H., et al., (1992) Science 258, 468-471) which is ameliorated when macrophage-specific apo E expression is initiated in Apo E-deficient mice (Spangenberg, J., et al., (1997) Biochem. Biophys. Acta 1349, 109-121).

Apo E is a protein that binds lipid and has two major domains (Mahley, R.W., et al. J. Lipid Res. 1999, 40:622-630). The 22 kDa amino terminal domain has been shown by X-ray crystallographic studies to be a 4-helix bundle (Wilson, C., et al. Science 1991;252:1817-1822) and to contain a positively-charged receptor binding domain. For this region to mediate very low-density lipoprotein (VLDL) binding to its receptors, the apolipoprotein must associate with the lipoprotein surface; this is enabled by the C-terminal amphipathic helical region. If the 4-helix bundle that contains the positively charged receptor-binding domain does not open up on the lipoprotein surface, then the VLDL is defective in binding to receptors. Thus, the positively charged arginine (Arg)-rich cluster domain of the Apo E and the C-terminal amphipathic helical domain, are both required for the enhanced uptake of atherogenic Apo E-containing lipoproteins.

Apo E is secreted as a 299 amino acid residue protein with a molecular weight of 34,200. Based on thrombin cleavage of Apo E into two fragments, a two-domain hypothesis was initially suggested to explain the fact that the C-terminal region of Apo E (192-299) is essential for its binding to hypertriglyceridemic VLDL and the N-terminal 22 kDa domain (1-191), binds to the LDL-R (Bradley, W. A., et al., (1986) J. Lipid Res. 27, 40-48). Additional physical-chemical characterization of the protein and its mutants have extended this concept and have shown that the region 192-211 binds to phospholipid while the amino terminal domain (1-191) is a globular structure that contains the LDL receptor binding domain in the 4-helix bundle (Wilson, C., et al., (1991) Science 252, 1817-1822). Studies with synthetic peptides (Sparrow et al. Biochemistry 31(4):1065-8, 1992) and monoclonal antibodies pinpointed the LDL receptor binding domain of apo E between residues 129-169, a domain enriched in positively charged amino acids, Arg and Lys (Rall, S. C., Jr., et al., (1982) PNAS USA 79, 4696-4700; Lalazar, A., et al., (1988) J. Biol. Chem. 263, 3542-2545; Dyer, C. A., et al., (1991) J. Biol. Chem. 296, 22803-22806; and Dyer, C. A., et al., (1991) J. Biol. Chem. 266, 15009-15015).

To test the hypothesis that a minimal arginine-rich Apo E receptor binding domain (141-150) was sufficient to enhance low density lipoprotein (LDL) and very low density lipoprotein (VLDL) uptake and clearance when covalently linked to a class A amphipathic helix, a peptide was synthesized in which the receptor binding domain of human Apo E, LRKLRKRLLR (SEQ ID NO:4) (hApo E[141-150] also referred to as "hE"), was linked to 18A, a well characterized high affinity lipid-associating peptide (DWLKAFYDKVAEKLKEAF (SEQ ID NO:5), also referred to as "18A") to produce a peptide denoted as hApo E[141-150]-18A (also referred to as "hE-18A") (see U.S. Patent No. 6,506,880). Also synthesized was an end protected analog of hE-18A, denoted Ac-hE18A-NH2. The importance of the lysine residues and the role of the hydrophobic residues in the receptor binding domain were also studied using two analogs, LRRLRRRLLR-18A (SEQ ID NO:6) (also referred to as "hE(R)-18A") and LRKMRKRLMR-18A (SEQ ID NO:7) (also referred to as "mE18A"), whereby the receptor binding domain of human Apo E was modified to substitute arginine (R) residues for lysine (K) residues at positions 143 and 146 (LRRLRRRLLR; SEQ ID NO:6) and whereby the receptor binding domain of mouse Apo E (LRKMRKRLMR; SEQ ID NO:7), were linked to 18A, respectively. The effect of the dual character peptides on the uptake and degradation of human LDL/VLDL by cells was then determined.

It was determined that in MEF 1 cells with induced LDL receptors, LDL internalization was enhanced three, five and seven times by Ac-mE-18A-NH2, Ac-hE-18A-NH2, and Ac-hE(R)-18A-NH2 respectively. All three peptides increased degradation of LDL by 100 percent. Both Ac-hE-18A- NH2 and the control peptide Ac-18A- NH2 interacted with VLDL to cause a displacement of apo E from VLDL. However, only Ac-hE-18A- NH2-associated VLDL enhanced the uptake of VLDL six fold and degradation three fold compared to VLDL alone in spite of the absence of apoE. The LDL binding to fibroblasts in the presence of these peptides was not saturable, however, over the LDL concentration range studied.

Furthermore, a similar enhancement of LDL internalization independent of the presence of the LDL receptor related protein (LRP) or LDL receptor or both was seen. Pretreatment of cells with heparinase and heparitinase however abolished greater than 80% of enhanced peptide-mediated LDL uptake and degradation by cells. The data indicated that the dual-domain peptides enhanced LDL uptake and degradation by binding to the LDL through the amphipathic lipid binding domain (18A). However, the minimal 141-150 Arg-rich domain did not decrease LDL levels but did so only in combination with 18A lipid associating domain, did not confer LDL-receptor binding but directed the LDL-peptide complex to the HSPG pathway for uptake and degradation by fibroblasts.

### ii. Single Domain Peptides

Disclosed are single-domain synthetic Apo E mimetics. The single-domain synthetic Apo E mimetics can consist of a receptor binding domain of Apo E or a lipid-associating peptide.

### a. Receptor binding domain peptides

The receptor binding domain peptide for the synthetic Apo E mimetics can be a human receptor binding domain peptide of Apo E. For example, receptor binding domain peptide of the disclosed synthetic Apo E mimetics can comprise the amino acid sequence of LRKLRKRLLR (SEQ ID NO:4), LRRLRRRLLR (SEQ ID NO:6), or LRKLRKRFFR (SEQ ID NO:7). The receptor binding domain peptide of such synthetic Apo E mimetics can also be from a species selected from the group consisting of mouse, rabbit, monkey, rat, bovine, pig and dog.

Examples of receptor binding domain peptides that can be used in the disclosed synthetic Apo E mimetics are provided in Table 1.

| **Table 1 - Disclosed Synthetic Apo E mimetics** | | |
|---|---|---|
| **Species** | **Starting Residue NO:** | **Sequence** |
| Human | 141 | **LR**KLR**KRL**L**R (SEQ ID NO:4)** |
| Rabbit | 134 | **LR**KLR**KRL**L**R (SEQ ID NO:4)** |
| Monkey | 141 | **LR**KLR**KRL**L**R (SEQ ID NO:4)** |
| Mouse | 133 | **LRK***M*R**KRL***M***R (SEQ ID NO:7)** |
| Rat | 133 | **LRK***M*R**KRL***M***R (SEQ ID NO:7)** |
| Bovine | 140 | **LR**KL***P*KRL**L**R (SEQ ID NO:8)** |
| Pig | 140 | **LR***N*V**RKRL***V***R (SEQ ID NO:9)** |
| Dog | 133 | *M*RKLRKR*V*LR (SEQ ID NO: 10) |
| R Modified | 141 | **LR***R*LR*R***RL**L**R (SEQ ID NO:11)** |
| F Modified | 141 | **LR**KLR**KR*FF*R (SEQ ID NO:12)** |
| ApoB | | ***RLTRKRGLK* (SEQ ID NO:13)** |

The italicized residues in Table 1 indicate changes from the human sequence; however, the property of the amino acid is conserved. The bold-italicized residues in Table 1 indicate the difference from the human sequence at that position.

The receptor binding domain peptide for the synthetic Apo E mimetics can also be the LDL receptor (LDLR) binding domain of apolipoprotein B (ApoB). The LDL receptor (LDLR) binding domain of ApoB can have the sequence RLTRKRGLK (SEQ ID NO:13). ApoB-100 is a 550,000 Da glycoprotein with nine amino acids (3359-3367) serving as the binding domain for the LDL receptor (Segrest et al., J. Lipid. Res. 42, pp. 1346-1367 (2001)). Upon binding to LDLR in clathrin coated pits, LDL is internalized via endocytosis and moves into the endosome where a drop in pH causes the receptor to dissociate from the LDL. The receptor is recycled back to the surface of the cell while the LDL is moved into the lysosome where the particle is degraded (Goldstein et al., Ann. Rev. Cell Biol. 1, pp. 1-39 (1985)). The LDL receptor (LDLR) binding domain of ApoB when used with the disclosed peptides can also be altered and/or modified as described throughout this application for Apo E. For example, LDL receptor (LDLR) binding domain of ApoB can be used with the disclosed lipid-associating peptides, wherein the LDL receptor (LDLR) binding domain of ApoB is covalently linked to said lipid-associating peptide. In addition, the LDL receptor (LDLR) binding domain of ApoB can be scrambled, reverse-oriented, can be part of a domain switched peptide as described below.

### b. Lipid-Associating Peptides

Lipid-associating peptides can be used alone or in combination with the Apo E-mimicking peptides. The lipid associating peptide for these synthetic Apo E mimetics can be, but are not limited to, class A amphipathic helical peptides, class A amphipathic helical peptide mimetics of apoA-I having aromatic or aliphatic residues in the non-polar face, small peptides including pentapeptides, tetrapeptides, tripeptides, dipeptides and pairs of amino acids, Apo-J (G* peptides), and peptide mimetics, e.g., as described below.

### (A) Class A Amphipathic Helical Peptides

In one aspect, the lipid-associating peptides for use in the disclosed methods include class A amphipathic helical peptides, e.g. as described in U.S. Pat. No. 6,664,230, and PCT Publications WO 02/15923 and WO 2004/034977. It was discovered that peptides comprising a class A amphipathic helix ("class A peptides"), are capable of mitigating one or more symptoms of atherosclerosis as well as treating other disorders.

Class A peptides are characterized by formation of an α-helix that produces a segregation of polar and non-polar residues thereby forming a polar and a nonpolar face with the positively charged residues residing at the polar-nonpolar interface and the negatively charged residues residing at the center of the polar face (see, e.g., Anantharamaiah (1986) Meth. Enzymol, 128: 626-668). It is noted that the fourth exon of apo A-I, when folded into 3.667 residues/turn produces a class A amphipathic helical structure.

One class A peptide, designated 18A (see, e.g., Anantharamaiah (1986) Meth. Enzymol, 128: 626-668) was modified as described herein to produce peptides orally administrable and highly effective at inhibiting or preventing one or more symptoms of atherosclerosis and/or other indications described herein. Without being bound by a particular theory, it is believed that the disclosed peptides can act in vivo by picking up seeding molecule(s) that mitigate oxidation of LDL.

Increasing the number of Phe residues on the hydrophobic face of 18A can increase lipid affinity as determined by the computation described by Palgunachari et al. (1996) Arteriosclerosis, Thrombosis, & Vascular Biol. 16: 328-338. Theoretically, a systematic substitution of residues in the nonpolar face of 18A with Phe could yield six peptides. Peptides with an additional 2, 3 and 4 Phe would have theoretical lipid affinity (λ) values of 13, 14 and 15 units, respectively. However, the λ values jumped four units if the additional Phe were increased from 4 to 5 (to 19 λ units). Increasing to 6 or 7 Phe would produce a less dramatic increase (to 20 and 21 λ units, respectively).

A number of these class A peptides were made including, the peptide designated 4F, D4F, 5F, and D5F, and the like. Various class A peptides inhibited lesion development in atherosclerosis-susceptible mice. In addition, the peptides show varying, but significant degrees of efficacy in mitigating one or more symptoms of the various pathologies described herein. A number of such peptides are illustrated in Table 2.

**Table 2: Class A peptides**

| Peptide Name | Amino Acid Sequence |
|---|---|
| 18F | D-W-L-K-A-F-Y-D-K-V-A-E-K-L-K-E-A-F (SEQ ID NO:5) |
| 2F | |
| 3F | |
| 3F14 | |
| 4F | |
| 5F | |
| 6F | |
| 7F | |
| | |
| | |
| | |
| | |
| | |
| | |
| | |
| | |
| | |
| | |
| | |
| | |
| | Ac-A-F-Y-D-K-V-A-E-K-L-K-E-A-F-NH₂ (SEQ ID NO:32) |
| | Ac-A-F-Y-D-K-V-A-E-K-F-K-E-A-F-NH₂ (SEQ ID NO:33) |
| | Ac-A-F-Y-D-K-V-A-E-K-F-K-E-A-F-NH₂ (SEQ ID NO:34) |
| | Ac-A-F-Y-D-K-F-F-E-K-F-K-E-F-F-NH₂ (SEQ ID NO:35) |
| | Ac-A-F-Y-D-K-F-F-E-K-F-K-E-F-F-NH₂ (SEQ ID NO:36) |
| | Ac-A-F-Y-D-K-V-A-E-K-F-K-E-A-F-NH₂ (SEQ ID NO:37) |
| | Ac-A-F-Y-D-K-V-A-E-K-L-K-E-F-F-NH₂ (SEQ ID NO:38) |
| | Ac-A-F-Y-D-K-V-F-E-K-F-K-E-A-F-NH₂ (SEQ ID NO:39) |
| | Ac-A-F-Y-D-K-V-F-E-K-L-K-E-F-F-NH₂ (SEQ ID NO:40) |
| | Ac-A-F-Y-D-K-V-A-E-K-F-K-E-F-F-NH₂ (SEQ ID NO:41) |
| | Ac-K-A-F-Y-D-K-V-F-E-K-F-K-E-F-NH₂ (SEQ ID NO:42) |
| | Ac-L-F-Y-E-K-V-L-E-K-F-K-E-A-F-NH₂ (SEQ ID NO:43) |
| | Ac-A-F-Y-D-K-V-A-E-K-F-K-E-A-F-NH₂ (SEQ ID NO:44) |
| | Ac-A-F-Y-D-K-V-A-E-K-L-K-E-F-F-NH₂ (SEQ ID NO:45) |
| | Ac-A-F-Y-D-K-V-F-E-K-F-K-E-A-F-NH₂ (SEQ ID NO:46) |
| | Ac-A-F-Y-D-K-V-F-E-K-L-K-E-F-F-NH₂ (SEQ ID NO:47) |
| | Ac-A-F-Y-D-K-V-A-E-K-F-K-E-F-F-NH₂ (SEQ ID NO:48) |
| | Ac-A-F-Y-D-K-V-F-E-K-F-K-E-F-F NH₂ (SEQ ID NO:49) |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | |
| | Ac-D-W-F-K-A-F-Y-D-K-V-A-E-K-F-NH₂ (SEQ ID NO:98) |
| | Ac-F-K-A-F-Y-D-K-V-A-E-K-F-K-E-NH₂ (SEQ ID NO:99) |
| | Ac-F-K-A-F-Y-E-K-V-A-E-K-F-K-E-NH₂ (SEQ ID NO:100) |
| | NMA-F-K-A-F-Y-D-K-V-A-E-K-F-K-E-NH₂ (SEQ ID NO:101) |
| | NMA-F-K-A-F-Y-E-K-V-A-E-K-F-K-E-NH₂ (SEQ ID NO:102) |
| | |
| | |
| | NMA-A-F-Y-D-K-V-A-E-K-F-K-E-A-F-NH₂ (SEQ ID NO:105) |
| | NMA-D-W-F-K-A-F-Y-D-K-V-A-E-K-F-NH₂ (SEQ ID NO:106) |
| | |
| | |
| | |
| | |
| | Ac-A-F-Y-D-K-V-F-E-K-F-K-E-F-F-NH₂ (SEQ ID NO:111) |
| | NMA-A-F-Y-D-K-V-F-E-K-F-K-E-F-F-NH₂ (SEQ ID NO:112) |
| | Ac-A-F-Y-E-K-V-F-E-K-F-K-E-F-F-NH₂ (SEQ ID NO:113) |
| | NMA-A-F-Y-E-K-V-F-E-K-F-K-E-F-F-NH₂ (SEQ ID NO:114) |
| | Ac-D-W-L-K-A-F-Y-D-K-V-F-E-K-F-NH₂ (SEQ ID NO:115) |
| | NMA-D-W-L-K-A-F-Y-D-K-V-F-E-K-F-NH₂ (SEQ ID NO:116) |
| | Ac-E-W-L-K-A-F-Y-E-K-V-F-E-K-F-NH₂ (SEQ ID NO:117) |
| | NMA-E-W-L-K-A-F-Y-E-K-V-F-E-K-F-NH₂ (SEQ ID NO:118) |
| | Ac-L-K-A-F-Y-D-K-V-F-E-K-F-K-E-NH₂ (SEQ ID NO:119) |
| | NMA-L-K-A-F-Y-D-K-V-F-E-K-F-K-E-NH₂ (SEQ ID NO:120) |
| | Ac-L-K-A-F-Y-E-K-V-F-E-K-F-K-E-NH₂ (SEQ ID NO:121) |
| | NMA-L-K-A-F-Y-E-K-V-F-E-K-F-K-E-NH₂ (SEQ ID NO:122) |

| | |
|---|---|
| *Linkers are underlined. NMA is N-Methyl Anthranilyl | |

In certain aspects, the peptides include variations of 4F (D-W-F-K-A-F-Y-D-K-V-A-E-K-F-K-E-A-F (SEQ ID NO:16) in Table 2), also known as L-4F, where all residues are L form amino acids) or D-4F where one or more residues are D form amino acids). In any of the peptides described herein, the C-terminus, and/or N-terminus, and/or internal residues can be blocked with one or more blocking groups as described herein.

While various peptides of Table 2, are illustrated with an acetyl group or an N-methylanthranilyl group protecting the amino terminus and an amide group protecting the carboxyl terminus, any of these protecting groups may be eliminated and/or substituted with another protecting group as described herein. The peptides can comprise one or more D-form amino acids as described herein. In certain aspects, every amino acid (e.g., every enantiomeric amino acid) of the peptides of Table 2 is a D-form amino acid.

It is also noted that Table 2 is not fully inclusive. Using the teachings provided herein, other suitable class A amphipathic helical peptides can routinely be produced (e.g., by conservative or semi-conservative substitutions (e.g., D replaced by E), extensions, deletions, and the like). Thus, for example, one embodiment utilizes truncations of any one or more of peptides shown herein (e.g., peptides identified as 2F, 3F, 3F14, 4F, 5F, 6F, or 7F--in Table 2). Thus, for example, A-F-Y-D-K-V-A-E-K-L-K-E-A-F (amino acids 5-18 of SEQ ID NO:5) illustrates a peptide comprising 14 amino acids from the C-terminus of 18A comprising one or more D amino acids, while others illustrate other truncations. Longer peptides are also suitable. Such longer peptides may entirely form a class A amphipathic helix, or the class A amphipathic helix (helices) can form one or more domains of the peptide. In addition, this invention contemplates multimeric versions of the peptides (e.g., concatamers). Thus, for example, the peptides illustrated herein can be coupled together (directly or through a linker (e.g., a carbon linker, or one or more amino acids) with one or more intervening amino acids). Illustrative polymeric peptides include 18A-Pro-18A and the peptides in the following table, in certain embodiments comprising one or more D amino acids, more preferably with every amino acid a D amino acid as described herein and/or having one or both termini protected.

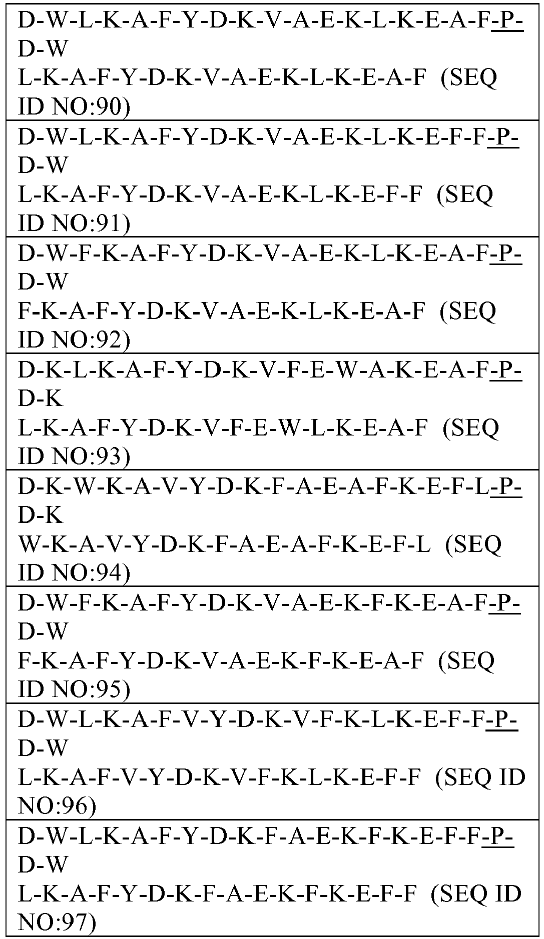

### (B) Class A Amphipathic Helical Peptide Mimetics of apoA-I Having Aromatic or Aliphatic Residues in the Non-Polar Face.

Also disclosed are modified class A amphipathic helix peptides. Certain preferred peptides incorporate one or more aromatic residues at the center of the nonpolar face, e.g., 3F^{Cπ}, (as present in 4F), or with one or more aliphatic residues at the center of the nonpolar face, e.g., 3F^{Iπ}, see, e.g., Table 3. Without being bound to a particular theory, the central aromatic residues on the nonpolar face of the peptide 3F^{Cπ}, due to the presence of π electrons at the center of the nonpolar face can allow water molecules to penetrate near the hydrophobic lipid alkyl chains of the peptide-lipid complex, which in turn would enable the entry of reactive oxygen species (such as lipid hydroperoxides) shielding them from the cell surface. The peptides with aliphatic residues at the center of the nonpolar face, e.g., 3F^{Iπ}, can act similarly but not quite as effectively as 3F^{Cπ}.

In one aspect, the peptides can convert pro-inflammatory HDL to anti-inflammatory HDL or make anti-inflammatory HDL more anti-inflammatory, and/or decrease LDL-induced monocyte chemotactic activity generated by artery wall cells equal to or greater than D4F or other peptides shown in Table 2.

**Table 3: Modified class A peptides**

| **Name** | **Sequence** |
|---|---|
| (3F^{cn}) | Ac-DKWKAVYDKFAEAFKEFL-NH₂ (SEQ ID NO:123) |
| (3F^{ln}) | Ac-DKLKAFYDKVFEWAKEAF-NH₂ (SEQ ID NO:124) |

### (C) Other class A and some Class Y Amphipathic Helical Peptides.

Class A amphipathic helical peptides that have an amino acid composition identical to one or more of the class A amphipathic helical peptides described above. Thus, for example, in certain embodiments this invention contemplates peptides having an amino acid composition identical to 4F. Thus, in certain embodiments, this invention includes active agents that comprise a peptide that consists of 18 amino acids, where the 18 amino acids consist of 3 alanines (A), 2 aspartates (D), 2 glutamates (E), 4 phenylalanines (F), 4 lysines (K), 1 valine (V), 1 tryptophan (W), and 1 tyrosine (Y); and where the peptide forms a class A amphipathic helix; and protects a phospholipid against oxidation by an oxidizing agent. In various embodiments, the peptides comprise least one "D" amino acid residue; and in certain embodiments, the peptides comprise all "D: form amino acid residues. A variety of such peptides are illustrated in Table 4. Reverse (retro-), inverse, retro-inverso-, and circularly permuted forms of these peptides are also contemplated.

**Table 4. Illustrative 18 amino acid length class A amphipathic helical peptides with the amino acid composition 3 alanines (A), 2 aspartates (D), 2 glutamates (E), 4 phenylalanines (F), 4 lysines (K), 1 valine (V), 1 tryptophan (W), and 1 tyrosine (Y).**

| Name | Sequence |
|---|---|
| | |
| [Switch D-E]-4F analogs | |
| [Switch D-E]-1-4F | Ac-**E**WFKAFY**E**KVA**D**KFK**D**AF-NH₂ (SEQ ID NO:125) |
| [Switch D-E]-2-4F | Ac-**E**WFKAFYDKVADKFK**E**AF-NH₂ (SEQ ID NO:126) |
| [Switch D-E]-3-4F | Ac-DWFKAFY**E**KVA**D**KFKEAF-NH₂ (SEQ ID NO:127) |
| [Switch D-E]-4-4F | Ac-DWFKAFY**E**KVAEKFK**D**AF-NH₂ (SEQ ID NO:128) |

| [W-2,F-3 positions reversed] | |
|---|---|
| 4F-2 | Ac-D**FW**KAFYDKVAEKFKEAF-NH₂ (SEQ ID NO:129) |
| [Switch D-E]-1-4F-2 | Ac-**E**FWKAFY**E**KVA**D**KFK**D**AF-NH₂ (SEQ ID NO:130) |
| [Switch D-E]-2-4F-2 | Ac-**E**FWKAFYDKVADKFK**E**AF-NH₂ (SEQ ID NO:131) |
| [Switch D-E]-3-4F-2 | Ac-DFWKAFY**E**KVA**D**KFKEAF-NH₂ (SEQ ID NO:132) |
| [Switch D-E]-4-4F-2 | Ac-DFWKAFY**E**KVAEKFK**D**AF-NH₂ (SEQ ID NO:133) |

| [F-6 and Y-7 positions switched] | |
|---|---|
| 4F-3 | Ac-DWFKA**YF**DKVAEKFKEAF-NH₂ (SEQ ID NO:134) |
| [Switch D-E]-1-4F-5 | Ac-**E**WFKAYF**E**KVA**D**KFK**D**AF-NH₂ (SEQ ID NO:135) |
| [Switch D-E]-2-4F-5 | Ac-**E**WFKAYFDKVADKFK**E**AF-NH₂ (SEQ ID NO:136) |
| [Switch D-E]-3-4F-5 | Ac-DWFKAYF**E**KVA**D**KFKEAF-NH₂ (SEQ ID NO:137) |
| [Switch D-E]-4-4F-5 | Ac-DWFKAYFEKVA**E**KFK**D**AF-NH₂ (SEQ ID NO:138) |

| [Y-land 10V positions switched] | |
|---|---|
| 4F-4 | Ac-DWFKAF**V**DK**Y**AEKFKEAF-NH₂ (SEQ ID NO:139) |
| [Switch D-E]-1-4F-4 | Ac-**E**WFKAFV**E**KYA**D**KFK**D**AF-NH₂ (SEQ IDN O:140) |
| [Switch D-E]-2-4F-4 | Ac-**E**WFKAFVDKYADKFK**E**AF-NH₂ (SEQ ID NO:141) |
| [Switch D-E]-3-4F-4 | Ac-DWFKAFV**E**KYA**D**KFKEAF-NH₂ (SEQ ID NO:142) |
| [Switch D-E]-4-4F | Ac-DWFKAFV**E**KYAEKFK**D**AF-NH₂ (SEQ ID NO: 143) |

| [V-10 and A-11 switched] | |
|---|---|
| 4-F-5 | Ac-DWFKAFYDK**AV**EKFKEAF-NH₂ (SEQ ID NO:144) |
| [Switch D-E]-1-4F-5 | Ac-**E**WFKAFY**E**KAV**D**KFK**D**AF-NH₂ (SEQ ID NO:145) |
| [Switch D-E]-2-4F-5 | Ac-**E**WFKAFYDKAVDKFK**E**AF-NH₂ (SEQ ID NO:146) |
| [Switch D-E]-3-4F-5 | Ac-DWFKAFY**E**KAV**D**KFKEAF-NH₂ (SEQ ID NO:147) |
| [Switch D-E]-4-4F-5 | Ac-DWFKAFY**E**KAVEKFK**D**AF-NH₂ (SEQ ID NO:148) |

| [A-11 and F-14 switched] | |
|---|---|
| 4F-6 | Ac-DWFKAFYDKV**F**EK**A**KEAF-NH₂ (SEQ ID NO:149) |
| [Switch D-E]-1-4F-6 | Ac-**E**WFKAFY**E**KVF**D**KAK**D**AF-NF1₂ (SEQ ID NO:150) |
| [Switch D-E]-2-4F-6 | Ac-**E**WFKAFYDKVFDKAK**E**AF-NH₂ (SEQ ID NO:151) |
| [Switch D-E]-3-4F-6 | Ac-DWFKAFY**E**KVF**D**KAKEAF-NH₂ (SEQ ID NO:152) |
| [Switch D-E]-4-4F-6 | Ac-DWFKAFY**E**KVFEKAK**D**AF-NH₂ (SEQ ID NO:153) |

| [F-14 and A-17 switched] | |
|---|---|
| 4F-7 | Ac-DWFKAFYDKVAEK**A**KE**F**F-NH₂ (SEQ ID NO:154) |
| [Switch D-E]-1-4F-7 | Ac-**E**WFKAFY**E**KVA**D**KAK**D**FF-NH₂ (SEQ ID NO:155) |
| [Switch D-E]-2-4F-7 | Ac-**E**WFKAFYDKVADKAK**E**FF-NH₂ (SEQ ID NO:156) |
| [Switch D-E]-3-4F-7 | Ac-DWFKAFY**E**KVA**D**KAKEFF-NH₂ (SEQ ID NO:157) |
| [Switch D-E]-4-4F-7 | Ac-DWFKAFY**E**KVAEKAK**D**FF-NH₂ (SEQ ID NO:158) |

| [A-17 and F-18 switched] | |
|---|---|
| 4F-8 | Ac-DWFKAFYDKVAEKFKE**FA**-NH₂ (SEQ ID NO:159) |
| [Switch D-E]-1-4F-8 | Ac-**E**WFKAFY**E**KVA**D**KFK**D**FA-NH₂ (SEQ ID NO:160) |
| [Switch D-E]-2-4F-8 | Ac-**E**WFKAFYDKVADKFK**E**FA-NH2 (SEQ ID NO:161) |
| [Switch D-E]-3-4F-8 | Ac-DWFKAFY**E**KVA**D**KFKEFA-NH₂ (SEQ ID NO:162) |
| [Switch D-E]-4-4F-8 | Ac-DWFKAFY**E**KVAEKFK**D**FA-NH₂ (SEQ ID NO:163) |

| [W-2 and A-17 switched] | |
|---|---|
| 4F-9 | Ac-D**A**FKAFYDKVAEKFKE**W**F-NH₂ (SEQ ID NO:164) |
| [Switch D-E]-1-4F-9 | Ac-**E**AFKAFY**E**KVA**D**KFK**D**WF-NH₂ (SEQ ID NO:165) |
| [Switch D-E]-2-4F-9 | Ac-**E**AFKAFYDKVADKFK**E**WF-NH₂ (SEQ ID NO:166) |
| [Switch D-E]-3-4F-9 | Ac-DAFKAFY**E**KVA**D**KFKEWF-NH₂ (SEQ ID NO:167) |
| [Switch D-E]-4-4F-9 | Ac-DAFKAFY**E**KVAEKFK**D**WF-NH₂ (SEQ ID NO:168) |

| [W-2 and A-11 switched] | |
|---|---|
| 4F-10 | Ac-D**A**FKAFYDKV**W**EKFKEAF-NH₂ (SEQ ID NO:169) |
| [Switch D-E]-1-4F-10 | Ac-**E**AFKAFY**E**KVW**D**KFK**D**AF-NH₂ (SEQ ID NO:170) |
| [Switch D-E]-2-4F-10 | Ac-**E**AFKAFYDKVWDKFK**E**AF-NH₂ (SEQ ID NO:171) |
| [Switch D-E]-3-4F-10 | Ac-DAFKAFY**E**KVW**D**KFKEAF-NH₂ (SEQ ID NO:172) |
| [Switch D-E]-4-4F-10 | Ac-DAFKAFY**E**KVWEKFK**D**AF-NH₂ (SEQ ID NO:173) |

| [W-2 and Y-7 switched] | |
|---|---|
| 4F-11 | Ac-D**Y**FKAF**W**DKVAEKFKEAF-NH₂ (SEQ ID NO:174) |
| [Switch D-E]-1-4F-11 | Ac-**E**YFKAFW**E**KVA**D**KFK**D**AF-NH₂ (SEQ ID NO:175) |
| [Switch D-E]-2-4F-11 | Ac-**E**YFKAFWDKVADKFK**E**AF-NH₂ (SEQ ID NO:176) |
| [Switch D-E]-3-4F-11 | Ac-DYFKAFW**E**KVA**D**KFKEAF-NH₂ (SEQ ID NO:177) |
| [Switch D-E]-4-4F-11 | Ac-DYFKAFW**E**KVAEKFK**D**AF-NH₂ (SEQ ID NO:178) |

| [F-3 and A-17 switched] | |
|---|---|
| 4F-12 | Ac-DW**A**KAFYDKVAEKFKE**F**F-NH₂ (SEQ ID NO:179) |
| [Switch D-E]-1-4F-12 | Ac-**E**WAKAFY**E**KVA**D**KFK**D**FF-NH₂ (SEQ ID NO:180) |
| [Switch D-E]-2-4F-12 | Ac-**E**WAKAFYDKVADKFK**E**FF-NH₂ (SEQ ID NO:181) |
| [Switch D-E]-3-4F-12 | Ac-DWAKAFY**E**KVA**D**KFKEFF-NH₂ (SEQ ID NO:182) |
| [Switch D-E]-4-4F-12 | Ac-DWAKAFY**E**KVAEKFK**D**FF-NH₂ (SEQ ID NO:183) |

| [F-6 and A-17 switched] | |
|---|---|
| 4F-13 | Ac-DWFKA**A**YDKVAEKFKE**F**F-NH₂ (SEQ ID NO:184) |
| [Switch D-E]-1-4F-13 | Ac-**E**WFKAAY**E**KVA**D**KFK**D**FF-NH₂ (SEQ ID NO:185) |
| [Switch D-E] -2-4F-13 | Ac-**E**WFKAAYDKVADKFK**E**FF-NH₂ (SEQ ID NO:186) |
| [Switch D-E]-3-4F-13 | Ac-DWFKAAY**E**KVA**D**KFKEFF-NH₂ (SEQ ID NO:187) |
| [Switch D-E]-4-4F-13 | Ac-DWFKAAY**E**KVAEKFK**D**FF-NH₂ (SEQ I DNO:188) |

| [Y-7 and A-17 switched | |
|---|---|
| 4F-14 | Ac-DWFKAF**A**DKVAEKFKE**Y**F-NH₂ (SEQ ID NO:189) |
| [Switch D-E]-1-4F-14 | Ac-**E**WFKAFA**E**KVA**D**KFK**D**YF-NH₂ (SEQ ID NO:190) |
| [Switch D-E]-2-4F-14 | Ac-**E**WFKAFADKVADKFK**E**YF-NH₂ (SEQ ID NO:191) |
| [Switch D-E]-3-4F-14 | Ac-DWFKAFA**E**KVA**D**KFKEYF-NH₂ (SEQ ID NO:192) |
| [Switch D-E] -4-4F | Ac-DWFKAFA**E**KVAEKFK**D**YF-NH₂ (SEQ ID NO:193) |

| [V-10 and A-17 switched] | |
|---|---|
| 4F-15 | Ac-DWFKAFYDK**A**AEKFKE**V**F-NH₂ (SEQ ID NO:194) |
| [Switch D-E]-1-4F-15 | Ac-**E**WFKAFY**E**KAA**D**KFK**D**VF-NH₂ (SEQ ID NO:195) |
| [Switch D-E]-2-4F-15 | Ac-**E**WFKAFYDKAADKFK**E**VF-NH₂ (SEQ ID NO:196) |
| [Switch D-E]-3-4F-15 | Ac-DWFKAFY**E**KAA**D**KFKEVF-NH₂ (SEQ ID NO:197) |
| [Switch D-E]-4-4F-15 | Ac-DWFKAFY**E**KAAEKFK**D**VF-NH₂ (SEQ ID NO:198) |

| [F3 and Y-7 switched] | |
|---|---|
| 4F-16 | Ac-DW**Y**KAF**F**DKVAEKFKEAF-NH₂ (SEQ ID NO:199) |
| [Switch D-E]-1-4F-16 | Ac-**E**WYKAFF**E**KVA**D**KFK**D**AF-NH₂ (SEQ ID NO:200) |
| [Switch D-E]-2-4F-16 | Ac-**E**WYKAFFDKVADKFK**E**AF-NH₂ (SEQ ID NO:201) |
| [Switch D-E]-3-4F-16 | Ac-DWYKAFF**E**KVA**D**KFKEAF-NH₂ (SEQ ID NO:202) |
| [Switch D-E]-4-4F-16 | Ac-DWYKAFF**E**KVAEKFK**D**AF-NH₂ (SEQ ID NO:203) |

| [F-3 and V-10 switched] | |
|---|---|
| 4F-17 | Ac-DW**V**KAFYDK**F**AEKFKEAF-NH₂ (SEQ ID NO:204) |
| [Switch D-E]-1-4F-17 | Ac-**E**WVKAFY**E**KFA**D**KFK**D**AF-NH₂ (SEQ ID NO:205) |
| [Switch D-E]-2-4F-17 | Ac-**E**WVKAFYDKFADKFK**E**AF-NH₂ (SEQ ID NO:206) |
| [Switch D-E]-3-4F-17 | Ac-DWVKAFY**E**KFA**D**KFKEAF-NH₂ (SEQ ID NO:207) |
| [Switch D-E]-4-4F-17 | Ac-DWVKAFY**E**KFAEKFK**D**AF-NH₂ (SEQ ID NO:208) |

| [Y-7 and F-14 switched] | |
|---|---|
| 4F-18 | Ac-DWFKA**F**FDKVAEK**Y**KEAF-NH₂ (SEQ ID NO: 209) |
| [Switch D-E]-1-4F-18 | Ac-**E**WFKAFF**E**KVA**D**KYK**D**AF-NH₂ (SEQ ID NO:210) |
| [Switch D-E]-2-4F-18 | Ac-**E**WFKAFFDKVADKYK**E**AF-NH₂ (SEQ ID NO:211) |
| [Switch D-E]-3-4F-18 | Ac-DWFKAFF**E**KVA**D**KYKEAF-NH₂ (SEQ ID NO:212) |
| [Switch D-E]-3-4F-18 | Ac-DWFKAFF**E**KVA**D**KYKEAF-NH₂ (SEQ ID NO:213) |

| [Y-7 and F-18 switched] | |
|---|---|
| 4F-19 | Ac-DWFKAF**F**DKVAEKFKEA**Y**-NH₂ (SEQ ID NO:214) |
| [Switch D-E]-1-4F-19 | Ac-**E**WFKAFF**E**KVA**D**KFK**D**AY-NH₂ (SEQ ID NO:215) |
| [Switch D-E]-2-4F-19 | Ac-**E**WFKAFFDKVADKFK**E**AY-NH₂ (SEQ ID NO:216) |
| [Switch D-E]-3-4F-19 | Ac-DWFKAFF**E**KVA**D**KFKEAY-NH₂ (SEQ ID NO:217) |
| [Switch D-E]-4-4F-19 | Ac-DWFKAFF**E**KVAEKFK**D**AY-NH2 (SEQ ID NO:218) |

| [V-10 and F-18 switched] | |
|---|---|
| 4F-20 | Ac-DWFKAFYDK**F**AEKFKEA**V**-NH₂ (SEQ ID NO:219) |
| [Switch D-E]-1-4F-20 | Ac-**E**WFKAFY**E**KFA**D**KFK**D**AV-NH₂ (SEQ ID NO:220) |
| [Switch D-E]-2-4F-20 | Ac-**E**WFKAFYDKFADKFK**E**AV-NH₂ (SEQ ID NO:221) |
| [Switch D-E]-3-4F-20 | Ac-DWFKAFY**E**KFA**D**KFKEAV-NH₂ (SEQ ID NO:222) |
| [Switch D-E]-4-4F-20 | Ac-DWFKAFY**E**KFAEKFK**D**AV-NH₂ (SEQ ID NO:223) |

| [W-2 and K13 switched] | |
|---|---|
| 4F-21 | Ac-D**K**FKAFYDKVAEKF**W**EAF-NH₂ (SEQ ID NO:224) |
| [Switch D-E]-1-4F-21 | Ac-**E**KFKAFY**E**KVA**D**KFW**D**AF-NH₂ (SEQ ID NO:225) |
| [Switch D-E]-2-4F-21 | Ac-**E**KFKAFYDKVADKFW**E**AF-NH₂ (SEQ ID NO:226) |
| [Switch D-E]-3-4F-21 | Ac-DKFKAFY**E**KVA**D**KFWEAF-NH2 (SEQ ID NO:227) |
| [Switch D-E]-4-4F-21 | Ac-DKFKAFY**E**KVAEKFW**D**AF-NH₂ (SEQ ID NO:228) |

| [W-3, F-13 and K-2 4F] | |
|---|---|
| 4F-22 | Ac-D**KW**KAFYDKVAEKF**F**EAF-NH₂ (SEQ ID NO:229) |
| [Switch D-E]-1-4F-22 | Ac-**E**KWKAFY**E**KVA**D**KFF**D**AF-NH₂ (SEQ ID NO:230) |
| [Switch D-E]-2-4F-22 | Ac-**E**KWKAFYDKVADKFF**E**AF-NH₂ (SEQ ID NO:231) |
| [Switch D-E]-3-4F-22 | Ac-DKWKAFY**E**KVA**D**KFFEAF-NH₂ (SEQ ID NO:232) |
| [Switch D-E]-4-4F-22 | Ac-DKWKAFYEKVA**E**KFF**D**AF-NH₂ (SEQ ID NO:233) |

| [K-2, W10, V-13] | |
|---|---|
| 4F-23 | Ac-DKF**K**AFYDK**W**AE**V**FKEAF-NH₂ (SEQ ID NO:234) |

| [Switch D-E]-4F analogs | |
|---|---|
| [Switch D-E]-1-4F-23 | Ac-**E**KFKAFY**E**KWA**D**VFK**D**AF-NH₂ (SEQ ID NO:235) |
| [Switch D-E]-2-4F-23 | Ac-EKFKAFYDKWADVFK**E**AF-NH₂ (SEQ ID NO:236) |
| [Switch D-E]-3-4F-23 | Ac-DKFKAFY**E**KWA**D**VFKEAF-NH₂ (SEQ ID NO:237) |
| [Switch D-E]-4-4F-23 | Ac-DKFKAFY**E**KWAEVFK**D**AF-NH₂ (SEQ ID NO:238) |

| [K-2, F-13, W-14 4F] | |
|---|---|
| 4F-24 | Ac-D**K**FKAFYDKVAE**FW**KEAF-NH₂ (SEQ ID NO:239) |

| [Switch D-E]-4F analogs | |
|---|---|
| [Switch D-E]-1-4F-24 | Ac-**E**KFKAFY**E**KVA**D**FWK**D**AF-NH₂ (SEQ ID NO:240) |
| [Switch D-E]-2-4F-24 | Ac-**E**KF1CAFYDKVADFWK**E**AF-NH₂ (SEQ ID NO:241) |
| [Switch D-E]-3-4F-24 | Ac-DKFKAFY**E**KVA**D**FWKEAF-NH₂ (SEQ ID NO:242) |
| [Switch D-E]-4-4F-24 | Ac-DKFKAFY**E**KVAEFWK**D**AF-NH₂ (SEQ ID NO:243) |

| Reverse 4F analogs | |
|---|---|
| Rev-4F | Ac-FAEKFKEAVKDYFAKFWD-NH₂ (SEQ ID NO:244) |
| [Switch D-E]-1-Rev-4F | Ac-FA**D**KFK**D**AVK**E**YFAKFW**E**-NH₂ (SEQ ID NO:245) |
| [Switch D-E]-2-Rev-4F | Ac-FA**D**KFKEAVKDYFAKFW**E**-NH₂ (SEQ ID NO:246) |
| [Switch D-E]-3-Rev-4F | Ac-FAEKFK**D**AVKEYFAKFWD-NH₂ (SEQ ID NO:247) |
| [Switch D-E]-4-Rev-4F | Ac-FAEKFK**D**AVKDYFAKFW**E**-NH₂ (SEQ ID NO:248) |

| [A-2 and W-17 switched] | |
|---|---|
| Rev-4F-1 | Ac-F**W**EKFKEAVKDYFAKF**A**D-NH₂ (SEQ ID NO:249) |
| [Switch D-E]-1-Rev-4F-1 | Ac-FW**D**KFK**D**AVK**E**YFAKFA**E**-NH₂ (SEQ ID NO:250) |
| [Switch D-E]-2-Rev-4F-1 | Ac-FA**D**KFKEAVKDYFAKFW**E**-NH₂ (SEQ ID NO:251) |
| [Switch D-E]-3-Rev-4F-1 | Ac-FAEKFK**D**AVK**E**YFAKFWD-NH₂ (SEQ ID NO:252) |
| [Switch D-E]-4-Rev-4F-1 | Ac-FAEKFK**D**AVKDYFAKFW**E**-NH₂ (SEQ ID NO:253) |

| [Switch A-2 and F-16] | |
|---|---|
| Rev-4F-2 | Ac-F**F**EKFKEAVKDYFAK**A**WD-NH₂ (SEQ ID NO:254) |
| [Switch D-E]-1-Rev-4F-2 | Ac-FF**D**KFK**D**AVK**E**YFAKA W**E**-NH₂ (SEQ ID NO:255) |
| [Switch D-E]-2-Rev-4F-2 | Ac-FF**D**KFKEAVKDYFAKAW**E**-NH₂ (SEQ ID NO:256) |
| [Switch D-E]-3-Rev-4F-2 | Ac-FFEKFK**D**AVK**E**YFAKAWD-NH₂ (SEQ ID NO:257) |
| [Switch D-E]-4-Rev-4F-2 | Ac-FFEKFK**D**AVKDYFAKAW**E**-NH₂ (SEQ ID NO:258) |

| [switch F-5 and A-8] | |
|---|---|
| Rev-4F-3 | Ac-FAEK**A**KE**F**VKDYFAKFWD-NH₂ (SEQ ID NO:259) |
| [Switch D-E]-1-Rev-4F-3 | Ac-F A**D**KAK**D**FVK**E**YFAKFW**E**-NH₂ (SEQ ID NO:260) |
| [Switch D-E]-2-Rev-4F-3 | Ac-F A**D**KAKEFVKDYFAKFW**E**-NH₂ (SEQ ID NO:261) |
| [Switch D-E]-3-Rev-4F-3 | Ac-FAEKAK**D**FVK**E**YFAKFWD-NH₂ (SEQ ID NO:262) |
| [Switch D-E]-4-Rev-4F-3 | Ac-FAEKAK**D**FVKDYFAKFW**E**-NH₂ (SEO ID NO:263) |

| [Switch A-8 and V9] | |
|---|---|
| Rev-4F-4 | Ac-FAEKFKE**VA**KDYFAKFWD-NH₂ (SEQ ID NO:264) |
| [Switch D-E]-1-Rev-4F-4 | Ac-FA**D**KFK**D**VAK**E**YFAKFW**E**-NH₂ (SEQ ID NO:265) |
| [Switch D-E]-2-Rev-4F-4 | Ac-FA**D**KFKEVAKDYFAKFW**E**-NH₂ (SEQ ID NO:266) |
| [Switch D-E]-3-Rev-4F-4 | Ac-FAEKFK**D**VAK**E**YFAKFWD-NH₂ (SEQ ID NO:267) |
| [Switch D-E]-4-Rev-4F-4 | Ac-FAEKFK**D**VAKDYFAKFW**E**-NH₂ (SEQ ID NO:268) |

| [Switch V-9 to Y-12] | |
|---|---|
| Rev-4F-5 | Ac-FAEKFKEA**Y**KD**V**FAKFWD-NH₂ (SEQ ID NO:267) |
| [Switch D-E]-1-Rev-4F-5 | Ac-FA**D**KFK**D**AYK**E**VFAKFW**E**-NH₂ (SEQ ID NO:268) |
| [Switch D-E]-2-Rev-4F-5 | Ac-FA**D**KFKEAYKDVFAKFW**E**-NH₂ (SEQ ID NO:269) |
| [Switch D-E]-3-Rev-4F-5 | Ac-FAEKFK**D**AYK**E**VFAKFWD-NH₂ (SEQ ID NO:270) |
| [Switch D-E]-4-Rev-4F-5 | Ac-FAEKFK**D**AYKDVFAKFW**E**-NH₂ (SEQ ID NO:271) |

| [Switch Y-12 and F-13] | |
|---|---|
| Rev-4F-6 | Ac-FAEKFKEAVKD**FY**AKFWD-NH₂ (SEQ ID NO:272) |
| [Switch D-E]-1-Rev-4F-6 | Ac-FA**D**KFK**D**AVKEFYAKFW**E**-NH₂ (SEQ ID NO:273) |
| [Switch D-E]-2-Rev-4F-6 | Ac-FA**D**KFKEAVKDFYAKFW**E**-NH₂ (SEQ ID NO:274) |
| [Switch D-E]-3-Rev-4F-6 | Ac-FAEKFK**D**AVK**E**FYAKFWD-NH₂ (SEQ ID NO:275) |
| [Switch D-E]-4-Rev-4F-6 | Ac-FAEKFK**D**AVKDFYAKFW**E**-NH₂ (SEQ ID NO:276) |

| [Switch K-6 and W-17] | |
|---|---|
| Rev-4F-7 | Ac-FAEKF**W**EAVKDYFAKF**K**D-NH₂ (SEQ ID NO:277) |
| [Switch D-E]-1-Rev-4F-7 | Ac-FA**D**KFW**D**AVK**E**YFAKFK**E**-NH₂ (SEQ ID NO:278) |
| [Switch D-E]-2-Rev-4F-7 | Ac-FA**D**KFWEAVKDYFAKFK**E**-NH₂ (SEQ ID NO:279) |
| [Switch D-E]-3-Rev-4F-7 | Ac-FAEKFW**D**AVK**E**YFAKFKD-NH₂ (SEQ ID NO:280) |
| [Switch D-E]-4-Rev-4F-7 | Ac-FAEKFW**D**AVKDYFAKFK**E**-NH₂ (SEQ ID NO:281) |

| [Switch F-1 and A-2] | |
|---|---|
| Rev-4F-8 | Ac-**A F**EKFKEAVKDYFAKFWD-NH₂ (SEQ ID NO:282) |
| [Switch D-E]-1-Rev-4F-8 | Ac-AF**D**KFK**D**AVK**E**YFAKFW**E**-NH₂ (SEQ ID NO:283) |
| [Switch D-E]-2-Rev-4F-8 | Ac-AF**D**KFKEAVKDYFAKFW**E**-NH₂ (SEQ ID NO:284) |
| [Switch D-E]-3-Rev-4F-8 | Ac-AFEKFK**D**AVK**E**YFAKFWD-NH₂ (SEQ ID NO:285) |
| [Switch D-E]-4-Rev-4F-8 | Ac-AFEKFK**D**AVKDYFAKFW**E**-NH₂ (SEQ ID NO:286) |

| [F-1 and V-9 are switched] | |
|---|---|
| Rev-F-9 | Ac-**V**AEKFKEA**F**KDYFAKFWD-NH₂ (SEQ ID NO:287) |
| [Switch D-E]-1-Rev-4F-9 | Ac-VA**D**KFK**D**AFK**E**YFAKFW**E**-NH₂ (SEQ ID NO:288) |
| [Switch D-E]-2-Rev-4F-9 | Ac-VA**D**KFKEAFKDYFAKFW**E**-NH₂ (SEQ ID NO:289) |
| [Switch D-E]-3-Rev-4F-9 | Ac-VAEKFK**D**AFK**E**YFAKFWD-NH₂ (SEQ ID NO:290) |
| [Switch D-E]-4-Rev-4F-9 | Ac-VAEKFK**D**AFKDYFAKFW**E**-NH₂ (SEQ ID NO:291) |

| [F-1 and Y-12 are switched] | |
|---|---|
| Rev-4F-10 | Ac-**Y**AEKFKEAVKD**F**FAKFWD-NH₂ (SEQ ID NO:292) |
| [Switch D-E]-1-Rev-4F-10 | Ac-YA**D**KFK**D**AVK**E**FFAKFW**E**-NH₂ (SEQ ID NO:293) |
| [Switch D-E]-2-Rev-4F-10 | Ac-YA**D**KFKEAVKDFFAKFW**E**-NH₂ (SEQ ID NO:294) |
| [Switch D-E]-3-Rev-4F-10 | Ac-YAEKFK**D**AVK**E**FFAKFWD-NH₂ (SEQ ID NO:295) |
| [Switch D-E]-4-Rev-4F-10 | Ac-YAEKFK**D**AVKDFFAKFW**E**-NH₂ (SEQ ID NO:296) |

| [F-1 and A-8 are switched] | |
|---|---|
| Rev-4F-11 | Ac-**A**AEKFKE**F**VKDYFAKFWD-NH₂ (SEQ ID NO:297) |
| [Switch D-E]-1-Rev-4F-11 | Ac-AA**D**KFK**D**FVK**E**YFAKFW**E**-NH₂ (SEQ ID NO:298) |
| [Switch D-E]-2-Rev-4F-11 | Ac-AA**D**KFKEFVKDYFAKFW**E**-NH₂ (SEQ ID NO:299) |
| [Switch D-E]-3-Rev-4F-11 | Ac-AAEKFK**D**FVK**E**YFAKFWD-NH₂ (SEQ ID NO:300) |
| Switch D-E]-4-Rev-4F-11 | Ac-AAEKFKDFVK**D**YFAKFW**E**-NH₂ (SEQ ID NO:301) |

| [A-2 and F-5 are switched] | |
|---|---|
| Rev-4F-12 | Ac-F**F**EK**A**KEAVKDYFAKFWD-NH₂ (SEQ ID NO:302) |
| [Switch D-E]-1-Rev-4F-12 | Ac-FF**D**KAK**D**AVK**E**YFAKFW**E**-NH₂ (SEQ ID NO:303) |
| [Switch D-E]-2-Rev-4F-12 | Ac-FF**D**KAKEAVKDYFAKFW**E**-NH₂ (SEQ ID NO:304) |
| [Switch D-E]-3-Rev-4F-12 | Ac-141-EKAK**D**AVK**E**YFAKFWD-NH₂ (SEQ ID NO:305) |
| [Switch D-E]-4-Rev-4F-12 | Ac-1-1-EKAK**D**AVKDYFAKFW**E**-NH₂ (SEQ ID NO:306) |

| [A-2 and Y12 are switched | |
|---|---|
| Rev-4F-13 | Ac-F**Y**EKFKEAVKD**A**FAKFWD-NH₂ (SEQ ID NO:307) |
| [Switch D-E]-1-Rev-4F-13 | Ac-FY**D**KFK**D**AVK**E**AFAKFW**E**-NH₂ (SEQ ID NO:308) |
| [Switch D-E]-2-Rev-4F-13 | Ac-FY**D**KFKEAVKDAFAKFW**E**-NH₂ (SEQ ID NO:309) |
| [Switch D-E]-3-Rev-4F-13 | Ac-FYEKFK**D**AVK**E**AFAKFWD-NH₂ (SEQ ID NO:310) |
| [Switch D-E]-4-Rev-4F-13 | Ac-FYEKFK**D**AVKDAFAKFW**E**-NH₂ (SEQ ID NO:311) |

| [A-2 and V-9 are switched] | |
|---|---|
| Rev-4F-14 | Ac-F**V**EKFKEA**A**KDYFAKFWD-NH₂ (SEQ ID NO:312) |
| [Switch D-E]-1-Rev-4F-14 | Ac-FV**D**KFK**D**AAK**E**YFAKFW**E**-NH₂ (SEQ ID NO:313) |
| [Switch D-E]-2-Rev-4F-14 | Ac-FV**D**KFKEAAKDYFAKFW**E**-NH₂ (SEQ ID NO:314) |
| [Switch D-E]-3-Rev-4F-14 | Ac-FVEKFK**D**AAK**E**YFAKFWD-NH₂ (SEQ ID NO:315) |
| [Switch D-E]-4-Rev-4F-14 | Ac-FVEKFK**D**AAKDYFAKFW**E**-NH₂ (SEQ ID NO:316) |

| [F-5 and Y-12 are switched] | |
|---|---|
| Rev-4F-15 | Ac-FAEK**Y**KEAVKD**F**FAKFWD-NH₂ (SEQ ID NO:317) |
| [Switch D-E]-1-Rev-4F-15 | Ac-FA**D**KYK**D**AVK**E**FFAKFW**E**-NH₂ (SEQ ID NO:318) |
| [Switch D-E]-2-Rev-4F-15 | Ac-FA**D**KYKEAVKDFFAKFW**E**-NH₂ (SEQ ID NO:319) |
| [Switch D-E]-3-Rev-4F-15 | Ac-FAEKYK**D**AVK**E**FFAKFWD-NH₂ (SEQ ID NO:320) |
| [Switch D-E]-4-Rev-4F-15 | Ac-FAEKYK**D**AVKDFFAKFW**E**-NH₂ (SEQ ID NO:321) |

| [F-5 and V-9 are switched] | |
|---|---|
| Rev-4F-16 | Ac-FAEK**V**KEA**F**KDYFAKFWD-NH₂ (SEQ ID NO:322) |
| [Switch D-E]-1-Rev-4F-16 | Ac-FA**D**KVK**D**AFK**E**YFAKFWE-NH₂ (SEQ ID NO:323) |
| [Switch D-E]-2-Rev-4F-16 | Ac-FA**D**KVKEAFKDYFAKFW**E**-NH₂ (SEQ ID NO:324) |
| [Switch D-E]-3-Rev-4F-16 | Ac-F AEKVK**D**AFK**E**YFAKFWD-NH₂ (SEQ ID NO:325) |
| [Switch D-E]-4-Rev-4F-16 | Ac-FAEKVK**D**AFKDYFAKFW**E**-NH₂ (SEQ ID NO:326) |

| [A-8 and Y-12 switched] | |
|---|---|
| Rev-4F-17 | Ac-FAEKFKE**Y**VKD**A**FAKFWD-NH₂ (SEQ ID NO:327) |
| [Switch D-E]-1-Rev-4F-17 | Ac-FA**D**KFK**D**YVK**E**AFAKFW**E**-NH₂ (SEQ ID NO:328) |
| [Switch D-E]-2-Rev-4F-17 | Ac-FA**D**KFKEYVKDAFAKFW**E**-NH₂ (SEQ ID NO:329) |
| [Switch D-E]-3-Rev-4F-17 | Ac-F AEKFK**D**YVK**E**AFAKFWD-NH₂ (SEQ ID NO:330) |
| [Switch D-E]-4-Rev-4F-17 | Ac-FAEKFK**D**YVKDAFAKFW**E**-NH₂ (SEQ ID NO:331) |

| [V-9 and F-13 are switched] | |
|---|---|
| Rev-4F-18 | Ac-FAEKFKEA**F**KDY**V**AKFWD-NH₂ (SEQ ID NO:332) |
| [Switch D-E]-1-Rev-4F-18 | Ac-FA**D**KFK**D**AFKEYVAKFW**E**-NH₂ (SEQ ID NO:333) |
| [Switch D-E]-2-Rev-4F-18 | Ac-F A**D**KFKEAFKDYVAKFW**E**-NH₂ (SEQ ID NO:334) |
| [Switch D-E]-3-Rev-4F-18 | Ac-FAEKFK**D**AFK**E**YVAKFWD-NH₂ (SEQ ID NO:335) |
| [Switch D-E]-4-Rev-4F-18 | Ac-FAEKFK**D**AFKDYVAKFW**E**-NH₂ (SEQ ID NO:336) |

| [V-9 and F-16 switched] | |
|---|---|
| Rev-4F-19 | Ac-FAEKFKEA**F**KDYFAK**V**WD-NH₂ (SEQ ID NO:337) |
| [Switch D-E]-1-Rev-4F-19 | Ac-FA**D**KFK**D**AFK**E**YFAKVW**E**-NH₂ (SEQ ID NO:338) |
| [Switch D-E]-2-Rev-4F-19 | Ac-FA**D**KFKEAFKDYFAKVW**E**-NH₂ (SEQ ID NO:339) |
| [Switch D-E]-3-Rev-4F-19 | Ac-FAEKFK**D**AFK**E**YFAKVWD-NH₂ (SEQ ID NO:340) |
| Switch D-E]-4-Rev-4F-19 | Ac-F AEKFK**D**AFKDYFAKVW**E**-NH₂ (SEQ ID NO:341) |

| [Y-12 and F-16 are switched | |
|---|---|
| Rev-4F-20 | Ac-FAEKFKEAVKD**F**FAK**Y**WD-NH₂ (SEQ ID NO:342) |
| [Switch D-E]-1-Rev-4F-20 | Ac-FA**D**KFK**D**AVK**E**FFAKYW**E**-NH₂ (SEQ ID NO:343) |
| [Switch D-E]-2-Rev-4F-20 | Ac-FA**D**KFKEAVKDFFAKYW**E**-NH₂ (SEQ ID NO:344) |
| [Switch D-E]-3-Rev-4F-20 | Ac-FAEKFK**D**AVK**E**FFAKYWD-NH₂ (SEQ ID NO:345) |
| [Switch D-E]-4-Rev-4F-20 | Ac-FAEKFK**D**AVKDFFAKYW**E**-NH₂ (SEQ ID NO:346) |

| [W-1, F-6 and K-17 Rev 4F] | |
|---|---|
| Rev-4F-21 | Ac-**W**AEKF**F**EAVKDYFAKF**K**D-NH₂ (SEQ ID NO:347) |
| [Switch D-E]-1-Rev-4F-7 | Ac-WA**D**KFF**D**AVKEYFAKFK**E**-NH₂ (SEQ ID NO:348) |
| [Switch D-E]-2-Rev-4F-7 | Ac-WA**D**KFFEAVKDYFAKFK**E**-NH₂ (SEQ ID NO:349) |
| [Switch D-E]-3-Rev-4F-7 | Ac-WAEKFF**D**AVK**E**YFAKFKD-NH₂ (SEQ ID NO:350) |
| Switch D-E]-4-Rev-4F-7 | Ac-WAEKFF**D**AVKDYFAKFK**E**-NH₂ (SEQ ID NO:351) |

| [W-5, F-6 and K-17 Rev-4F] | |
|---|---|
| Rev-4F-22 | Ac-FAEK**WF**EAVKDYFAKF**K**D-NH₂ (SEQ ID NO:352) |
| [Switch D-E]-1-Rev-4F-22 | Ac-FA**D**KWF**D**AVK**E**YFAKFK**E**-NH₂ (SEQ ID NO:353) |
| [Switch D-E]-2-Rev-4F-22 | Ac-FA**D**KWFEAVKDYFAKFK**E**-NH₂ (SEQ ID NO:354) |
| [Switch D-E]-3-Rev-4F-22 | Ac-FAEKWF**D**AVK**E**YFAKFKD-NH₂ (SEQ ID NO:355) |
| [Switch D-E]-4-Rev-4F-22 | Ac-FAEKWF**D**AVKDYFAKFK**E**-NH₂ (SEQ ID NO:356) |

| [V-6, W-9, K-17 Rev-4F] | |
|---|---|
| Rev-4F-23 | Ac-FAEKF**V**EA**W**KDYFAKF**K**D-NH₂ (SEQ ID NO:357) |
| [Switch D-E]-1-Rev-4F-23 | Ac-FA**D**KFV**D**AWK**E**YFAKFK**E**-NH₂ (SEQ ID NO:358) |
| [Switch D-E]-2-Rev-4F-23 | Ac-FA**D**KFVEAWKDYFAKFK**E**-NH₂ (SEQ ID NO:359) |
| [Switch D-E]-3-Rev-4F-23 | Ac-FAEKFV**D**AWK**E**YFAKFKD-NH₂ (SEQ ID NO:360) |
| [Switch D-E]-4-Rev-4F-23 | Ac-FAEKFV**D**AWKDYFAKFK**E**-NH₂ (SEQ ID NO:361) |

| [Y-2, A-4, W-12, K-17 Rev-4F] | |
|---|---|
| Rev-4F-24 | Ac-F**Y**EKF**A**EAVKD**W**FAKF**K**D-NH₂ (SEQ ID NO:362) |
| [Switch D-E]-1-Rev-4F-24 | Ac-FY**D**KFA**D**AVK**E**WFAKFKE-NH₂ (SEQ ID NO:363) |
| [Switch D-E]-2-Rev-4F-24 | Ac-FY**D**KFAEAVKDWFAKFK**E**-NH₂ (SEQ ID NO:364) |
| [Switch D-E]-3-Rev-4F-24 | Ac-FYEKFA**D**AVK**E**WFAKFKD-NH₂ (SEQ ID NO:365) |
| [Switch D-E]-4-Rev-4F-24 | Ac-FYEKFA**D**AVKDWFAKFK**E**-NH₂ (SEQ ID NO:366) |

It is possible to readily identify biologically active and useful peptides. Thus, for example, the following peptides have been accurately identified as active: 3F1; 3F2; 4F the reverse (retro) forms thereof and the retro-inverso forms thereof. Lipid-associating peptides can comprise a peptide that is 18 amino acids in length and forms a class A amphipathic helix where the peptide has the amino acid composition 2 aspartates, 2 glutamates, 4 lysines, 1 tryptophan, 1 tyrosine, no more than one leucine, no more than 1 valine, no less than 1 and no more than 3 alanines, and with 3 to 6 amino acids from the group: phenylalanine, alpha-naphthalanine, beta-naphthalanine, histidine, and contains either 9 or 10 amino acids on the polar face in a helical wheel representation of the class A amphipathic helix including 4 amino acids with positive charge at neutral pH with two of the positively charged residues residing at the interface between the polar and non-polar faces and with two of the four positively charged residues on the polar face that are contiguous and on the non-polar face two of the amino acid residues from the group: phenylalanine, alpha-naphthalanine, beta-naphthalanine, histidine are also contiguous and if there are 4 or more amino acids from this group on the non-polar face there are also at least 2 residues from this group that are not contiguous.

Certain class Y as well as class A amphipathic helical peptides are disclosed. Class Y amphipathic helical peptides are known to those of skill in the art (see, e.g., Segrest et al. (1992) J. Lipid Res. 33: 141-166; Oram and Heinecke (2005) Physiol Rev. 85: 1343-1372, and the like). These peptides include, but are not limited to, an 18 amino acid peptide that forms a class A amphipathic helix or a class Y amphipathic helix described by formula I:

D X X K Y X X D K X Y D KX K D Y X (SEQ ID NO:367) I

where the D's are independently Asp or Glu; the Ks are independently Lys or Arg; the Xs are independently Leu, norLeu, Val, Ile, Trp, Phe, Tyr, β-Nal, or α-Nal and all X residues are on the non-polar face (e.g., when viewed in a helical wheel diagram) except for one that can be on the polar face between two K residues; the Y's are independently Ala, His, Ser, Gln, Asn, or Thr non-polar f ace (e.g., when viewed in a helical wheel diagram) and the Y's are independently one Ala on the polar face, one His, one Ser, one Gln one Asn, or one Thr on the polar face (e.g., when viewed in a helical wheel diagram), where no more than two K are be contiguous (e.g., when viewed in a helical wheel diagram); and where no more than 3 D's are contiguous (e.g., when viewed in a helical wheel diagram) and the fourth D is be separated from the other D's by a Y. Illustrative peptides of this kind which include peptides with histidine, and/or alpha- and/or beta-napthalanine are shown in Table 5. Reverse (retro-), inverse, retro-inverso-, and circularly permuted forms of these peptides are also contemplated.

| Table 5 | |
|---|---|
| Short Name | Peptide Sequence |
| [A-5>H]4F | Ac-DWFKHFYDKVAEKFKEAF-NH₂ (SEQ ID NO:368) |
| [A-5>H, D-E switched] 4F | Ac-EWFKHFYEKVADKFKDAF-NH₂ (SEQ ID NO:369) |
| [A-5>H, D-1>E]4F | Ac-EWFKHFYDKVAEKFKEAF-NH₂ (SEQ ID NO:370) |
| [A-5>H, D-8>E]4-F | Ac-DWFKHFYEKVAEKFKEAF-NH₂ (SEQ ID NO:371) |
| [A-5>H, E-12>D]4F | Ac-DWFKHFYDKVADKFKEAF-NH₂ (SEQ ID NO:372) |
| [A-5>H, E-16>D]4F | Ac-DWFKHFYDKVAEKFKDAF-NH₂ (SEQ ID NO:373) |
| [F-3>H, A-5>F]-4F | Ac-DWHKFFYDKVAEKFKEAF-NH₂ (SEQ ID NO:374) |
| [F-3>H, A-5>F, D-E switched]-4F | Ac-EWHKFFYEKVADKFKDAF-NH₂ (SEQ ID NO:375) |
| [F-3>H, A-5>F, D-1>E]-4F | Ac-EWHKFFYDKVAEKFKEAF-NH₂ (SEQ ID NO:376) |
| [F-3>H, A-5>F, D-8>E]-4F | Ac-DWHKFFYEKVAEKFKEAF-NH₂ (SEQ ID NO:377) |
| [F-3>H, A-5>F, E-12>D]-4F | Ac-DWHKFFYDKVADKFKEAF-NH₂ (SEQ ID NO:378) |
| [F-3>H, A-5>F, E-16>D]-4F | Ac-DWHKFFYDKVAEKFKDAF-NH₂ (SEQ ID NO:379) |
| [A-5>F, F-6>H]4F | Ac-DWFKFHYDKVAEKFKEAF-NH₂ (SEQ ID NO:380) |
| [A-5>F, F-6>H, D-E switched]4F | Ac-EWFKFHYEKVADKFKDAF-NH₂ (SEQ ID NO:381) |
| [[A-5>F, F-6>H, D-1>E]4F | Ac-EWFKFHYDKVAEKFKEAF-NH₂ (SEQ ID NO:382) |
| [A-5>F, F-6>H, D-8>E]4F | Ac-DWFKFHYEKVAEKFKEAF-NH₂ (SEQ ID NO:383) |
| [A-5>F, F-6>H, E-12>D]4F | Ac-DWFKFHYDKVADKFKEAF-NH₂ (SEQ ID NO:384) |
| [A-5>F, F-6>H, E-16>D]4F | Ac-DWFKFHYDKVAEKFKDAF-NH₂ (SEQ ID NO:385) |
| [A-5>V, V-10>H]4F | Ac-DWFKVFYDKHAEKFKEAF-NH₂ (SEQ ID NO:386) |
| [A-5>V, V-10>H, D-E switched]4F | Ac-EWFKVFYEKHADKFKDAF-NH₂ (SEQ ID NO:387) |
| [A-5>V, V-10>H, D-1>E]4F | Ac-EWFKVFYDKHAEKFKEAF-NH₂ (SEQ ID NO:388) |
| [A-5>V, V-10>H, D-8>E]4F | Ac-DWFKVFYEKHAEKFKEAF-NH₂ (SEQ ID NO:389) |
| [A-5>V, V-10>H, E-12>D]4F | Ac-DWFKVFYDKHADKFKEAF-NH₂ (SEQ ID NO:390) |
| [A-5>V, V-10>H, E16>D]4F | Ac-DWFKVFYDKHAEKFKDAF-NH₂ (SEQ ID NO:391) |
| [[A-17>H]4F | Ac-DWFKAFYDKVAEKFKEHF-NH₂ (SEQ ID NO:392) |
| [A-17>H, D-E switched]4F | Ac-EWFKAFYEKVADKFKDHF-NH₂ (SEQ ID NO:393) |
| [[A-17>H, D-1>E]4F | Ac-EWFKAFYDKVAEKFKEHF-NH₂ (SEQ ID NO:394) |
| [[A-17>H, D-8>E]4F | Ac-DWFKAFYEKVAEKFKEHF-NH₂ (SEQ ID NO:395) |
| [[A-17>H, E-12>D]4F | Ac-DWFKAFYDKVADKFKEHF-NH₂ (SEQ ID NO:396) |
| [[A-17>H, E16>D]4F | Ac-DWFKAFYDKVAEKFKDHF-NH₂ (SEQ ID NO:397) |
| [A-17>F, F-18>H]4F | Ac-DWFKAFYDKVAEKFKEFH-NH₂ (SEQ ID NO:398) |
| [A-17>F, F-18>H, D-E switched]4F | Ac-EWFKAFYEKVADKFKDFH-NH₂ (SEQ ID NO:399) |
| [A-17>F, F-18>H, D-1>E]-4F | Ac-EWFKAFYDKVAEKFKEFH-NH₂ (SEQ ID NO:400) |
| [A-17>F, F-18>H]4F | Ac-DWFKAFYDKVAEKFKEFH-NH₂ (SEQ ID NO:401) |
| [A-17>F, F-18>H, D-8>E]-4F | Ac-DWFKAFYEKVAEKFKEFH-NH₂ (SEQ ID NO:402) |
| [A-17>F, F-18>H, E-12>D]4F | Ac-DWFKAFYDKVAEKFKEFH-NH₂ (SEQ ID NO:403) |
| [A-17>F, F-18>H], E-16>D]-4F | Ac-DWFKAFYDKVAEKFKDFH-NH₂((SEQ ID NO:404) |
| Rev-4 F | Ac-FAEKFKEAVKDYFAKFWD-NH₂ (SEQ ID NO:405) |
| [A-2>H]Rev4F | Ac-FHEKFKEAVKDYFAKFWD-NH₂ (SEQ ID NO:406) |
| Rev-[A-2>H, D>E]-4F | Ac-FHEKFKEAVKEYFAKFWE-NH₂ (SEQ ID NO:407) |
| Rev-[A-2>H, E>D]4F | Ac-FHDKFKDAVKDYFAKFWD-NH₂ (SEQ ID NO:408) |
| [A-2>H, D-E switched]Rev-4F | Ac-FHDKFKDAVKEYFAKFWE-NH₂ (SEQ ID NO:409) |
| [A-2>H, E-3>D]Rev-4F | Ac-FHDKFKEAVKDYFAKFWD-NH₂ (SEQ ID NO:410) |
| [A-2>H, E-7>D]Rev-4F | Ac-FHEKFKDAVKDYFAKFWD-NH₂ (SEQ ID NO:411) |
| [A-2>2H, D-11>E]Rev-4F | Ac-FHEKFKEAVKEYFAKFWD-NH₂ (SEQ ID NO:412) |
| [A-2>H, D-18>E]Rev-4F | Ac-FHEKFKEAVKDYFAKFWE-NH₂ (SEQ ID NO:413) |
| [F-1>H, A-2>F]Rev-4F | Ac-HFEKFKEAVKDYFAKFWD-NH₂ (SEQ ID NO:414) |
| [F-1>H, A-2>F,D-E switched]Rev-4F | Ac-HFDKFKDAVKEYFAKFWE-NH₂ (SEQ ID NO:415) |
| [F-1>H, A-2>F, D>E]Rev-4F | Ac-HFEKFKEAVKEYFAKFWE-NH₂ (SEQ ID NO:416) |
| [F-1>H, A-2>F, E-3>D]Rev-4F | Ac-HFDKFKEAVKDYFAKFWD-NH₂ (SEQ ID NO:417) |
| [F-1>H, A-2>F, E-7>D]Rev-4F | Ac-HFEKFKDAVKDYFAKFWD-NH₂ (SEQ ID NO:418) |
| [F-1>H, A-2>F, D-11>E]Rev-4F | Ac-HFEKFKEAVKEYFAKFWD-NH₂ (SEQ ID NO:419) |
| [F-1>H, A-2>F, D-18>E]Rev-4F | Ac-HFEKFKEAVKDYFAKFWE-NH₂ (SEQ ID NO:420) |
| [A-2>F, F-5>H] Rev D-4F | Ac-FFEKHKEAVKDYFAKFWD-NH₂ (SEQ ID NO:421) |
| [A-2>F, F-5>H, D-E switched]Rev D-4F | Ac-FFDKHKDAVKEYFAKFWE-NH₂ (SEQ ID NO:422) |
| [A-2>F, F-5>H, D>E]Rev D-4F | Ac-FFEKHKEAVKEYFAKFWE-NH₂ (SEQ ID NO:423) |
| [A-2>F, F-5>H, E>D]Rev D-4F [ | Ac-FFDKHKDAVKDYFAKFWD-NH₂ (SEQ ID NO:424) |
| A-2>F, F-5>H, E-3>D]Rev | Ac-FFDKHKEAVKDYFAKFWD-NH₂ (SEQ ID NO:425) |
| D-4F [A-2>F, F-5>H, D-11>E]Rev D-4F | Ac-FFEKHKEAVKEYFAKFWD-NH₂ (SEQ ID NO:426) |
| [A-2>F, F-5>H, D-18>E]Rev D-4F | Ac-FFEKHKEAVKDYFAKFWE-NH₂ (SEQ ID NO:427) |
| [A-2>V, V-9>H]Rev D-4F | Ac-FVEKFKEAHKDYFAKFWD-NH₂ (SEQ ID NO:428) |
| [A-2>V, V-9>H, D- E switched]Rev D-4 | Ac-FVDKFKDAHKEYFAKFWE-NH₂ (SEQ ID NO:429) |
| [A-2>V, V-9>H, D>E]Rev D-4F | Ac-FVEKFKEAHKEYFAKFWE-NH₂ (SEQ ID NO:430) |
| [A-2>V, V-9>H, E>D]Rev D-4F | Ac-FVDKFKDAHKDYFAKFWD-NH₂ (SEQ ID NO:431) |
| [A-2>V, V-9>H, E-3>D]Rev D-4F | Ac-FVDKFKEAHKDYFAKFWD-NH₂ (SEQ ID NO:432) |
| [A-2>V, V-9>H, E-7>D]Rev D-4F | Ac-FVEKFKDAHKDYFAKFWD-NH₂ (SEQ ID NO:433) |
| [A-2>V, V-9>H, D-11>E]Rev D-4F | Ac-FVEKFKEAHKEYFAKFWD-NH₂ (SEQ ID NO:434) |
| [A-2>V, V-9>H, D-18>E]Rev D-4F | Ac-FVEKFKEAHKDYFAKFWE-NH₂ (SEQ ID NO:435) |
| [A-8>H]Rev-4F | Ac-FAEKFKEHVKDYFAKFWD-NH₂ (SEQ ID NO:436) |
| [A-8>H,D-E switched]Rev-4F | Ac-FADKFKDHVKEYFAKFWE-NH₂ (SEQ ID NO:437) |
| [A-8>H, D>E]Rev-4F | Ac-FAEKFKEHVKEYFAKFWE-NH₂ (SEQ ID NO:438) |
| [A-8>H, E>D]Rev-4F | Ac-FADKFKDHVKDYFAKFWD-NH₂ (SEQ ID NO:439) |
| [A-8>H, E-3>D]Rev-4F | Ac-FADKFKEHVKDYFAKFWD-NH₂( (SEQ ID NO:440) |
| [A-8>H, E-7>D]Rev-4F | Ac-FAEKFKDHVKDYFAKFWD-NH₂ (SEQ ID NO:441) |
| [A-8>H, D-11>E]Rev-4F | Ac-FAEKFKEHVKEYFAKFWD-NH₂ (SEQ ID NO:442) |
| [A-8>H, D-18>E]Rev-4F | Ac-FAEKFKEHVKDYFAKFWE-NH₂ (SEQ ID NO:443) |
| [A-8>F, F-13>H]Rev-4F | Ac-FAEKFKEFVKDYHAKFWD-NH₂ (SEQ ID NO:444) |
| [A-8>F, F-13>H, D-E switched]Rev-4F | Ac-FADKFKDFVKEYHAKFWE-NH₂ (SEQ ID NO:445) |
| [A-8>F, F-13>H, E-3>D]Rev-4F | Ac-FADKFKEFVKDYHAKFWD-NH₂ (SEQ ID NO:446) |
| [A-8>F, F-13>H, E-7>D]Rev-4F | Ac-FAEKFKDFVKDYHAKFWD-NH₂ (SEQ ID NO:447) |
| [A-8>F, F-13>H, E>D]Rev-4F | Ac-FADKFKDFVKDYHAKFWD-NH₂ (SEQ ID NO:448) |
| [A-8>F, F-13>H, D>E]Rev-4F | Ac-FAEKFKEFVKEYHAKFWE-NH₂ (SEQ ID NO:449) |
| [A-8>F, F-13>H, D-11>E]Rev-4F | Ac-FAEKFKEFVKEYHAKFWD-NH₂ (SEQ ID NO:450) |
| [A-8>F, F-13>H, D-18>E]Rev-4F | Ac-FAEKFKEFVKDYHAKFWE-NH₂ (SEQ ID NO:451) |
| [A-8>F, F16>H]Rev-4F | Ac-FAEKFKEFVKDYFAKHWD-NH₂ (SEQ ID NO:452) |
| [A-8>F, F16>H, D-E switched]Rev-4F | Ac-FADKFKDFVKEYFAKHWE-NH₂ (SEQ ID NO:453) |
| [A-8>F, F16>H, D>E]Rev-4F | Ac-FAEKFKEFVKEYFAKHWE-NH₂ (SEQ ID NO:454) |
| [A-8>F, F16>H, E>D]Rev-4F | Ac-FADKFKDFVKDYFAKHWD-NH₂ (SEQ ID NO:455) |
| [A-8>F, F16>H, E-3>D]Rev-4F | Ac-FADKFKEFVKDYFAKHWD-NH₂ (SEQ ID NO:456) |
| [A-8>F, F16>H, E-7>D]Rev-4F | Ac-FAEKFKDFVKDYFAKHWD-NH₂ (SEQ ID NO:457) |
| [A-8>F, F16>H, D- 1 1>E]Rev-4F | Ac-FAEKFKEFVKEYFAKHWD-NH₂ (SEQ ID NO:458) |
| [A-8>F, F16>H, D-18>E]Rev-4F | Ac-FAEKFKEFVKDYFAKHWE-NH₂ (SEQ ID NO:459) |

Examples of class A 4F and Rev 4F analogs with beta-Nph. Similarly, alpha-Nph analogs can be designed. Similarly to the above analogs, His can be incorporated to Nph analogs. D>E analogs, E>D analogs and D-E switch analogs are additional possibilities similarly to the above described analogs.

| | |
|---|---|
| 4Nph | Ac-DW**Nph**KA**Nph**YDKVAEK**Nph**KEA**Nph**-NH2 (SEQ ID NO:460) |
| [D-E switched] 4Nph | Ac-**E**W**Nph**KA**Nph**Y**E**KVA**D**K**Nph**K**D**A**Nph**-NH2 (SEQ ID NO:461) |
| [D>E]4Nph | Ac-**E**W**Nph**KA**Nph**Y**E**KVAEK**Nph**KEA**Nph**-NH2 (SEQ ID NO:462) |
| [E>D]4Nph | Ac-DW**Nph**KA**Nph**YDKVA**D**K**Nph**K**D**A**Nph**-NH2 (SEQ ID NO:463) |
| [D-1>E]4Nph | Ac-**E**W**Nph**KA**Nph**YDKVAEK**Nph**KEA**Nph**-NH2 (SEQ ID NO:464) |
| [D-8>E]4Nph | Ac-DW**Nph**KA**Nph**Y**E**KVAEK**Nph**KEA**Nph**-NH2 (SEQ ID NO:465) |
| [E-12>D]4Nph | Ac-DW**Nph**KA**Nph**YDKVA**D**K**Nph**KEA**Nph**-NH2 (SEQ ID NO:466) |
| [E-16>D]4Nph | Ac-DW**Nph**KA**Nph**YDKVAEK**Nph**K**D**A**Nph**-NH2 (SEQ ID NO:467) |

As described above for 4Nph, a minimum of 7 additional analogs for each of the analogs given below.

| | |
|---|---|
| [F-3, 6,>Nph]4F | Ac-DW**Nph**KA**Nph**YDKVAEKFKEAF-NH2 (SEQ ID NO:468) |
| [F-14, 18>Nph]4F | Ac-DWFKAFYDKVAEK**Nph**KEA**Nph**-NH2 (SEQ ID NO:469) |
| [[F-3>Nph]4F | Ac-DW**Nph**KAFYDKVAEKFKEAF-NH2 (SEQ ID NO:470) |
| [F-6>Nph]4F | Ac-DWFKA**Nph**YDKVAEKFKEAF-NH2 (SEQ ID NO:471) |
| [F-14>Nph]4F | Ac-DWFKAFYDKVAEK**Nph**KEAF-NH2 (SEQ ID NO:472) |
| [F-18>Nph]4F | Ac-DWFKAFYDKVAEKFKEA**Nph**-NH2 (SEQ ID NO:473) |

For each of the analog described below, a minimum of 7 additional analogs are possible as described above by switching D-E, D>E and E>D and single D or E analogs.

| | |
|---|---|
| Rev-4Nph | Ac-**Nph**AEK**Nph**KEAVKDY**Nph**AK**Nph**WD-NH2 (SEQ ID NO:474) |
| [F-3, 6>Nph]Rev | Ac-**Nph**AEK**Nph**KEAVKDYFAKFWD-NH2 (SEQ ID NO:475) |
| 4F [F-13, 16]Rev-4F | Ac-FAEKFKEAVKDY**Nph**AK**Nph**WD-NH2 (SEQ ID NO:476) |
| [F-3>Nph]Rev-4F | Ac-**Nph**AEKFKEAVKDYFAKFWD-NH2 (SEQ ID NO:477) |
| [F-6>Nph]Rev-4F | Ac-FAEK**Nph**KEAVKDYFAKFWD-NH2 (SEQ ID NO:478) |
| [F-13>Nph]Rev-4F | Ac-FAEKFKEAVKDY**Nph**AKFWD-NH2 (SEQ ID NO:479) |
| [F-16>Nph]Rev-4F | Ac-FAEKEKEAVKDYFAK**Nph**WD-NH2 (SEQ ID NO:480) |

For the analogs described below, additional analogs are possible by incorporating His or alpha-Nph and beta-Nph

| | | |
|---|---|---|
| Rev-[D>E]-4F | Ac-FAEKFKEAVK**E**YFAKFW**E**-NH2 (SEQ ID NO:481) | |
| Rev-[E>D]4F | Ac-FA**D**KFK**D**AVKDYFAKFWD-NH2 (SEQ ID NO:482) | |
| Rev-R4-4F | Ac-FAE**R**FREAVKDYFAKFWD-NH2 (SEQ ID NO:483) | |
| Rev-R6-4F | Ac-FAEKF**R**EAVKDYFAKFWD-NH2 (SEQ ID NO:484) | |
| | | |
| Rev-R10-4F | Ac-FAEKFKEAV**R**DYFAKFWD-NH2 (SEQ ID NO:485) | |
| Rev-R14-4F | Ac-FAEKFKEAVKDYFA**R**FWD-NH2 (SEQ ID NO:486) | |
| Rev-[D>E]-4F | Ac-FAEKFKEAVK**E**YFAKFW**E**-NH2 (SEQ ID NO:481) | |
| | | |
| Rev-[E>D]4F | Ac-FA**D**KFK**D**AVKDYFAKFWD-NH2 (SEQ ID NO:482) | |
| Rev-R4-4F | Ac-FAE**R**FREAVKDYFAKFWD-NH2 | (SEQ ID NO:483) |
| Rev-R6-4F | Ac-FAEKF**R**EAVKDYFAKFWD-NH2 | (SEQ ID NO:484) |
| | | |
| Rev-R10-4F | Ac-FAEKFKEAV**R**DYFAKFWD-NH2 | (SEQ ID NO:485) |
| Rev-R14-4F | Ac-FAEKFKEAVKDYFA**R**FWD-NH2 | (SEQ ID NO:486) |
| Rev-[D>E]-4F | Ac-FAEKFKEAVK**E**YFAKFW**E**-NH2 | (SEQ ID NO:481) |
| Rev-[E>D]4F | Ac-FA**D**KFK**D**AVKDYFAKFWD-NH2 | (SEQ ID NO:482) |
| Rev-R4-4F | Ac-FAE**R**FREAVKDYFAKFWD-NH2 | (SEQ ID NO:483) |
| Rev-R6-4F | Ac-FAEKF**R**EAVKDYFAKFWD-NH2 | (SEQ ID NO:484) |
| | | |
| Rev-R10-4F | Ac-FAEKFKEAV**R**DYFAKFWD-NH2 | (SEQ ID NO:485) |
| Rev-R14-4F | Ac-FAEKFKEAVKDYFA**R**FWD-NH2 | (SEQ ID NO:486) |
| Rev-R4-4F | Ac-FAE**R**FREAVKDYFAKFWD-NH2 | (SEQ ID NO:483) |
| Rev-R6-4F | Ac-FAEKF**R**EAVKDYEAKFWD-NH2 | (SEQ ID NO:487) |
| Rev-R10-4F | Ac-FAEKFKEAV**R**DYFAKFWD-NH2 | (SEQ ID NO:485) |
| Rev-R14-4F | Ac-FAEKFKEAVKDYFA**R**FWD-NH2 | (SEQ ID NO:486) |
| Rev-[D>E]-4F | Ac-FAEKFKEAVK**E**YFAKFW**E**-NH2 | (SEQ ID NO:481) |
| | | |
| Rev-[E>D]4F | Ac-FA**D**KFK**D**AVKDYFAKFWD-NH2 | (SEQ ID NO:482) |
| Rev-R4-4F | Ac-FAE**R**FREAVKDYFAKFWD-NH2 | (SEQ ID NO:483) |
| Rev-R6-4F | Ac-FAEKF**R**EAVKDYFAKFWD-NH2 | (SEQ ID NO:484) |
| | | |
| Rev-R10-4F | Ac-FAEKFKEAV**R**DYFAKFWD-NH2 | (SEQ ID NO:485) |
| Rev-R14-4F | Ac-FAEKFKEAVKDYFA**R**FWD-NH2 | (SEQ ID NO:486) |

For each of the analogs below, additional H and Nph analogs are possible using the examples described above. Each analog can yield 7 analogs with the changes described in the examples given above.

| | |
|---|---|
| Rev3F-2 | Ac-LFEKFAEAFKDYVAKWKD-NH2 (SEQ ID NO:488) |
| RevR4-3F-2 | Ac-LFE**R**FAEAFKDYVAKWKD-NH2 (SEQ ID NO:489) |
| RevR10-3F2 | Ac-LFEKFAEAF**R**DYVAKWKD-NH2 (SEQ ID NO:490) |
| RevR15-3F-2 | Ac-LFEKFAEAFKDYVA**R**WKD-NH2 (SEQ ID NO:491) |
| RevR17-3F-2 | Ac-LFEKFAEAFKDYVAKW**R**D-NH2 (SEQ ID NO:492) |
| Rev[D>E]3F2 | Ac-LFEKFAEAFK**E**YVAKWK**E**-NH2 (SEQ ID NO:493) |
| Rev[E>D]3F-2 | Ac-LF**D**KFA**D**AFKDYVAKWKD-NH2 (SEQ ID NO:494) |
| Rev-[E3>D]-3F-2 | Ac-LF**D**KFAEAFKDYVAKWKD-NH2 (SEQ ID NO:495) |
| Rev-[E7>D]-3F-2 | Ac-LFEKFA**D**AFKDYVAKWKD-NH2 (SEQ ID NO:496) |
| Rev[D11>E]3F-2 | Ac-LFEKFAEAFK**E**YVAKWKD-NH2 (SEQ ID NO:497) |
| Rev-[D18>E]3F-2 | Ac-LFEKFAEAFKDYVAKWK**E**-NH2 (SEQ ID NO:498) |
| Rev3F-1 | Ac-FAEKAWEFVKDYFAKLKD-NH2 (SEQ ID NO:499) |
| RevR4-3F-1 | Ac-FAE**R**AWEFVKDYFAKLKD-NH2 (SEQ ID NO:500) |
| RevR10-3F-1 | Ac-FAEKAWEFV**K**DYFAKLKD-NH2 (SEQ ID NO:501) |
| RevR15-3F-1 | Ac-FAEKAWEFVKDYFA**K**LKD-NH2 (SEQ ID NO:502) |
| RevR17-3F-1 | Ac-FAEKAWEFVKDYFAKL**R**D-NH2 (SEQ ID NO:503) |
| | |
| Rev[D>E]3F-1 | Ac-FAEKAWEFVK**E**YFAKLK**E**-NH2 (SEQ ID NO:504) |
| Rev[E>D}3F-1 | Ac-FA**D**KAW**D**FVKDYFAKLKD-NH2 (SEQ ID NO:505) |
| Rev[E3>D]-3F-1 | Ac-FA**D**KAWEFVKDYFAKLKD-NH2 (SEQ ID NO:506) |
| Rev[E7>D]3F-1 | Ac-FAEKAW**D**FVKDYFAKLKD-NH2 (SEQ ID NO:507) |
| Rev-[D11>E]3F-1 | Ac-FAEKAWEFVK**E**YFAKLKD-NH2 (SEQ ID NO:508) |
| Rev-[D18>E]3F-1 | Ac-FAEKAWEFVKDYFAKLK**E**-NH2 (SEQ ID NO:509) |
| Rev-5F | Ac-FFEKFKEFVKDYFAKLWD-NH2 (SEQ ID NO:510) |
| Rev-[D>E]5F | Ac-FFEKFKEFVK**E**YFAKLW**E**-NH2 (SEQ ID NO:511) |
| Rev-[E>D]5F | Ac-FF**D**KFK**D**FVKDYFAKLWD-NH2 (SEQ ID NO:512) |
| Rev-R4-5F | Ac-FFE**R**FKEFVKDYFAKLWD-NH2 (SEQ ID NO:513) |
| Rev-R6-5F | Ac-FFEKF**R**EFVKDYFAKLWD-NH2 (SEQ ID NO:514) |
| Rev-R10-5F | Ac-FFEKFKEFV**R**DYFAKLWD-NH2 (SEQ ID NO:515) |
| Rev-R15-5F | Ac-FFEKFKEFVKDYFA**R**LWD-NH2 (SEQ ID NO:516) |
| Rev-[E3>D]-5F | Ac-FF**D**KFKEFVKDYFAKLWD-NH2 (SEQ ID NO:517) |
| Rev-[E7>D]5F | Ac-FFEKFK**D**FVKDYFAKLWD-NH2 (SEQ ID NO:518) |
| Rev-[D11>E]-5F | Ac-FFEKFKEFVK**E**YFAKLWD-NH2 (SEQ ID NO:519) |
| Rev-[D18>E]-5F | Ac-FFEKFKEFVKDYFAKLW**E**-NH2 (SEQ ID NO:520) |
| Rev-5F-2 | Ac-F**L**EKFKEFVKDYFAK**F**WD-NH2 (SEQ ID NO:521) |
| Rev-[D>E]-5F-2 | Ac-FLEKFKEFVK**E**YFAKFW**E**-NH2 (SEQ ID NO:522) |
| Rev-[E>D]-5F-2 | Ac-FL**D**KFK**E**FVKDYFAKFWD-NH2 (SEQ ID NO:523) |
| Rev-[E3>D]-5F-2 | Ac-FL**D**KFKEFVKDYFAKFWD-NH2 (SEQ ID NO:524) |
| Rev-[E7>D]-5F-2 | Ac-FLEKFK**D**FVKDYFAKFWD-NH2 (SEQ ID NO:525) |
| Rev-[D11>E]-5F-2 | Ac-FLEKFKEFVK**E**YFAKFWD-NH2 (SEQ ID NO:526) |
| Rev-[D18>E]-5F-2 | Ac-FLEKFKEFVKDYFAKFW**E**-NH2 (SEQ ID NO:527) |
| Rev-R4-5F-2 | Ac-FLE**R**FKEFVKDYFAKFWD-NH2 (SEQ ID NO:528) |
| Rev-R6-5F-2 | Ac-FLEKF**R**EFVKDYFAKFWD-NH2 (SEQ ID NO:529) |
| RevR10-5F-2 | Ac-FLEKFKEFV**R**DYFAKFWD-NH2 (SEQ ID NO:530) |
| Rev-R16-5F-2 | Ac-FLEKFKEFVKDYFA**R**FWD-NH2 (SEQ ID NO:531) |
| Rev-6F | Ac-F**F**EK**F**KE**FF**KDYFAKLWD-NH2 (SEQ ID NO:532) |
| Rev-[D>E]-6F | Ac-FFEKFKEFFK**E**YFAKLW**E**-NH2 (SEQ ID NO:533) |
| Rev-[E>D]-6F | Ac-FF***D***KFK***D***FFKDYFAKLWD-NH2 (SEQ ID NO:534) |
| Rev-R4-6F | Ac-FFE**R**FKEFFKDYFAKLWD-NH2 (SEQ ID NO:535) |
| Rev-R6-6F | Ac-FFEKF**R**EFFKDYFAKLWD-NH2 (SEQ ID NO:536) |
| Rev-R10-6F | Ac-FFE**K**FKEFF**R**DYFAKLWD-NH2 (SEQ ID NO:537) |
| Rev-R14-6F | Ac-FFERFKEFFKDYFA**R**LWD-NH2 (SEQ ID NO:538) |
| Rev-[E3>D]-6F | Ac-FF**D**KFKEFFKDYFAKLWD-NH2 (SEQ ID NO:539) |
| Rev-[E7>D]-6F | Ac-FFEKEK**D**FFKDYFAKLWD-NH2 (SEQ ID NO:540) |
| Rev-[D11>E]-6F | Ac-FFEKFKEFFK**E**YFAKLWD-NH2 (SEQ ID NO:541) |
| Rev-[D18>E]-6F | Ac-FFEKFKEFFKDYFAKLW**E**-NH2 (SEQ ID NO:542) |
| Rev-4F | Ac-FAEKFKEAVKDYFAKFWD-NH2 (SEQ ID NO:543) |
| Rev-[D>E]-4F | Ac-FAEKFKEAVK**E**YFAKFW**E**-NH2 (SEQ ID NO:481) |
| | |
| Rev-[E>D]4F | Ac-FA**D**KFK**D**AVKDYFAKFWD-NH2 (SEQ ID NO:482) |
| Rev-R4-4F | Ac-FAE**R**FREAVKDYFAKFWD-NH2 (SEQ ID NO:483) |
| Rev-R6-4F | Ac-FAEKF**R**EAVKDYFAKFWD-NH2 (SEQ ID NO:484) |
| | |
| Rev-R10-4F | Ac-FAEKFKEAV**R**DYFAKFWD-NH2 (SEQ ID NO:485) |
| Rev-R14-4F | Ac-FAEKFKEAVKDYFA**R**FWD-NH2 (SEQ ID NO:486) |
| 4F-2 | Ac-DKWKAVYDKFAEAFKEFF-NH2 (SEQ ID NO:544) |
| [D>E]-4F-2 | Ac-EKWKAVYEKFAEAFKEFF-NH2 (SEQ ID NO:545) |
| [E>D]-4F-2 | Ac-DKWKAVYDKFA**D**AFK**D**FF-NH2 (SEQ ID NO:546) |
| R2-4F-2 | Ac-D**R**WKAVYDKFAEAFKEFF-NH2 (SEQ ID NO:547) |
| R4-4F-2 | Ac-DKW**R**AVYDKFAEAFKEFF-NH2 (SEQ ID NO:548) |
| R9-4F-2 | Ac-DKWKAVYD**R**FAEAFKEFF-NH2 (SEQ ID NO:549) |
| R14-4F-2 | Ac-DKWKAVYDKFAEAF**R**EFF-NH2 (SEQ ID NO:550) |
| Rev4F-2 | Ac-FFEKFAEAFKDYVAKWKD-NH2 (SEQ ID NO:551) |
| Rev-[D>E]-4F-2 | Ac-FFEKFAEAFK**E**YVAKWK**E**-NH2 (SEQ ID NO:552) |
| Rev-[E>D]-3F-2 | Ac-FF**D**KFA**D**AFKDYVAKWKD-NH2 (SEQ ID NO:553) |
| Rev-R4-4F-2 | Ac-FFE**R**FAEAFKDYVAKWKD-NH2 (SEQ ID NO:554) |
| Rev-R10-4F-2 | Ac-EFERFAEAF**R**DYVAKWKD-NH2 (SEQ ID NO:555) |
| Rev-R15-4F-2 | Ac-FFEKFAEAFKDYVA**R**WKD-NH2 (SEQ ID NO:556) |
| Rev-R17-4F-2 | Ac-FFE**R**FAEAFKDYVAKW**R**D-NH2 (SEQ ID NO:557) |
| Rev-[E3>D]-4F-2 | Ac-FF**D**KFAEAFKDYVAKWKD-NH2 (SEQ ID NO:558) |
| Rev-[E7>D]-4F-2 | Ac-FFEKFA**D**AFKDYVAKWKD-NH2 (SEQ ID NO:559) |
| Rev-[D11>E]-4F-2 | Ac-FFERFAEAFK**E**YVAKWKD-NH2 (SEQ ID NO:560) |
| Rev-[D18>E]-4F-2 | Ac-FFERFAEAFKDYVAKWKE-NH2 (SEQ ID NO:561) |
| Rev-7F | Ac-FFEKFKEFFKDYFAKFWD-NH2 (SEQ ID NO:562) |
| Rev-[E>D]-7F | Ac-FF**D**KFK**D**FFKDYFAKFWD-NH2 (SEQ ID NO:563) |
| Rev-[D>E]-7F | Ac-FFEKFKEFFK**E**YFAKFW**E**-NH2 (SEQ ID NO:564) |
| Rev-R4-7F | Ac-FFE**R**FKEFFKDYFAKFWD-NH2 (SEQ ID NO:565) |
| Rev-R6-7F | Ac-FFEKF**R**EFFKDYFAKFWD-NH2 (SEQ ID NO:566) |
| Rev-R10-7F | Ac-FFEKFKEFF**R**DYFAKFWD-NH2 (SEQ ID NO:567) |
| Rev-R14-7F | Ac-FFEKFKEFFKDYFA**R**FWD-NH2 (SEQ ID NO:568) |
| Rev-[E3>D]-7F | Ac-FF**D**KFKEFFKDYFAKFWD-NH2 (SEQ ID NO:569) |
| Rev-[E7>D]7F | Ac-FFEKFK**D**FFKDYFAKFWD-NH2 (SEQ ID NO:570) |
| Rev-[D11>E]-7F | Ac-FFEKFKEFFK**E**YFAKFWD-NH2 (SEQ ID NO:571) |
| Rev-[D18>E]-7F | Ac-FFEKFKEFFKDYFAKFW**E**-NH2 (SEQ ID NO:572) |

It is also noted that any of the peptides described herein can comprise non-natural amino acids in addition to or instead of the corresponding the natural amino acids identified herein. Such modifications include, but are not limited to acetylation, amidation, formylation, methylation, sulfation, and the like. Illustrative non-natural amino acids include, but are not limited to Ornithine, norleucine, norvaline, N-methylvaline, 6-N-methyllysine, N-methylisoleucine, N-methylglycine, sarcosine, inosine, allo-isoleucine, isodesmolysine, 4-hydroxyproline, 3-hydroxyproline, allo-hydroxylysine, hydoxylisine, N-ethylasparagine, N-ethylglycine, 2,3-diaminopropionic acid, 2,2'-diaminopropionic acid, desmosine, 2,4-diaminobutyric acid, 2-aminopimelic acid, 3-aminoisobutyric acid, 2-aminoisobutyric acid, 2-aminoheptanoic acid, 6-aminocaproic acid, 4-aminobutyric acid, 2-aminobutyric acid, beta-alanine, 3-aminoadipic acid, 2-aminoadipic acid, and the like. In certain embodiments and one or more of the "natural" amino acids of the peptides described herein, can be substituted with the corresponding non-natural amino acid (e.g. as describe above).

In certain embodiments, this invention contemplates particularly the use of modified lysines. Such modifications include, but are not limited to, biotin modification of epsilon lysines and/or methylation of the epsilon lysines. Illustrative peptide comprising epsilon methylated lysines include, but are not limited to: Ac-D-W-F-K(eCH₃)₂-A-F-Y-D-K(eCH₃)₂-V-A-E-K(eCH₃)-₂-F-K(eCH₃)₂-E-A-F-NH(CH₃)₂ (SEQ ID NO:573) and: Ac-DWFK(eCH₃)₂AFYDK(eCH₃)₂VAEK(eCH₃)₂FK(eCH₃)₂EAF-NH(CH₃) (SEQ ID NO:574). Other modified amino acids include but are not limited to ornithine analogs and homoaminoalanine analogs (instead of (CH₂)₄--NH₂ for Lys it can be --(CH₂)₂--NH₂ for Haa and --(CH₂)₃--NH₂ for Orn] and the like. It is noted that these modifications are illustrative and not intended to be limiting. Illustrative 4F analogues that possess modified amino acids are shown in Table 6.

**TABLE 6: Illustrative 4F analogs that comprise modified amino acids.**

| εN-Dimethyl-Lys derivative of 4F (εN-Dime) |
|---|
| |
| |
| |
| |

| εN-Diethyl-Lys derivatives of 4F (εN-Diet) |
|---|
| |
| |
| |

| εN-Monomethyl-Lys derivative of 4F (εN -Me) |
|---|
| |
| |
| |

| εN-ethylLys derivative of 4F (εN -Et) |
|---|
| |
| |
| |

| HomoLys analogs of 4F (hK) (--CH₂)₅-NH₂ |
|---|
| |
| |
| |
| |
| |
| |
| |
| |
| |
| |
| |

| 4F analogs in which K is replaced O (O=Ornithine, --(CH₂)₃-NH₂) |
|---|
| Ac-D-W-F-O-A-F-Y-D-O-V-A-E-O-F-O-E-A-F-NH₂(SEQ ID NO:598) |
| |
| |
| |
| |
| |
| |
| |
| |
| |
| |

### iii. Dual Domain Peptides

Dual domain peptides are also disclosed. Dual domain peptides can be synthetic Apo E mimetics, consisting of a combination of the disclosed receptor binding domains of apolipoprotein E and the disclosed lipid-associating peptides, wherein said receptor binding domain is covalently linked to said lipid-associating peptide.

Also disclosed are synthetic Apo E mimetics, consisting of a combination of the disclosed receptor binding domains of apolipoprotein B and the disclosed lipid-associating peptides, wherein said receptor binding domain is covalently linked to said lipid-associating peptide. Non-limiting examples of the disclosed synthetic Apo E mimetics are provided in Table 7. The disclosed synthetic Apo E mimetics can also be N-terminally protected using acetyl and amino groups.

| **Table 7-Non-limiting Examples of the Disclosed Synthetic Apo E mimetics** | |
|---|---|
| **Receptor Binding Domains of Apo E** | **Lipid-Associating Peptides** |
| **LR**KLR**KRL**L**R (SEQ ID NO:4)** | DWLKAFYDKVAEKLKEAF (SEQ ID NO:5) |
| **LR**KLR**KRL**L**R (SEQ ID NO:4)** | DWLKAFYDKVAEKLKEAF (SEQ ID NO:5) |
| **LR**KLR**KRL**L**R (SEQ ID NO:4)** | DWLKAFYDKVAEKLKEAF (SEQ ID NO:5) |
| **LR**K*M*R**KRL***M***R (SEQ ID NO:7)** | DWLKAFYDKVAEKLKEAF (SEQ ID NO:5) |
| **LR**K*M*R**KRL***M***R (SEQ ID NO:7)** | DWLKAFYDKVAEKLKEAF (SEQ ID NO:5) |
| **LR**KL***P*KRL**L**R (SEQ ID NO:8)** | DWLKAFYDKVAEKLKEAF (SEQ ID NO:5) |
| **LR***N*V**RKRL***V***R (SEQ ID NO:9)** | DWLKAFYDKVAEKLKEAF (SEQ ID NO:5) |
| *M*RKLRKR*V*LR **(SEQ ID NO:10)** | DWLKAFYDKVAEKLKEAF (SEQ ID NO:5) |
| **LR***R*LR*R***RL**L**R (SEQ ID NO:11)** | DWLKAFYDKVAEKLKEAF (SEQ ID NO:5) |
| **LR**KLR**KR*FF*R (SEQ ID NO:12)** | DWLKAFYDKVAEKLKEAF (SEQ ID NO:5) |
| **LR**KLR**KRL**L**R (SEQ ID NO:4)** | DWFKAFYDKVAEKFKEAF (SEQ ID NO:16) |
| **LR**KLR**KRL**L**R (SEQ ID NO:4)** | DWFKAFYDKVAEKFKEAF (SEQ ID NO:16) |
| **LR**KLR**KRL**L**R (SEQ ID NO:4)** | DWFKAFYDKVAEKFKEAF (SEQ ID NO:16) |
| **LR**K*M*R**KRL***M***R (SEQ ID NO:7)** | DWFKAFYDKVAEKFKEAF (SEQ ID NO:16) |
| **LR**K*M*R**KRL***M***R (SEQ ID NO:7)** | DWFKAFYDKVAEKFKEAF (SEQ ID NO:16) |
| **LR**KL***P*KRL**L**R (SEQ ID NO:8)** | DWFKAFYDKVAEKFKEAF (SEQ ID NO:16) |
| **LR***N*VR**KRL***V***R (SEQ ID NO:9)** | DWFKAFYDKVAEKFKEAF (SEQ ID NO:16) |
| *M*RKLRKR*V*LR **(SEQ ID NO:10)** | DWFKAFYDKVAEKFKEAF (SEQ ID NO:16) |
| **LR***R*LR*R***RL**L**R (SEQ ID NO:11)** | DWFKAFYDKVAEKFKEAF (SEQ ID NO:16) |
| **LR**KLR**KR*FF*R (SEQ ID NO:12)** | DWFKAFYDKVAEKFKEAF (SEQ ID NO:16) |

In one aspect of the disclosure, the synthetic Apo E mimetics, consist of: a receptor binding domain of apolipoprotein E comprising the amino acid sequence of LRKLRKRFFR (SEQ ID NO:12); and a lipid-associating peptide, wherein said receptor binding domain is covalently linked to said lipid-associating peptide. As such, the receptor binding domain replaced the two leucine (L) residues at positions 148 and 149 of LRKLRKRLLR (SEQ ID NO:4) (hApoE[141-150], LRKLRKRLLR; SEQ ID NO:4) with two phenylalanine (F) residues.

In another aspect of the disclosure, the synthetic Apo E mimetics, comprise: a lipid binding domain of apolipoprotein E comprising the amino acid sequence of E-W-L-K-A-F-Y-D-K-V-F-E-K-F-K-E-A-F (SEQ ID NO:28); and a receptor binding domain peptide, wherein said lipid binding domain is covalently linked to said receptor binding domain peptide. As such, the lipid binding domain replaced the two leucine (L) residues of DWLKAFYDKVAEKLKEAF (SEQ ID NO:5) (18A) with two phenylalanine (F) residues resulting in the sequence DWFKAFYDKVAEKFKEAF (SEQ ID NO:16), also referred to as modified 18A or m18A).

### a. Domain switched peptides

Also disclosed are synthetic Apo E mimetics, consisting of a combination of the disclosed receptor binding domains of apolipoprotein E and the disclosed lipid-associating peptides, wherein said receptor binding domain is covalently linked to said lipid-associating peptide in a domain switched orientation. Also disclosed are synthetic Apo E mimetics, consisting of a combination of the disclosed receptor binding domains of apolipoprotein B and the disclosed lipid-associating peptides, wherein said receptor binding domain is covalently linked to said lipid-associating peptide in a domain switched orientation. These peptides can be referred to as "domain switched" "switched domain", or "switched" peptides. For example, disclosed are synthetic Apo E mimetics, consisting of a combination of the disclosed receptor binding domains of apolipoprotein E and the disclosed lipid-associating peptides, wherein said receptor binding domain is covalently linked to said lipid-associating peptide in a domain switched orientation to those described above and in Table 7. Specifically, the lipid-associating peptide is covalently linked to the receptor binding domain of apolipoprotein E such that the lipid-associating peptide is at the N-terminus of the synthetic apolipoprotein E-mimicking peptide. Non-limiting examples of the disclosed synthetic Apo E mimetics are provided in Table 8.

| **Table 8 - Non-limiting Examples of Disclosed Synthetic Apo E mimetics** | |
|---|---|
| **Lipid-Associating Peptides** | **Receptor Binding Domains of Apo E** |
| DWLKAFYDKVAEKLKEAF (SEQ ID NO:5) | **LR**KLR**KRL**L**R (SEQ ID NO:6)** |
| DWLKAFYDKVAEKLKEAF (SEQ ID NO:5) | **LR**KLR**KRL**L**R (SEQ ID NO:6)** |
| DWLKAFYDKVAEKLKEAF (SEQ ID NO:5) | **LR**KLR**KRL**L**R (SEQ ID NO:6)** |
| DWLKAFYDKVAEKLKEAF (SEQ ID NO:5) | **LR**K*M***RKRL***M***R (SEQ ID NO:7)** |
| DWLKAFYDKVAEKLKEAF (SEQ ID NO:5) | **LR**K*M*R**KRL*M*R (SEQ ID NO:7)** |
| DWLKAFYDKVAEKLKEAF (SEQ ID NO:5) | **LR**KL***P*KR**LL**R (SEQ ID NO:8**) |
| DWLKAFYDKVAEKLKEAF (SEQ ID NO:5) | **LR***NV***RKRL***V***R (SEQ ID NO:9)** |
| DWLKAFYDKVAEKLKEAF (SEQ ID NO:5) | *M*RKLRKR*V*LR (SEQ ID NO:10) |
| DWLKAFYDKVAEKLKEAF (SEQ ID NO:5) | **LR***R*LR*R***RL**LR **(SEQ ID NO:11)** |
| DWLKAFYDKVAEKLKEAF (SEQ ID NO:5) | **LR**KLR**KR*FF*R (SEQ ID NO:12)** |
| DWFKAFYDKVAEKFKEAF (SEQ ID NO:16) | **LR**KLR**KRL**L**R (SEQ ID NO:4)** |
| DWFKAFYDKVAEKFKEAF (SEQ ID NO:16) | **LR**KLR**KRL**L**R (SEQ ID NO:4)** |
| DWFKAFYDKVAEKFKEAF (SEQ ID NO:16) | **LR**KL**RKRL**L**R (SEQ ID NO:4)** |
| DWFKAFYDKVAEKFKEAF (SEQ ID NO:16) | **LR**K*M*R**KRL***M***R (SEQ ID NO:7)** |
| DWFKAFYDKVAEKFKEAF (SEQ ID NO:16) | **LR**K*M*R**KRL***M***R (SEQ ID NO:7)** |
| DWFKAFYDKVAEKFKEAF (SEQ ID NO:16) | **LR**KL***P*KRL**LR **(SEQ ID NO:8)** |
| DWFKAFYDKVAEKFKEAF (SEQ ID NO:16) | **LR***NV***RKR**L*V*R **(SEQ ID NO:9)** |
| DWFKAFYDKVAEKFKEAF (SEQ ID NO:16) | *M*RKLRKR*V*LR (SEQ ID NO:10) |
| DWFKAFYDKVAEKFKEAF (SEQ ID NO:16) | **LR***R*LR*R***RL**L**R (SEQ ID NO:11)** |
| DWFKAFYDKVAEKFKEAF (SEQ ID NO:16) | **LR**KLR**KR*FF*R (SEQ ID NO:12)** |

The disclosed domain switched synthetic Apo E mimetics can also be N-terminally protected using acetyl and amino groups.

### b. Peptides with reverse orientation

Also disclosed are synthetic Apo E mimetics, consisting of a combination of the disclosed receptor binding domains of apolipoprotein E and the disclosed lipid-associating peptides, wherein said receptor binding domain is covalently linked to said lipid-associating peptide in a reversed orientation. For example, disclosed are synthetic Apo E mimetics, consisting of a combination of the disclosed receptor binding domains of apolipoprotein E and the disclosed lipid-associating peptides, wherein either the sequence of the receptor binding domain or the sequence of the lipid-associating peptide or both sequences are in the reversed orientation. Also disclosed are synthetic Apo E mimetics, consisting of a combination of the disclosed receptor binding domains of apolipoprotein B and the disclosed lipid-associating peptides, wherein said receptor binding domain is covalently linked to said lipid-associating peptide in a reversed orientation. Non-limiting examples of the disclosed synthetic Apo E mimetics are provided in Table 9.

| **Table 9-Non-limiting Examples of Synthetic Apo E mimetics** | |
|---|---|
| **Receptor Binding Domains of Apo E** | **Lipid-Associating Peptides** |
| RLLRKRLKRL (SEQ ID NO:609) | DWLKAFYDKVAEKLKEAF (SEQ ID NO:5) |
| RLLRKRLKRL (SEQ ID NO:609) | DWLKAFYDKVAEKLKEAF (SEQ ID NO:5) |
| RLLRKRLKRL (SEQ ID NO:609) | DWLKAFYDKVAEKLKEAF (SEQ ID NO:5) |
| RMLRKRMKRL (SEQ ID N0:610) | DWLKAFYDKVAEKLKEAF (SEQ ID NO:5) |
| RMLRKRMKRL (SEQ ID N0:610) | DWLKAFYDKVAEKLKEAF (SEQ ID NO:5) |
| RLLRKPLKRL (SEQ ID NO:611) | DWLKAFYDKVAEKLKEAF (SEQ ID NO:5) |
| RVLRKRVNRL (SEQ ID NO:612) | DWLKAFYDKVAEKLKEAF (SEQ ID NO:5) |
| RLVRKRLKRM (SEQ ID NO:613) | DWLKAFYDKVAEKLKEAF (SEQ ID NO:5) |
| RLLRRRLRRL (SEQ ID NO:614) | DWLKAFYDKVAEKLKEAF (SEQ ID NO:5) |
| RFFRKRLKRL (SEQ ID NO:615) | DWLKAFYDKVAEKLKEAF (SEQ ID NO:5) |
| RLLRKRLKRL (SEQ ID NO:609) | DWFKAFYDKVAEKFKEAF (SEQ ID NO:16) |
| RLLRKRLKRL (SEQ ID NO:609) | DWFKAFYDKVAEKFKEAF (SEQ ID NO:16) |
| RLLRKRLKRL (SEQ ID NO:609) | DWFKAFYDKVAEKFKEAF (SEQ ID NO:16) |
| RMLRKRMKRL (SEQ ID N0:610) | DWFKAFYDKVAEKFKEAF (SEQ ID NO:16) |
| RMLRKRMKRL (SEQ ID N0:610) | DWFKAFYDKVAEKFKEAF (SEQ ID NO:16) |
| RLLRKPLKRL (SEQ ID NO:611) | DWFKAFYDKVAEKFKEAF (SEQ ID NO:16) |
| RVLRKRVNRL (SEQ ID NO:612) | DWFKAFYDKVAEKFKEAF (SEQ ID NO:16) |
| RLVRKRLKRM (SEQ ID NO:613) | DWFKAFYDKVAEKFKEAF (SEQ ID NO:16) |
| RLLRRRLRRL (SEQ ID NO:614) | DWFKAFYDKVAEKFKEAF (SEQ ID NO:16) |
| RFFRKRLKRL (SEQ ID NO:615) | DWFKAFYDKVAEKFKEAF (SEQ ID NO:16) |
| LRKLRKRLLR (SEQ ID NO:4) | FAEKLKEAVKDYFAKLWD (SEQ ID NO:616) |
| LRKLRKRLLR (SEQ ID NO:4) | FAEKLKEAVKDYFAKLWD (SEQ ID NO:616) |
| LRKLRKRLLR (SEQ ID NO:4) | FAEKLKEAVKDYFAKLWD (SEQ ID NO:616) |
| LRK*M*RKRL*M*R (SEQ ID NO:7) | FAEKLKEAVKDYFAKLWD (SEQ ID NO:616) |
| LRK*M*RKRL*M*R (SEQ ID NO:7) | FAEKLKEAVKDYFAKLWD (SEQ ID NO:616) |
| LRKL*P*KRLLR (SEQ ID NO:4) | FAEKLKEAVKDYFAKLWD (SEQ ID NO:616) |
| LR*NV*RKRL*V*R (SEQ ID NO:9) | FAEKLKEAVKDYFAKLWD (SEQ ID NO:616) |
| *M*RKLRKR*V*LR (SEQ ID NO:10) | FAEKLKEAVKDYFAKLWD (SEQ ID NO:616) |
| LR*R*LR*R*RLLR (SEQ ID NO:6) | FAEKLKEAVKDYFAKLWD (SEQ ID NO:616) |
| LRKLRKR*FF*R (SEQ ID NO: 12) | FAEKLKEAVKDYFAKLWD (SEQ ID NO:616) |
| LRKLRKRLLR (SEQ ID NO:4) | FAEKFKEAVKDYFAKFWD (SEQ ID NO:617) |
| LRKLRKRLLR (SEQ ID NO:4) | FAEKFKEAVKDYFAKFWD (SEQ ID NO:617) |
| LRKLRKRLLR (SEQ ID NO:4) | FAEKFKEAVKDYFAKFWD (SEQ ID NO:617) |
| LRK*M*RKRL*M*R (SEQ ID NO:7) | FAEKFKEAVKDYFAKFWD (SEQ ID NO:617) |
| LRK*M*RKRL*M*R (SEQ ID NO:7) | FAEKFKEAVKDYFAKFWD (SEQ ID NO:617) |
| LRKL*P*KRLLR (SEQ ID NO:8) | FAEKFKEAVKDYFAKFWD (SEQ ID NO:617) |
| LR*NV*RKRL*V*R (SEQ ID NO:9) | FAEKFKEAVKDYFAKFWD (SEQ ID NO:617) |
| *M*RKLRKR*V*LR (SEQ ID NO:10) | FAEKFKEAVKDYFAKFWD (SEQ ID NO:617) |
| LR*R*LR*R*RLLR (SEQ ID NO:4) | FAEKFKEAVKDYFAKFWD (SEQ ID NO:617) |
| LRKLRKR*FF*R (SEQ ID NO: 12) | FAEKFKEAVKDYFAKFWD (SEQ ID NO:617) |

### c. Scrambled peptides

Also disclosed are synthetic Apo E mimetics, consisting of: a receptor binding domain of apolipoprotein E and a lipid-associating peptide, wherein said receptor binding domain is covalently linked to said lipid-associating peptide, wherein the receptor binding domain of apolipoprotein E is scrambled. For example, disclosed is a synthetic apolipoprotein E-mimicking peptide, consisting of: a receptor binding domain of apolipoprotein E comprising the amino acid sequence of D-W-L-K-A-F-V-Y-D-K-V-F-K-L-K-E-F-F (SEQ ID NO:69); and a lipid-associating peptide, wherein said receptor binding domain is covalently linked to said lipid-associating peptide. Also disclosed are synthetic Apo E mimetics, consisting of: a receptor binding domain of apolipoprotein B and a lipid-associating peptide, wherein said receptor binding domain is covalently linked to said lipid-associating peptide, wherein the receptor binding domain of apolipoprotein B is scrambled.

Also disclosed are synthetic Apo E mimetics, consisting of: a receptor binding domain of apolipoprotein E and a lipid-associating peptide, wherein said receptor binding domain is covalently linked to said lipid-associating peptide, wherein the lipid-associating peptide is scrambled. For example, disclosed herein is a synthetic Apo E mimetics, comprising: a lipid binding domain of apolipoprotein E comprising the amino acid sequence of E-W-L-K-A-F-V-Y-E-K-V-F-K-L-K-E-F-F (SEQ ID NO:70) and a receptor binding domain peptide, wherein said lipid binding domain is covalently linked to said receptor binding domain peptide.

Also disclosed are synthetic Apo E mimetics, consisting of: a receptor binding domain of apolipoprotein E and a lipid-associating peptide of apolipoprotein E, wherein receptor binding domain is covalently linked to said lipid-associating peptide, wherein both the receptor binding domain and the lipid-associating peptide are scrambled. Non-limiting examples of the disclosed scrambled synthetic Apo E mimetics are provided in Table 10.

| **Table 10 - Scrambled Synthetic Apolipoprotein E-Mimicking Peptides** | | |
|---|---|---|
| **Name** | **Receptor Binding Domains of Apo E** | **Lipid-Associating Peptides** |
| hE-Sc 18A (hE with Sc18A also referred to as Sc2F) | LRKLRKRLLR (SEQ ID NO:4) | KAFEEVLAKKFYDKALWD (SEQ ID NO:618) |
| SchE-18A | LRLLRKLKRR (SEQ ID NO:619) | DWLKAFYDKVAEKLKEAF (SEQ ID NO:5) |

The disclosed scrambled synthetic Apo E mimetics can also be N-terminally and C-terminally protected using acetyl and amide groups. The disclosed scrambled synthetic Apo E mimetics can also be reverse-oriented as described above.

### d. Linkages

Any suitable linker can be used in accordance with the present invention. The peptide linkages can be selected from the group consisting of: --CH₂NH--, --CH₂S--, --CH₂-CH₂--, --CH=CH-- (cis and trans), --COCH₂--, --CH(OH)CH₂--, --CH₂SO--, etc. by methods known in the art and further described in the following references: Spatola (1983) p. 267 in Chemistry and Biochemistry of Amino Acids, Peptides, and Proteins, B. Weinstein, eds., Marcel Dekker, New York; Spatola (1983) Vega Data 1(3) Peptide Backbone Modifications. (general review); Morley (1980) Trends Pharm Sci pp. 463-468 (general review); Hudson et al. (1979) Int J PeptProt Res 14:177-185 (--CH₂NH--, CH₂CH₂--); Spatola et al. (1986) Life Sci 38:1243-1249 (--CH₂--S); Hann, (1982) J ChemSoc Perkin Trans I 307-314 (--CH--CH--, cis and trans); Almquist et al. (1980) J Med. Chem. 23:1392-1398 (--COCH₂--); Jennings-White et al. (1982) Tetrahedron Lett. 23:2533 (--COCH₂--); Szelke et al., European Appln. EP 45665 (1982) CA: 97:39405 (1982) (--CH(OH)CH2-); Holladay et al. (1983) Tetrahedron Lett 24:4401-4404 (--C(OH)CH₂--); and Hruby (1982) Life Sci., 31:189-199 (--CH₂--S--)).

One particularly preferred non-peptide linkage is --CH₂NH--. Such peptide mimetics may have significant advantages over polypeptide embodiments, including, for example: more economical production, greater chemical stability, enhanced pharmacological properties (half-life, absorption, potency, efficacy, etc.), reduced antigenicity, and others.

In one aspect, the linker is a cleavable linker. To give but a few examples, cleavable linkers include protease cleavable peptide linkers, nuclease sensitive nucleic acid linkers, lipase sensitive lipid linkers, glycosidase sensitive carbohydrate linkers, pH sensitive linkers, hypoxia sensitive linkers, photo-cleavable linkers, heat-labile linkers, enzyme cleavable linkers (e.g., esterase cleavable linker), ultrasound-sensitive linkers, x-ray cleavable linkers, etc.

### iv. Variants

The receptor binding domain or the lipid-associating peptide can be modified or altered as described above. For example, the receptor binding domain or the lipid-associating peptide can be mutated, scrambled, and/or reverse-oriented. Any other modifications or alterations disclosed herein for the dual-domain polypeptides can also be used for the single-domain peptides.

Numerous other variants or derivatives of the peptides disclosed herein are also contemplated. For example, scrambled peptides can also be reverse-oriented, or can be in a switched orientation. Additionally, reverse-oriented peptides can be in a switched orientation. All other combinations of the disclosed peptides are also contemplated. Non-limiting examples of the peptides have been described herein (see Tables 1-5, for example). As used herein, the term "analog" is used interchangeably with "variant" and "derivative." Variants and derivatives are well understood to those of skill in the art and can involve amino acid sequence modifications. Such, amino acid sequence modifications typically fall into one or more of three classes: substantial; insertional; or deletional variants. Insertions include amino and/or carboxyl terminal fusions as well as intrasequence insertions of single or multiple amino acid residues. Insertions ordinarily are smaller insertions than those of amino or carboxyl terminal fusions, for example, on the order of one to four residues. These variants ordinarily are prepared by site-specific mutagenesis of nucleotides in the DNA encoding the protein, thereby producing DNA encoding the variant, and thereafter expressing the DNA in recombinant cell culture. Techniques for making substitution mutations at predetermined sites in DNA having a known sequence are well known, for example M13 primer mutagenesis and PCR mutagenesis. Amino acid substitutions are typically of single residues, but can occur at a number of different locations at once. Substitutions, deletions, insertions or any combination thereof may be combined to arrive at a final derivative or analog. Substitutional variants are those in which at least one residue has been removed and a different residue inserted in its place. Such substitutions generally are made in accordance with Tables 11 and 12 and are referred to as conservative substitutions.

Substantial changes in function or immunological identity are made by selecting substitutions that are less conservative than those in Table 11, i.e., selecting residues that differ more significantly in their effect on maintaining (a) the structure of the polypeptide backbone in the area of the substitution, for example as a sheet or helical conformation, (b) the charge or hydrophobicity of the molecule at the target site, or (c) the bulk of the side chain. The substitutions which in general are expected to produce the greatest changes in the protein properties are those in which: (a) the hydrophilic residue, e.g. seryl or threonyl, is substituted for (or by) a hydrophobic residue, e.g., leucyl, isoleucyl, phenylalanyl, valyl or alanyl; Tryptophan, Tyrosinyl (b) a cysteine or proline is substituted for (or by) any other residue; (c) a residue having an electropositive side chain, e.g., lysyl, arginyl, or hystidyl, is substituted for (or by) an electronegative residue, e.g. glutamyl or aspartyl; or (d) a residue having a bulky side chain, e.g., phenylalanine, is substituted for (or by) one not having a side chain, e.g., glycine, in this case, or (e) by increasing the number of sites for sulfation and/or glycosylation.

| **Table 11 - Amino Acid Substitutions** | |
|---|---|
| **Original Residue** | **Non-limiting Exemplary Conservative Substitutions** |
| Ala | Ser |
| Arg | Gly; Gln; Lys |
| Asn | Gln; His |
| Asp | Glu |
| Cys | Ser |
| Gln | Asn; Lys |
| Glu | Asp |
| Gly | Ala |
| His | Asn; Gln |
| Ile | Leu; Val |
| Leu | Ile; Val |
| Lys | Arg; Gln |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

| **TABLE 12 - Amino Acid Abbreviations** | |
|---|---|
| **Amino Acid** | **Abbreviations** |
| Alanine | Ala (A) |
| Allosoleucine | AIle |
| Arginine | Arg (R) |
| Asparagine | Asn (N) |
| Aspartic Acid | Asp (D) |
| Cysteine | Cys (C) |
| Glutamic Acid | Glu (E) |
| Glutamine | Gln (Q) |
| Glycine | Gly (G) |
| Histidine | His (H) |
| Isolelucine | He (I) |
| Leucine | Leu (L) |
| Lysine | Lys (K) |
| Phenylalanine | Phe (F) |
| Praline | Pro (P) |
| Pyroglutamic Acid | PGlu (U) |
| Serine | Ser (S) |
| Threonine | Thr (T) |
| Tyrosine | Tyr (Y) |
| Tryptophan | Trp (W) |
| Valine | Val (V) |

It is understood that one way to define the variants and derivatives of the disclosed proteins herein is to define them in terms of homology/identity to specific known sequences. Specifically disclosed are variants of synthetic Apo E mimetics and other proteins or peptides herein disclosed which have at least, 70% or at least 75% or at least 80% or at least 85% or at least 90% or at least 95% homology to the synthetic Apo E mimetics specifically recited herein. Those of skill in the art readily understand how to determine the homology of two proteins.

The polypeptides can be modified by either natural processes, such as posttranslational processing, or by chemical modification techniques which are well known in the art. Modifications can occur anywhere in the polypeptide, including the peptide backbone, the amino acid side-chains and the amino or carboxyl termini. The same type of modification can be present in the same or varying degrees at several sites in a given polypeptide. Also, a given polypeptide can have many types of modifications. Modifications include, without limitation, acetylation, acylation, ADP-ribosylation, amidation, covalent cross-linking or cyclization, covalent attachment of flavin, covalent attachment of a heme moiety, covalent attachment of a nucleotide or nucleotide derivative, covalent attachment of a lipid or lipid derivative, covalent attachment of a phosphytidylinositol, disulfide bond formation, demethylation, formation of cysteine or pyroglutamate, formylation, gamma-carboxylation, glycosylation, GPI anchor formation, hydroxylation, iodination, methylation, yristolyation, oxidation, pergylation, proteolytic processing, phosphorylation, prenylation, racemization, selenoylation, sulfation, and transfer-RNA mediated addition of amino acids to protein such as arginylation. (See Proteins - Structure and Molecular Properties 2nd Ed., T.E. Creighton, W.H. Freeman and Company, New York (1993); Posttranslational Covalent Modification of Proteins, B.C. Johnson, Ed., Academic Press, New York, pp. 1-12 (1983)).

Variants can also include peptidomimetics. As used herein, "peptidomimetic" means a mimetic of a function of a protein which includes some alteration of the normal peptide chemistry. Peptidomimetics typically are short sequences of amino acids that in biological properties, mimic one or more function(s) of a particular protein. Peptide analogs enhance some property of the original peptide, such as increases stability, increased efficacy, enhanced delivery, increased half-life, etc. Methods of making peptidomimetics based upon a known polypeptide sequence is described, for example, in U.S. Patent Nos. 5,631,280; 5,612,895; and 5,579,250. Use of peptidomimetics can involve the incorporation of a non-amino acid residue with non-amide linkages at a given position. One embodiment of the present invention is a peptidomimetic wherein the compound has a bond, a peptide backbone or an amino acid component replaced with a suitable mimic. Some non-limiting examples of unnatural amino acids which may be suitable amino acid mimics include β-alanine, L-α-amino butyric acid, L-γ-amino butyric acid, L-α-amino isobutyric acid, L-ε-amino caproic acid, 7-amino heptanoic acid, L-aspartic acid, L-glutamic acid, N-ε-Boc-N-α-CBZ-L-lysine, N-ε-Boc-N-α-Fmoc-L-lysine, L-methionine sulfone, L-norleucine, L-norvaline, N-α-Boc-N-δCBZ-L-ornithine, N-δ-Boc-N-α-CBZ-L-omithine, Boc-p-nitro-L-phenylalanine, Boc-hydroxyproline, and Boc-L-thioproline.

### v. Nucleic Acids

As this specification discusses various peptide sequences it is understood that the nucleic acids that can encode those polypeptide sequences are also disclosed. This would include all degenerate sequences related to a specific polypeptide sequence, i.e. all nucleic acids having a sequence that encodes one particular polypeptide sequence as well as all nucleic acids, including degenerate nucleic acids, encoding the disclosed variants and derivatives of the protein sequences. Thus, while each particular nucleic acid sequence may not be written out herein, it is understood that each and every sequence is in fact disclosed and described herein through the disclosed polypeptide sequences.

### vi. Blocking/Protecting Groups and D Residues

While the various compositions described herein may be shown with no protecting groups, in certain embodiments (*e.g.*, particularly for oral administration), they can bear one, two, three, four, or more protecting groups. The protecting groups can be coupled to the C- and/or N-terminus of the peptide(s) and/or to one or more internal residues comprising the peptide(s) (*e.g.,* one or more R-groups on the constituent amino acids can be blocked). Thus, for example, in certain embodiments, any of the peptides described herein can bear, *e.g.,* an acetyl group protecting the amino terminus and/or an amide group protecting the carboxyl terminus. One example of such a "dual protected peptide" is Ac-LRKLRKRLLRDWLKAFYDKVAEKLKEAF-NH₂ (SEQ ID NO:1) with blocking groups), either or both of these protecting groups can be eliminated and/or substituted with another protecting group as described herein. Without being bound by a particular theory, it was a discovery of this invention that blockage, particularly of the amino and/or carboxyl termini of the subject peptides of this invention can improve oral delivery and can also increase serum half-life.

A wide number of protecting groups are suitable for this purpose. Such groups include, but are not limited to acetyl, amide, and alkyl groups with acetyl and alkyl groups being particularly preferred for N-terminal protection and amide groups being preferred for carboxyl terminal protection. For example, the protecting groups can include, but are not limited to alkyl chains as in fatty acids, propeonyl, formyl, and others. Carboxyl protecting groups include amides, esters, and ether-forming protecting groups can also be used. For example, an acetyl group can be used to protect the amino terminus and an amide group can be used to protect the carboxyl terminus. These blocking groups enhance the helix-forming tendencies of the peptides. Additional blocking groups include alkyl groups of various lengths, e.g., groups having the formula: CH₃(CH₂)ₙCO where n ranges from about 1 to about 20, preferably from about 1 to about 16 or 18, more preferably from about 3 to about 13, and most preferably from about 3 to about 10.

Additionally, the protecting groups include, but are not limited to alkyl chains as in fatty acids, propeonyl, formyl, and others. For example, carboxyl protecting groups can include amides, esters, and ether-forming protecting groups. These blocking groups can enhance the helix-forming tendencies of the peptides. Blocking groups can include alkyl groups of various lengths, e.g., groups having the formula: CH₃(CH₂)ₙCO where n ranges from about 3 to about 20, preferably from about 3 to about 16, more preferably from about 3 to about 13, and most preferably from about 3 to about 10.

Other protecting groups include, but are not limited to Fmoc, t-butoxycarbonyl (t-BOC), 9-fluoreneacetyl group, 1-fluorenecarboxylic group, 9-florenecarboxylic group, 9-fluorenone-1-carboxylic group, benzyloxycarbonyl, Xanthyl (Xan), Trityl (Trt), 4-methyltrityl (Mtt), 4-methoxytrityl (Mmt), 4-methoxy-2,3,6-trimethyl-benzenesulphonyl (Mtr), Mesitylene-2-sulphonyl (Mts), 4,4-dimethoxybenzhydryl (Mbh),Tosyl (Tos), 2,2,5,7,8-pentamethyl chroman-6-sulphonyl (Pmc), 4-methylbenzyl (MeBzl), 4-methoxybenzyl (MeOBzl), Benzyloxy (BzlO), Benzyl (Bzl), Benzoyl (Bz), 3-nitro-2-pyridinesulphenyl (Npys), 1-(4,4-dimentyl-2,6-diaxocyclohexylidene)ethyl (Dde), 2,6-dichlorobenzyl (2,6-DiCl-Bzl), 2-chlorobenzyloxycarbonyl (2-C1-Z), 2-bromobenzyloxycarbonyl (2-Br-Z), Benzyloxymethyl (Bom), cyclohexyloxy (cHxO),t-butoxymethyl (Bum), t-butoxy (tBuO), t-Butyl (tBu), Acetyl (Ac), and Trifluoroacetyl (TFA).

Protecting/blocking groups are well known to those of skill as are methods of coupling such groups to the appropriate residue(s) comprising the peptides of this invention (see, *e.g.,* Greene et al., (1991) Protective Groups in Organic Synthesis, 2nd ed., John Wiley & Sons, Inc. Somerset, N.J.). For example, acetylation can be accomplished during the synthesis when the peptide is on the resin using acetic anhydride. Amide protection can be achieved by the selection of a proper resin for the synthesis.

The compositions disclosed herein can also comprise one or more D-form (dextro rather than levo) amino acids as described herein. For example, at least two enantiomeric amino acids, at least 4 enantiomeric amino acids or at least 8 or 10 enantiomeric amino acids can be in the "D" form amino acids. Additionally, every other, or even every amino acid (*e.g.,* every enantiomeric amino acid) of the peptides described herein is a D-form amino acid.

Additionally, at least 50% of the enantiomeric amino acids can be "D" form, at least 80% of the enantiomeric amino acids are "D" form, at least 90%, or even all of the enantiomeric amino acids can be in the "D" form amino acids.

### C. Methods of Treating

Methods of treating atherosclerosis, peripheral or cerebral vascular disease by administering an effective amount of an Apo E mimetic for at least one treatment cycle followed by a rest phase are provided. Thus, the disclosed methods involve administering an Apo E mimetic using one or more of the disclosed dosing regimens. Thus, any of the disclosed treatment cycles or rest phases can be used in the disclosed methods. The methods disclosed herein can allow for prolonged therapeutic effects even in the absence of the therapeutic. The disclosed methods can include the administration of an effective amount of Apo E mimetic. The effective amount of an Apo E mimetic can be an amount that allows for sustained therapeutic effects after the Apo E mimetic has been withdrawn.

Disclosed herein are methods of treating atherosclerosis comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase.

Disclosed herein are methods of treating atherosclerosis comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, wherein the rest phase is at least four weeks.

Disclosed herein are methods of treating atherosclerosis comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, further comprising a second treatment cycle after the rest phase. The second treatment cycle can be administered after a four week rest phase or one year from the beginning of the initial treatment cycle.

Disclosed herein are methods of treating atherosclerosis comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, wherein an atherosclerosis therapeutic other than an Apo E mimetic is administered during the rest phase. The atherosclerosis therapeutic other than an Apo E mimetic can be a conventional lipid lowering therapy and the conventional lipid lowering therapy can be a statin.

Disclosed herein are methods of treating atherosclerosis comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the Apo E mimetic consists of the amino acid sequence LRKLRKRLLRDWLKAFYDKVAEKLKEAF (SEQ ID NO:1), wherein the treatment cycle is followed by a rest phase, wherein Apo E mimetic is not administered during the rest phase.

Disclosed herein are methods of treating atherosclerosis comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, wherein the treatment cycle comprises administration of an effective amount of an Apo E mimetic once a week for three months.

Disclosed herein are methods of treating peripheral vascular disease comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase.

Peripheral vascular disease (PVD) can also refer to peripheral artery disease (PAD) or peripheral artery occlusive disease (PAOD) or peripheral obliterative arteriopathy. Peripheral vascular disease refers to the obstruction of large arteries not within the coronary, aortic arch vasculature, or brain. PVD can result from atherosclerosis, inflammatory processes leading to stenosis, an embolism, orthrombus formation. PVD can cause either acute or chronic ischemia (lack of blood supply). PVD can also refer to atherosclerotic blockages found in the lower extremity

Peripheral artery occlusive disease is commonly divided in the Fontaine stages, introduced by René Fontaine in 1954 for ischemia. The Fontain stages are provide below in the following Table 13.

**Table 13.**

| **Fontain Stage** | **Substage** | **Description** |
|---|---|---|
| Stage I | | Asymptomatic, incomplete blood vessel obstruction |
| Stage II | | Mild claudication |
| | Stage IIA | Claudication at a distance of greater than 200 metres |
| | Stage IIB | Claudication distance of less than 200 metres |
| Stage III | | Rest pain, mostly in the feet |
| Stage IV | | Necrosis and/or gangrene of the limb |

Peripheral artery occlusive disease can also refer to a classification designed by Rutherford which consists of three grades and six categories. The Rutherford stages are provide below in the following Table 14.

**Table 14.**

| **Rutherford Stage** | **Description** |
|---|---|
| Category 0 | Asymptomatic |
| Category 1 | Mild claudication |
| Category 2 | Moderate claudication |
| Category 3 | Severe claudication |
| Category 4 | Rest pain |
| Category 5 | Minor tissue loss; Ischemic ulceration not exceeding ulcer of the digits of the foot |
| Category 6 | Major tissue loss; Severe ischemic ulcers or frank gangrene |

Disclosed herein are methods of treating peripheral vascular disease comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, wherein the rest phase is at least four weeks.

Disclosed herein are methods of treating peripheral vascular disease comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, further comprising a second treatment cycle after the rest phase. The second treatment cycle can be administered after a four week rest phase or one year from the beginning of the initial treatment cycle.

Disclosed herein are methods of treating peripheral vascular disease comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, wherein a peripheral vascular disease therapeutic other than an Apo E mimetic is administered during the rest phase. The atherosclerosis therapeutic other than an Apo E mimetic can be a conventional lipid lowering therapy and the conventional lipid lowering therapy can be one or more of the following: a statin, an antiplatelet drus, aspirin, clopidogrel, Cilostazol, or pentoxifylline. The atherosclerosis therapeutic other than an Apo E mimetic can also include surgical intervention, smoking cessation, management of diabetes, management of hypertension management of cholesterol, exercise, Angioplasty (PTA or percutaneous transluminal angioplasty), plaque excision, bypass grafting, sympathectomy, amputation, or thrombolysis.

Disclosed herein are methods of treating peripheral vascular disease comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the Apo E mimetic consists of the amino acid sequence LRKLRKRLLRDWLKAFYDKVAEKLKEAF (SEQ ID NO:1), wherein the treatment cycle is followed by a rest phase, wherein Apo E mimetic is not administered during the rest phase.

Disclosed herein are methods of treating peripheral vascular disease comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, wherein the treatment cycle comprises administration of an effective amount of an Apo E mimetic once a week for three months.

Disclosed herein are methods of treating critical limb ischemia comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase.

Critical limb ischemia (CLI) is the most severe form of peripheral arterial disease (PAD) caused by chronic inflammatory processes associated with atherosclerosis that result in markedly reduced blood flow to the legs, feet and hands. There are an estimated 10-12 million people with PAD in the United States, and over 1 million people with CLI.

Disclosed herein are methods of treating critical limb ischemia comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, wherein the rest phase is at least four weeks.

Disclosed herein are methods of treating critical limb ischemia comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, further comprising a second treatment cycle after the rest phase. The second treatment cycle can be administered after a four week rest phase or one year from the beginning of the initial treatment cycle.

Disclosed herein are methods of treating critical limb ischemia comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, wherein an peripheral vascular disease therapeutic other than an Apo E mimetic is administered during the rest phase. The critical limb ischemia therapeutic other than an Apo E mimetic can be a conventional lipid lowering therapy and the conventional lipid lowering therapy can be one or more of the following: a statin, an antiplatelet drus, aspirin, clopidogrel, Cilostazol, or pentoxifylline. The atherosclerosis therapeutic other than an Apo E mimetic can also include surgical intervention, smoking cessation, management of diabetes, management of hypertension management of cholesterol, exercise, Angioplasty (PTA or percutaneous transluminal angioplasty), plaque excision, bypass grafting, sympathectomy, amputation, or thrombolysis.

Disclosed herein are methods of treating critical limb ischemia comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the Apo E mimetic consists of the amino acid sequence LRKLRKRLLRDWLKAFYDKVAEKLKEAF (SEQ ID NO:1), wherein the treatment cycle is followed by a rest phase, wherein Apo E mimetic is not administered during the rest phase.

Disclosed herein are methods of treating critical limb ischemia comprising administering to a subject an effective amount of an Apo E mimetic for at least one treatment cycle, wherein the treatment cycle comprises administering an effective amount of an Apo E mimetic to allow for a sustained therapeutic effect after withdrawal of the Apo E mimetic, wherein the treatment cycle is followed by a rest phase, wherein Apo E mimetic is not administered during the rest phase, wherein the treatment cycle comprises administration of an effective amount of an Apo E mimetic once a week for three months.

Any of the disclosed Apo E mimetics can be used in these methods. For example, the Apo E mimetic can be hE18A (LRKLRKRLLRDWLKAFYDKVAEKLKEAF; SEQ ID NO:1), in particular the Apo E mimetic can be the hE18A peptide that has an acetyl group on the amino terminus and an amide group at the carboxyl terminus.

The disclosed methods of treating can occur at different times depending on the subject. In particular, treatment can occur in a subject considered to be of high, or high residual risk of a cardio- or cerebrovascular event. In one instance, the treatment can be initiated after a subject is stabilized following an acute coronary event. In one instance, the treatment can be initiated immediately after the acute coronary event, or 3, 6, 9, or 12 months after the acute coronary event. The treatment can be initiated following acute interventional cardiology procedures such as coronary artery bypass surgery (CABG), percutaneous coronary intervention (angioplasty, PCI), or implant of a stent into a coronary artery. Subjects considered as high risk can be those individuals that have homozygous familial hypercholesterolemia (FH), severe refractory FH, diabetes or an individual following acute coronary syndrome (ACS). In high risk subjects, treatment can be extended.

### 1. Treatment cycle

The treatment cycle, as previously described with respect to the dosing regimens, can vary in length of time. The treatment cycle can be at least four weeks but can last up to six months. In one instance, the disclosed methods have a treatment cycle that involves the administration of an effective amount of an Apo E mimetic once a week for one month (four weeks), three months (12 weeks) or six months (24 weeks). A treatment cycle can include the administration of Apo E mimetic once, twice or three times a week. In some aspects, the Apo E mimetic can be administered daily. In some aspects, the Apo E mimetic can be administered once every two weeks or even once a month. In some instances, the Apo E mimetic can be administered every two weeks for 4, 6, 8, 10, 12 , 14, 16, 18, 20, 22, or 24 weeks. Each treatment cycle can include an established length of time for administration as well as an established dosing schedule during that time frame.

The methods can further include a second treatment cycle after the rest phase. In one aspect, the second treatment cycle can be administered after a four week rest phase. In another aspect, the second treatment cycle can be administered at least one year from the beginning of the initial treatment cycle.

### 2. Rest phase

The rest phase, as previously described with regards to the dosing regimen, can be at least four weeks but can last for several years. Apo E mimetic is not administered during the rest phase.

The length of the rest phase is dependent on how long the sustained therapeutic effects of the Apo E mimetic administered during the treatment cycle last. In some instances the rest phase can be at least 1, 2, 3, 4, 5, 6, 7, 8, 9, 10, 11, or 12 months. In some instances the rest phase can be at least 1, 2, 3, 4, 5, 6, 7, 8, 9, or 10 years. For example, the rest phase can be at least four weeks (one month).

In one aspect, the rest phase can be decreased or extended depending on the dose of Apo E mimetic administered during the treatment cycle. For example, the rest phase can be extended if the dose of Apo E mimetic during the treatment cycle is increased. The length of the rest phase can also vary based on the length of the treatment cycle. For instance, if a subject receives a certain dose of Apo E mimetic once a week for three months then the rest phase may be shorter than a subject that receives the same dose of Apo E mimetic once a week for six months.

Although an Apo E mimetic is not administered during the rest phase, an atherosclerosis therapeutic other than an Apo E mimetic can be administered during the rest phase. The atherosclerosis therapeutic other than an Apo E mimetic can be a conventional lipid lowering therapy, such as a statin, or bile acid sequestrant, and/or a therapeutic such as a PCSK9 inhibitor, a VLDL synthesis inhibitor and/or a CETP inhibitor.

### 3. Atherosclerosis

The combination of LDL accumulation in a vessel wall and an inflammatory response to the LDL's is responsible for initiating atherosclerosis. The LDL within the vessel wall becomes oxidized which damages the vessel wall and triggers an immune response. Immune cells, such as macrophages, are not able to process the oxidized-LDL and eventually rupture which leads to more oxidized cholesterol in the artery wall. This cycle continues which causes more and more damage to the vessel walls. The increase in cholesterol leads to plaques which ultimately results in hardening and narrowing of the vessel wall. The disclosed methods are useful for treating atherosclerosis.

### 4. Delivery

In the methods described herein, administration or delivery of the Apo E mimetics can be via a variety of mechanisms. As defined above, disclosed herein are dosing regimens and methods of using those dosing regimens to treat atherosclerosis. The dosing regimens and methods include compositions containing any one or more of the polypeptides or nucleic acids described herein that can also include a carrier such as a pharmaceutically acceptable carrier. For example, disclosed are pharmaceutical compositions, comprising the Apo E mimetics disclosed herein, and a pharmaceutically acceptable carrier.

The disclosed Apo E mimetics can be in solution or in suspension (for example, incorporated into microparticles, liposomes, or cells). These compositions can be targeted to a particular cell type via antibodies, receptors, or receptor ligands. One of skill in the art knows how to make and use such targeting agents with the disclosed compositions. A targeting agent can be a vehicle such as an antibody conjugated liposomes; receptor mediated targeting of DNA through cell specific ligands, and highly specific retroviral targeting of cells in vivo. Any such vehicles can be part of the compositions herein. For example, targeting agents that direct the Apo E mimetic to the blood vessel walls can be included in the compositions.

Any suitable route of administration can be used for the disclosed compositions. Suitable routes of administration can, for example, include topical, enteral, local, systemic, or parenteral. For example, administration can be epicutaneous, inhalational, enema, conjunctival, eye drops, ear drops, alveolar, nasal, intranasal, enteral, oral, intraoral, transoral, intestinal, rectal, intrarectal, transrectal, injection, infusion, intravenous, intraarterial, intramuscular, intracerebral, intraventricular, intracerebroventricular, intracardiac, subcutaneous, intraosseous, intradermal, intrathecal, intraperitoneal, intravesical, intracavernosal, intramedullar, intraocular, intracranial, transdermal, transmucosal, transnasal, inhalational, intracisternal, epidural, peridural, intravitreal, etc. The disclosed compositions can be used in and with any other therapy.

The compositions described herein can comprise a pharmaceutically acceptable carrier. By "pharmaceutically acceptable" is meant a material or carrier that would be selected to minimize any degradation of the active ingredient and to minimize any adverse side effects in the subject, as would be well known to one of skill in the art. Examples of carriers include dimyristoylphosphatidyl (DMPC), phosphate buffered saline or a multivesicular liposome. For example, PG:PC:Cholesterol:peptide or PC:peptide can be used as carriers in this invention. Other suitable pharmaceutically acceptable carriers and their formulations are described in Remington: The Science and Practice of Pharmacy (19th ed.) ed. A.R. Gennaro, Mack Publishing Company, Easton, PA 1995. Typically, an appropriate amount of pharmaceutically-acceptable salt is used in the formulation to render the formulation isotonic. Other examples of the pharmaceutically-acceptable carrier include, but are not limited to, saline, Ringer's solution and dextrose solution. The pH of the solution can be from about 5 to about 8, or from about 7 to about 7.5. Further carriers include sustained release preparations such as semi-permeable matrices of solid hydrophobic polymers containing the composition, which matrices are in the form of shaped articles, e.g., films, stents (which are implanted in vessels during an angioplasty procedure), liposomes or microparticles. It will be apparent to those persons skilled in the art that certain carriers may be more preferable depending upon, for instance, the route of administration and concentration of composition being administered. These most typically would be standard carriers for administration of drugs to humans, including solutions such as sterile water, saline, and buffered solutions at physiological pH.

Pharmaceutical compositions may also include carriers, thickeners, diluents, buffers, preservatives and the like, as long as the intended activity of the polypeptide, peptide, nucleic acid, vector of the invention is not compromised. Pharmaceutical compositions may also include one or more active ingredients (in addition to the composition of the invention) such as antimicrobial agents, anti-inflammatory agents, anesthetics, and the like. The pharmaceutical composition may be administered in a number of ways depending on whether local or systemic treatment is desired, and on the area to be treated.

Preparations of parenteral administration include sterile aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium choloride solution, Ringer's dextrose, dextrose and sodium choloride, lactated Ringer's, or fixed oils. Intravenous vehicles include fluid and nutrient replenishers, electrolyte replenishers (such as those based on Ringer's dextrose), and the like. Preservatives and other additives may also be present such as, for example, antimicrobials, anti-oxidants, chelating agents, and inert gases and the like.

Compositions for oral administration include powders or granules, suspensions or solutions in water or non-aqueous media, capsules, sachets, or tablets. Thickeners, flavorings, diluents, emulsifiers, dispersing aids, or binders may be desirable. Some of the compositions may potentially be administered as a pharmaceutically acceptable acid- or base-addition salt, formed by reaction with inorganic acids such as hydrochloric acid, hydrobromic acid, perchloric acid, nitric acid, thiocyanic acid, sulfuric acid, and phosphoric acid, and organic acids such as formic acid, acetic acid, propionic acid, glycolic acid, lactic acid, pyruvic acid, oxalic acid, malonic acid, succinic acid, maleic acid, and fumaric acid, or by reaction with an inorganic base such as sodium hydroxide, ammonium hydroxide, potassium hydroxide, and organic bases such as mon-, di-, trialkyl and aryl amines and substituted ethanolamines.

Unlike typical peptide formulations, the peptides of this invention comprising D-form amino acids can be administered, even orally, without protection against proteolysis by stomach acid, etc. Nevertheless, in certain embodiments, peptide delivery can be enhanced by the use of protective excipients. This is typically accomplished either by complexing the polypeptide with a composition to render it resistant to acidic and enzymatic hydrolysis or by packaging the polypeptide in an appropriately resistant carrier such as a liposome. Means of protecting polypeptides for oral delivery are well known in the art (see, e.g., U.S. Pat. No. 5,391,377 describing lipid compositions for oral delivery of therapeutic agents).

Elevated serum half-life can be maintained by the use of sustained-release protein "packaging" systems. Such sustained release systems are well known to those of skill in the art. In one preferred embodiment, the ProLease biodegradable microsphere delivery system for proteins and peptides (Tracy (1998) Biotechnol. Prog., 14: 108; Johnson et al. (1996) Nature Med. 2: 795; Herbert et al. (1998), Pharmaceut. Res. 15, 357) a dry powder composed of biodegradable polymeric microspheres containing the active agent in a polymer matrix that can be compounded as a dry formulation with or without other agents.

The ProLease microsphere fabrication process was specifically designed to achieve a high encapsulation efficiency while maintaining integrity of the active agent. The process consists of (i) preparation of freeze-dried drug particles from bulk by spray freeze-drying the drug solution with stabilizing excipients, (ii) preparation of a drug-polymer suspension followed by sonication or homogenization to reduce the drug particle size, (iii) production of frozen drug-polymer microspheres by atomization into liquid nitrogen, (iv) extraction of the polymer solvent with ethanol, and (v) filtration and vacuum drying to produce the final dry-powder product. The resulting powder contains the solid form of the active agents, which is homogeneously and rigidly dispersed within porous polymer particles. The polymer most commonly used in the process, poly(lactide-co-glycolide) (PLG), is both biocompatible and biodegradable.

Encapsulation can be achieved at low temperatures (e.g., -40° C.). During encapsulation, the protein is maintained in the solid state in the absence of water, thus minimizing water-induced conformational mobility of the protein, preventing protein degradation reactions that include water as a reactant, and avoiding organic-aqueous interfaces where proteins may undergo denaturation. A preferred process uses solvents in which most proteins are insoluble, thus yielding high encapsulation efficiencies (e.g., greater than 95%).

In another embodiment, one or more components of the solution can be provided as a "concentrate", e.g., in a storage container (e.g., in a premeasured volume) ready for dilution, or in a soluble capsule ready for addition to a volume of water.

The foregoing formulations and administration methods are intended to be illustrative and not limiting. It will be appreciated that, using the teaching provided herein, other suitable formulations and modes of administration can be readily devised.

### 5. Combination Therapy

In one aspect of the disclosed methods, the Apo E mimetics can be administered alone or in combination with one or more additional therapeutic agents. The additional therapeutic agents are selected based on the disease or symptom to be treated. A description of the various classes of suitable pharmacological agents and drugs may be found in Goodman and Gilman, The Pharmacological Basis of Therapeutics, (11th Ed., McGraw-Hill Publishing Co.) (2005). For example, pharmaceutical compositions containing Apo E mimetics can be administered in combination with one or more known therapeutic agents for treating atherosclerosis. Therapeutic agents for treating atherosclerosis include, but are not limited to, cholesterol-lowering agents, blood pressure-lowering agents, blood thinning agents (i.e. medicines that prevent blood clots), anti-inflammatory agents, and anti-atherogenic agents. Examples of cholesterol-lowering agents include, but are not limited to, a cholesterol absorption inhibitor, a bile acid sequestrant, a fibrate, a PCSK9 inhibitor, a microsomal triglyceride transfer protein inhibitor, an apolipoprotein B synthesis inhibitor, or a CETP inhibitor.

In one aspect of the disclosed methods, the Apo E mimetics can be administered alone or in combination with one or more additional peripheral vascular disease therapeutic agents. For example, pharmaceutical compositions containing Apo E mimetics can be administered in combination with one or more known therapeutic agents for treating peripheral vascular disease. Therapeutic agents for treating peripheral vascular disease include, but are not limited to, cholesterol-lowering agents, blood pressure-lowering agents, blood thinning agents (i.e. medicines that prevent blood clots), anti-inflammatory agents, and anti-atherogenic agents. Examples of cholesterol-lowering agents include, but are not limited to, a cholesterol absorption inhibitor, a bile acid sequestrant, a fibrate, a PCSK9 inhibitor, a microsomal triglyceride transfer protein inhibitor, an apolipoprotein B synthesis inhibitor, or a CETP inhibitor.

In one aspect of the disclosed methods, the Apo E mimetics can be administered alone or in combination with one or more additional interventional therapies such as angioplasty, artherectomy or grafting.

In one aspect of the disclosed methods, the Apo E mimetics can be administered alone or in combination with one or more additional critical limb ischemia therapeutic agents. For example, pharmaceutical compositions containing Apo E mimetics can be administered in combination with one or more known therapeutic agents for treating critical limb ischemia. Therapeutic agents for treating critical limb ischemia include, but are not limited to, cholesterol-lowering agents, blood pressure-lowering agents, blood thinning agents (i.e. medicines that prevent blood clots), anti-inflammatory agents, and anti-atherogenic agents. Examples of cholesterol-lowering agents include, but are not limited to, a cholesterol absorption inhibitor, a bile acid sequestrant, a fibrate, a PCSK9 inhibitor, a microsomal triglyceride transfer protein inhibitor, an apolipoprotein B synthesis inhibitor, or a CETP inhibitor.

The critical limb ischemia therapeutic other than an Apo E mimetic can be a conventional lipid lowering therapy and the conventional lipid lowering therapy can be one or more of the following: a statin, an antiplatelet drus, aspirin, clopidogrel, Cilostazol, or pentoxifylline. The atherosclerosis therapeutic other than an Apo E mimetic can also include surgical intervention, smoking cessation, management of diabetes, management of hypertension management of cholesterol, exercise, Angioplasty (PTA or percutaneous transluminal angioplasty), plaque excision, bypass grafting, sympathectomy, amputation, or thrombolysis.

The Apo E mimetics can be administered in conjunction with or followed by any of the disclosed additional therapeutics. The treatments can be administered in conjunction with or followed by LDL apheresis.

The combination therapies can include administering the Apo E mimetic and an additional therapeutic agent during the treatment cycle of a dosing regimen. The combination therapies can also include administering the Apo E mimetic during the treatment cycle and an additional therapeutic agent during the rest phase.

### Examples

It is understood that the disclosed method and compositions are not limited to the particular methodology, protocols, and reagents described as these may vary. It is also to be understood that the terminology used herein is for the purpose of describing particular embodiments only, and is not intended to limit the scope of the present invention which will be limited only by the appended claims.

Those skilled in the art will recognize, or be able to ascertain using no more than routine experimentation, many equivalents to the specific embodiments of the method and compositions described herein. Such equivalents are intended to be encompassed by the following claims.

### A. Example 1: Study One - Prolonged effect of Apo E mimetics (a comparison of hE18A with mR18L, a single domain cationic peptide)

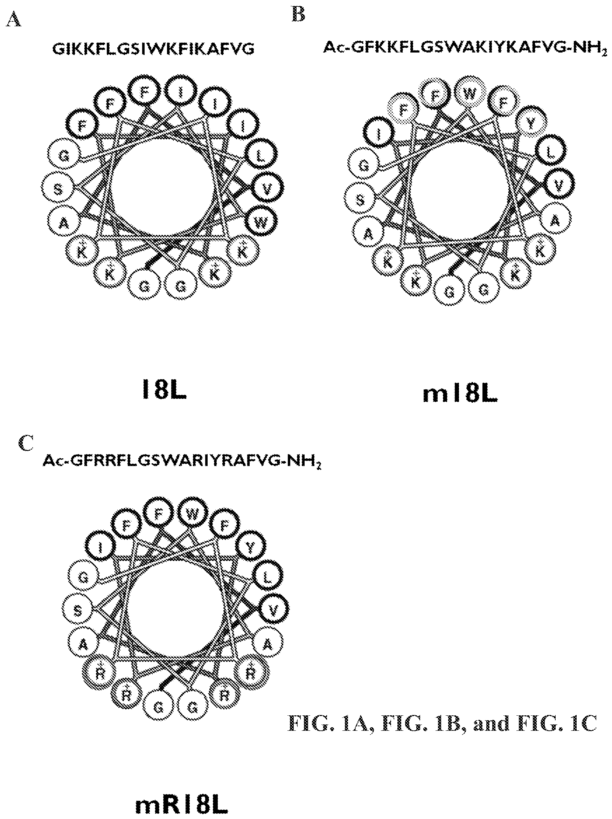

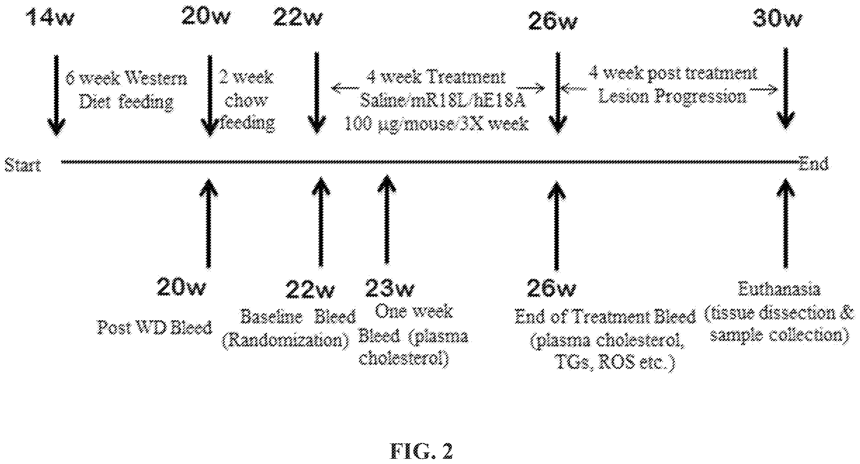

Figure 1 shows the design of mR18L (Tytler et al. JBC, 268:22112-22118, 1993; Handattu et al. J. Lipid Res, 51:3491-3499, 2010). The experimental protocol was performed in Apo E null mice as shown in Figure 2. The mice were fed a Western diet for 6 weeks to elevate cholesterol. The animals were then switched to a chow diet for two weeks after which they were treated with peptide or saline for 4 weeks. Treatment was then withdrawn for 4 weeks while the animals remained on a chow diet. After the 4 week post treatment phase, the mice were euthanized and tissue dissection and sample collection was performed.

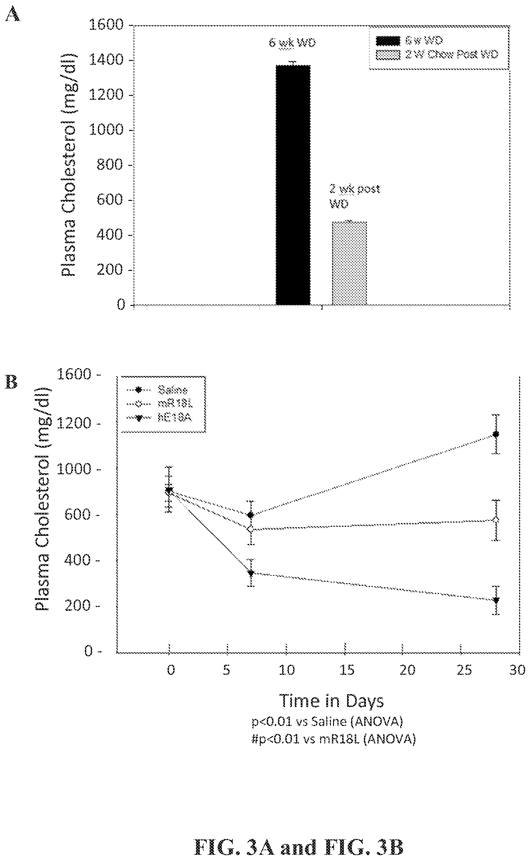

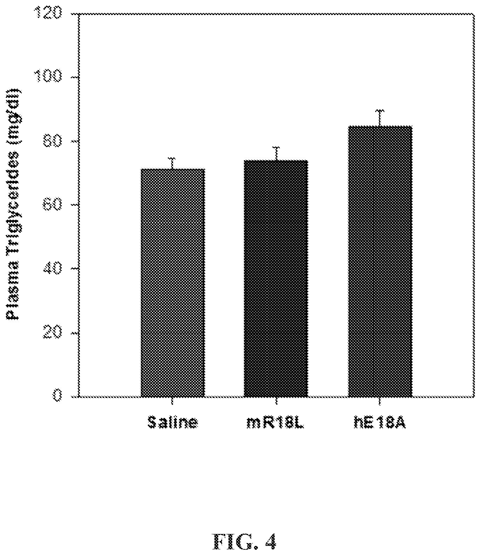

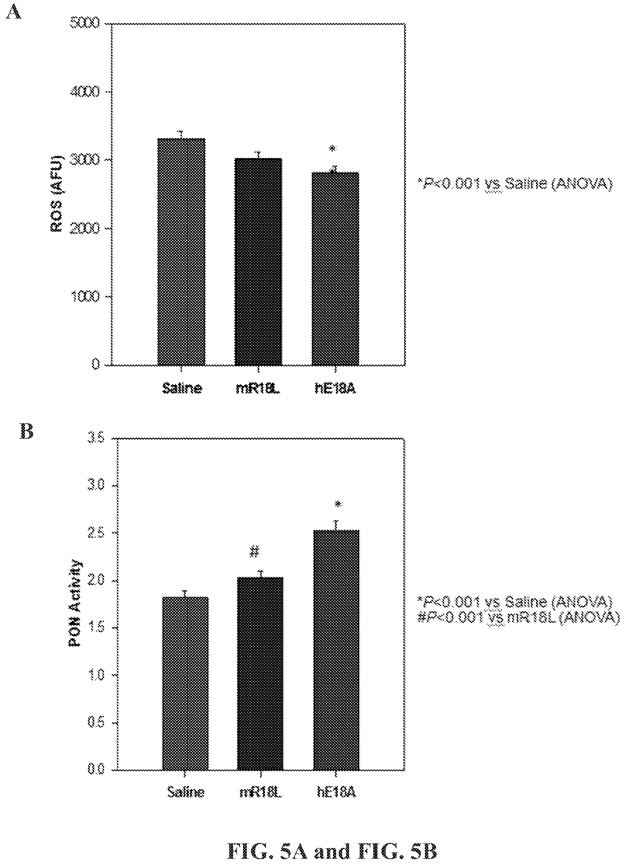

Blood was drawn after 4 weeks of treatment at week 26 and analyzed for cholesterol. At the end of treatment, hE18A was better in reducing plasma cholesterol levels in Apo E null mice compared to mR18L and saline (Figures 3A and 3B). Apo E mimetic peptide treatment did not change plasma triglyceride levels (Figure 4). However, Apo E mimetic treatment did improve plasma marker of oxidative stress, such as ROS and PON activity. Figure 5A shows a significant decrease in reactive oxygen species (ROS) in the hE18A treated mice compared to the saline treated mice. Because ROS have been implicated in the pathogenesis of atherosclerosis, the decrease in ROS by treatment with an Apo E mimetic is beneficial.

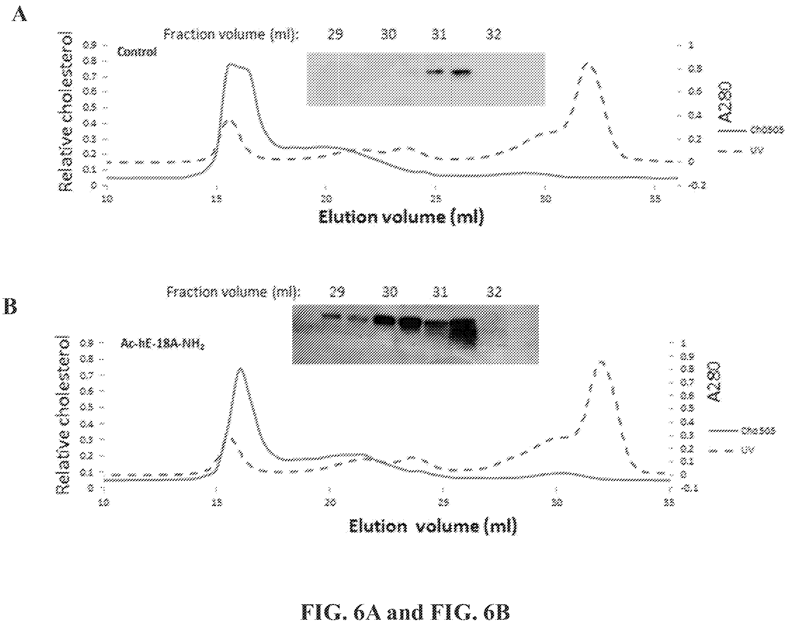

Figure 5B shows a significant increase in paraoxonase (PON) activity in hE18A treated mice compared to saline and mR18L treated mice. The PON gene family has anti-atherosclerotic effects, such as preventing oxidation of LDLs. Thus, the increase in PON activity in hE18A treated mice is beneficial in the atherosclerosis animal model. Figures 6A and 6B further shows the dramatic increase in PON-1 levels in HDL fractions in Apo E null mice treated with Ac-hE18A-NH₂.

The mice were euthanized at week 30, which is four weeks post treatment with Apo E mimetic.

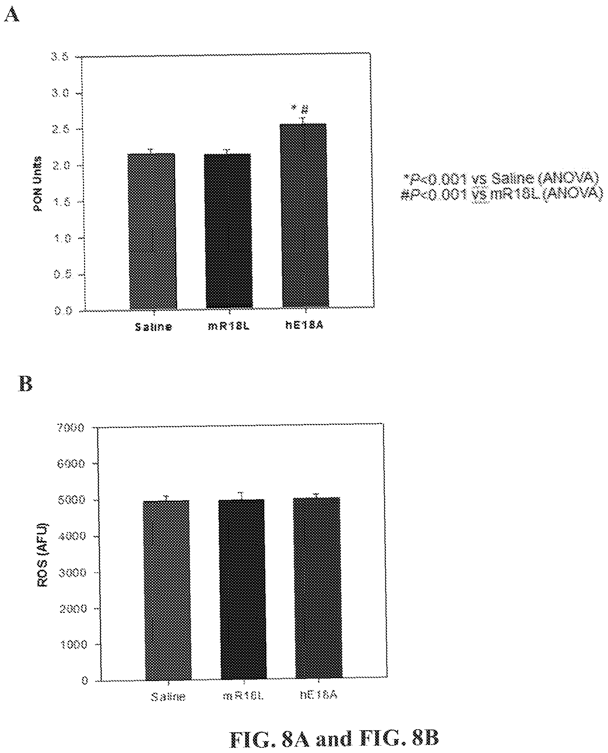

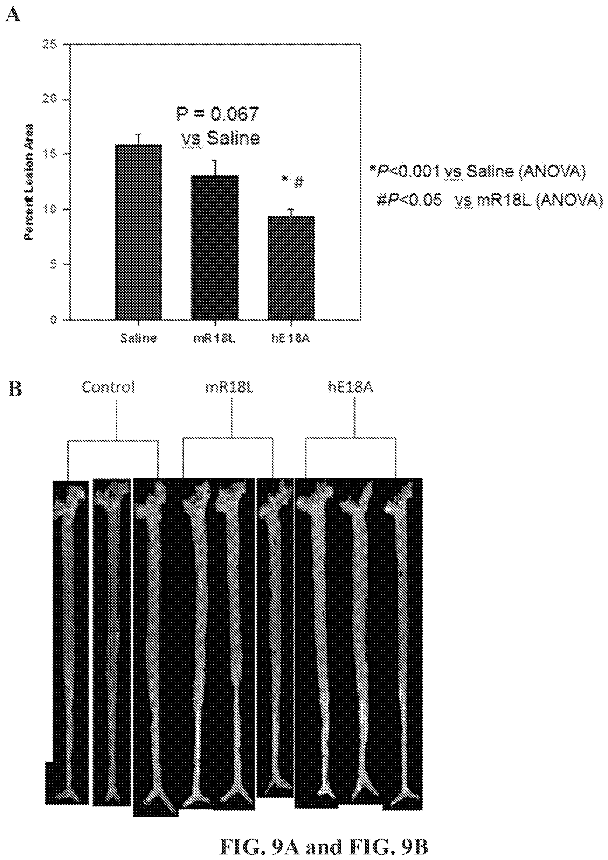

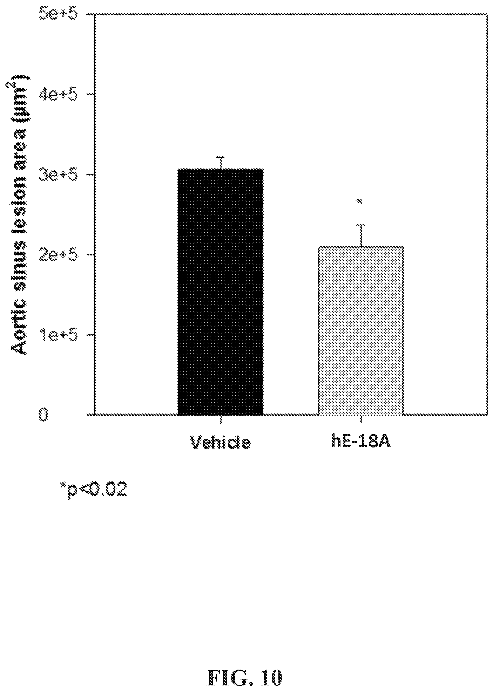

Figures 7 and 8B show that cholesterol levels and ROS levels, respectively, were not significantly different four weeks after the end of treatment period. However, PON-1 activity was still increased in the hE18A treated mice over the saline and mR18L treated mice 4 weeks post treatment (Figure 8A). Importantly, the atherosclerotic lesion area was significantly reduced in the hE18A treated mice compared to the saline or mR18L treated mice (Figures 9A and 9B). Atherosclerotic lesion area in the aortic sinus was also significantly decreased by hE18A (Figure 10), but not by mR18L. There was also a non-significant trend towards a decrease in macrophage coverage of the aortic sinus with hE18A (Figure 11).

### B. Example 2: Study Two - Late Intervention Study in Apo E -/- mice

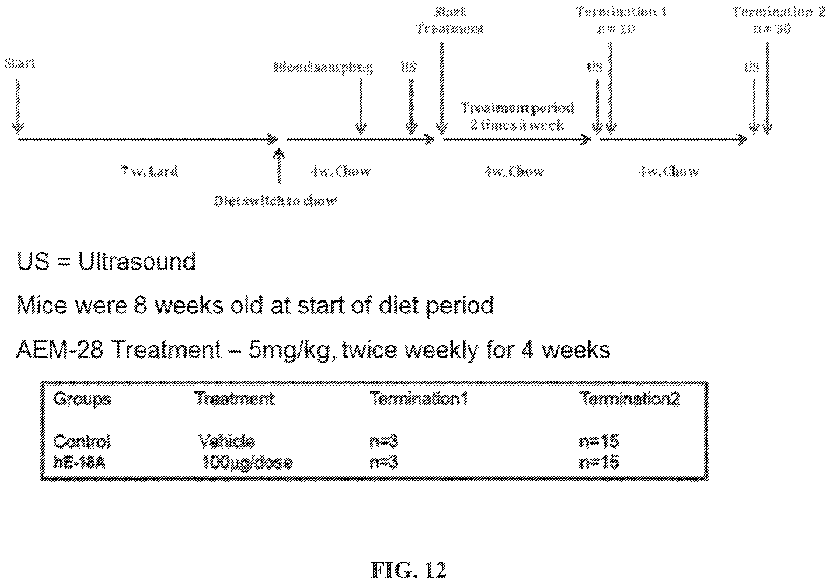

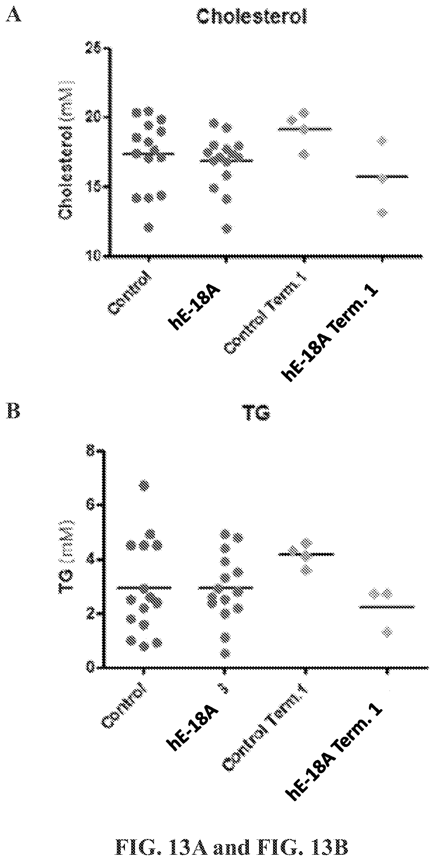

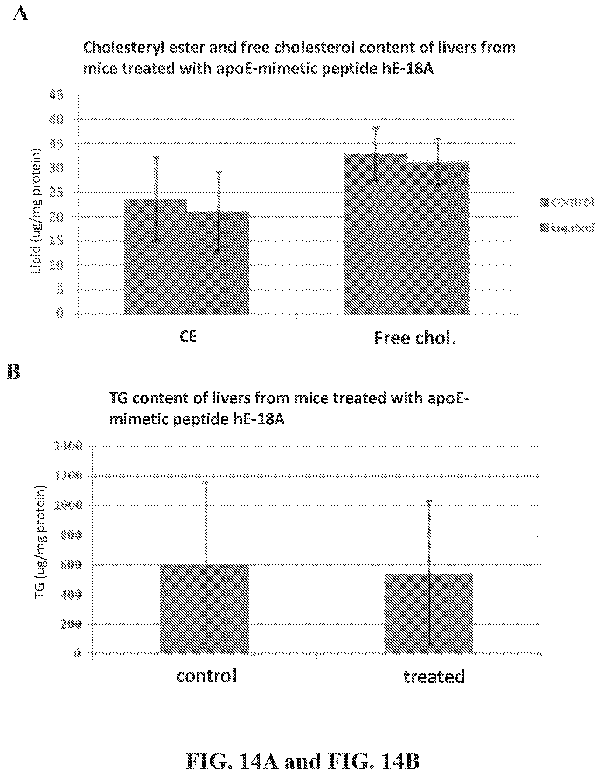

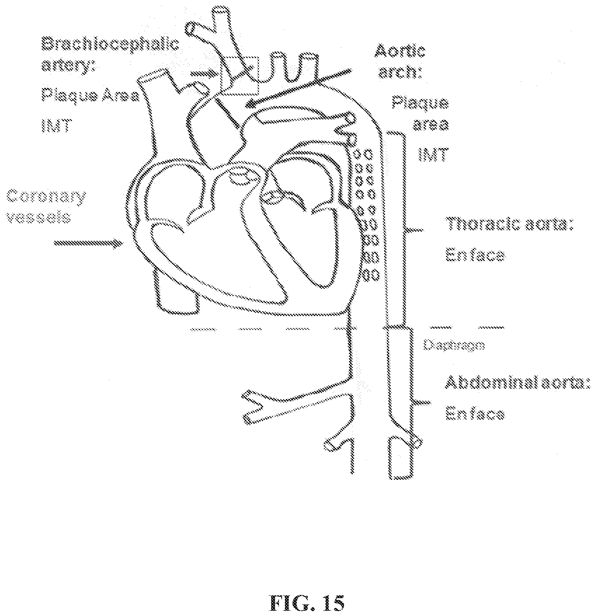

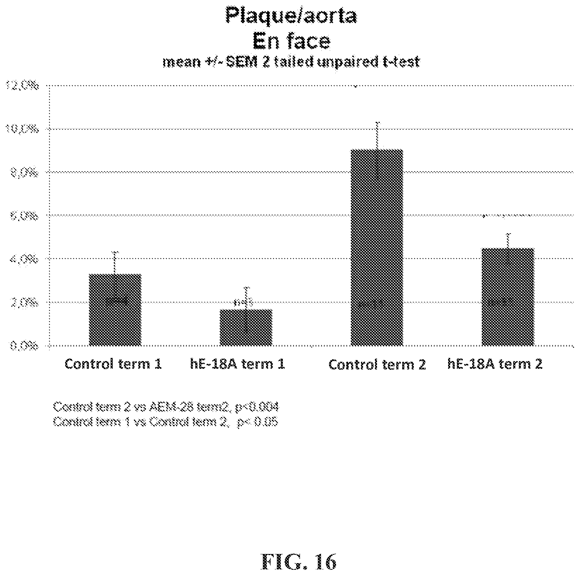

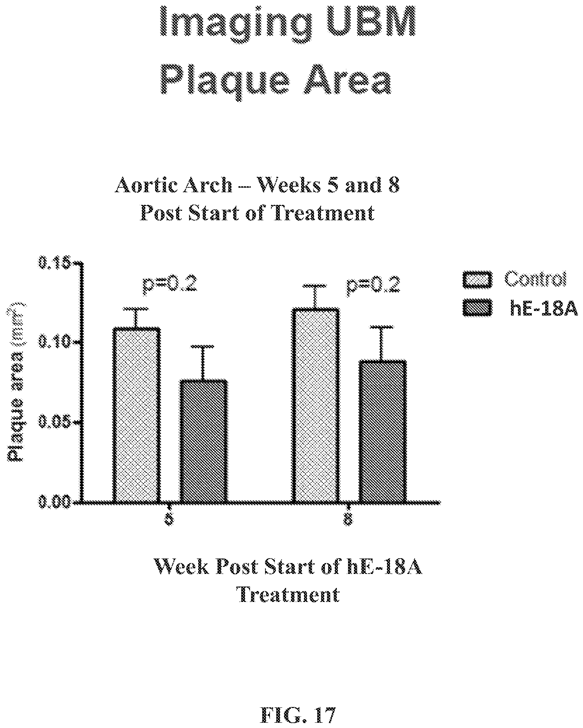

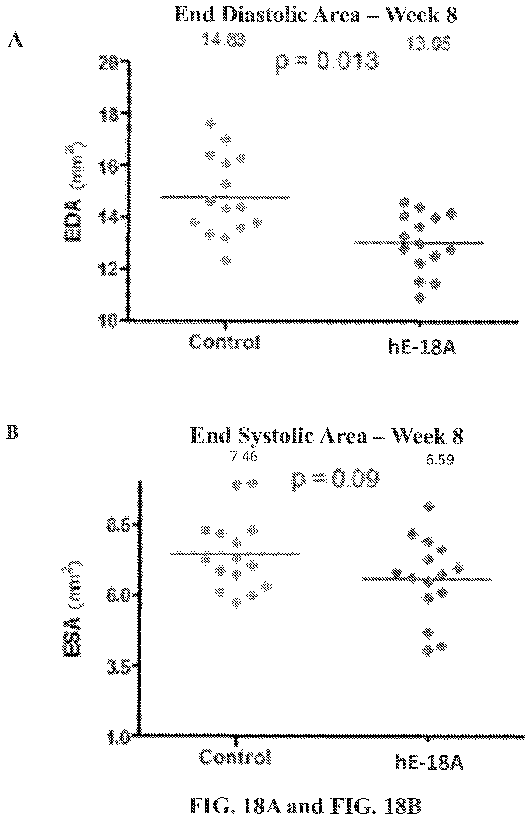

Figure 12 shows the study design used to treat Apo E -/- mice. The mice were fed a lard diet for seven weeks and then were switched to a chow diet for four weeks. The mice were treated two times a week for four weeks and then treatment was withdrawn for four weeks. Termination 1 represents the time point immediately following termination of the Apo E mimetic treatment. Termination 2 represents the time point immediately following the four weeks of treatment withdrawal. Lipids were analyzed in the plasma (cholesterol, triglycerides) and in the liver (cholesterol esters, unesterified cholesterol, triglycerides). A cardiovascular analysis was also performed and the different regions of the aorta that were analyzed are shown in Figure 15.
Treatment with the Apo E mimetic lowered lipid levels during the treatment period but did not appear to have sustained effects (Figures 13 and 14). However, the cardiovascular analysis showed that the percentage of plaques in the aorta was significantly decreased even four weeks after treatment with Apo E mimetic was withdrawn (Figures 16 and 17). Furthermore, both end-diastolic and end-systolic cross sectional areas of the heart were decreased four weeks after treatment with Apo E mimetics were withdrawn (Figures 18A and 18B). Ejection fraction remained the same. There was no increase in heart rate of ventricle wall thickness, indicating that the hearts were operating with a greater cardiac reserve to deliver the same amount of blood.

### Summary

Both studies showed similar results. Although the animals had cholesterol levels of about 650 mg/dl four weeks after cessation of treatment, the animals treated with 5 mg/kg of AchE18ANH2 had decreased atherosclerotic lesions in the thoracic aorta by about 50%, and reduced lesions in the aortic arch by about 35% at the end of treatment.

Four weeks after the cessation of treatment, the end diastolic and end systolic areas were reduced in the treated animals. Ejection fraction and heart rate was unchanged. As a result, cardiac output was maintained at a lower heart volume, increasing cardiac reserve and efficiency. This is indicative of decreased arterial stiffness and enhanced vasorelaxation.

Four weeks after cessation of treatment, PON-1 activity remained elevated in the Apo E mimetic treated animals. PON-1 is a major anti-atherosclerotic component of HDL, and thus improves HDL function. All of these sustained benefits indicate that a course of treatment with Ac-hE18A-NH2 reduces atherosclerotic burden, improves the health of the artery wall, and rejuvenates the artery wall, regressing atherosclerosis caused by chronic hypercholesterolemia and inflammation.

## Claims

1. Apolipoprotein-E mimicking peptide for use in treating atherosclerosis, wherein the dosing regimen comprises at least one treatment cycle followed by a rest phase, wherein the treatment cycle comprises administering an effective amount of an Apolipoprotein-E mimicking peptide once a week for at least four weeks, wherein the Apolipoprotein-E mimicking peptideis not administered during the rest phase.

2. Apolipoprotein-E mimickingpeptide for use according to claim 1, wherein the treatment cycle comprises a second treatment cycle after the rest phase.

3. Apolipoprotein-E mimickingpeptide for use according to claim 1, wherein the rest phase is at least four weeks.

4. Apolipoprotein-E mimicking peptide for use according to claim 2, wherein a second treatment cycle is administered one year from the beginning of the initial treatment cycle, or after a four week rest phase.

5. Apolipoprotein-E mimickingpeptide for use according to claim 1, wherein an atherosclerosis therapeutic other than an Apolipoprotein-E mimicking peptideis administered during the rest phase.

6. Apolipoprotein-E mimickingpeptide for use according to claim 5, wherein the atherosclerosis therapeutic other than an Apolipoprotein-E mimicking peptideis a conventional lipid lowering therapy.

7. Apolipoprotein-E mimickingpeptide for use according to claim 6, wherein the conventional lipid lowering therapy is a statin.

8. Apolipoprotein-E mimickingpeptide for use according to claims 1 to 7, wherein the Apolipoprotein-E mimicking peptideis LRKLRKRLLRDWLKAFYDKVAEKLKEAF (SEQ ID NO:1).

9. Apolipoprotein-E mimickingpeptide for use according to claim 8, wherein the amino acid sequence LRKLRKRLLRDWLKAFYDKVAEKLKEAF (SEQ ID NO:1) is end protected with an acetyl group protecting the amino terminus and an amide group protecting the carboxyl terminus.

## Patentansprüche

1. Apolipoprotein-E-nachahmendes Peptid zur Verwendung bei der Behandlung von Atherosklerose, wobei das Dosierungsschema mindestens einen Behandlungszyklus gefolgt von einer Ruhephase umfasst, wobei der Behandlungszyklus die Verabreichung einer wirksamen Menge eines Apolipoprotein-E-nachahmenden Peptids einmal pro Woche, für mindestens vier Wochen umfasst, wobei das Apolipoprotein-E nachahmende Peptid während der Ruhephase nicht verabreicht wird.

2. Apolipoprotein-E nachahmendes Peptid zur Verwendung gemäß Anspruch 1, wobei der Behandlungszyklus einen zweiten Behandlungszyklus nach der Ruhephase umfasst.

3. Apolipoprotein-E nachahmendes Peptid zur Verwendung gemäß Anspruch 1, wobei die Ruhephase mindestens vier Wochen beträgt.

4. Apolipoprotein-E-nachahmendes Peptid zur Verwendung gemäß Anspruch 2, wobei ein zweiter Behandlungszyklus ein Jahr nach Beginn des anfänglichen Behandlungszyklus oder nach einer vierwöchigen Ruhephase verabreicht wird.

5. Apolipoprotein-E nachahmendes Peptid zur Verwendung gemäß Anspruch 1, wobei ein anderes Atherosklerosetherapeutikum als ein Apolipoprotein-E nachahmendes Peptid während der Ruhephase verabreicht wird.

6. Apolipoprotein-E-nachahmendes Peptid zur Verwendung gemäß Anspruch 5, wobei das andere Atherosklerosetherapeutikum als ein Apolipoprotein-E nachahmendes Peptid, eine herkömmliche lipidsenkende Therapie ist.

7. Apolipoprotein-E nachahmendes Peptid zur Verwendung gemäß Anspruch 6, wobei die herkömmliche lipidsenkende Therapie ein Statin ist.

8. Apolipoprotein-E-nachahmendes Peptid zur Verwendung gemäß den Ansprüchen 1 bis 7, wobei das Apolipoprotein-E-nachahmende Peptid LRKLRKRLLRDWLKAFYDKV AEKLKEAF (SEQ ID NO: 1) ist.

9. Apolipoprotein-E nachahmendes Peptid zur Verwendung gemäß Anspruch 8, wobei die Aminosäuresequenz LRKLRKRLLRDWLKAFYDKVAEKLKEAF (SEQ ID NO: 1) endgeschützt ist mit einer Acetylgruppe, die den Aminoterminus schützt, und einer Amidgruppe, die den Carboxylterminus schützt.

## Revendications

1. Peptide mimétique de l'apolipoprotéine-E à utiliser dans le traitement de l'athérosclérose, dans lequel le schéma posologique comprend au moins un cycle de traitement suivi d'une phase de repos, dans lequel le cycle de traitement comprend l'administration d'une quantité efficace d'un peptide mimétique de l'apolipoprotéine-E une fois par semaine pendant au moins quatre semaines, dans lequel le peptide mimétique de l'Apolipoprotéine-E n'est pas administré pendant la phase de repos.

2. Peptide mimétique de l'apolipoprotéine-E à utiliser selon la revendication 1, dans lequel le cycle de traitement comprend un deuxième cycle de traitement après la phase de repos.

3. Peptide mimétique de l'apolipoprotéine-E à utiliser selon la revendication 1, dans lequel la phase de repos est d'au moins quatre semaines.

4. Peptide mimétique de l'apolipoprotéine-E à utiliser selon la revendication 2, dans lequel un deuxième cycle de traitement est administré un an après le début du cycle de traitement initial, ou après une phase de repos de quatre semaines.

5. Peptide mimétique de l'apolipoprotéine-E à utiliser selon la revendication 1, dans lequel une athérosclérose thérapeutique autre qu'un peptide mimétique de l'apolipoprotéine-E est administré pendant la phase de repos.

6. Peptide mimétique de l'apolipoprotéine-E à utiliser selon la revendication 5, dans lequel une athérosclérose thérapeutique autre qu'un peptide mimétique de l'apolipoprotéine-E est un traitement hypolipémiant conventionnel.

7. Peptide mimétique de l'apolipoprotéine-E à utiliser selon la revendication 6, dans lequel le traitement hypolipémiant conventionnel est une statine.

8. Peptide mimétique de l'apolipoprotéine-E à utiliser selon les revendications 1 à 7, dans lequel le peptide mimétique de l'apolipoprotéine-E est LRKLRKRLLRDWLKAFYDKVAEKLKEAF (SEQ ID N °: 1).

9. Peptide mimétique de l'apolipoprotéine-E à utiliser selon la revendication 8, dans lequel la séquence d'acides aminés LRKLRKRLLDDWLKAFYDKVAEKLKEAF (SEQ ID NO: 1) est protégée à l'extrémité avec un groupe acétyle protégeant l'extrémité amino terminale et un groupe amide protégeant l'extrémité carboxy-terminale.
